# EUROPEAN PATENT APPLICATION

(11) **EP 1 690 548 A2**
(43) Date of publication of application: **16.08.2006**
(21) Application number: 05077789.5
(22) Date of filing: 23.10.2002
(51) Int. Cl.: A61K 38/17, C07K 16/18, C07K 14/00, C12N 15/00, C12N 15/10, G01N 33/48

(54) **Cell cycle progression proteins**

(30) Priority: 05.11.2001 GB 0126506; 27.11.2001 GB 0128384; 11.02.2002 GB 0203185
(62) Divisional of application: 02802672.2
(71) Applicant: Cyclacel Limited, London N1 7JQ (GB)
(72) Inventor: Deak, Peter, P.O. Box 521 6701 Szeged (HU); Frenz, Lisa, James Lindsay Place Dundee DD1 5JJ (GB); Glover, David, James Lindsay Place Dundee DD1 5JJ (GB); Midgley, Carol, James Lindsay Place Dundee DD1 5JJ (GB)
(74) Representative: Furlong, Isla Jane

(57) **Abstract**

Polynucleotides encoding a number of *Drosophila* gene products are provided. Polynucleotide probes derived from these nucleotide sequences, polypeptides encoded by the polynucleotides and antibodies that bind to the polypeptides are also provided.

## Description

The present invention relates to a number of genes implicated in the processes of cell cycle progression, including mitosis and meiosis.

We have now identified a number of genes in the X chromosome of *Drosophila,* mutations in which disrupt cell cycle progression, for example the processes of mitosis and/or meiosis. We have determined the phenotypes of these mutations and relate the mutations to the total genome sequence and so identify individual genes essential for cell cycle progression.

According to one aspect of the present invention, we provide a use of a polynucleotide as set out in Table 5, or a polypeptide encoded by the polypeptide, in a method of prevention, treatment or diagnosis of a disease in an individual.

Preferably, the polynucleotide comprises a human polypeptide as set out in column 3 of Table 5. In preferred embodiments, the polynucleotide or polypeptide is used to identify a substance capable of binding to the polypeptide, which method comprises incubating the polypeptide with a candidate substance under suitable conditions and determining whether the substance binds to the polypeptide.

Alternatively or in addition, the polynucleotide or polypeptide is used to identify a substance capable of modulating the function of the polypeptide, the method comprising the steps of: incubating the polypeptide with a candidate substance and determining whether activity of the polypeptide is thereby modulated.

The polynucleotide or polypeptide may be administered to an individual in need of such treatment. Alternatively, or in addition, the substance identified by the method is administered to an individual in need of such treatment.

The use may be for a method of diagnosis, in which the presence or absence of a polynucleotide is detected in a biological sample in a method comprising: (a) bringing the biological sample containing nucleic acid such as DNA or RNA into contact with a probe comprising a fragment of at least 15 nucleotides of the polynucleotide as set out in Table 5 under hybridising conditions; and (b) detecting any duplex formed between the probe and nucleic acid in the sample.

Alternatively, or in addition, the presence or absence of a polypeptide is detected in a biological sample in a method comprising: (a) providing an antibody capable of binding to the polypeptide; (b) incubating a biological sample with said antibody under conditions which allow for the formation of an antibody-antigen complex; and (c) determining whether antibody-antigen complex comprising said antibody is formed.

In highly preferred embodiments, the disease comprises a proliferative disease such as cancer.

In a further aspect of the invention, we provide a method of modulating, preferably down-regulating, the expression of a polynucleotide as set out in Table 5 in a cell, the method comprising introducing a double stranded RNA (dsRNA) corresponding to the polynucleotide, or an antisense RNA corresponding to the polynucleotide, or a fragment thereof, into the cell.

According to another aspect of the present invention, we provide a polynucleotide selected from: (a) polynucleotides comprising any one of the nucleotide sequences set out in Example 19, preferably Shp2 polynucleotide, or the complement thereof; (b) polynucleotides comprising a nucleotide sequence capable of hybridising to the nucleotide sequences set out in Example 19, preferably Shp2 polynucleotide, or a fragment thereof; (c) polynucleotides comprising a nucleotide sequence capable of hybridising to the complement of the nucleotide sequences set out in Example 19, preferably Shp2 polynucleotide, or a fragment thereof; (d) polynucleotides comprising a polynucleotide sequence which is degenerate as a result of the genetic code to the polynucleotides defined in (a), (b) or (c).

There is provided, according to a further aspect of the present invention, a polynucleotide selected from: (a) polynucleotides comprising any one of the nucleotide sequences set out in Example 28, preferably Dlg1 or Dlg2 polynucleotide, or the complement thereof; (b) polynucleotides comprising a nucleotide sequence capable of hybridising to the nucleotide sequences set out in Example 28, preferably Dlg1 or Dlg2 polynucleotide, or a fragment thereof; (c) polynucleotides comprising a nucleotide sequence capable of hybridising to the complement of the nucleotide sequences set out in Example 28, preferably Dlg1 or Dlg2 polynucleotide, or a fragment thereof; (d) polynucleotides comprising a polynucleotide sequence which is degenerate as a result of the genetic code to the polynucleotides defined in (a), (b) or (c).

We provide, according to another aspect of the present invention, a polynucleotide selected from: (a) polynucleotides comprising any one of the nucleotide sequences set out in Table 5 or the complement thereof; (b) polynucleotides comprising a nucleotide sequence capable of hybridising to the nucleotide sequences set out in Table 5, or a fragment thereof; (c) polynucleotides comprising a nucleotide sequence capable of hybridising to the complement of the nucleotide sequences set out in Table 5, or a fragment thereof; (d) polynucleotides comprising a polynucleotide sequence which is degenerate as a result of the genetic code to the polynucleotides defined in (a), (b) or (c).

As a further aspect of the present invention, there is provided a polynucleotide selected from: (a) polynucleotides comprising any one of the nucleotide sequences set out in Examples 1 to 18, 20 to 27 and 29 or the complement thereof; (b) polynucleotides comprising a nucleotide sequence capable of hybridising to the nucleotide sequences set out in Examples 1 to 18, 20 to 27 and 29, or a fragment thereof; (c) polynucleotides comprising a nucleotide sequence capable of hybridising to the complement of the nucleotide sequences set out in Examples 1 to 18, 20 to 27 and 29, or a fragment thereof; (d) polynucleotides comprising a polynucleotide sequence which is degenerate as a result of the genetic code to the polynucleotides defined in (a), (b) or (c).

We provide, according to a further aspect of the present invention, a polynucleotide selected from: (a) polynucleotides comprising any one of the nucleotide sequences set out in Examples 1, 2, 2A, 2B and 2C or the complement thereof; (b) polynucleotides comprising a nucleotide sequence capable of hybridising to the nucleotide sequences set out in Examples 1, 2, 2A, 2B and 2C, or a fragment thereof; (c) polynucleotides comprising a nucleotide sequence capable of hybridising to the complement of the nucleotide sequences set out in Examples 1, 2, 2A, 2B and 2C, or a fragment thereof; (d) polynucleotides comprising a polynucleotide sequence which is degenerate as a result of the genetic code to the polynucleotides defined in (a), (b) or (c).

The present invention, in another aspect, provides polynucleotide selected from:
(a) polynucleotides comprising any one of the nucleotide sequences set out in Examples 3 to 9 and 9A or the complement thereof; (b) polynucleotides comprising a nucleotide sequence capable of hybridising to the nucleotide sequences set out in Examples 3 to 9 and 9A, or a fragment thereof; (c) polynucleotides comprising a nucleotide sequence capable of hybridising to the complement of the nucleotide sequences set out in Examples 3 to 9 and 9A, or a fragment thereof; (d) polynucleotides comprising a polynucleotide sequence which is degenerate as a result of the genetic code to the polynucleotides defined in (a), (b) or (c).

In a further aspect of the present invention, there is provided polynucleotide selected from: (a) polynucleotides comprising any one of the nucleotide sequences set out in Examples 10 to 29 or the complement thereof; (b) polynucleotides comprising a nucleotide sequence capable of hybridising to the nucleotide sequences set out in Examples 10 to 29, or a fragment thereof; (c) polynucleotides comprising a nucleotide sequence capable of hybridising to the complement of the nucleotide sequences set out in Examples 10 to 29, or a fragment thereof; (d) polynucleotides comprising a polynucleotide sequence which is degenerate as a result of the genetic code to the polynucleotides defined in (a), (b) or (c).

As a further aspect of the invention, we provide a polynucleotide probe which comprises a fragment of at least 15 nucleotides of a polynucleotide according to any of the above aspects of the invention.

The present invention also provides a polypeptide which comprises any one of the amino acid sequences set out in Examples I to 29 or in any of Examples 1 to 2, 2A, 2B and 2C, Examples 3 to 9 and 9A and Examples 10 to 29, or a homologue, variant, derivative or fragment thereof.

Preferably the polypeptide is encoded by a cDNA sequence obtainable from a eukaryotic cDNA library, preferably a metazoan cDNA library (such as insect or mammalian) said DNA sequence comprising a DNA sequence being selectively detectable with a nucleotide sequence, preferably a *Drosophila* nucleotide sequence, as shown in any one of Examples 1 to 29.

The term "selectively detectable" means that the cDNA used as a probe is used under conditions where a target cDNA is found to hybridize to the probe at a level significantly above background. The background hybridization may occur because of other cDNAs present in the cDNA library. In this event background implies a level of signal generated by interaction between the probe and a non-specific cDNA member of the library which is less than 10 fold, preferably less than 100 fold as intense as the specific interaction observed with the target cDNA. The intensity of interaction may be measured, for example, by radiolabelling the probe, e.g. with ³²P. Suitable conditions may be found by reference to the Examples, as well as in the detailed description below.

A polynucleotide encoding a polypeptide as described here is also provided.

We further provide a vector comprising a polynucleotide of the invention, for example an expression vector comprising a polynucleotide of the invention operably linked to a regulatory sequence capable of directing expression of said polynucleotide in a host cell.

Also provided is an antibody capable of binding such a polypeptide.

In a further aspect the present invention provides a method for detecting the presence or absence of a polynucleotide of the invention in a biological sample which method comprises: (a) bringing the biological sample containing DNA or RNA into contact with a probe comprising a nucleotide of the invention under hybridising conditions; and (b) detecting any duplex formed between the probe and nucleic acid in the sample.

In another aspect the invention provides a method for detecting a polypeptide of the invention present in a biological sample which comprises: (a) providing an antibody of the invention; (b) incubating a biological sample with said antibody under conditions which allow for the formation of an antibody-antigen complex; and (c) determining whether antibody-antigen complex comprising said antibody is formed.

Knowledge of the genes involved in cell cycle progression allows the development of therapeutic agents for the treatment of medical conditions associated with aberrant cell cycle progression. Accordingly, the present invention provides a polynucleotide of the invention for use in therapy. The present invention also provides a polypeptide of the invention for use in therapy. The present invention further provides an antibody of the invention for use in therapy.

In a specific embodiment, the present invention provides a method of treating a tumour or a patient suffering from a proliferative disease, comprising administering to a patient in need of treatment an effective amount of a polynucleotide, polypeptide and/or antibody of the invention.

The present invention also provides the use of a polypeptide of the invention in a method of identifying a substance capable of affecting the function of the corresponding gene. For example, in one embodiment the present invention provides the use of a polypeptide of the invention in an assay for identifying a substance capable of inhibiting cell cycle progression. The assay involves contacting the polypeptide with a candidate substance or molecule, and detecting modulation of activity of the polypeptide. In preferred embodiments, further steps of isolating or synthesising the substance so identified are carried out.

The substance may inhibit any of the steps or stages in the cell cycle, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, and cytokinesis functions. For example, possible functions of genes of the invention for which it may be desired to identify substances which affect such functions include chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways.

In a further aspect the present invention provides a method for identifying a substance capable of binding to a polypeptide of the invention, which method comprises incubating the polypeptide with a candidate substance under suitable conditions and determining whether the substance binds to the polypeptide.

In an additional aspect, the invention provides kits comprising polynucleotides, polypeptides or antibodies of the invention and methods of using such kits in diagnosing the presence of absence of polynucleotides and polypeptides of the invention including deleterious mutant forms.

Also provided is a substance identified by the above methods of the invention. Such substances may be used in a method of therapy, such as in a method of affecting cell cycle progression, for example mitosis and/or meiosis.

The invention also provides a process comprising the steps of: (a) performing one of the above methods; and (b) preparing a quantity of those one or more substances identified as being capable of binding to a polypeptide of the invention.

Also provided is a process comprising the steps of: (a) performing one of the above methods; and (b) preparing a pharmaceutical composition comprising one or more substances identified as being capable of binding to a polypeptide of the invention.

We further provide a method for identifying a substance capable of modulating the function of a polypeptide of the invention or a polypeptide encoded by a polynucleotide of the invention, the method comprising the steps of: incubating the polypeptide with a candidate substance and determining whether activity of the polypeptide is thereby modulated.

A substance identified by a method or assay according to any of the above methods or processes is also provided, as is the use of such a substance in a method of inhibiting the function of a polypeptide. Use of such a substance in a method of regulating a cell division cycle function is also provided.

We further provide a method of identifying a human nucleic acid sequence, by: (a) selecting a *Drosophila* polypeptide identified in any of Examples 1 to 29; (b) identifying a corresponding human polypeptide; (c) identifying a nucleic acid encoding the polypeptide of (b).

Preferably, a human homologue of the *Drosophila* sequence, or a human sequence similar to the *Drosophila* sequence, is identified in step (b).

Preferably, the human polypeptide has at least one of the biological activities, preferably substantially all the biological activities of the *Drosophila* polypeptide.

We provide a human polypeptide identified by a method according to the previous aspect of the invention.

### BRIEF DESCRIPTION OF THE FIGURES

Figure 1 shows mitotic index after RNAi knockdown of Corkscrew (CG3954) in Dmel-2 *Drosophila* cultured cells. Values are an average of triplicate samples. Positive controls are siRNA with the mitotic genes Polo kinase and Orbit, negative controls are siRNA with water and with an siRNA against non-endogenous gene GL3
Figure 2 shows a BLASTP alignment of Drosophila Corkscrew (CG3954) (query sequence), identified in Example 19 as a cell cycle gene, and human Shp2 Protein-tyrosine phosphatase, non-receptor type 11 (genbank accession D13540) (subject sequence).
Figure 3 shows a histogram of Facs analysis of cell cycle compartment as determined by DNA content in U20S cells after human Shp2 siRNA transfection for 48 hours. The negative control is transfection with siRNA against the non-endogenous gene GL3.
Figure 4 shows fluorescence micrographs showing the effect of Shp2 siRNAi in U2OS cells. A) Irregular nuclear shape, B) Increase in apoptosis.
Figure 5 shows Mitotic index after RNAi knockdown of Drosophila discs large 1 Dlg1 (CG1725) in Dmel-2 *Drosophila* cultured cells. Values are an average of triplicate samples. Positive controls are siRNA with the mitotic genes Polo kinase and Orbit, negative controls are siRNA with water and with an siRNA against non-endogenous gene GL3
Figure 6A shows a BLASTP alignment of Drosophila discs large 1 DIg1 (CG1725) , identified in Example 28 as a cell cycle gene, and human discs, large (Drosophila) homolog 1 (genbank accession U13896).
Figure 6B shows a ClustalW alignment of Drosophila discs large 1 Dlg1 (CG1725) and human discs, large (Drosophila) homolog 1 (genbank accession U13896).
Figure 6C shows a BLASTP alignment of Drosophila discs large 1 Dlg1 (CG1725), and human discs, large (drosophila) homolog 2 (genbank accession U32376).
Figure 6D shows a ClustalW alignment of Drosophila discs large 1 Dlg1 (CG1725) and human discs, large (drosophila) homolog 2 (genbank accession U32376).
Figure 7 shows a ClustalW alignment Drosophila Dlgl and 5 human Dlg genes (Dlg 1-5) so far described.
Figure 8 shows a histogram of FACS analysis of cell cycle status after siRNA in U2OS cells. Negative control is siRNA against the non-endogenous GL3 gene.
Figure 9 fluorescence micrographs showing the dominant phenotype observed with Dlg1 COD1654 siRNAi in U2OS cells. A) Multicentrosomal cells at prometaphase and anaphase. B) Cytokinesis defect
Figure 10 fluorescence micrographs showing the dominant phenotype observed with Dlg2 COD 1652 siRNAi in U2OS cells. A) Multicentrosomal cell at telophase. B) Cytokinesis defects.

### DETAILED DESCRIPTION

We provide for polynucleotide sand polypeptides whose sequences are set out, or which are referred to, in any of Examples 1 to 29, including *Drosophila* and human sequences. In particular, we provide for the sequences, including human sequences, and their use in diagnosis and treatment of disease (including prevention and treatment of diseases, syndromes and symptoms) as described in further detail below. A particularly suitable disease for treatment or diagnosis is a proliferative disease such as cancer or any tumour. The polynucleotides and polypeptides disclosed here may be used in screening assays to identify compounds which are capable of binding to, or inhibiting an activity of, the polypeptide or polynucleotide.

Particularly preferred polypeptides include those set out in Example 19 and referred to as Shp2, as well as those set out in Example 28 and referred to as Dlgland Dlg2. Accordingly, we provide for Shp2 polypeptide and polynucleotide, as well as Dlg1 and Dlg2 polypeptide and polynucleotide, for the treatment and diagnosis of diseases such as cancer, as described in further detail below.

By the term "Shp2", we mean a sequence as set out in Example 19 and having the accession number NM_002834, together with its variants, homologues, derivatives, fragments and complements as described in further detail below. Preferably, the term "Shp2" should be taken to refer to the human sequence itself. Two transcript variants (variants 1 and 2 as set out in Example 19) are known, and both are encompassed in the term "Shp2". Shp2 is also known as *Homo sapiens* protein tyrosine phosphatase, non-receptor type 11 (PTPN11). Furthermore, various sequences differing in length are known for Shp2, and each of these is intended to be included for the uses and compositions described here.

As used in this document, the terms "Dlg1" and "Dlg2" mean the sequences as set out in Example 28 and having the GENBANK accession numbers U13896 and U32376 respectively. Variants, homologues, derivatives, fragments and complements (as described in further detail below) of each of these sequences are also included within the meaning of these terms.

Dlg1 is also known as "human discs, large (Drosophila) homolog 1" while Dlg2 is also known as "human discs, large (Drosophila) homolog 2, chapsyn-110 channel-associated protein of synapses-110'''. Various sequences differing in length are known for Dlg1 and Dlg2, and each of these is intended to be included for the uses and compositions described here.

Preferably, the polypeptides and polynucleotides are such that they give rise to or are associated with defined phenotypes when mutated.

For example, mutations in the polypeptides and polynucleotides may be associated with female sterility; such polypeptides and polynucleotides are conveniently categorised as "Category 1". Phenotypes associated with Category 1 polypeptides and polynucleotides include any one or more of the following, singly or in combination: Female semi-sterile, brown eggs laid; female sterile, few eggs laid, several fully matured eggs in ovarioles; female semi-sterile, lays eggs, but arrest before cortical migration; "Female sterile, no eggs laid. Fully mature eggs, but "retained eggs" phenotype. Also has a mitotic phenotype: higher mitotic index, uneven chromosome staining, tangled and badly defined chromosomes with frequent bridges"; Female sterile (semi-sterile), 2-3 fully matured eggs in each of the ovarioles.

Alternatively, mutations in the polypeptides and polynucleotides may be associated with male sterility; such polypeptides and polynucleotides are conveniently categorised as "Category 2". Phenotypes associated with Category 2 polypeptides and polynucleotides include any one or more of the following, singly or in combination: Lethal phase pharate adult, cytokinesis defect - some onion stage cysts with large nebenkerns; reduced adult viability, cytokinesis defect - onion stage cysts have variable sized Nebenkerns - mitotic phenotype: tangled unevenly condensed chromosomes, anaphases with lagging chromosomes and bridges; semi-lethal male and female, cytokinesis defect - in some cysts, variable sized Nebenkerns; male sterile, cytokinesis defect, different meiotic stages within one cyst, variable sized nuclei, 2-4 nuclei, mitotic phenotype: semi-lethal, rod-like overcondensed chromosomes, high mitotic index, lagging chromosomes and bridges; male sterile, asynchronous meiotic divisions, cysts with large Nebenkern and 1-2 larger nuclei, testis from 2-3 old males become smaller, h igh mitotic index, colchicine type overcondensaton, many anaphases and telophases, no decondensation in telophase, mitotic phenotype: high mitotic index, colchicines-type overcondensed chromosomes, many ana- and relophases, no decondensation in telophase; cytokinesis defect, small testis, no meiosis observed, variable sized Nebenkerns with 2-4N nuclei; male sterile, cytokinesis defect, larger Nebenkerns with 2-4N nuclei; Male sterile, Cytokinesis defect: variable sized Nebenkerns with 4N nuclei, some nuclei detached from Nebenkern.

Mutations in the polypeptides and polynucleotides may be associated with a mitotic (neuroblast) phenotype ("Category 3"). Phenotypes associated with Category 3 polypeptides and polynucleotides include any one or more of the following, singly or in combination: lethal phase between pupil and pharate adult (P-pA), high mitotic index, rod-like overcondensed chromosomes, a few circular metaphases, many overcondensed anaphases and telophases, a few tetraploid cells; lethal phase pharate adult, high mitotic index, rod-like overcondensed chromosomes, lagging chromosomes and bridges in anaphase, highly condensed; lethal phase pupal - pharate adult, high mitotic index, colchicines- type overcondensation, high frequency of polyploids; lethal phase pupal - pharate adult, high mitotic index, colchicines-type overcondensed chromosomes, many strongly stained nuclei; lethal phase larval stage 3 - pre-pupal-pupal, small optic lobes, missing or small imaginal discs, badly defined chromosomes; lethal phase pharate adult, Dot and rod-like overcondensed chromosomes, high mitotic index, overcondensed anaphases some with lagging chromosomes, a few tetraploid cells with overcondensed chromosomes, XYY males; lethal phase embryonic larval phase3-pre-pupal-pupal, high mitotic index, dot-like chromosomes, strong metaphase arrest; lethal phase larval phase 3 D pre-pupal - pupal - pharate adult-adult, high mitotic index, dot and rod-like overcondensed chromosomes, high frequency of polyploids; lethal phase larval stage 3 (few pupae), high mitotic index, colchicine-type overcondensation of chromosomes, polyploid cells, mininuclei formation; lethal phase larval stage 1-2, low mitotic index, few cells in mitosis, metaphase with separated chromosomes; viable, high mitotic index, colchicines-type overcondensed chromosomes, a few polyploid cells; lethal phase pharate adult, high mitotic index, rod like overcondensed chromosomes, few anaphases with lagging chromosomes; lethal phase larval stage 3-pharate adult, small brain and optic lobes, high mitotic index, rod-like overcondensed chromosomes, fewer ana- and telophases, overcondensed chromosomes in ana- and telophase; lethal phase larval stage 3, small brain, few cells in mitosis, badly defined chromosomes, weak chromosome condensation, abnormal anaphases with broken chromosomes; lethal phase larval stage 3, small brain, high mitotic index, rod-like overcondensed chromosomes, fewer ana- and telophases; semilethal male and female, Low mitotic index, badly defined chromosomes, weak/uneven staining, fewer ana- and telophases; lethal phase pupal to pharate adult, lagging chromosomes and bridges in ana- and telophase; lethal phase, pupal, uneven chromosome condensation, lagging chromosomes in anaphase; lethal phase pupal, higher mitotic index, colchicine-like overcondensed chromosomes, many ana- and telophases, lagging chromosomes; lethal phase, prepupal - pupal, high mitotic index, colchicines-like chromosome condensation, metaphase arrest.

The polypeptides and polynucleotides described here may also be categorised according to their function, or their putative function.

For example, the polypeptides described here preferably comprise, and the polynucleotides described here are ones which preferably encode polypeptides comprising, any one or more of the following: CREB-binding proteins, transcription factors, casein kinases, serine threonine kinases, preferably involved in replication and cell cycle, protein phosphatases, membrane associated proteins, preferably involved in priming synaptic vesicles, dynein light chains, microtubule motor proteins, protein phosphatases, protein phosphatases with p53 dependent expression, proteins capable of inhibiting cell division, ribosomal proteins, motor proteins, cytoskeletal binding proteins linking to plama membrane, proteins involved in cytokinesis and cell shape, phosphatidylinositol 3-kinases, C-myc oncogenes, transcription factors, dehydrogenases, thioredoxin reductases, cell cycle regulators preferably involved in cyclin degradation; centrosome components, protein tyrosine phosphatases, Wnt oncogenes, ubiquitin ligases, ubiquitin conjugating enzymes, vesicle trafficking proteins, protein kinases (including protein kinases which regulate the G1/S phase transition and/or DNA replication in mammalian cells), serine/threonine kinases, including serine/threonine kinases involved in winglwess signaling pathway, components of cell junctions, including components of cell junctions having a role in proliferation and Ras associated effector proteins; hydroxymethyltransferase; glycosylation/membrane protein; hydrogen transporting ATP synthase; role in cell cycle progression.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual: Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3. Each of these general texts is herein incorporated by reference.

### POLYPEPTIDES

It will be understood that polypeptides as described here are not limited to polypeptides having the amino acid sequence set out in Examples 1 to 29 or fragments thereof but also include homologous sequences obtained from any source, for example related viral/bacterial proteins, cellular homologues and synthetic peptides, as well as variants or derivatives thereof.

Thus polypeptides also include those encoding homologues from other species including animals such as mammals (e.g. mice, rats or rabbits), especially primates, more especially humans. More specifically, such homologues include human homologues.

Thus, we describe variants, homologues or derivatives of the amino acid sequence set out in Examples 1 to 29, as well as variants, homologues or derivatives of the nucleotide sequence coding for the amino acid sequences as described here.

In the context of this document, a homologous sequence is taken to include an amino acid sequence which is at least 15, 20, 25, 30, 40, 50, 60, 70, 80 or 90% identical, preferably at least 95 or 98% identical at the amino acid level over at least 50 or 100, preferably 200, 300, 400 or 500 amino acids with any one of the polypeptide sequences shown in the Examples. In particular, homology should typically be considered with respect to those regions of the sequence known to be essential for protein function rather than non-essential neighbouring sequences. This is especially important when considering homologous sequences from distantly related organisms.

Although homology can also be considered in terms of similarity (i.e. amino acid residues having similar chemical properties/functions), in the context of this document, it is preferred to express homology in terms of sequence identity.

Homology comparisons can be conducted by eye, or more usually, with the aid of readily available sequence comparison programs. These publicly and commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al.,* 1999 *ibid -* Chapter 18), FASTA (Atschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The terms "variant" or "derivative" in relation to the amino acid sequences includes any substitution of, variation of, modification of, replacement of, deletion of or addition of one (or more) amino acids from or to the sequence providing the resultant amino acid sequence retains substantially the same activity as the unmodified sequence, preferably having at least the same activity as the polypeptides presented in the sequence listings in the Examples.

Polypeptides having the amino acid sequence shown in the Examples, or fragments or homologues thereof may be modified for use in the methods and compositions described here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Alternatively, modifications may be made to deliberately inactivate one or more functional domains of the polypeptides described here. Amino acid substitutions may include the use of non-naturally occurring analogues, for example to increase blood plasma half-life of a therapeutically administered polypeptide.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Polypeptides also include fragments of the full length sequences mentioned above. Preferably said fragments comprise at least one epitope. Methods of identifying epitopes are well known in the art. Fragments will typically comprise at least 6 amino acids, more preferably at least 10, 20, 30, 50 or 100 amino acids.

Proteins as described here are typically made by recombinant means, for example as described below. However they may also be made by synthetic means using techniques well known to skilled persons such as solid phase synthesis. Proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the function of the protein of interest sequence. Proteins as described here may also be obtained by purification of cell extracts from animal cells.

The proteins may be in a substantially isolated form. It will be understood that the protein may be mixed with carriers or diluents which will not interfere with the intended purpose of the protein and still be regarded as substantially isolated. A protein may also be in a substantially purified form, in which case it will generally comprise the protein in a preparation in which more than 90%, e.g. 95%, 98% or 99% of the protein in the preparation is a protein as described in this document.

A polypeptide may be labeled with a revealing label. The revealing label may be any suitable label which allows the polypeptide to be detected. Suitable labels include radioisotopes, e.g. ¹²⁵I, enzymes, antibodies, polynucleotides and linkers such as biotin. Labeled polypeptides as described here may be used in diagnostic procedures such as immunoassays to determine the amount of a polypeptide in a sample. Polypeptides or labeled polypeptides may also be used in serological or cell-mediated immune assays for the detection of immune reactivity to said polypeptides in animals and humans using standard protocols.

A polypeptide or labeled polypeptide or fragment thereof may also be fixed to a solid phase, for example the surface of an immunoassay well or dipstick. Such labeled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

The polypeptides described here may be used in *in vitro* or *in vivo* cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell by *in situ* expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (e.g. myristolation, glycosylation, truncation, lapidation and tyrosine, serine or threonine phosphorylation) as may be needed to confer optimal biological activity on recombinant expression products. Such cell culture systems in which such polypeptides are expressed may be used in assay systems to identify candidate substances which interfere with or enhance the functions of the polypeptides described here in the cell.

### POLYNUCLEOTIDES

We demonstrate here that mutations in genes encoding the polypeptides disclosed in the Examples demonstrate a cell cycle defect, and that accordingly these genes and the proteins encoded by them are responsible for cell cycle function.

Polynucleotides as described in this document include polynucleotides that comprise any one or more of the nucleic acid sequences encoding the polypeptides set out in Examples 1 to 29 and fragments thereof. Such polynucleotides also include polynucleotides encoding the polypeptides described here. It is straightforward to identify a nucleic acid sequence which encodes such a polypeptide, by reference to the genetic code. Furthermore, computer programs are available which translate a nucleic acid sequence to a polypeptide sequence, and/or *vice versa*. Each and all of sequences which are capable of encoding the polypeptides disclosed in the Examples is considered disclosed in this document, and the disclosure of a polypeptide sequence includes a disclosure of all nucleic acids (and their sequences) which encodes that polypeptide sequence.

It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

In preferred embodiments, the polynucleotides comprise those polypeptides, such as cDNA, mRNA, and genomic DNA of the relevant organism, which encode the polypeptides disclosed in the Examples. Such polynucleotides may typically comprise *Drosophila* cDNA, mRNA, and genomic DNA, *Homo sapiens* cDNA, mRNA, and genomic DNA, etc. Accession numbers are provided in the Examples for the polypeptide sequences, and it is straightforward to derive the encoding nucleic acid sequences by use of such accession numbers in a relevant database, such as a *Drosophila* sequence database, a human sequence database, including a Human Genome Sequence database, GadFly, FlyBase, etc. in particular, the annotated *Drosophila* sequence database of the Berkeley *Drosophila* Genome Project (GadFly: Genome Annotation Database of Drosophil at http://www.fruitfly.org/annot/) may be used to identify such *Drosophila* and human polynucleotide sequences. Relevant sequences may also be obtained by searching sequence databases such as BLAST with the polypeptide sequences. In particular, a search using TBLASTN may be employed.

Furthermore, we provide a method of identifying a human nucleic acid sequence, by: (a) selecting a *Drosophila* polypeptide identified in any of Examples 1 to 29; (b) identifying a corresponding human polypeptide; (c) identifying a nucleic acid encoding the polypeptide of (b). Step (b) may in particular involve identifying a human homologue of the *Drosophila* sequence, or a human sequence similar to the *Drosophila* sequence. Preferably, such a polypeptide has at least one of the biological activities, preferably substantially all the biological activities (such as identified in the Examples) of the *Drosophila* polypeptide. Preferably, the human polypeptide is involved in an aspect of cell cycle control. A human polypeptide identified as above, as well as a sequence of the human polypeptide and a sequence of the human nucleic acid are also provided.

Polynucleotides as described here may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of this document, it is to be understood that the polynucleotides described herein may be modified by any method available in the art. Such modifications may be carried out in order to enhance the *in vivo* activity or life span of polynucleotides.

The terms "variant", "homologue" or "derivative" in relation to a nucleotide sequence include any substitution of, variation of, modification of, replacement of, deletion of or addition of one (or more) nucleic acid from or to the sequence. Preferably said variant, homologues or derivatives code for a polypeptide having biological activity.

As indicated above, with respect to sequence homology, preferably there is at least 50 or 75%, more preferably at least 85%, more preferably at least 90% homology to the sequences shown in the sequence listing herein. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

This document also encompasses nucleotide sequences that are capable of hybridising selectively to the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides which capable of selectively hybridising to the nucleotide sequences presented herein, or to their complement, will be generally at least 70%, preferably at least 80 or 90% and more preferably at least 95% or 98% homologous to the corresponding nucleotide sequences presented herein over a region of at least 20, preferably at least 25 or 30, for instance at least 40, 60 or 100 or more contiguous nucleotides.

The term "selectively hybridizable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screening. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with ³²P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

In a preferred aspect, we describe nucleotide sequences that can hybridise to the nucleotide sequence as described here under stringent conditions (e.g. 65°C and 0.1xSSC {1xSSC = 0.15 M NaCl, 0.015 M Na₃ Citrate pH 7.0).

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

Polynucleotides which are not 100% homologous to the sequences of described here but are encompassed can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to sequences which encode the polypeptides shown in the Examples. Such sequences may be obtained by probing cDNA libraries made from or genomic DNA libraries from other animal species, and probing such libraries with probes comprising all or part of any on of the sequences under conditions of medium to high stringency. The nucleotide sequences of or which encode the human homologues described in the Examples, may preferably be used to identify other primate/mammalian homologues since nucleotide homology between human sequences and mammalian sequences is likely to be higher than is the case for the *Drosophila* sequences identified herein.

Similar considerations apply to obtaining species homologues and allelic variants of the polypeptide or nucleotide sequences described here.

Variants and strain/species homologues may also be obtained using degenerate PCR which will use primers designed to target sequences within the variants and homologues encoding conserved amino acid sequences within the sequences described here. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences. It will be appreciated by the skilled person that overall nucleotide homology between sequences from distantly related organisms is likely to be very low and thus in these situations degenerate PCR may be the method of choice rather than screening libraries with labeled fragments.

In addition, homologous sequences may be identified by searching nucleotide and/or protein databases using search algorithms such as the BLAST suite of programs. This approach is described below and in the Examples.

Alternatively, such polynucleotides may be obtained by site directed mutagenesis of characterised sequences, such as the sequences encoding polypeptides disclosed in the Examples. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides. For example, further changes may be desirable to represent particular coding changes found in the sequences coding polypeptides disclosed in the Examples which give rise to mutant genes which have lost their regulatory function. Probes based on such changes can be used as diagnostic probes to detect such mutants.

The polynucleotides described here may be used to produce a primer, e.g. a PCR primer, a primer for an alternative amplification reaction, a probe e.g. labeled with a revealing label by conventional means using radioactive or non-radioactive labels, or the polynucleotides may be cloned into vectors. Such primers, probes and other fragments will be at least 8, 9, 10, or 15, preferably at least 20, for example at least 25, 30 or 40 nucleotides in length, and are also encompassed by the term "polynucleotides" as used herein.

Polynucleotides such as a DNA polynucleotides and probes as described here may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

The polynucleotides or primers may carry a revealing label. Suitable labels include radioisotopes such as ³²P or ³⁵S, enzyme labels, or other protein labels such as biotin. Such labels may be added to the polynucleotides or primers and may be detected using by techniques known *per se.*

Polynucleotides or primers or fragments thereof labeled or unlabeled may be used by a person skilled in the art in nucleic acid-based tests for detecting or sequencing polynucleotides in the human or animal body.

Such tests for detecting generally comprise bringing a biological sample containing DNA or RNA into contact with a probe comprising a polynucleotide or primer as described here under hybridising conditions and detecting any duplex formed between the probe and nucleic acid in the sample. Such detection may be achieved using techniques such as PCR or by immobilising the probe on a solid support, removing nucleic acid in the sample which is not hybridised to the probe, and then detecting nucleic acid which has hybridised to the probe. Alternatively, the sample nucleic acid may be immobilised on a solid support, and the amount of probe bound to such a support can be detected. Suitable assay methods of this and other formats can be found in for example WO89/03891 and WO90/13667.

Tests for sequencing nucleotides include bringing a biological sample containing target DNA or RNA into contact with a probe comprising a polynucleotide or primer under hybridising conditions and determining the sequence by, for example the Sanger dideoxy chain termination method (see Sambrook *et al*.).

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and dTTP, a buffer and ATP. Dideoxynucleotides are used for selective termination.

Tests for detecting or sequencing nucleotides in a biological sample may be used to determine particular sequences within cells in individuals who have, or are suspected to have, an altered gene sequence, for example within cancer cells including leukaemia cells and solid tumours such as breast, ovary, lung, colon, pancreas, testes, liver, brain, muscle and bone tumours. Cells from patients suffering from a proliferative disease may also be tested in the same way.

In addition, the identification of the genes described in the Examples will allow the role of these genes in hereditary diseases to be investigated. In general, this will involve establishing the status of the gene (e.g. using PCR sequence analysis), in cells derived from animals or humans with, for example, neurological disorders or neoplasms.

The probes as described here may conveniently be packaged in the form of a test kit in a suitable container. In such kits the probe may be bound to a solid support where the assay format for which the kit is designed requires such binding. The kit may also contain suitable reagents for treating the sample to be probed, hybridising the probe to nucleic acid in the sample, control reagents, instructions, and the like.

### HOMOLOGY SEARCHING

Sequence homology (or identity) may be determined using any suitable homology algorithm, using for example default parameters.

Advantageously, the BLAST algorithm is employed, with parameters set to default values. The BLAST algorithm is described in detail at http://www.ncbi.nih.gov/BLAST/blast_help.html, which is incorporated herein by reference. The search parameters are defined as follows, and are advantageously set to the defined default parameters.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nih.gov/BLAST/blast_help.html) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul *et al*. (1994).

The five BLAST programs available at http://www.ncbi.nlm.nih.gov perform the following tasks:
**blastp** compares an amino acid query sequence against a protein sequence database;
**blastn** compares a nucleotide query sequence against a nucleotide sequence database;
**blastx** compares the six-frame conceptual translation products of a nucleotide query sequence (both strands) against a protein sequence database;
**tblastn** compares a protein query sequence against a nucleotide sequence database dynamically translated in all six reading frames (both strands).
**tblastx** compares the six-frame translations of a nucleotide query sequence against the six-frame translations of a nucleotide sequence database.
BLAST uses the following search parameters:
   HISTOGRAM Display a histogram of scores for each search; default is yes. (See parameter H in the BLAST Manual).
   DESCRIPTIONS Restricts the number of short descriptions of matching sequences reported to the number specified; default limit is 100 descriptions. (See parameter V in the manual page). See also EXPECT and CUTOFF.
   ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
   EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
   CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
   MATRIX Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.
   STRAND Restrict a TBLASTN search to just the top or bottom strand of the database sequences; or restrict a BLASTN, BLASTX or TBLASTX search to just reading frames on the top or bottom strand of the query sequence.
   FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at http://www.ncbi.nlm.nih.gov/BLAST.

### NUCLEIC ACID VECTORS

Polynucleotides as described in this document can be incorporated into a recombinant replicable vector. The vector may be used to replicate the nucleic acid in a compatible host cell. Thus in a further embodiment, we provide a method of making polynucleotides by introducing a polynucleotide as described here into a replicable vector, introducing the vector into a compatible host cell, and growing the host cell under conditions which bring about replication of the vector. The vector may be recovered from the host cell. Suitable host cells include bacteria such as *E*. *coli,* yeast, mammalian cell lines and other eukaryotic cell lines, for example insect Sf9 cells.

Preferably, a polynucleotide in a vector is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under condition compatible with the control sequences.

The control sequences may be modified, for example by the addition of further transcriptional regulatory elements to make the level of transcription directed by the control sequences more responsive to transcriptional modulators.

Vectors as described here may be transformed or transfected into a suitable host cell as described below to provide for expression of a protein. This process may comprise culturing a host cell transformed with an expression vector as described above under conditions to provide for expression by the vector of a coding sequence encoding the protein, and optionally recovering the expressed protein. Vectors will be chosen that are compatible with the host cell used.

The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used, for example, to transfect or transform a host cell.

Control sequences operably linked to sequences encoding a polypeptide described here include promoters/enhancers and other expression regulation signals. These control sequences may be selected to be compatible with the host cell for which the expression vector is designed to be used in. The term promoter is well-known in the art and encompasses nucleic acid regions ranging in size and complexity from minimal promoters to promoters including upstream elements and enhancers.

The promoter is typically selected from promoters which are functional in mammalian cells, although prokaryotic promoters and promoters functional in other eukaryotic cells, such as insect cells, may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase). They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

The polynucleotides may also be inserted into the vectors described above in an antisense orientation to provide for the production of antisense RNA. Antisense RNA or other antisense polynucleotides may also be produced by synthetic means. Such antisense polynucleotides may be used in a method of controlling the levels of RNAs transcribed from genes comprising any one of the polynucleotides as described.

### HOST CELLS

The vectors and polynucleotides may be introduced into host cells for the purpose of replicating the vectors/polynucleotides and/or expressing the polypeptides encoded by the polynucleotides described here. Although such polypeptides may be produced using prokaryotic cells as host cells, it is preferred to use eukaryotic cells, for example yeast, insect or mammalian cells, in particular mammalian cells.

Vectors/polynucleotides as described here may be introduced into suitable host cells using a variety of techniques known in the art, such as transfection, transformation and electroporation. Where vectors/polynucleotides are to be administered to animals, several techniques are known in the art, for example infection with recombinant viral vectors such as retroviruses, herpes simplex viruses and adenoviruses, direct injection of nucleic acids and biolistic transformation.

### PROTEIN EXPRESSION AND PURIFICATION

Host cells comprising polynucleotides as described here may be used to express polypeptides. Host cells may be cultured under suitable conditions which allow expression of the proteins. Expression of the polypeptides as described may be constitutive such that they are continually produced, or inducible, requiring a stimulus to initiate expression. In the case of inducible expression, protein production can be initiated when required by, for example, addition of an inducer substance to the culture medium, for example dexamethasone or IPTG.

Polypeptides can be extracted from host cells by a variety of techniques known in the art, including enzymatic, chemical and/or osmotic lysis and physical disruption.

The polypeptides may also be produced recombinantly in an *in vitro* cell-free system, such as the TnT™ (Promega) rabbit reticulocyte system.

### ANTIBODIES

We also provide monoclonal or polyclonal antibodies to polypeptides as described here, or fragments thereof. Thus, we further provide a process for the production of monoclonal or polyclonal antibodies to polypeptides.

If polyclonal antibodies are desired, a selected mammal (e.g., mouse, rabbit, goat, horse, etc.) is immunised with an immunogenic polypeptide bearing an epitope(s) from a polypeptide as described here. Serum from the immunised animal is collected and treated according to known procedures. If serum containing polyclonal antibodies to an epitope from a polypeptide contains antibodies to other antigens, the polyclonal antibodies can be purified by immunoaffinity chromatography. Techniques for producing and processing polyclonal antisera are known in the art. In order that such antibodies may be made, we also provide polypeptides as described here, or fragments thereof, haptenised to another polypeptide for use as immunogens in animals or humans.

Monoclonal antibodies directed against epitopes in the polypeptides described here can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against epitopes in the polypeptides can be screened for various properties; i.e., for isotype and epitope affinity.

An alternative technique involves screening phage display libraries where, for example the phage express scFv fragments on the surface of their coat with a large variety of complementarity determining regions (CDRs). This technique is well known in the art.

Antibodies, both monoclonal and polyclonal, which are directed against epitopes from polypeptides described here are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the antigen of the agent against which protection is desired.

Techniques for raising anti-idiotype antibodies are known in the art. These anti-idiotype antibodies may also be useful in therapy.

For the purposes of this document, the term "antibody", unless specified to the contrary, includes fragments of whole antibodies which retain their binding activity for a target antigen. Such fragments include Fv, F(ab') and F(ab')₂ fragments, as well as single chain antibodies (scFv). Furthermore, the antibodies and fragments thereof may be humanised antibodies, for example as described in EP-A-239400.

Antibodies may be used in method of detecting polypeptides as described in this document present in biological samples by a method which comprises: (a) providing an antibody as described here; (b) incubating a biological sample with said antibody under conditions which allow for the formation of an antibody-antigen complex; and (c) determining whether antibody-antigen complex comprising said antibody is formed.

Suitable samples include extracts tissues such as brain, breast, ovary, lung, colon, pancreas, testes, liver, muscle and bone tissues or from neoplastic growths derived from such tissues.

Such antibodies may be bound to a solid support and/or packaged into kits in a suitable container along with suitable reagents, controls, instructions and the like.

### ASSAYS

We also provide assays that are suitable for identifying substances which bind to polypeptides as described here and which affect, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, cytokinesis functions, chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways.

In addition, assays suitable for identifying substances that interfere with binding of polypeptides as described here, where appropriate, to components of cell division cycle machinery. This includes not only components such as microtubules but also signalling components and regulatory components as indicated above. Such assays are typically *in vitro.* Assays are also provided that test the effects of candidate substances identified in preliminary *in vitro* assays on intact cells in whole cell assays. The assays described below, or any suitable assay as known in the art, may be used to identify these substances.

In particular, we provide for the use of a polynucleotide as set out in Table 5, or a polypeptide encoded by the polypeptide, in a method of identifying a substance capable of binding to the polypeptide, which method comprises incubating the polypeptide with a candidate substance under suitable conditions and determining whether the substance binds to the polypeptide.

We further provide for use of a polynucleotide as set out in Table 5, or a polypeptide encoded by the polypeptide, in a method of identifying a substance capable of modulating the function of the polypeptide, the method comprising the steps of: incubating the polypeptide with a candidate substance and determining whether activity of the polypeptide is thereby modulated.

The substance identified may be isolated or synthesised, and used for prevention, treatment or diagnosis of a disease in an individual. The substance may be adminstered to an individual in need of such treatment. Alternatively or in addition, the substance identified by the assay is administered to an individual in need of such treatment. Preferably, the polynucleotide comprises a human polypeptide as set out in column 3 of Table 5.

Therefore, we provide one or more substances identified by any of the assays described below, *viz*, mitosis assays, meiotic assays, polypeptide binding assays, microtubule binding/polymerisation assays, microtubule purification and binding assays, microtubule organising centre (MTOC) nucleation activity assays, motor protein assay, assay for spindle assembly and function, assays for dna replication, chromosome condensation assays, kinase assays, kinase inhibitor assays, and whole cell assays, each as described in further detail below.

### CANDIDATE SUBSTANCES

A substance that inhibits cell cycle progression as a result of an interaction with a polypeptide as described here may do so in several ways. For example, if the substance inhibits cell division, mitosis and/or meiosis, it may directly disrupt the binding of a polypeptide as described here to a component of the spindle apparatus by, for example, binding to the polypeptide and masking or altering the site of interaction with the other component. A substance which inhibits DNA replication may do so by inhibiting the phosphorylation or de-phosphorylation of proteins involved in replication. For example, it is known that the kinase inhibitor 6-DMAP (6-dimethylaminopurine) prevents the initiation of replication (Blow, JJ, 1993, J Cell Biol122,993-1002). Candidate substances of this type may conveniently be preliminarily screened by *in vitro* binding assays as, for example, described below and then tested, for example in a whole cell assay as described below. Examples of candidate substances include antibodies which recognise a polypeptide as described in this document.

A substance which can bind directly to such a polypeptide may also inhibit its function in cell cycle progression by altering its subcellular localisation and hence its ability to interact with its normal substrate. The substance may alter the subcellular localisation of the polypeptide by directly binding to it, or by indirectly disrupting the interaction of the polypeptide with another component. For example, it is known that interaction between the p68 and p180 subunits of DNA polymerase alpha-primase enzyme is necessary in order for p180 to translocate into the nucleus (Mizuno et al (1998) Mol Cell Biol 18,3552-62), and accordingly, a substance which disrupts the interaction between p68 and p180 will affect nuclear translocation and hence activity of the primase. A substance which affects mitosis may do so by preventing the polypeptide and components of the mitotic apparatus from coming into contact within the cell.

These substances may be tested using, for example the whole cells assays described below. Non-functional homologues of a polypeptide as described here may also be tested for inhibition of cell cycle progression since they may compete with the wild type protein for binding to components of the cell division cycle machinery whilst being incapable of the normal functions of the protein or block the function of the protein bound to the cell division cycle machinery. Such non-functional homologues may include naturally occurring mutants and modified sequences or fragments thereof.

Alternatively, instead of preventing the association of the components directly, the substance may suppress the biologically available amount of a polypeptide as described here. This may be by inhibiting expression of the component, for example at the level of transcription, transcript stability, translation or post-translational stability. An example of such a substance would be antisense RNA or double-stranded interfering RNA sequences which suppresses the amount of mRNA biosynthesis.

Suitable candidate substances include peptides, especially of from about 5 to 30 or 10 to 25 amino acids in size, based on the sequence of the polypeptides described in the Examples, or variants of such peptides in which one or more residues have been substituted. Peptides from panels of peptides comprising random sequences or sequences which have been varied consistently to provide a maximally diverse panel of peptides may be used.

Suitable candidate substances also include antibody products (for example, monoclonal and polyclonal antibodies, single chain antibodies, chimeric antibodies and CDR-grafted antibodies) which are specific for a polypeptide as described here. Furthermore, combinatorial libraries, peptide and peptide mimetics, defined chemical entities, oligonucleotides, and natural product libraries may be screened for activity as inhibitors of binding of a polypeptide as described here to the cell division cycle machinery, for example mitotic/meiotic apparatus (such as microtubules). The candidate substances may be used in an initial screen in batches of, for example 10 substances per reaction, and the substances of those batches which show inhibition tested individually. Candidate substances which show activity in *in vitro* screens such as those described below can then be tested in whole cell systems, such as mammalian cells which will be exposed to the inhibitor and tested for inhibition of any of the stages of the cell cycle.

### POLYPEPTIDE BINDING ASSAYS

One type of assay for identifying substances that bind to a polypeptide as described here involves contacting a polypeptide as described here, which is immobilised on a solid support, with a non-immobilised candidate substance determining whether and/or to what extent the polypeptide as described here and candidate substance bind to each other. Alternatively, the candidate substance may be immobilised and the polypeptide non-immobilised.

In a preferred assay method, the polypeptide is immobilised on beads such as agarose beads. Typically this is achieved by expressing the component as a GST-fusion protein in bacteria, yeast or higher eukaryotic cell lines and purifying the GST-fusion protein from crude cell extracts using glutathione-agarose beads (Smith and Johnson, 1988). As a control, binding of the candidate substance, which is not a GST-fusion protein, to the immobilised polypeptide is determined in the absence of the polypeptide as described here. The binding of the candidate substance to the immobilised polypeptide is then determined. This type of assay is known in the art as a GST pulldown assay. Again, the candidate substance may be immobilised and the polypeptide non-immobilised.

It is also possible to perform this type of assay using different affinity purification systems for immobilising one of the components, for example Ni-NTA agarose and histidine-tagged components.

Binding of the polypeptide as described here to the candidate substance may be determined by a variety of methods well-known in the art. For example, the non-immobilised component may be labeled (with for example, a radioactive label, an epitope tag or an enzyme-antibody conjugate). Alternatively, binding may be determined by immunological detection techniques. For example, the reaction mixture can be Western blotted and the blot probed with an antibody that detects the non-immobilised component. ELISA techniques may also be used.

Candidate substances are typically added to a final concentration of from 1 to 1000 nmol/ml, more preferably from 1 to 100 nmol/ml. In the case of antibodies, the final concentration used is typically from 100 to 500 µg/ml, more preferably from 200 to 300 µg/ml.

### Microtubule Binding/Polymerisation Assays

In the case of polypeptides as described here that bind to microtubules, another type of *in vitro* assay involves determining whether a candidate substance modulates binding of such a polypeptide to microtubules . Such an assay typically comprises contacting a polypeptide as described here with microtubules in the presence or absence of the candidate substance and determining if the candidate substance has an affect on the binding of the polypeptide as described here to the microtubules. This assay can also be used in the absence of candidate substances to confirm that a polypeptide as described here does indeed bind to microtubules. Microtubules may be prepared and assays conducted as follows:

### Microtubule Purification and Binding Assays

Microtubules are purified from 0-3h-old *Drosophila* embryos essentially as described previously (Saunders, *et al*., 1997). About 3 ml of embryos are homogenized with a Dounce homogenizer in 2 volumes of ice-cold lysis buffer (0.1 M Pipes/NaOH, pH6.6, 5 mM EGTA, 1 mM MgSO4, 0.9 M glycerol, 1 mM DTT, 1 mM PMSF, 1 µg/ml aprotinin, 1 µg/ml leupeptin and 1 µg/ml pepstatin). The microtubules are depolymerized by incubation on ice for 15 min, and the extract is then centrifuged at 16,000 g for 30 min at 4°C. The supernatant is recentrifuged at 135,000 g for 90 min at 4°C. Microtubules in this later supernatant are polymerized by addition of GTP to 1 mM and taxol to 20 µM and incubation at room temperature for 30 min. A 3 ml aliquot of the extract is layered on top of 3 ml 15% sucrose cushion prepared in lysis buffer. After centrifuging at 54,000g for 30 min at 20°C using a swing out rotor, the microtubule pellet is resuspended in lysis buffer.

Microtubule overlay assays are performed as previously described (Saunders *et al.,* 1997). 500 ng per lane of recombinant Asp, recombinant polypeptide, and bovine serum albumin (BSA, Sigma) are fractionated by 10% SDS-PAGE and blotted onto PVDF membranes (Millipore). The membranes are preincubated in TBST (50mM Tris pH 7.5, 150 mM NaCl, 0.05% Tween 20) containing 5% low fat powdered milk (LFPM) for 1 h and then washed 3 times for 15 min in lysis buffer. The filters are then incubated for 30 minutes in lysis buffer containing either 1 mM GDP, 1 mM GTP, or 1 mM GTP-γ-S. MAP-free bovine brain tubulin (Molecular Probes) is polymerised at a concentration of 2 µg/ml in lysis buffer by addition of GTP to a final concentration of 1 mM and incubated at 37°C for 30 min. The nucleotide solutions are removed and the buffer containing polymerised microtubules added to the membanes for incubation for 1h at 37°C with addition of taxol at a final concentration of 10 µM for the final 30 min. The blots are then washed 3 times with TBST and the bound tubulin detected using standard Western blot procedures using anti-β-tubulin antibodies (Boehringer Manheim) at 2.5 µg/ml and the Super Signal detection system (Pierce).

It may be desirable in one embodiment of this type of assay to deplete the polypeptide as described here from cell extracts used to produce polymerise microtubules. This may, for example, be achieved by the use of suitable antibodies.

A simple extension to this type of assay would be to test the effects of purified polypeptide as described here upon the ability of tubulin to polymerise *in vitro* (for example, as used by Andersen and Karsenti, 1997) in the presence or absence of a candidate substance (typically added at the concentrations described above). *Xenopus* cell-free extracts may conveniently be used, for example as a source of tubulin.

### Microtubule Organising Centre (MTOC) Nucleation Activity Assays

Candidate substances, for example those identified using the binding assays described above, may be screening using a microtubule organising centre nucleation activity assay to determine if they are capable of disrupting MTOCs as measured by, for example, aster formation. This assay in its simplest form comprises adding the candidate substance to a cellular extract which in the absence of the candidate substance has microtubule organising centre nucleation activity resulting in formation of asters.

In a preferred embodiment, the assay system comprises (i) a polypeptide as described here and (ii) components required for microtubule organising centre nucleation activity except for functional polypeptide as described here, which is typically removed by immunodepletion (or by the use of extracts from mutant cells). The components themselves are typically in two parts such that microtubule nucleation does not occur until the two parts are mixed. The polypeptide as described here may be present in one of the two parts initially or added subsequently prior to mixing of the two parts.

Subsequently, the polypeptide as described here and candidate substance are added to the component mix and microtubule nucleation from centrosomes measured, for example by immunostaining for the polypeptide and visualising aster formation by immuno-fluorescence microscopy. The polypeptide may be preincubated with the candidate substance before addition to the component mix. Alternatively, both the polypeptide as described here and the candidate substance may be added directly to the component mix, simultaneously or sequentially in either order.

The components required for microtubule organising centre formation typically include salt-stripped centrosomes prepared as described in Moritz *et al.,* 1998. Stripping centrosome preparations with 2 M KI removes the centrosome proteins CP60, CP190, CNN and γ-tubulin. Of these, neither CP60 nor CP 190 appear to be required for microtubule nucleation. The other minimal components are typically provided as a depleted cellular extract, or conveniently, as a cellular extract from cells with a non-functional variant of a polypeptide as described here. Typically, labeled tubulin (usually β-tubulin) is also added to assist in visualising aster formation.

Alternatively, partially purified centrosomes that have not been salt-stripped may be used as part of the components. In this case, only tubulin, preferably labeled tubulin is required to complete the component mix.

Candidate substances are typically added to a final concentration of from 1 to 1000 nmol/ml, more preferably from 1 to 100 nmol/ml. In the case of antibodies, the final concentration used is typically from 100 to 500 µg/ml, more preferably from 200 to 300 µg/ml.

The degree of inhibition of aster formation by the candidate substance may be determined by measuring the number of normal asters per unit area for control untreated cell preparation and measuring the number of normal asters per unit area for cells treated with the candidate substance and comparing the result. Typically, a candidate substance is considered to be capable of disrupting MTOC integrity if the treated cell preparations have less than 50%, preferably less than 40, 30, 20 or 10% of the number of asters found in untreated cells preparations. It may also be desirable to stain cells for γ-tubulin to determine the maximum number of possible MTOCs present to allow normalisation between samples.

### Motor Protein Assay

The polypeptides may interact with motor proteins such as the Eg5-like motor protein *in vitro*. The effects of candidate substances on such a process may be determined using assays wherein the motor protein is immobilised on coverslips. Rhodamine labeled microtubules are then added and their translocation can be followed by fluorescent microscopy. The effect of candidate substances may thus be determined by comparing the extent and/or rate of translocation in the presence and absence of the candidate substance. Generally, candidate substances known to bind to a polypeptide as described here, would be tested in this assay. Alternatively, a high throughput assay may be used to identify modulators of motor proteins and the resulting identified substances tested for affects on a polypeptide as described above.

Typically this assay uses microtubules stabilised by taxol (e.g. Howard and Hyman 1993; Chandra and Endow, 1993 - both chapters in "Motility Assays for Motor Proteins" Ed Jon Scholey, pub Academic Press). If however, a polypeptide as described here were to promote stable polymerisation of microtubules (see above) then these microtubules could be used directly in motility assays.

Simple protein-protein binding assays as described above, using a motor protein and a polypeptide as described here may also be used to confirm that the polypeptide binds to the motor protein, typically prior to testing the effect of candidate substances on that interaction.

### Assay for Spindle Assembly and Function

A further assay to investigate the function of polypeptide as described here and the effect of candidate substances on those functions is an assay which measures spindle assembly and function. Typically, such assays are performed using *Xenopus* cell free systems, where two types of spindle assembly are possible. In the "half spindle" assembly pathway, a cytoplasmic extract of CSF arrested oocytes is mixed with sperm chromatin. The half spindles that form subsequently fuse together. A more physiological method is to induce CSF arrested extracts to enter interphase by addition of calcium, whereupon the DNA replicates and kinetochores form. Addition of fresh CSF arrested extract then induces mitosis with centrosome duplication and spindle formation (for discussion of these systems see Tournebize and Heald, 1996).

Again, generally, candidate substances known to bind to a polypeptide as described here, or non-functional polypeptide variants, would be tested in this assay. Alternatively, a high throughput assay may be used to identify modulators of spindle formation and function and the resulting identified substances tested for affects binding of the polypeptide as described above.

### Assays for DNA Replication

Another assay to investigate the function of polypeptide as described here and the effect of candidate substances on those functions is as assay for replication of DNA. A number of cell free systems have been developed to assay DNA replication. These can be used to assay the ability of a substance to prevent or inhibit DNA replication, by conducting the assay in the presence of the substance. Suitable cell-free assay systems include, for example the SV-40 assay (Li and Kelly, 1984, Proc. Natl. Acad Sci USA 81, 6973-6977; Waga and Stillman, 1994, Nature 369, 207-212.). A *Drosophila* cell free replication system, for example as described by Crevel and Cotteril (1991), EMBO J. 10, 4361-4369, may also be used. A preferred assay is a cell free assay derived from *Xenopus* egg low speed supernatant extracts described in Blow and Laskey (1986, Cell 47,577-587) and Sheehan et al. (1988, J. Cell Biol. 106, 1-12), which measures the incorporation of nucleotides into a substrate consisting of *Xenopus* sperm DNA or HeLa nuclei. The nucleotides may be radiolabelled and incorporation assayed by scintillation counting. Alternatively and preferably, bromo-deoxy-uridine (BrdU) is used as a nucleotide substitute and replication activity measured by density substitution. The latter assay is able to distinguish genuine replication initiation events from incorporation as a result of DNA repair. The human cell-free replication assay reported by Krude, et al (1997), Cell 88, 109-19 may also be used to assay the effects of substances on the polypeptides.

### Other In Vitro Assays

Other assays for identifying substances that bind to a polypeptide as described here are also provided. For example, substances which affect chromosome condensation may be assayed using the *in vitro* cell free system derived from *Xenopus* eggs, as known in the art.

Substances which affect kinase activity or proteolysis activity are of interest. It is known, for example, that temporal control of ubiquitin-proteasome mediated protein degradation is critical for normal G1 and S phase progression (reviewed in Krek 1998, Curr Opin Genet Dev 8, 36-42). A number of E3 ubiquitin protein ligases, designated SCFs (Skpl-cullin-F-box protein ligase complexes), confer substrate specificity on ubiquitination reactions, while protein kinases phosphorylate substrates destined for destruction and convert them into preferred targets for ubiquitin modification catalyzed by SCFs. Furthermore, ubiquitin-mediated proteolysis due to the anaphase-promoting complex/cyclosome (APC/C) is essential for separation of sister chromatids during mitosis, and exit from mitosis (Listovsky et al., 2000, Exp Cell Res 255, 184-191).

Substances which inhibit or affect kinase activity may be identified by means of a kinase assay as known in the art, for example, by measuring incorporation of ³²P into a suitable peptide or other substrate in the presence of the candidate substance. Similarly, substances which inhibit or affect proteolytic activity may be assayed by detecting increased or decreased cleavage of suitable polypeptide substrates.

Assays for these and other protein or polypeptide activities are known to those skilled in the art, and may suitably be used to identify substances which bind to a polypeptide and affect its activity.

### Whole Cell Assays

Candidate substances may also be tested on whole cells for their effect on cell cycle progression, including mitosis and/or meiosis. Preferably the candidate substances have been identified by the above-described *in vitro* methods. Alternatively, rapid throughput screens for substances capable of inhibiting cell division, typically mitosis, may be used as a preliminary screen and then used in the *in vitro* assay described above to confirm that the affect is on a particular polypeptide.

The candidate substance, i.e. the test compound, may be administered to the cell in several ways. For example, it may be added directly to the cell culture medium or injected into the cell. Alternatively, in the case of polypeptide candidate substances, the cell may be transfected with a nucleic acid construct which directs expression of the polypeptide in the cell. Preferably, the expression of the polypeptide is under the control of a regulatable promoter.

Typically, an assay to determine the effect of a candidate substance identified by the method as described here on a particular stage of the cell division cycle comprises administering the candidate substance to a cell and determining whether the substance inhibits that stage of the cell division cycle. Techniques for measuring progress through the cell cycle in a cell population are well known in the art. The extent of progress through the cell cycle in treated cells is compared with the extent of progress through the cell cycle in an untreated control cell population to determine the degree of inhibition, if any. For example, an inhibitor of mitosis or meiosis may be assayed by measuring the proportion of cells in a population which are unable to undergo mitosis/meiosis and comparing this to the proportion of cells in an untreated population.

The concentration of candidate substances used will typically be such that the final concentration in the cells is similar to that described above for the *in vitro* assays.

A candidate substance is typically considered to be an inhibitor of a particular stage in the cell division cycle (for example, mitosis) if the proportion of cells undergoing that particular stage (i.e., mitosis) is reduced to below 50%, preferably below 40, 30, 20 or 10% of that observed in untreated control cell populations.

### THERAPEUTIC USES

Many tumours are associated with defects in cell cycle progression, for example loss of normal cell cycle control. Tumour cells may therefore exhibit rapid and often aberrant mitosis. One therapeutic approach to treating cancer may therefore be to inhibit mitosis in rapidly dividing cells. Such an approach may also be used for therapy of any proliferative disease in general. Thus, since the polypeptides described here appear to be required for normal cell cycle progression, they represent targets for inhibition of their functions, particularly in tumour cells and other proliferative cells.

The term proliferative disorder is used herein in a broad sense to include any disorder that requires control of the cell cycle, for example, cardiovascular disorders such as restenosis and cardiomyopathy, auto-immune disorders such as glomerulonephritis and rheumatoid arthritis, dermatological disorders such as psoriasis, anti-inflammatory, antifungal, antiparasitic disorders such as malaria, emphysema and alopecia.

One possible approach is to express anti-sense constructs directed against polynucleotides described in this document, preferably selectively in tumour cells, to inhibit gene function and prevent the tumour cell from progressing through the cell cycle. Anti-sense constructs may also be used to inhibit gene function to prevent cell cycle progression in a proliferative cell. Such anti-sense constructs may comprise anti-sense molecules corresponding to any of the polynucleotides, in particular, those identified in Table 5.

Alternatively, or in addition, RNAi may be used to modulate expression of the polynucleotide in a cell. Double stranded RNA may be made as described in the Examples, e.g., by transcribing both strands of a polynucleotide sequence in a suitable vector (e.g., from T7 or other promoters on either side of the cloned sequence), denatured and annealed. The double stranded RNA (ds RNA) may then be introduced into a relevant cell to inhibit the transcription or expression of the relevant polynucleotide or polypeptide.
We therefore describe a method of modulating, preferably down-regulating, the expression of a polynucleotide as described here, preferably a polynucleotide as set out in Table 5 in a cell, the method comprising introducing a double stranded RNA (dsRNA) corresponding to the polynucleotide, or an antisense RNA corresponding to the polynucleotide, or a fragment thereof, into the cell.

Another approach is to use non-functional variants of the polypeptides that compete with the endogenous gene product for cellular components of cell cycle machinery, resulting in inhibition of function. Alternatively, compounds identified by the assays described above as binding to a polypeptide may be administered to tumour or proliferative cells to prevent the function of that polypeptide. This may be performed, for example, by means of gene therapy or by direct administration of the compounds. Suitable antibodies may also be used as therapeutic agents.

Alternatively, double-stranded (ds) RNA is a powerful way of interfering with gene expression in a range of organisms that has recently been shown to be successful in mammals (Wianny and Zernicka-Goetz, 2000, Nat Cell Biol 2000, 2, 70-75). Double stranded RNA corresponding to the sequence of a polynucleotide can be introduced into or expressed in oocytes and cells of a candidate organism to interfere with cell division cycle progression.

In addition, a number of the mutations described herein exhibit aberrant meiotic phenotypes. Aberrant meiosis is an important factor in infertility since mutations that affect only meiosis and not mitosis will lead to a viable organism but one that is unable to produce viable gametes and hence reproduce. Consequently, the elucidation of genes involved in meiosis is an important step in diagnosing and preventing/treating fertility problems. Thus the polypeptides identified in mutant *Drosophila* having meiotic defects (as is clearly indicated in the Examples) may be used in methods of identifying substances that affect meiosis. In addition, these polypeptides, and corresponding polynucleotides, may be used to study meiosis and identify possible mutations that are indicative of infertility. This will be of use in diagnosing infertility problems.

### ADMINISTRATION

Substances identified or identifiable by the assay methods described here may preferably be combined with various components to produce compositions. Preferably the compositions are combined with a pharmaceutically acceptable carrier or diluent to produce a pharmaceutical composition (which may be for human or animal use). Suitable carriers and diluents include isotonic saline solutions, for example phosphate-buffered saline. The composition as described here may be administered by direct injection. The composition may be formulated for parenteral, intramuscular, intravenous, subcutaneous, intraocular or transdermal administration. Typically, each protein may be administered at a dose of from 0.01 to 30 mg/kg body weight, preferably from 0.1 to 10 mg/kg, more preferably from 0.1 to 1 mg/kg body weight.

Polynucleotides/vectors encoding polypeptide components (or antisense constructs) for use in inhibiting cell cycle progression, for example, inhibiting mitosis or meiosis, may be administered directly as a naked nucleic acid construct. They may further comprise flanking sequences homologous to the host cell genome. When the polynucleotides/vectors are administered as a naked nucleic acid, the amount of nucleic acid administered may typically be in the range of from 1 µg to 10 mg, preferably from 100 µg to 1 mg. It is particularly preferred to use polynucleotides/ vectors that target specifically tumour or proliferative cells, for example by virtue of suitable regulatory constructs or by the use of targeted viral vectors.

Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam™ and transfectam™). Typically, nucleic acid constructs are mixed with the transfection agent to produce a composition.

Preferably the polynucleotide, polypeptide, compound or vector described here may be conjugated, joined, linked, fused, or otherwise associated with a membrane translocation sequence.

Preferably, the polynucleotide, polypeptide, compound or vector, etc described here may be delivered into cells by being conjugated with, joined to, linked to, fused to, or otherwise associated with a protein capable of crossing the plasma membrane and/or the nuclear membrane (i.e., a membrane translocation sequence). Preferably, the substance of interest is fused or conjugated to a domain or sequence from such a protein responsible for the translocational activity. Translocation domains and sequences for example include domains and sequences from the HIV-1-trans-activating protein (Tat), *Drosophila* Antennapedia homeodomain protein and the herpes simplex-1 virus VP22 protein. In a highly preferred embodiment, the substance of interest is conjugated with penetratin protein or a fragment of this. Penetratin comprises the sequence RQIKIWFQNRRMKWKK and is described in Derossi, et al., (1994), J. Biol. Chem. 269, 10444-50; use of penetratin-drug conjugates for intracellular delivery is described in WO/00/01417. Truncated and modified forms of penetratin may also be used, as described in WO/00/29427.

Preferably the polynucleotide, polypeptide, compound or vector is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular or transdermal administration.

The routes of administration and dosages described are intended only as a guide since a skilled practitioner will be able to determine readily the optimum route of administration and dosage for any particular patient and condition.

### FURTHER ASPECTS

Further aspects of the invention are set out in the following numbered paragraphs; it is to be understood that the invention includes these aspects.

Paragraph 1. A polynucleotide selected from: (a) polynucleotides encoding any one of the polypeptide sequences set out in Examples 1 to 30 or the complement thereof; (b) polynucleotides comprising a nucleotide sequence capable of hybridising to the polynucleotides defined in (a) above, or a fragment thereof; (c) polynucleotides comprising a nucleotide sequence capable of hybridising to the complement of the polynucleotides defined in (a) above, or a fragment thereof; (d) polynucleotides comprising a polynucleotide sequence which is degenerate as a result of the genetic code to the polynucleotides defined in (a), (b) or (c).

Paragraph 2. A polynucleotide selected from: (a) polynucleotides encoding any one of the polypeptide sequences set out in Examples 1, 2, 2A, 2B and 2C or the complement thereof; (b) polynucleotides comprising a nucleotide sequence capable of hybridising to the polynucleotides defined in (a) above, or a fragment thereof; (c) polynucleotides comprising a nucleotide sequence capable of hybridising to the complement of the polynucleotides defined in (a) above, or a fragment thereof; (d) polynucleotides comprising a polynucleotide sequence which is degenerate as a result of the genetic code to the polynucleotides defined in (a), (b) or (c).

Paragraph 3. A polynucleotide selected from: (a) polynucleotides encoding any one of the polypeptide sequences set out in Examples 3 to 9 and 9A or the complement thereof; (b) polynucleotides comprising a nucleotide sequence capable of hybridising to the polynucleotides defined in (a) above, or a fragment thereof; (c) polynucleotides comprising a nucleotide sequence capable of hybridising to the complement of the polynucleotides defined in (a) above, or a fragment thereof; (d) polynucleotides comprising a polynucleotide sequence which is degenerate as a result of the genetic code to the polynucleotides defined in (a), (b) or (c).

Paragraph 4. A polynucleotide selected from: (a) polynucleotides encoding any one of the polypeptide sequences set out in Examples 10 to 29 or the complement thereof; (b) polynucleotides comprising a nucleotide sequence capable of hybridising to the polynucleotides defined in (a) above, or a fragment thereof; (c) polynucleotides comprising a nucleotide sequence capable of hybridising to the complement of the polynucleotides defined in (a) above, or a fragment thereof; (d) polynucleotides comprising a polynucleotide sequence which is degenerate as a result of the genetic code to the polynucleotides defined in (a), (b) or (c).

Paragraph 5. A polynucleotide probe which comprises a fragment of at least 15 nucleotides of a polynucleotide according to any of Paragraph s 1 to 4.

Paragraph 6. A polypeptide which comprises any one of the amino acid sequences set out in Examples 1 to 30 or in any of Examples 1 to 2, 2A, 2B and 2C, Examples 3 to 9 and 9A and Examples 10 to 29 or a homologue, variant, derivative or fragment thereof.

Paragraph 7. A polynucleotide encoding a polypeptide according to Paragraph 6.

Paragraph 8. A vector comprising a polynucleotide according to any of Paragraph s 1 to 5 and 7.

Paragraph 9. An expression vector comprising a polynucleotide according to any of Paragraph s 1 to 5 and 7 operably linked to a regulatory sequence capable of directing expression of said polynucleotide in a host cell.

Paragraph 10. An antibody capable of binding a polypeptide according to Paragraph 6.

Paragraph 11. A method for detecting the presence or absence of a polynucleotide according to any of Paragraph s 1 to 5 and 7 in a biological sample which comprises: (a) bringing the biological sample containing DNA or RNA into contact with a probe according to Paragraph 5 under hybridising conditions; and (b) detecting any duplex formed between the probe and nucleic acid in the sample.

Paragraph 12. A method for detecting a polypeptide according to Paragraph 6 present in a biological sample which comprises: (a) providing an antibody according to Paragraph 10; (b) incubating a biological sample with said antibody under conditions which allow for the formation of an antibody-antigen complex; and (c) determining whether antibody-antigen complex comprising said antibody is formed.

Paragraph 13. A polynucleotide according to according to any of Paragraph s 1 to 5 and 7 for use in therapy.

Paragraph 14. A polypeptide according to Paragraph 6 for use in therapy.

Paragraph 15. An antibody according to Paragraph 10 for use in therapy.

Paragraph 16. A method of treating a tumour or a patient suffering from a proliferative disease comprising administering to a patient in need of treatment an effective amount of a polynucleotide according to any of Paragraph s 1 to 5 and 7.

Paragraph 17. A method of treating a tumour or a patient suffering from a proliferative disease, comprising administering to a patient in need of treatment an effective amount of a polypeptide according to Paragraph 6.

Paragraph 18. A method of treating a tumour or a patient suffering from a proliferative disease, comprising administering to a patient in need of treatment an effective amount of an antibody according to Paragraph 10 to a patient.

Paragraph 19. Use of a polypeptide according to Paragraph 6 in a method of identifying a substance capable of affecting the function of the corresponding gene.

Paragraph 20. Use of a polypeptide according to Paragraph 6 in an assay for identifying a substance capable of inhibiting the cell division cycle.

Paragraph 21. Use as Paragraph ed in Paragraph 20, in which the substance is capable of inhibiting mitosis and/or meiosis.

Paragraph 22. A method for identifying a substance capable of binding to a polypeptide according to Paragraph 6, which method comprises incubating the polypeptide with a candidate substance under suitable conditions and determining whether the substance binds to the polypeptide.

Paragraph 23. A method for identifying a substance capable of modulating the function of a polypeptide according to Paragraph 6 or a polypeptide encoded by a polynucleotide according to any of Paragraph s 1 to 5 and 7, the method comprising the steps of: incubating the polypeptide with a candidate substance and determining whether activity of the polypeptide is thereby modulated.

Paragraph 24. A substance identified by a method or assay according to any of Paragraph s 19 to 23.

Paragraph 25. Use of a substance according to Paragraph 24 in a method of inhibiting the function of a polypeptide.

Paragraph 26. Use of a substance according to Paragraph 24 in a method of regulating a cell division cycle function.

Paragraph 27. A method of identifying a human nucleic acid sequence, by: (a) selecting a *Drosophila* polypeptide identified in any of Examples 1 to 30; (b) identifying a corresponding human polypeptide; (c) identifying a nucleic acid encoding the polypeptide of (b).

Paragraph 28. A method according to Paragraph 27, in which a human homologue of the *Drosophila* sequence, or a human sequence similar to the *Drosophila* sequence, is identified in step (b).

Paragraph 29. A method according to Paragraph 27 or 28, in which the human polypeptide has at least one of the biological activities, preferably substantially all the biological activities of the *Drosophila* polypeptide.

Paragraph 30. A human polypeptide identified by a method according to Paragraph 27, 28 or 29.

The invention will now be further described by way of Examples, which are meant to serve to assist one of ordinary skill in the art in carrying out the invention and are not intended in any way to limit the scope of the invention.

### EXAMPLES

### EXAMPLES SECTION A: IDENTIFICATION OF HUMAN CELL CYCLE GENES

### Introduction

In order to identify new cell cycle regulatory genes in *Drosophila* and their human counterparts, we investigated 33 fly lines obtained by P-element mutagenesis carried out on the X chromosome. All those fly lines are screened directly for mitotic phenotypes at developmental stages where division is crucial (i.e. the syncytial embryo, larval brains, and male and female meiosis). In each case, the P-element insertion site is identified leading to the selection of 62 genes flanking the insertion site.¹
¹ Deleted sentence and moved below.

In order to clarify the identity of the mutated "mitotic genes", we use an RNAi-based knockdown approach in cultured *Drosophila* cells followed by FACS analysis, mitotic index evaluation (Cellomics Arrayscan) and immunofluorescence observations of mitotic phenotypes for all 63 genes.

The microscope phenotyping approach led to the identification of 30 gene candidates that are required for cell cycle progression, some of which are also detected as presenting some changes in the FACS profile and/or in the mitotic index (see Table 5 for a full summary). ²Data relating to these genes is presented in Examples Section B, Examples 1 to 29 below.
² Sentence inserted from above

These genes encode a variety of novel proteins: 6 protein kinases; 2 protein phosphatases, 2 proteins of the ubiquitin-mediated protein degradation pathway, a cytosketal protein, a microtubule-binding protein, a homologue of a suspected kinesin-like protein, a RNA polymerase 2 associated cyclin, a ribosomal protein; a protein involved in retrograde (Golgi to ER) transport, a member of the family of thioredoxin reductases, a hydroxymethyltransferase, a Cdk associated protein, an RNA binding protein, an O-acetyl transferase and 9 other novel proteins with no particularly characteristic identifying features.

Human counterparts of the selected genes are identified and tested as described below. A short list of *Drosophila* and human genes and proteins useful for screening for anti-proliferative molecules is presented as Table 5.

**Table 5:**

| Short list of potentially new interesting gene candidates³ | | |
|---|---|---|
| Drosophila Gene Name | Human Homologue Gene Name | Human Homologue Accession Number |
| CG2028 | Casein kinase I | P48729 |
| CG3011 | Serine hydroxymethyl transferase | AAA63258 |
| CG15309 | DiGeorge syndrome related protein FKSG4 | AAL09354 |
| CG15305 | Human homologue of CG15305 | None |
| CG2222 | Hypothetical protein FLJ13912 | NP_073607 |
| CG2938 | CAS1 O-acetyltransferase | NP_075051 |
| CG1524 | Ribosomal protein S14 | A25220 |
| CG10778 | Hypothetical protein FLJ13102 (kinesin like) | NP_079163 |
| CG18292 | Cdk associated protein 1 (deleted in oral cancer) | BAA22937 |
| CG10701 | Moesin | A41289 |
| CG10648 | Mak 16-like RNA binding protein | NP_115898 |
| CG2854 | CAD38627 hypothetical protein | CAD38627 |
| CG2845 | B-raf | AAA35609 |
| CG1486 | BAA19780 novel protein | BAA19780 |
| CG10964 | 11-cis retinal dehydrogenase | AAC50725 |
| CG2151 | Thioredoxin reductase beta | XP_033135 |
| CG10988 | Gamma tubulin ring complex 3 | AAC39727 |
| CG1558 | Human homologue of CG1558 | NONE |
| CG11697 | Novel protein | BAB14444 unamed protein - similar to a hypothetical protein in the region deleted in human familial |
| CG3954 | Protein tyrosine phosphatase non- receptor type 11 (Shp2) | AAH08692 |
| CG16903 | Cyclin L ania-6a | AAD53184 |
| CG16983 | Skp1 ubiquitin ligase | XP_054159 |
| CG13363 | CGI-85 | NP_057112 |
| CG18319 | Ubc13 ubiquitin conjugating enzyme | BAA11675 |
| CG14813 | archain | CAA57071 |
| CG8655 | Cdc7 | AAB97512 |
| CG2621 | GSK 3 beta | NP_002084 |
| CG1725 | Dlg1/Dlg2 | XP_012060 |
| CG1594 | JAK-2 Janus kinase 2 | NP_004963 |
| CG2096 | Protein phosphatase 1 | NP_002700 |

| | | |
|---|---|---|
| ³ Replaced with Table from email | | |

### Results

Table 6 shows all significant cell cycle phenotypes observed after RNAi with the *Drosophila* genes flanking P-element insertion sites identified in Examples 1 to 29. The PCR primers used to create the double stranded RNA (see Materials and Methods above) are shown in each case together with the RNA ID number. Results derived from Facs analysis of cell cycle compartment, mitotic index as determined by the Cellomics mitotic index assay, and cellular phenotypes determined by microscopy are shown.

### FACS analysis of cell cycle

FACS analysis is used to assess the effects of *Drosophila* gene specific RNAi on the cell cycle. Through the determination of the DNA content by propidium iodide quantitation, any changes in the cell cycle distribution in sub-G1 (apoptotic), G1, G2/M can be observed. 24 genes in the Facs assessment present some changes in cell cycle distribution. (Table 6).

### Mitotic index evaluation with Cellomics Arrayscan

An evaluation of mitotic index.is performed using the Cellomics arrayscan and the Cellomics proprietary mitotic index HitKit procedure (see Materials and Methods above).

The basic principle of this method is that cells in mitosis are decorated by an antibody directed against a specific mitotic marker. Their proportion relatively to the total number of cells is determined, giving a proportion of cells in mitosis. This automated method presents the advantage of being more rapid than the microscope observations, however it only measures one feature of the cycling cells. Some mitotic genes that do not significantly affect the overall proportion of cells in mitosis will therefore not be detected.
The reverse is also true as the knockdown of some gene products might affect the mitotic index without displaying any obvious increase in chromosomal or spindle defects. Table 6 presents data only where there was a statistically significant variation in the mitotic index (determined by a Ttest value of ⁴< 0.1) as compared to the RFP RNAi control.
⁴ Changed to less than (email said right angle bracker)

An increase in mitotic index can indicate that the knockdown of a gene essential for completion of mitosis has blocked more cells in mitosis, however many of the gene knockdowns listed in Table 6 result in a decrease in the mitotic index, suggesting that the population of cells overall are spending less time in mitosis. Possible interpretations of this, are that defects in the centrosome duplication cycle block some cells in G1/S and they are unable to enter mitosis, or that defects in cytokinesis block cells on the exit from mitosis at a point after the assay specific marker is lost. The loss of checkpoints at mitosis may also allow cells to move faster through mitosis. The increase in mitotic defects observed for most of these genes might then be the result of this lack of checkpoint control.

13 genes in the phenotype assessment present some changes in the mitotic index (Table 6).

### Microscope Observation and Cellular Phenotyping

The primary goal of the cell phenotype assessment is to find abnormalities in the following: chromosome number in prometaphase (ploidy), chromosome behaviour in metaphase or anaphase, spindle morphology, number of centrosomes, and cell viability. The secondary goal of the assessment is to evaluate and quantify these abnormalities, this is an essential step as control cells also present some defects.

The wild-type *Drosophila* DMEL2 cells present a large range and a significant proportion of chromosomal defects (between 30-40 %). Therefore, between 300 and 500 mitotic cells were counted for each experiment in order to obtain a statistically significant evaluation of any change in the proportion of defects. The cells categorized as presenting chromosomal defects in the study encompass aneuploid and polyploid prometaphase cells, cells that apparently fail to align their chromosomes at metaphase and the cells with lagging or stretched chromosomes in anaphase. Spindle defects are also noted, but not quantified in the same group. Some candidates are also noted as presenting a significant decrease in the number of mitotic cells (mitotic index) or as affecting the viability of the cells (decrease in cell confluency or presence of apoptotic cells)..

A noteworthy observation is that it is difficult to find a unique representative phenotype for most of the genes tested. Rather than one gene = one phenotype, an overall increase in the different categories of chromosomal defects is observed. However, one can often see a more significant increase in one particular subcategory of defects as for example in the proportion of lagging chromatids or the number of centrosomes.

Table 6 describes the data obtained from these studies for genes where a significant phenotype is observed. 30 of the candidate genes show a significant phenotype, 26 of which show an increase in chromosomal defects. This increase in mitotic chromosome behaviour abnormalities is sometimes associated with an increase in mitotic spindle defects. Of the remaining 4 with no increase in chromosomal defects, CG1725 (RNA528/529) shows a clear increase in spindle defects, with CG1524 (RNA 482/483) there are not enough mitotic cells to do a proper quantification (as the gene product is a ribosomal protein, it is highly probable that its inactivation results in a net increase in the proportion of cell death explaining the drop in cell confluency also observed) and for CG14813 (RNA 586/587), a large proportion of cells are dying and there is an obvious decrease in the number of mitotic cells, this might affect the relative proportion of normal and abnormal mitotic cells. Finally CG10648 (RNA 488/489) had a lower proportion of chromosomal defects but a high proportion of monopolar and small spindles. The proportion of prometaphase cells and apoptotic cells was also high.

### Conclusion

From a collection of *Drosophila* P-element insertion lines which display phenotypes consistent with an effect on mitosis we derived a series of novel *Drosophila* and human genes which represent targets for the development of anti-proloiferative therapies. We used three different approaches to validate the role of each gene in the cell cycle and to gather phenotype information following an RNAi-based gene knockdown approach.

Table 5 shows a short list of 30 new interesting human genes demonstrated to play a role in mitosis. This short list is mainly based on the results of the detailed microscope phenotype evaluation (see Table 6), although all of the 42 genes listed in Table 6 show a cell cycle related phenotype in one or more of the 3 assays.

### MATERIALS AND METHODS

### Generation and Identification of Lethal, Semi-Lethal and Sterile X Chromosome Mutants Having Defects in Mitosis and/or Meiosis

### P-Element Mutagenesis

Transposable elements are widely used for mutagenesis in *Drosophila melanogaster* as they couple the advantages of providing effective genetic lesions with ease of detecting disrupted genes for the purpose of molecular cloning. To achieve near saturation of the genome with mutations resulting from mobilisation of the P-lacW transposon (a P-element marked with a mini-white gene, bearing the *E. coli lacZ* gene as an enhancer trap, and an *E.coli* replicon and ampicillin resistance gene to facilitate 'plasmid rescue' of sequences at the site of the P-insertion), *Drosophila* females that are homozygous for *P-lacW* (inserted on the second chromosome) are crossed with males carrying the transposase source P(Δ2-3) (Deak et al., 1997). Random transpositions of the mutator element are then 'captured' in lines lacking transposase activity. Stable, or balanced, stocks bearing single lethal *P-lacW* insertions are made to give a collection of 501 lines (Peter et al., submitted) and a further 73 lines that are either sterile or carry a mutation giving a visible morphological phenotype.

### Screening for Mitotic and Meiotic Defects

About half of the mutants in the collection are embryonic lethals.

Screens for mutants affecting spermatogenesis within this collection of 501 recessive lethal, semi-lethal and sterile mutants were carried out.

We have carried out cytological screens of the lines that comprise late larval lethals, pupal lethals, pharate and adult semi-lethals and steriles for defective mitosis in the developing larval CNS. This has identified 20 complementation groups that affect all stages of the mitotic cycle. The cytological screens involve examining orcein-stained squashed preparations of the larval CNS to detect abnormal mitotic cells. In lines where defects are identified, the larval CNS is subjected to immunostaining to identify centromeres, spindle microtubules and DNA for further examination. This leads to clarification of the mitotic defect.

As a set of common functions are essential to both mitosis and meiosis, we then identify mutations resulting in sterility and failed progression through male meiosis. This involves examining squashed preparations larval, pupal or adult testes by phase contrast microscopy. We examine "onion stage" spermatids in the 24 pupal and pharate lethal lines and adult "semi-lethal" and viable lines for variations in size and number of nuclei which provides an indication of whether there have been defects in either chromosome segregation or cytokinesis, respectively. A total of 8 lines show such defects.

Further phenotype information for each mutant described in the results section, as observed by phase contrast microscopy of dividing meiocytes, is provided in the "Phenotype" field.

We then examined the ovaries and eggs of females that when homozygous are either sterile or produce embryos that fail to develop. Dissected ovaries are examined by microscopy for defects in the mitotic divisions that lead to the formation of the 16 cell egg chambers, for defects in the endoreduplication of 15 nurse cell nucleic; for cytoskeletal defects in the development of the egg chamber; for defects in meiosis; and for mitotic defects in embryos derived from mutant mothers.

We examined 24 lines that show female sterility or maternal effect lethality when homozygous and identify 5 that display defects of the type described above. In the Examples 1 to 29 below, lines exhibiting mitotic and meiotic phenotypes are categorised generally into three categories:
Category 1 : Female Sterile
Category 2 : Male Sterile
Category 3: Mitotic (Neuroblast) Phenotypes
Category 1 phenotypes are exhibited by mutations in Examples 1, 2, 2A, 2B and 2C; while Category 2 phenotypes are exhibited by mutations in Examples 3 to 9 and 9A. Category 3 phenotypes are exhibited by mutations in Examples 10 to 29.

### Plasmid Rescue of P-Elements from Mutant Drosophila Lines

Genomic DNA was isolated from adult flies by the method of Jowett et al., 1986. Inverse PCR is used to identify flanking chromosomal sequences. The position of the inserted P-element is indicated in the Examples.

### Sequence Analysis of P Element Insertion Lines

The open reading frame(s) (ORF(s)) immediately adjacent to the insertion site are identified from the annotated total genome sequence of *Drosophila* with reference to the 'GADFLY' section of the 'FLYBASE' *Drosophila* genome database (database of the Berkeley *Drosophila* Genome Project). The site of P element insertion and the GenBank accession number of the genomic file which contains the insertion site are included in the results section.

Where the insertion site was within a gene or close to the 5' end of a gene, disruption of this gene is likely to be responsible for the phenotype, and it is included in the results section under the field heading "Annotated *Drosophila* Genome Complete Genome Candidate", as both an accession number and an amino acid sequence. Where the insertion site indicates that the P-element may be affecting expression of two diverging genes (on opposite strands of the DNA) both are included in the results section.

The *Drosophila* gene sequence is then used to identify a human homologue. Data on homologues is derived from the Blink ("BLAST Link") facility provided by the NCBI (National Center for Biotechnology Information) database. Where homologues are not apparent, further searches are made against the NCBI database using BLASTX (which compares the nucleotide query sequence virtually translated in all 6 frames against an amino acid database) or TBLASTN (amino acid query sequence against a nucleotide database virtually translated in all 6 frames) or TBLASTX (nucleotide query sequence against nucleotide database, both virtually translated in all 6 frames). Human homologues are included in the results section under the heading "Human Homologue of Complete Genome Candidate", as both an accession number and an amino acid.

### Additional Sequence Analysis using the Annotated D. melanogaster Sequence (GadFly)

As indicated above, rescue sequences are also used to search the fully annotated version of the Drosophila genome (GadFly; Adams, et al., 2000, Science 287, 2185-2195), using GlyBLAST at the Berkeley Drosophila Genome Projects web site (http://www.fruitfly.org/annot/) to identify the genome segment (usually approximately 200-250 kb) containing the P-element insertion site. The graphic representation of the genomic fragment available at GadFly allows the identification of all real and theoretical genes which flank the site of insertion. Candidate genes where the P-element is either inserted within the gene or close to the 5' end of the gene are identified. In GadFly, the *Drosophila* genes are given the designation CG (Complete gene) and usually details of human homologues are also given. Such human sequences may also be obtained using the fly sequences to screen databases using the BLAST series of programs. They may also be found by nucleic acid hybridisation techniques. In both cases homologies are defined using the parameters taught earlier in this patent. In most cases, this data confirms the data derived from the sequence analysis procedure described above, and in some cases new data is obtained. Where available both sets of data are included in the individual Examples described below.

### Confirmation of Cell Cycle Involvement of Candidate Genes Using Double

### Stranded RNA Interference (RNAi)

P-elements usually insert into the region 5' to a *Drosophila* gene. This means that there is sometimes more than one candidate gene affected, as the P-element can insert into the 5' regions of two diverging genes (one on each DNA strand). In order to confirm which of the candidate genes is responsible for the cell cycle phenotype observed in the fly line, we use the technique of double stranded RNA interference to specifically knock out gene expression in *Drosophila* cells in tissue culture (Clemens, et al., 2000, *Proc. Natl. Acad Sci. USA*, 6499-6503). The overall strategy is to prepare double stranded RNA (dsRNA) specific to each gene of interest and to transfect this into Schneider's *Drosophila* line 2 (Dmel-2) to inhibit the expression of the particular gene. The dsRNA is prepared from a double stranded, gene specific PCR product with a T7 RNA polymerase binding site at each end. The PCR primers consist of 25-30 bases of gene specific sequence fused to a T7 polymerase binding site (TAATACGACTCACTATAGGGACA), and are designed to amplify a DNA fragment of around 500bp. Although this is the optimal size, the sequences in fact range from 450 bp to 650 bp. Where possible, PCR amplification is performed using genomic DNA purified from Schneider's *Drosophila* line 2 (Dmel-2) as a template. This is only feasible where the gene has an exon of 450 bp or more. In instances where the gene possesses only short exons of less than 450 bp, primers are designed in different exons and PCR amplification is performed using cDNA derived from Schneider's *Drosophila* line 2 (Dmel-2) as a template.

A sample of PCR product is analysed by horizontal gel electrophoresis and the DNA purified using a Qiagen QiaQuick PCR purification kit. 1µg of DNA is used as the template in the preparation of gene specific single stranded RNA using the Ambion T7 Megascript kit. Single stranded RNA is produced from both strands of the template and is purified and immediately annealed by heating to 90 degrees C for 15 mins followed by gradual cooling to room temperature overnight. A sample of the dsRNA is analysed by horizontal gel electrophoresis.

3µg of dsRNA is transfected into Schneider's *Drosophila* line 2 (Dmel-2) using the transfection agent, Transfect (Gibco) and the cells incubated for 72 hours prior to fixation. The DNA content of the cells is analysed by staining with propidium iodide and standard FACS analysis for DNA content. The cells in G1 and G2/S phases of the cell cycle are visualised as two separate population peaks in normal cycling S2 cells. In each experiment, Red Fluorescent Protein dsRNA is used as a negative control.

### Preparation of dsRNA

RNA is prepared using an Ambion T7 Megascript kit in the following reaction: µl 10x T7 reaction buffer, 2 µl 75 mM ATP, 2 µl 75 mM GTP, 2 µl 75 mM UTP, 2 µl 75 mM CTP, 2 µl T7 RNA polymerase enzyme mix, 8 µl purified PCR product

Incubate at 37oC for 6 hours. For convenience this can be done overnight in a PCR machine, such that the reaction is due to finish the next day e.g. 10 hrs 4°C, 6 hrs 37°c, 4°C ∞ (prog. LISA6)

To degrade the DNA, add 1 ml DNase I (2U/ml) and incubate at 37°C for 15 mins.

Add 115 µl DEPC-treated water and 15 µl ammonium acetate stop solution (5M ammonium acetate, 100 mM EDTA)

Extract with an equal volume of phenol/chloroform, an equal volume of chloroform and then precipitate the RNA by adding 1 volume of isopropanol. Chill at - 20°C for 15-30 mins, then spin at top speed in a microfuge at 4°C. Remove the supernatant avoiding the RNA pellet, which appears as a clear, jelly-like pellet at the base of the tube. Dry briefly then dissolve the RNA in 20-100 µl DEPC-treated water, depending on the size of the pellet.

At this stage there are 2 complimentary single stranded RNAs. To anneal these, incubate the tube at 90°C for 10 mins, then cool slowly, by transferring to a hot block at 37°C and then setting the thermostat to room temperature.

Once the hot block has reduced to room temperature, spin down the liquid to the bottom of the tube and run 1 µl on a 1% agarose TBE horizontal gel to check the RNA yield and size.

### Transfection of Schneider line 2 (Dmel-2) cells with dsRNA (adherent protocol)

Transfect 3 µg dsRNA into Schneider line 2 (Dmel-2) cells using Promega Transfast transfectjon reagent.

Schneider line 2 (Dmel-2) cells are grown in Schneider's medium + 10% FCS + penicillin/Streptomycin, at 25°C. For the purpose of transfection with dsRNA, 25ml of a healthy growing culture should be sufficient for 24-30 transfections. Knock off cells adhering to the bottom of the flask by banging it sharply against the side of the bench, then aliquot 1ml into each well of 5 six-well plates. Add an additional 2 ml Schneider's medium + 10% FCS + penicillin/Streptomycin to each well and incubate the plates overnight in a humid chamber at 25°C.

Vortex the Transfast, then add 9 µl to a sterile eppendorf containing the 3 µg dsRNA. Add 1 ml Schneider's medium (no additives), vortex immediately and incubate at room temperature for 15 mins. In the mean time, carefully remove the Schneider's medium from the six-well plates and replace with Schneider's medium (no additives); ~1 ml / well.

Once the dsRNA+ Transfast has finished its 15 min incubation, remove the medium from the cells in the six-well plates, replace with the 1 ml dsRNA/Transfast/Schneider's medium and incubate at 25°C for 1 hr in a humid chamber.

Add 2 ml Schneider's medium containing 10%FCS + pen/strep and return to humid chamber in 25°C incubator for 24-72 hrs.

Initially, observations of the affects of dsRNA transfection on the Schneider line 2 cell cycle are made after 72 hrs incubation, but where a significant phenotype is observed, additional transfections are performed and observations made at earlier time points.

For each experiment, transfection with RFP dsRNA is used as a negative control. Cells which have been treated with transfast, but which have not been transfected with dsRNA are also included as a control. Transfection with polo or orbit dsRNA, shown in preliminary studies to have an observable affect on Schneider line 2 cell cycle, is used as a positive control in each experiment.

### Immunostaining of DMEL-2 cells for microscopic analysis

- For microscopic analysis of DMEL-2 insect cell line, ~4x10⁶ cells (0.5x10⁶ cells for 3 day incubations) are grown on coverslips in the bottom of the wells of six-well plates
- Following any required treatments, the media is carefully removed and replaced with 1 ml PHEMgSO₄ fixation buffer (60 mM PIPES, 25 mM Hepes, 10 mM EGTA, 4 mM MgSO₄, pH to 6.8 with KOH) + 3.7% formaldehyde. Until the cells are fixed they do not adhere strongly to the coverslip, so it is important to pipette gently at this stage.
- The cells are left to fix for 20 mins, then the buffer replaced with PBS + 0.1% Triton X-100 for 2 mins to permeablise the cells.
- Cells are then blocked using PBS + 0.1% Triton X-100 + 1% BSA (freshly prepared) and incubated for 1 hr at RT.
- Next cells are incubated with the primary rat α-tubulin antibody YL1/2 (1:300 dil.) (+ any other primary antibodies to be used, ex: gamma-tub at 1/500) in PBS + 0.1% Triton X-100 + 1% BSA 2-3 hrs at RT or alternatively overnight at 4°C.
- Wash the cells 3 times for 5 mins in PBS + 0.1% Triton X-100 and then incubate with the secondary antibody, TRTTC-donkey anti-rat (1:500 dil.) (+ any other secondary antibodies to be used) in PBS + 0.1% Triton X-100 + 1% BSA, at room temperature for 1 hr.
- Wash the cells 3 times for 5 mins in PBS + 0.1 % Triton X-100 and once in PBS alone, then mount on a slide on a drop of N-propyl gallate mounting medium containing DAPI to stain the DNA and seal with nail varnish
- View using fluorescent microscopy.

Primary antibodies: anti α-tub, 1:300 (rat YL1/2; SEROTEC); anti γ-tub, 1:500 (mouse; Sigma GTU-88)

Secondary antibodies: TRITC donkey anti-rat IgG at 1:300 (Jackson Immunoresearch, 712-026-150); AlexaFluor 488 goat anti-mouse, 1:300 (Molecular Probes; A-11001)

Transfections of S2 cells were carried out in 6 well tissue culture plates using 3 µg ds RNA per gene. The cells were harvested following three days for immunostaining.

### Microscope observations and cellular phenotyping

All studies were performed using a standard operating procedure. For every gene, each phenotypic test was performed following a 48 hours period of RNAi induction in duplicate and in two independent sets of experiments. The observations were carried out using a Zeiss Axioskop 2 motorized microscope with a 63X/1.4 plan-apochromat Zeiss objective.

Cells were fixed and stained with DAPI, alpha-tubulin and gamma-tubulin to visualise the nucleus/DNA, the microtubule network/spindle and the centrosomes respectively (see immunostaining section).

For each experiment, the number of normal looking mitotic cells in prophase/prometaphase, metaphase, anaphase and telophase is quantified as well as the abnormal looking ones in those various stages. These comprise abnormal chromosome number in prometaphase, misaligned chromosomes and lagging chromosomes in metaphase and anaphase respectively. Also, the abnormalities in the spindle morphology and the number of centrosomes are carefully noted. To get a more complete characterisation of the phenotype, the cell viability (cell confluency and number of apoptotic cells) is also assessed as well as the number of multinucleated interphase cells and the nucleus and cell morphology if different from control. If a phenotype appears to be more representative some images were stored for presentation of data.

### FACS analysis of transfected Schneider line 2 cells

Following transfection and incubation for the desired length of time, then transfer the cells to a 15 ml centrifuge tube and pellet by spinning at 2000rpm for 5 mins. Remove the supernatant, resuspend the cell pellet in 1 ml PBS and pellet a second time by spinning at 2000rpm for 5 mins. Remove 900 µl of the PBS, resuspend the cells in the remaining PBS and then add 900 µl ethanol drop-wise while vortexing the tube. Transfer the cells to an eppendorf tube and store at -20°C.

On the day of analysis, pellet the cells by spinning in a microfuge for 5 mins at 2000rpm, remove the supernatant, resuspend the cells in the residual ethanol and add 500 µl PBS. To remove clumps take the cells up through a 25 gauge needle and transfer to FACS tube. Add 3 µl 6 mg/ml Rnase A (Pharmacia) and 2.5 µl 25 mg/ml propidium iodide and incubate at 37°c for 30 mins, then store on ice.

Analyse DNA content of the Schneider line 2 cells using FACSCalibur at Babraham Institute. Mutant phenotypes are determined by comparing profiles relative to cells transfected with RFP dsRNA.

### Cellomics Mitotic Index HitKit procedure

- To Packard Viewplates containing pre-aliquoted dsRNA samples (1000ng/well) add 35 µl of logarithmically growing D.Mel-2 cells diluted to 2.3x10⁵ cells/ml in fresh *Drosophila*-SFM/glutamine/Pen-Strep pre-warmed to 28°C.
- Incubate the cells with the dsRNA (60nM) in a humid chamber at 28°C for 1 hr.
- Add 100 µl *Drosophila*-SFM/glutamine/Pen-Strep pre-warmed to 28°C and return the cells containing the dsRNA to the humid chamber at 28°C for 72 hrs.
- Gently remove the medium and slowly add 100 µl Fixation Solution (3.7% formaldehyde, 1.33mM CaCl₂, 2.69mM KCI, 1.47mM KH₂PO₄, 0.52mM MgCl₂-6H₂O, 137mM NaCl, 8.50mM Na₂HPO₄-7H₂O) pre-warmed to 28°C. Incubate in the fume hood for 15 minutes. It is imperative to use care when manipulating cells before and during fixation.
- Remove the Fixation Solution and wash with 100 µl Wash Buffer (1.33mM CaCl₂, 2.69mM KCI, 1.47mM KH₂PO₄, 0.52mM MgCl₂-6H₂O, 137mM NaCl, 8.50mM Na₂HPO₄-7H₂O).
- Remove the Wash buffer, add 100 µl Permeabilisation Buffer (30.8mM NaCl, 0.31mM KH₂PO₄, 0.57mM Na₂HPO₄-7H₂O, 0.02% Triton X-100), and incubate for 15 minutes.
- Remove the Permeabilisation Buffer and wash with 100 µl Wash Buffer.
- Remove the Wash Buffer and add 50 µl of Staining Solution (1 µg/ml Hoechst 33258, 1.33mM CaCl₂, 2.69mM KCI, 1.47mM KH₂PO₄, 0.52mM MgCl₂-6H₂0, 137mM NaCl, 8.50mM Na₂HPO₄-7H₂O) per well. Incubate for 1 hour protected from the light.
- Remove the Staining Solution and wash twice with 100 µl Wash Buffer.
- Remove the Wash Buffer and replace with 200 µL Wash Buffer containing 0.02% sodium azide.
- Seal the plates and analyse the transfection efficiency using the ArrayScan HCS System, running the Application protocol Percent_Transfection_200602_10x_p2.0 with the 10x objective and the QuadBGRFR filter set.

**Table 6**

| Results of Facs, Mitotic Index, and Cell phenotype assays after siRNA gene knockdown in Dmel-2 cells | | | | | | | | |
|---|---|---|---|---|---|---|---|---|
| **Example number** | **Fly Line** | **Drosophila gene** | **RNA ID** | **RNAi primers** | **RNAi phenotype** | | | **Human homologue** |
| | | | | | **Facs** | **Mitotic Index (% of RFP control)** | **Microscopy** | |
| 1 | 464 | CG15319 | 452 | TAATACGACTCACTATAGGGAGAACGGCACTTCTTTTTCTTGTCACCT | Fewer G1 cells, with corresponding increase in G2/M | wt | wt | AAC51331 - CREB-binding protein |
| | | | 453 | TAATACGACTCACTATAGGGAGAATGATGAGCAGCTCCAGCAGTCTCT | | | | |
| 2 | 492 | CG2028 | 458 | TAATACGACTCACTATAGGGAGAGAAGCGGATCGTTTGGCGACATTTA | Fewer cells in G2/M, with a corresponding increase in sub-G1 events | | 20% increase in chromosomal defects Some bright spots scattered in the cytoplasm in the DAPI channel, most of the nuclei are irregularly shaped, MI decreases, and DNA appears hypocondensed Shape of the cells is also very affected. | P48729 Casein kinase I, alpha isoform |
| | | | 459 | TAATACGACTCACTATAGGGAGAAGATGGGCATTGATCGAGGCATAG | | | | |
| 2A | ccr-a2 | CG3011 | 598 | TAATACGACTCACTATAGCGACATCGCAACGAGTACATCGACCGCATA | wt | 91% | 12% increase in chromosomal defects Multipolar and tripolar spindles | AAA63258 - serine hydroxymethyltransfer ase |
| | | | 599 | TAATACGACTCACTATAGGGAGATACCTTGTCTCCATTGGCCTTGGTG | | | | |
| 2B | ewv-b | CG2446 | 602 | TAATACGACTCACTATAGGGAGACCCCAAGGCGATAGATACCACGAT | wt | 74% | wt | none |
| | | | 603 | TAATACGACTCACTATAGGGAGAATCTCTGGTATGGCCATCAGGCACT | | | | |
| 2C | Fs(I)06 | CG15309 | 608 | TAATACGACTCACTATAGGGAGAGGTGAAGACGTTTCAGGCCTATCTA | wt | 111% | 20% increase in chromosomal defects spindle defects, some bipolar spindle | AAL09354 DiGeorge syndrome-related protein FKSG4 |
| | | | 609 | TAATACGACTCACTATAGGGAGATCCCAGCCGTTCTCCTTGATCATGT | | | | |
| 3 | 167 | CG15305 | 462 | TAATACGACTCACTATAGGGAGATATGTGCATCCATTCGAAAGACTTT | Very slightly fewer cycling cells & a corresponding increase in sub-G1 cells | wt | 20% increase in chromosomal defects Difficult to see a normal spindle | None |
| | | | 463 | TAATACGACTCACTATAGCGAGAATAGGGGAGGTTGTTCTTAGATTGA | | | | |
| 4 | 224 | CG2096 | 468 | TAATACGACTCACTATAGGGAGATGAAACCATCCGAGAAGAAGGCCAA | wt | wt | ⁵20% increase in chromosomal defects, no defects incentrosomes or spindle | NP_002700 protein phosphatase 1 |
| | | | 469 | TAATACGACTCACTATAGGGAGACAGATAATCATCAAATGCAGGAATC | | | | |
| | | CG2222 | 464 | TAATACGACTCACTATAGGGAGAACGGAATGAACTATTTTCCGAACTATTACT | wt | Not done | 40 % increase in chromosomal defects Multipolar and monopolar spindles Many polyploid cells Some hypercondensed chromosomes | NP_073607 hypothetical protein FLJ13912 |
| | | | 465 | TAATACCACTCACTATAGGGAGAGATGTACTGACTGTTGGTGCGCACT | | | | |
| 5 | 231 | CG2941 | 470 | TAATACGACTCACTATAGGCACAATCTGTAGACAGACGGCAGAATTGC | Fewer cells in G2/M, with a corresponding increase in sub-G1 events | wt | wt | None |
| | | | 471 | TAATACGACTCACTATAGGGAGACGCAATAGCAGTACTTCCATCTTGT | | | | |
| | | CG2938 | 474 | TAATACGACTCACTATAGGGAGAATTGGATTGCGAATCGCTCAGGATC | wt | wt | 10% increase in chromosomal defects Fewer cells indicating cell death Multipolar spindles | NP_075051 Cas1 O-acetyltransferase |
| | | | 475 | TAATACGACTCACTATAGGGAGATTTTCGCGAAGGACATCAATATCAG | | | | |
| 6 | 248 | CG6998 | 476 | TAATACGACTCACTATAGGGAGAGGCCTACATCAAGAAGGAGTTCGAC | Very slightly fewer cells in G2/M & a corresponding increase in sub-G1 cells | wt | wt | AAH10744 Similar to RIKEN cDNA 6720463E02 gene |
| | | | 477 | TAATACGACTCACTATAGGGAGATGGTTAGTTGTATTTGCGAATCTTC | | | | |
| 8 | ms(l)04 | CG1524 | 482 | TAATACGACTCACTATAGGGAGAGTTGCTGATCGACAAACAAACCCAG | Fewer G2/M events, with a corresponding increase in sub-G1 events and a different G1 profile | 63% | Only 38 mitotic cells remained on the slide, cells are very scattered and some are dying. Nuclei are degraded | A25220 ribosomal protein S14 |
| | | | 483 | TAATACGACTCACTATAGGGAGACTTTCCAGATACTGCCATCTACAGA | | | | |
| | | CG10778 | 484 | TAATACGACTCACTATAGGGAGAGAGTGTCGCGTGTAGAGGCATTCTT | wt | 78% | 20% increase in chromosomal defects High number of multipolar spindles | hypothetical protein FCJ13102 (54%)Similarity to Mouse kinesin-like protein KIF4 |
| | | | 485 | TAATACGACTCACTATAGGGAGAAAGTACACATGGACGGAGCGGATAG | | | | |
| 9 | thb-a | CG1453 | 556 | TAATACGACTCACTATAGGGAGAGGTGCCGTTTTTCCTTTTTGTATCC | Slight increase in G1 and sub-G1 cells, but no obvious corresponding decrease in S or G2/M cells | wt | wt | (CG1453) -CAA69621 - kinesin-2 |
| | | | 557 | TAATACGACTCACTATAGGGAGATGATCCTTCCTCTTTGACTCCACCTGTT | | | | |
| | | CG18292 | 558 | TAATACGACTCACTATAGGGAGACGCTAAAAACTAGTAGTTTTGTGTGCCAGG | wt | 91% | 20% increase in chromosomal defects Possible decrease in mitotic index Some multipolar spindles, few normal looking spindles | BAA22937 - cdk2-associated protein 1; cdk2ap1, deleted in oral cancer I |
| | | | 559 | TAATACGACTCACTATAGGGAGAACCACCATTGCTGGAGCACATGTTG | | | | |
| 9A | ms(1)13 | CG5941 | 610 | TAATACGACTCACTATAGGGAGAGGATTAGCACCGTCGACCACGAAAA | Very slight decrease in G1 peak, but no other obvious variation from wt profile | wt | wt | MCT-1(multiple copies in a T-cell malignancies) (BAA86055), |
| | | | 611 | TAATACGACTCACTATAGGGAGAAATTTCCTGTGTGGATAACGTGAGGAGTCC | | | | |
| 10 | 187 | CG10701 | 490 | TAATACGACTCACTATAGGGAGACGTTCCTGCTGTTTGGCATTCTTCT | Fewer G2/M events with a corresponding increase in sub- G1 events | wt | 20% increase in chromosomal defects, misaligned chromosome (40%), spindle with free extracentrosome, cells with more than one spindle. | A41289 human moesin |
| | | | 491 | TAATACGACTCACTATAGGGAGAACCACAATAAGACCACCCACACAGC | | | | |
| | | CG10648 | 488 | TAATACGACTCACTATAGGGAGACACCITCTGCCGCCATGAGTACAAT | wt | wt | Proportion of mitotic chromosomal defects a bit lower than normal, high proportion of monopolar spindles and small spindles. Very high proportion of prometaphase cells Cell death | NP_115898 Mak16-like RNA binding protein |
| | | | 489 | TAATACGACTCACTATAGGGAGATTCCGCCTCCAGAGCCTTGTTGAAA | | | | |
| 11 | 226 | CG2865 | 492 | TAATACGACTCACTATAGGGAGATCAAGGCGTCCATGATCACCTCGAAAT | Fewer cells in G2/M and also S. Increased percentage of cells in sub-G1 and G1 | wt | wt | none |
| | | | 493 | TAATACGACTCACTATAGGGAGAACCTGTCCAGCTGCAACTTGGTCAA | | | | |
| | | CG2854 | 494 | TAATACGACTCACTATAGGGAGAGGAGATGGAAAAGGAGCTCGGAAAA | wt | wt | 17% increase in chromosomal defects Higher level of polyploid, prometaphase cells and misaligned chromosomes, anaphase normal | CAD38627 hypothetical protein |
| | | | 495 | TAATACGACTCACTATAGGGAGATCTCAATCCGTATGCCAAGGAGCAC | | | | |
| | | CG2845 | 496 | TAATACGACTCACTATAGGGAGAAGTTGACCTCCAAGCTCCACGAACT | wt | wt | More than 20% increase in chromosomal defects More multipolar spindles | AAA35609. B-raf protein |
| | | | 497 | TAATACGACTCACTATAGGGAGACTGGTGCTTGATGTGTGTCCTAATG | | | | |
| 12 | 269 | CG1696 | 500 | TAATACGACTCACTATAGGGAGACACTTGGCGATTGAACATGAAACAA | Fewer cells in G2/M and also S. Increased percentage of cells in sub-G1 and G1 | wt | wt | NP_056158 hypothetical protein |
| | | | 501 | TAATACGACTCACTATAGGGAGAATATAAAAAGCCCCCAAAAGAATTG | | | | |
| | | CG1486 | 502 | TAATACGACTCACTATAGGGAGAATTGCACTTTGATTGCAGTCGATTGCG | wt | wt | 10% increase in chromosomal defects More prometaphase cells | BAA19780 Similar to a C.elegans protein in cosmid C14H10 |
| | | | 503 | TAATACGACTCACTATAGGGAGAGATGTGGAATGGTGTGACCGTAGTG | | | | |
| 13 | 291 | CG10798 | 504 | TAATACGACTCACTATAGGGAGAGACAGGCATATAACTCAGGAACTTA | Fewer cells in G2/M. | wt | wt | CAA23831 c-myc |
| | | | 505 | TAATACGACTCACTATAGGGAGACTTGATGATCACCGGCATGTTCTCG | Increased percentage of cells in sub- G1 and G1 | | | oncogene |
| 15 | 379 | CG10964 | 552 | TAATACGACTCACTATAGGGAGACGGAGTGCCGTCGTAGTTGACAAAA | wt | wt | 15% increase in chromosomal defects high number of disorganised spindles | AAC50725 11-cis retinol dehydrogenase |
| | | | 553 | TAATACGACTCACTATAGGGAGATGACCAAGGACCAAGGCCTCAATGT | | | | |
| | | CG2151 | 554 | TAATACGACTCACTATAGGGAGAAGCCCACTGTGATGGTGCGTTCTAT | wt | 81% | 20%increase in chromosomal defects High proportion of polyploid cells | XP_033135 thioredoxin reductase beta |
| | | | 555 | TAATACGACTCACTATAGGGAGAATCTCATCGGCTCCGAACTGCTTGA | | | | |
| 17 | 121 | CG10988 | 560 | TAATACGACTCACTAGGGAGACATTTAAGCAAAATGATTGCCGCCAATAGT | wt | wt | 22% increase of chromosomal defects Main feature is a high proportion of metaphase figures with misaligned chromosomes (75% vs 20% in normal cells) Some cells without any centrosomes | AAC39727 - spindle pole body protein spc98 homolog GCP3 |
| | | | 561 | TAATACGACTCACTATAGGGAGATCTCAATCCGATGCTGGACTGTGTG | | | | |
| 18 | 237 | CG1558 | 562 | TAATACGACTCACTATAGGGAGAGCCCAGAAGGAGCAGCAAAAGTTCT | wt | 117% | 18% increase in chromosomal defects Abnormal spindle structures (increased number of centrosomes) | none |
| | | | 563 | TAATACGACTCACTATAGCGAGATAAGTTACCTGCATCGAGGCATTGT | | | | |
| | | CG11697 | 564 | TAATACGACTCACTATAGGGAGAATGATTTATGCGATCGTGATACACA | Fewer G2/M events, with a corresponding increase in sub-G1 events. Also a different G1 profile from wt. | wt | 18%increase in chromosomal defects More polyploid cells | BAB 14444 unamed protein-similar to a hypothetical protein in the region deleted in human familial adenomatous polyposis I |
| | | | 565 | TAATACGACTCACTATAGGGAGACCGCTTCTCTTCCAACTGCCTTTTG | | | | |
| 19 | 171 | CG3954 | 566 | TAATACGACTCACTATAGGGAGAGGAGCCGAGTACATCAATGCCAACT | Very slight increase in G1 and sub-G1 cells, but no obvious corresponding decrease in S or G2/M cells | 45% | 20% increase in chromosomal defects Spindle and centrosome seem normal. Higher level of aneuploidy and polyploidy | AAH08692 - protein tyrosine phosphatase, non-receptor type 11 |
| | | | 567 | TAATACGACTCACTATAGGGAGAATGTAGGTCTTAAACATCTCGCGCT | | | | |
| | | CG16903 | 568 | TAATACGACTCACTATAGGGAGAGGAAATCTCGCCCATGGTGCTAGAT | wt | wt | 20% increase in chromosomal defects Clear decrease in mitotic index. A lot of spindles seem to be affected in their structure, poles not well defined and microtubule array irregular Many cells with fused interphase or decondensed nuclei | AAD53184 - cyclin L ania-6a |
| | | | 569 | TAATACGACTCACTATAGGGAGATGTTCCGATCCACGGTGATTACAGC | | | | |
| 20 | 500 | CG4399 | 570 | TAATACGACTCACTATAGGGAGATGCCCCCCTGGATGATAATGCCAAT | Fewer cells in G2/M, with a corresponding increase in sub-G1 events. Also a different G1 profile from wt. | 88% | wt | AAF13722 -neurofilament protein |
| | | | 571 | TAATACGACTCACTATAGGGAGAACTTGCAGCTCGTGACTCTGATGCT | | | | |
| | | CG4406 | 572 | TAATACGACTCACTATAGGGAGAATGCTTGTTAAATTTGTTGTCATCTTTGCC | Slight decrease in G2/M and corresponding slight increase in sub-G1 cells. | wt | wt | XP_131206 similar to GPI-anchor transamidase |
| | | | 573 | TAATACGACTCACTATAGGGAGAATCTCCTCCGAGTCCTGGAACTTGA | | | | |
| 23 | 37 | CG16983 | 580 | TAATACGACTCACTATAGGGAGAATGCOCAGCATCAAGTTGCAATCTT | Significant decrease in sub-G1 & G1 peaks, with a corresponding increase in the G2/M peak, indicating mitotic arrest. | wt | 30% increase in chromosomal defects All types of spindle and chromosomal defects are visible but no obvious main one Higher proportion of aneuploid and polyploid cells Possible decrease in mitotic index Cells with excess centrosomes | XP_054159 -hypothetical protein |
| | | | 581 | TAATACGACTCACTATAGGGAGACGAAATGCCGCGCTTTACTTCTCCT | | | | |
| | | CG13363 | 582 | TAATACGACTCACTATAGGGAGATCCGATACCTGCGCGTCTTTGACAA | wt | wt | 40% increase in chromosomal defects A lot of polyploid cells, multicentrosome but some normal spindle also | NP_057112 CGI-85 protein |
| | | | 583 | TAATACGACTCACTATAGGGAGAGCCATTATTACCAGGTCCACTGCTG | | | | |
| 24 | 186 | CG18319 | 584 | TAATACGACTCACTATAGGGAGACTCAACGAGAAGGTCCAGACTCAAC | Significant decrease in sub-G1 & G1 peaks, but no corresponding increase in the G2/M peak. Probably indicates mitotic arrest. | 91% | 30% increase in chromosomal defects Various chromosomal defects ranging from number of centrosomes, spindle structure and stretched/lagging chromatids. High number of abnormal anaphases 75% ofanaphases (compared to 10-15 % in normal cells) | BAA11675 -ubiquitin-conjugating enzyme E2 UbcH-bcn |
| | | | 585 | TAATACGACTCACTATAGGGAGATCGACGGCATATTTCTGGGTCCACT | | | | |
| 25 | 301 | CG14813 | 586 | TAATACGACTCACTATAGGGAGAAATGTGCAGCCTTCGGTGGCGGAGTACGAC | Fewer G1 events, with an increased number of cells in G2/M indicating mitotic arrest | 81% | Cell death Lower proportion of chromosomal defects | CAA57071 - archain |
| | | | 587 | TAATACGACTCACTATAGGGAGACAATTACTCGCTCTGAGAAGCTGTC | | | | |
| 26 | 148 | CG8655 | 590 | TAATACGACTCACTATAGGGAGAATGCCCTTCATGGCACATGACCGAT | very slight decrease in G1 and G2/M peaks, but no significant increase in sub-G1 cells or polypoid cells. | wt | 40% increase in chromosomal defects Some chromosomal defects in spindle structure but no clear -single phenotype | AAB97512 - HsCdc7 |
| | | | 591 | TAATACGACTCACTATAGGGAGATTGCTGCTCTTGCTGCTAGCTGT | | | | |
| 27 | 335 | CG2621 | 594 | TAATACGACTCACTATAGGGAGAAATAATAATAACAACUTTATAAGCCAGCCG | wt | wt | 20% increase in chromosomal defects Many obvious mitotic chromosomal defects and too many centrosomes per cell Very difficult to find a normal looking mitotic spindle Most of the anaphases are abnormal with lagging chromosomes | NP_002084 - glycogen synthase kinase 3 beta |
| | | | 595 | TAATACGACTCACTATAGGGAGATAATGCGGCTGCGCAAGATGCTGTT | | | | |
| 28 | 342 | CG1725 | 528 | TAATACGACTCACTATAGGGAGAGCCACGTTGAAATCGATCACCGACA | Essentially wt profile. Very slight reduction in G1 peak, but no obvious corresponding increase in other peaks | | No increase in chromosomal defects but many with more than two centrosomes | XP_012060 - discs. large (Drosophila) homolog 2 |
| | | CT4934 | 529 | TAATACGACTCACTATAGGGAGAATAGAAGGAGTTGGCGGGTGGAGAT | | | | |
| | | CT41310 | 530 | TAATACGACTCACTATAGGGAGATCTCTTTCGATTTCTTCTCTTCTGT | | | | |
| | | | 531 | TAATACGACTCACTATAGGGAGATTGATGAACACGGCGACGGGATACA | | | | |
| | | CG1594 | 532 | TAATACGACTCACTATAGGGAGAAGGGAATCGTGTGGAAAGACTCGCA | Very slight reduction in G1 peak, with a corresponding increase in sub-G1 cells. | wt | 20% increase in chromosomal defects Polyploid cells Abnormal number of centrosomes in many cells but some normal bipolar spindles | NP_004963 JAK-2 kinase (Janus kinase 2), involved in cytokine receptor signaling |
| | | | 533 | TAATACGACTCACTATAGGGAGAACAAGGACAAATCAACGGGACTGGC | | | | |
| 29 | 419 | CG12638 | 596 | TAATACGACTCACTATAGGGAGATGTTTGCCATATCATTGCAGCTGCT | Decrease in the number of cells in G2/M, with an increase in the sub-G1 population. The G1 peak differs in profile from wt. | 94% | wt | B38637 - Ras inhibitor (clone JC265) - human (fragment) |
| | | | 597 | TAATACGACTCACTATAGGGAGAGATGTCATATTGGCCAGGTCACTGG | | | | |
| | | | | | | | | |

| | | | | | | | | |
|---|---|---|---|---|---|---|---|---|
| ⁵ I only changed columns 6 and 8 - and have assumed that everything else (sequences etc) are unchanged | | | | | | | | |

### EXAMPLES SECTION B: P-ELEMENT SCREENING RESULTS

The layout of a typical entry in the results section is shown below. Not all fields present in the actual results section contain information for each individual *Drosophila* line described.

### Results Layout (Examples 1 to 29)

**Line ID**
   *(Drosophila* line designation)
**Phenotype**
   (Description of *Drosophila* phenotype)
**Annotated *Drosophila* genome genomic segment containing P element insertion site (and map position)**
   (Accession number, map position according to the Bridges map, Lefevre, 1976)
**P element Insertion site**
   (Base pair position within genomic segment)
**Annotated *Drosophila* Genome Complete Genome candidate**
   (derived from GADFLY Berkley Drosophila Genome Project database, accession number, mRNA sequence (complete CDS) and Peptide sequence)
**Human homologue of Complete Genome candidate**
   (Derived from Blink and BLAST searches, accession number, mRNA sequence (complete CDS) and peptide sequence)
**Putative function**
   (Derived from homologies or Drosophila experimental data)

A specific example is as follows (Example 5, Category 2):
**Line ID** - 231
**Phenotype** - Semi-lethal male and female, cytokinesis defect. In some cysts, variable sized Nebenkerns
**Annotated *Drosophila* genome genomic segment containing P element insertion site (and map position)** - AE003429 (3F)
**P element insertion site** - 153,730
**Annotated *Drosophila* genome Complete Genome candidate -** CG5014 - vap-33-1 vesicle associated membrane protein 
CACATCACTAGCTGACAGAATATATGGCTTTTTACATTTTGCGTTTTCA
ACTGAAGTTTGCGAAGAAACCGAAGCGTGGTAAACCACTGAAATCGAAAA
TATCGACAGAAAAGCGACCTAAAGTCGGTGAAGAAGTCGCACGTTGATCG
TTGTGTTTTTTCCCGAAATTTTCTGCAAAAAGCCCGTGCGTGCGTGAGT
TTCTCTGGCTCTTGCTTTTTTTTTGTCCATGCGTGTGTGTGTGGTCGCAT
AAATTTACCGATATTTCGCCTGTGAGAGCGAAACGAACGAAAAACGAAAG
AAAAAAAGAGAGACGAGTAAAGTAAAACGAAACAGGCATAAAAACAGCAG
CAGTTTTCTTGATATATTTGGCTAAAAAACGCAAACCAAACAGCCAGCAA
GAACAACAAATAGCTGGGCAAAAACAGGACGCACAAAAAATAAAATTAAA
ACGATAAGAGGCGAAAAGCGGAGAGAGTGAAATTCTCGGCAGCAACAACG
ACAAGAACAACACCAGGAGCAGCAGCAACAACAACAACAAAAGCCAGCCG
CCACAATGAGCAAATCACTCTTTGATCTTCCGTTGACCATTGAACCAGAA
CATGAGTTGCGTTTTGTGGGTCCCTTCACCCGACCCGTTGTCACAATCAT
GACTCTGCGCAACAACTCGGCTCTGCCTCTGGTCTTCAAGATCAAGACAA
CCGCCCCGAAACGCTACTGCGTACGTCCAAACATCGGCAAGATAATTCCC
TTTCGATCAACCCAGGTGGAGATCTGCCTTCAGCCATTCGTCTACGATCA
GCAGGAGAAGAACAAGCACAAGTTCATGGTGCAGAGCGTCCTGGCACCCA
TGGATGCTGATCTAAGCGATTTAAATAAATTGTGGAAGGATCTGGAGCCC
GAGCAGCTGATGGACGCCAAACTGAAGTGCGTTTTCGAGATGCCCACCGC
TGAGGCAAATGCTGAGAACACCAGCGGTGGTGGTGCCGTTGGCGGCGGAA
CCGGAGCTGCCGGAGGCGGAAGCGCGGGTGCCAATACTAGCTCAGCCAGC
GCTGAGGCGCTCGAGAGCAAGCCGAAGCTCTCCAGCGAGGATAAGTTTAA
GCCATCCAATTTGCTCGAAACGTCTGAGAGTCTGGACTTGCTGTCCGGAG
AGATCAAAGCGCTGCGTGAATGCAACATTGAATTGCGAAGAGAGAATCTT
CACTTGAAGGATCAAATCACACGTTTCCGGAGCTCGCCGGCCGTCAAACA
GGTGAATGAGCCCTATGCCCCAGTCCTGGCTGAGAAGCAGATTCCGGTCT
TTTACATTGCAGTTGCCATTGCTGCGGCCATCGTTAGCCTCCTGCTGGGC
AAATTCTTTCTCTGA
 
MSKSLFDLPLTIEPEHELRFVGPFTRPVVTIMTLRNNSALPLVFKIKTTA
PKRYCVRPNIGKIIPFRSTQVEICLQPFVYDQQEKNKHKFMVQSVLAPMD
ADLSDLNKLWKDLEPEQLMDAKLKCVFEMPTAEANAENTSGGGAVGGG
TGAAGGGSAGANTSSASAEALESKPKLSSEDKFKPSNLLETSESLDLLSGEI
KALRECNIELRRENLHLKDQITRFRSSPAVKQVNEPYAPVLAEKQIPVFY
IAVAIAAAIVSLLLGKFFL
**Human homologue of Complete Genome candidate**
   AAD13577 VAMP-associated protein B

1 gcgcgcccac ccggtagagg acccccgccc gtgccccgac cggtccccgc ctttttgtaa
61 aacttaaagc gggcgcagca ttaacgcttc ccgccccggt gacctctcag ggtctcccc
121 gccaaaggtg ctccgccgct aaggaacatg gcgaaggtgg agcaggtcct gagcctcgag
181 ccgcagcacg agctcaaatt ccgaggtccc ttcaccgatg ttgtcaccac caacctaaag
241 cttggcaacc cgacagaccg aaatgtgtgt tttaaggtga agactacagc accacgtagg
301 tactgtgtga ggcccaacag cggaatcatc gatgcagggg cctcaattaa tgtatctgtg
361 atgttacagc ctttcgatta tgatcccaat gagaaaagta aacacaagtt tatggttcag
421 tctatgtttg ctccaactga cacttcagat atggaagcag tatggaagga ggcaaaaccg
481 gaagacctta tggattcaaa acttagatgt gtgtttgaat tgccagcaga gaatgataaa
541 ccacatgatg tagaaataaa taaaattata tccacaactg catcaaagac agaaacacca
601 atagtgtcta agtctctgag ttcttctttg gatgacaccg aagttaagaa ggttatggaa
661 gaatgtaaga ggctgcaagg tgaagttcag aggctacggg aggagaacaa gcagttcaag
721 gaagaagatg gactgcggat gaggaagaca gtgcagagca acagccccat ttcagcatta

781 gccccaactg ggaaggaaga aggccttagc acccggctct tggctctggt ggttttgttc
841 tttatcgttg gtgtaattat tgggaagatt gccttgtaga ggtagcatgc acaggatggt
901 aaattggatt ggtggatcca ccatatcatg ggatttaaat ttatcataac catgtgtaaa
961 aagaaattaa tgtatgatga catctcacag gtcttgcctt taaattaccc ctccctgcac
1021 acacatacac agatacacac acacaaatat aatgtaacga tcttttagaa agttaaaaat
1081 gtatagtaac tgattgaggg ggaaaagaat gatctttatt aatgacaagg gaaaccatga
1141 gtaatgccac aatggcatat tgtaaatgtc attttaaaca ttggtaggcc ttggtacatg
1201 atgctggatt acctctctta aaatgacacc cttcctcgcc tgttggtgct ggcccttggg
1261 gagctggagc ccagcatgct ggggagtgcg gtcagctcca cacagtagtc cccacgtggc
1321 ccactcccgg cccaggctgc tttccgtgtc ttcagttctg tccaagccat cagctccttg
1381 ggactgatga acagagtcag aagcccaaag gaattgcact gtggcagcat cagacgtact
1441 cgtcataagt gagaggcgtg tgttgactga ttgacccagc gctttggaaa taaatggcag
1501 tgctttgttc acttaaaggg accaagctaa atttgtattg gttcatgtag tgaagtcaaa
1561 ctgttattca gagatgttta atgcatattt aacttattta atgtatttca tctcatgttt
1621 tcttattgtc acaagagtac agttaatgct gcgtgctgct gaactctgtt gggtgaactg
1681 gtattgctgc tggagggctg tgggctcctc tgtctctgga gagtctggtc atgtggaggt
1741 ggggtttatt gggatgctgg agaagagctg ccaggaagtg ttttttctgg gtcagtaaat
1801 aacaactgtc ataggcaggg aaattctcag tagtgacagt caactctagg ttaccttttt
1861 taatgaagag tagtcagtct tctagattgt tcttatacca cctctcaacc attactcaca
1921 cttccagcgc ccaggtccaa gtttgagcct gacctcccct tggggaccta gcctggagtc
1981 aggacaaatg gatcgggctg caaagggtta gaagcgaggg caccagcagt tgtgggtggg
2041 gagcaaggga agagagaaac tcttcagcga atccttctag tactagttga gagtttgact
2101 gtgaattaat tttatgccat aaaagaccaa cccagttctg tttgactatg tagcatcttg
2161 aaaagaaaaa ttataataaa gccccaaaat taaga

1 
61 
121 
181 
241 

### Putative function

Membrane associated protein which may be involved in priming synaptic vesicles

### Results Layout for Examples 2A, 2B, 2C and 9A

The results layout for Examples 2A, 2B, 2C and 9A includes, in place of the fourth field "P Element Insertion Site", a field "P Element Insertion Site Sequence". This field shows the actual sequence of the insertion site which is determined experimentally, as opposed to the base pair position within genomic segment present in the other Examples.

### CATEGORY 1 - FEMALE STERILE

### Example 1 (Category 1)

**Line ID** - 464
**Phenotype -** Female semi-sterile, brown eggs laid
**Annotated *Drosophila* genome genomic segment containing P element insertion site** (and map position) - AE003448 (8F)
**Pelement Insertion site -** 44,575
**Annotated *Drosophila* genome Complete Genome candidate -** CG15319 - nejire (CREB binding protein, p300/CBP) 
CTTAACCAAACAAACAACCTGTGCAACAATTGTCAAAGTGCTAGGCGACA
AATAATTTCTGAAAGAAGATTTGACAAGTTCCAATAACGAAAATATCAGA
ACACACTCGAACTCCAACATAGACGGATCATTGGAGAGTTAGTGAAAAAA
AAAAGCGAAAAATCAGAAAAACTTTATAAACTAATAGAAACAATACTACT
CAGATTTTTCGAACGTTTTTCGTCTGCGTTTCTGTTTTTTTCCGAATCGA
AAGAATCAAACTAACTCTATATGATGGCCGATCACTTAGACGAACCGCCC
CAAAAGCGGGTTAAAATGGATCCAACGGATATCTCTTACTTTCTGGAGGA
GAACCTGCCCGATGAGCTGGTGTCCTCGAATAGTGGCTGGTCGGATCAGC
TGACCGGCGGAGCAGGCGGTGGCAATGGAGGTGGCGGCGCCTCCGGTGTA
ACCACAAATCCCACATCCGGCCCAAATCCCGGTGGCGGACCCAACAAGCC
GGCAGCCCAAGGACCCGGCTCTGGCACAGGCGGAGTCGGTGTTGGAGTGA
ATGTGGGTGTCGGCGGTGTTGTTGGCGTCGGCGTTGTGCCTTCCCAGATG
AACGGAGCCGGCGGCGGCAACGGATCCGGAACGGGTGGCGACGACGGCAG
TGGCAACGGCTCAGGAGCGGGCAACAGAATCAGTCAAATGCAACACCAGC
AACTGCAGCACCTACTCCAGCAGCAGCAGCAGGGCCAGAAGGGCGCCATG
GTGGTGCCCGGCATGCAGCAGCTGGGCAGCAAGTCGCCCAACCTGCAGTC
ACCCAACCAGGGCGGCATGCAGCAGGTGGTGGGCACTCAGATGGGTATGG
TCAACTCAATGCCCATGTCAATATCGAATAATGGCAACAATGGCATGAAC
GCCATACCAGGCATGAACACCATTGCGCAGGGCAATCTGGGAAACATGGT
GCTGACCAACAGCGTTGGCGGCGGCATGGGCGGCATGGTTAATCATCTTA
AGCAGCAGCCTGGCGGCGGCGGCGGTGGGATGATCAATTCCGTTTCAGTA
CCCGGAGGACCTGGAGCAGGAGCTGGTGGCGTTGGAGCTGGCGGCGGAGG
AGCCGTTGCCGCAAACCAAGGCATGCATATGCAGAACGGCCCAATGATGG
GACGCATGGTGGGGCAACAGCATATGCTTCGTGGCCCGCATCTCATGGGT
GCCTCTGGAGGAGCTGGTGGGCCAGGAAACGGGCCTGGTGGCGGAGGACC
ACGCATGCAGAATCCGAACATGCAAATGACTCAACTCAACAGTCTGCCCT
ACGGAGTGGGTCAGTATGGTGGCCCAGGCGGTGGTAACAATCCTCAGCAA
CAGCAGCAGCAACAGCAGCAACAACTTCTCGCCCAGCAGATGGCCCAAAG
AGGTGGCGTCGTACCGGGCATGCCGCAGGGTAATCGGCCCGTTGGCACAG
TGGTGCCCATGTCCACACTCGGCGGCGATGGATCAGGGCCCGCGGGGCAG
CTGGTAAGCGGGAATCCTCAGCAGCAGCAGATGCTGGCGCAGCAGCAAAC
CGGAGCCATGGGCCCGCGTCCTCCGCAACCAAACCAGCTGCTCGGTCATC
CCGGCCAGCAGCAGCAGCAGCAACAGCAGCCTGGCACCTCGCAGCAGCAG
CAACAGCAGCAGGGAGTCGGAATCGGAGGAGCAGGCGTTGTGGCCAATGC
AGGAACCGTGGCTGGCGTGCCGGCAGTGGCAGGCGGCGGAGCCGGTGGTG
CCGTACAATCTAGCGGCCCTGGTGGCGCCAATCGCGATGTGCCCGACGAC
CGTAAGCGACAGATCCAGCAGCAACTGATGCTGCTCCTCCATGCACACAA
ATGCAATCGCAGGGAGAACCTGAATCCGAACAGGGAAGTGTGCAACGTTA
ACTACTGCAAGGCGATGAAATCCGTGCTGGCCCACATGGGCACTTGCAAA
CAGAGCAAGGACTGCACCATGCAGCATTGTGCCTCTTCGCGCCAAATTCT
 
GTTGCATTATAAAACGTGCCAGAACAGTGGCTGCGTCATTTGCTATCCCT
TCCGGCAGAATCATTCGGTTTTTCAAAATGCGAATGTGCCGCCAGGAGGC
GGACCGGCAGGAATTGGAGGTGCGCCACCAGGTGGCGGCGGAGCGGGTGG
TGGAGCGGCTGGAGCAGGCGGTAATCTTCAGCAGCAACAGCAGCAGCAAC
AACAGCAGCAGCAGAACCAGCAGCCCAATCTGACGGGTCTGGTAGTGGAT
GGCAAGCAAGGACAGCAGGTTGCACCGGGAGGTGGCCAAAATACTGCCAT
AGTTCTTCCCCAGCAACAGGGAGCGGGCGGTGCACCGGGTGCGCCGAAAA
CGCCTGCGGATATGGTGCAACAATTGACCCAACAGCAGCAGCAGCAGCAA
CAGCAGGTTCACCAGCAACAGGTTCAGCAACAGGAACTCCGTCGATTCGA
TGGCATGAGCCAGCAAGTCGTAGCAGGTGGTATGCAACAGCAGCAGCAGC
AGGGTTTGCCTCCTGTGATTCGCATTCAAGGCGCTCAGCCGGCCGTCAGG
GTACTGGGACCAGGTGGTCCCGGCGGCCCAAGTGGACCAAATGTTCTGCC
GAACGATGTTAACAGCCTGCATCAACAACAGCAACAAATGCTGCAACAGC
AGCAGCAACAGGGCCAGAATCGACGACGCGGTGGCCTGGCCACCATGGTG
GAGCAACAACAGCAGCATCAGCAACAACAGCAGCAACCCAATCCCGCCCA
GCTGGGTGGCAACATTCCAGCACCACTCTCTGTCAACGTCGGTGGCTTTG
GCAATACCAATTTCGGTGGTGCAGCTGCCGGCGGAGCCGTGGGAGCCAAC
GATAAGCAGCAACTGAAGGTGGCCCAAGTGCATCCGCAGAGCCATGGCGT
AGGAGCGGGCGGTGCATCAGCGGGCGCCGGGGCGAGTGGTGGTCAAGTGG
CAGCCGGTTCCAGTGTCCTGATGCCAGCCGATACCACGGGCAGTGGTAAT
GCGGGCAATCCCAACCAGAATGCAGGCGGTGTAGCTGGAGGTGCCGGCGG
TGGCAATGGCGGAAACACTGGACCTCCGGGCGACAACGAGAAAGACTGGC
GGGAATCGGTGACCGCCGATCTGCGCAACCACCTCGTCCACAAACTGGTG
CAGGCCATCTTCCCCACCTCGGATCCTACGACCATGCAGGACAAACGGAT
GCATAATCTCGTTTCATACGCGGAAAAGGTCGAGAAGGACATGTACGAAA
TGGCCAAGTCCAGATCGGAGTACTATCACCTGCTGGCCGAGAAGATCTAC
AAGATTCAAAAGGAGCTGGAGGAGAAGCGACTGAAGCGTAAGGAGCAGCA
TCAGCAGATGCTGATGCAGCAACAGGGCGTTGCGAATCCAGTGGCTGGAG
GAGCGGCTGGCGGAGCAGGCAGTGCAGCTGGTGTAGCGGGCGGTGTAGTC
TTGCCCCAGCAGCAACAGCAGCAGCAACAACAACAGCAGCAGCAGGGTCA
GCAGCCTCTGCAGAGCTGTATCCATCCAAGCATCAGTCCAATGGGCGGTG
TGATGCCGCCGCAGCAGCTGCGTCCACAGGGACCACCTGGAATACTGGGC
CAACAGACGGCAGCAGGCCTGGGCGTCGGCGTGGGAGTGACCAACAATAT
GGTTACCATGCGCAGTCATTCGCCCGGTGGCAACATGCTCGCCTTGCAGC
AACAACAGCGCATGCAGTTCCCGCAACAACAGCAGCAACAACCGCCAGGG
TCTGGAGCCGGCAAAATGCTGGTCGGTCCACCAGGACCCAGTCCCGGTGG
CATGGTGGTCAATCCCGCGCTCTCGCCTTACCAGACGACCAATGTGCTCA
CCAGTCCGGTGCCAGGACAGCAGCAACAGCAGCAGTTCATTAATGCGAAC
GGCGGCACTGGCGCCAATCCTCAACTGAGCGAAATCATGAAGCAGCGTCA
CATTCACCAGCAGCAGCAGCAACAACAACAGCAGCAGCAGCAGGGAATGT
TGTTGCCGCAGTCGCCATTTAGCAATTCAACACCTCTACAACAACAACAG
CAGCAGCAGCAGCAACAACAGCAGCAGCAGGCGACTAGCAACAGTTTTAG
CTCACCAATGCAGCAACAGCAGCAAGGTCAGCAACAGCAACAACAGAAGC
CCGGCAGTGTGCTGAATAATATGCCGCCCACGCCCACGAGTCTGGAAGCC
CTGAATGCGGGGGCCGGAGCGCCGGGAACTGGAGGATCCGCCTCCAATGT
AACGGTTTCAGCTCCGAGCCCATCGCCTGGCTTCTTGTCCAACGGCCCGT
CGATTGGCACGCCCTCCAACAATAATAATAATAGTAGTGCTAACAACAAC
CCGCCCTCGGTGAGCAGTCTAATGCAACAGCCGCTGAGCAATCGGCCGGG
TACGCCTCCTTACATACCCGCTTCCCCAGTGCCGGCGACAAGTGCCTCCG
GATTAGCGGCGAGCAGTACGCCCGCATCAGCAGCAGCCACCTGTGCGAGT
AGTGGCAGTGGCAGCAATAGCAGCAGCGGAGCAACTGCAGCGGGTGCAAG
TTCCACGTCATCATCTTCCTCGGCGGGCTCGGGTACACCACTCAGCTCGG
TATCGACTCCTACATCGGCCACGATGGCCACCAGCAGCGGTGGTGGTGGT
GGTGGTGGGGGCAATGCAGGAGGCGGATCATCCACTACGCCCGCTAGCAA
TCCACTGCTCCTCATGTCTGGAGGAACGGCAGGAGGCGGAACGGGAGCAA
CGACCACCACATCGACATCCTCGAGCAGTCGCATGATGAGCAGCTCCAGC
AGTCTCTCCTCACAGATGGCTGCCCTGGAGGCTGCGGCGCGAGACAACGA
CGATGAGACGCCCTCGCCATCCGGCGAGAATACGAACGGCAGTGGTGGCA
 
GTGGAAATGCCGGCGGTATGGCCTCCAAGGGCAAACTGGACTCCATTAAG
CAAGATGATGATATCAAGAAGGAGTTTATGGATGACAGCTGTGGCGGAAA
TAACGATAGCTCGCAGATGGATTGCTCGACGGGTGGTGGCAAGGGCAAGA
ATGTGAACAACGACGGAACAAGCATGATCAAAATGGAGATCAAGACGGAG
.GATGGACTCGATGGCGAGGTAAAGATCAAAACGGAGGCCATGGATGTGGA
CGAGGCTGGAGGATCGACAGCCGGAGAGCATCATGGCGAAGGTGGCGGCG
GCAGTGGTGTTGGCGGCGGTAAGGATAACATAAATGGTGCGCACGATGGC
GGAGCGACAGGCGGTGCTGTGGACATAAAACCCAAGACGGAGACGAAACC
ACTCGTACCGGAGCCACTGGCACCCAATGCAGGTGACAAGAAAAAGAAGT
GCCAATTCAATCCCGAGGAACTGCGCACCGCTCTCCTGCCAACGCTAGAG
AAGCTCTACAGGCAGGAGCCCGAATCCGTGCCCTTTCGCTACCCAGTTGA
TCCCCAGGCGCTGGGCATACCTGATTACTTTGAAATCGTTAAGAAGCCCA
TGGACCTGGGCACTATACGCACCAACATCCAGAATGGAAAGTACAGTGAT
CCCTGGGAATATGTGGACGACGTTTGGCTGATGTTCGACAATGCCTGGCT
GTATAATCGCAAAACATCGCGGGTCTATCGCTATTGCACAAAGCTTTCCG
AAGTCTTTGAGGCGGAGATTGATCCTGTGATGCAGGCACTGGGATATTGC
TGCGGCAGGAAGTACACATTCAATCCACAGGTGCTATGCTGCTACGGCAA
GCAGCTCTGCACGATTCCGCGGGATGCCAAGTACTACAGCTACCAGAACA
GTCTAAAGGAATACGGTGTCGCCTCAAATAGATACACCTACTGCCAAAAG
TGCTTTAACGACATCCAGGGCGATACGGTCACACTGGGCGACGATCCACT
GCAATCGCAAACCCAAATCAAAAAGGATCAGTTCAAGGAGATGAAGAACG
ATCACCTCGAACTGGAGCCGTTTGTCAATTGCCAGGAGTGCGGACGCAAA
CAGCACCAAATCTGCGTACTCTGGCTGGATTCTATCTGGCCCGGTGGCTT
CGTGTGCGATAACTGCCTGAAAAAGAAGAACTCAAAGCGGAAGGAGAACA
AGTTCAATGCGAAACGCCTGCCCACCACCAAGCTGGGCGTGTACATAGAG
ACGCGGGTGAATAATTTCCTCAAGAAGAAGGAGGCTGGTGCCGGCGAGGT
GCACATTCGTGTGGTCAGCTCATCGGACAAGTGTGTAGAGGTGAAGCCCG
GCATGCGTCGACGATTCGTCGAGCAGGGCGAGATGATGAACGAGTTCCCA
TACCGAGCCAAAGCGCTCTTTGCCTTCGAGGAGGTGGATGGCATCGATGT
GTGCTTCTTTGGCATGCACGTTCAGGAGTATGGATCCGAGTGCCCGGCGC
CGAATACGCGGCGTGTGTATATTGCCTATTTGGATTCCGTTCATTTCTTC
CGGCCAAGACAGTACCGTACAGCGGTATATCACGAAATCCTGCTCGGCTA
TATGGACTACGTGAAACAGCTGGGCTACACAATGGCCCATATCTGGGCCT
GTCCGCCATCCGAGGGCGATGACTACATCTTTCACTGCCATCCCACGGAC
CAGAAGATACCCAAGCCCAAGCGCCTGCAGGAGTGGTACAAAAAGATGCT
TGACAAGGGAATGATCGAGCGCATCATACAGGACTACAAGGATATCCTGA
AGCAGGCGATGGAGGACAAACTGGGCTCTGCCGCAGAGCTGCCCTACTTT
GAGGGCGACTTCTGGCCCAATGTGCTGGAGGAGAGCATCAAGGAACTGGA
CCAGGAGGAGGAAGAGAAGCGCAAACAGGCCGAGGCCGCGGAAGCAGCAG
CTGCGGCAAATCTTTTCTCTATCGAGGAAAATGAAGTAAGCGGCGATGGC
AAAAAGAAGGGCCAGAAGAAGGCCAAAAAGTCGAACAAATCGAAAGCGGC
GCAGCGTAAGAACAGCAAAAAGTCCAACGAACATCAGTCGGGCAATGATC
TCTCCACAAAGATATATGCGACCATGGAGAAGCACAAGGAGGTCTTCTTC
GTTATCCGTCTGCATTCGGCGCAGTCGGCAGCTAGTTTAGCGCCCATCCA
GGATCCCGATCCGCTGCTCACATGCGATCTGATGGATGGACGCGATGCCT
TCCTCACGCTCGCCCGCGACAAGCACTTTGAGTTCTCGTCGCTGCGGCGC
GCACAATTCTCCACTCTGTCCATGTTGTATGAGCTGCATAACCAGGGTCA
GGACAAGTTTGTTTACACCTGCAACCACTGCAAGACGGCCGTGGAGACGC
GCTACCACTGTACTGTTTGTGATGACTTCGATCTGTGTATCGTGTGCAAG
GAGAAGGTTGGCCATCAGCACAAGATGGAGAAGCTCGGCTTCGACATCGA
CGACGGCTCTGCGCTGGCGGATCACAAGCAGGCTAATCCACAGGAGGCCC
GCAAGCAATCCATCCAGCGTTGCATCCAATCGCTGGCGCACGCCTGCCAG
TGTCGCGATGCCAACTGCCGCCTGCCATCGTGCCAGAAGATGAAGCTCGT
TGTCCAGCATACGAAGAACTGCAAGCGCAAGCCCAACGGAGGATGCCCCA
TTTGCAAGCAGCTTATCGCACTCTGTTGCTATCACGCGAAGAACTGTGAG
GAGCAGAAGTGCCCCGTGCCGTTCTGTCCCAACATCAAGCACAAGCTCAA
GCAGCAGCAGTCACAGCAGAAATTCCAGCAGCAGCAGTTGCTGCGTCGCC
GTGTGGCGCTCATGTCGCGTACAGCAGCTCCAGCGGCTCTGCAAGGCCCA
 
GCTGCAGTAAGCGGTCCGACCGTCGTCTCTGGAGGAGTGCCCGTGGTGGG
CATGTCCGGTGTGGCAGTTAGCCAACAGGTGATCCCGGCCAGGCGGGTA
TACTGCCTCCAGGGGCGGGTGGCATGTCGCCATCTACCGTGGCAGTTCCA
TCGCCTGTTTCAGGAGGAGCGGAGCCGGTGGAATGGGTGGAATGACATC
ACCACATCCGCATCAACCAGGTATAGGTATGAAACCTGGTGGCGGTCACT
CGCCGTCTCCAAATGTCCTACAAGTGGTGAAGCAGGTCCAGGAAGAGGCA
GCTCGTCAGCAGGTATCGCATGGCGGTGGCTTCGGCAAGGGCGTACCCAT
GGCGCCGCCCGTAATGAATCGACCAATGGGCGGCGCTGGGCCCAACCAAA
ATGTTGTTAATCAACTTGGTGGCATGGGCGTTGGAGTTGAAGGTGTCGGT
GGTGTTGGCGTCGGAGGCGTTGGTGGAGTGGGTGTTAATCAACTGAATTC
GGGTGGTGGCAATACACCCGGTGCACCCATTTCCGGTCCCGGAATGAATG
TCAATCATCTAATGTCCATGGATCAGTGGGGCGGTGGCGGAGCCGGCGGC
GGAGGTGCCAATCCCGGCGGTGGCAATCCACAAGCCCGCTATGCCAACAA
TACCGGCGGCATGCGCCAACCCACCCATGTGATGCAAACGAATCTGATAC
CGCCGCAGCAACAGCAACAGATGATGGGCGGACTGGGCGGACCCAACCAA
CTGGGAGGTGGCCAAATGCCAGTCGGCGGACAGCATGGAGGAATGGGAAT
GGGCATGGGAGCACCACCAATGGCCGGAACTGTTGGCGGAGTGCGTCCAT
CTCCCGGAGCAGGAGGTGGAGGTGGAAGTGCGACTGGGGGCGGTCTAAAT
ACGCAACAACTCGCCCTGATTATGCAAAAGATTAAGAACAATCCCACCAA
CGAGAGCAACCAGCACATCCTTGCCATACTAAAACAGAATCCGCAGATCA
TGGCGGCGATCATCAAGCAGCGCCAGCAGTCGCAGAACAATGCGGCAGCG
GGCGGAGGAGCACCTGGCCCAGGTGGAGCCCTACAGCAGCAGCAGGCCGG
TAACGGACCGCAAAATCCTCAACAGCAGCAGCAGCAGCAGCAACAGCAAC
AGGTGATGCAGCAACAGCAGATGCAGCACATGATGAACCAGCAGCAGGGC
GGCGGCGGTCCACAGCAGATGAATCCCAACCAGCAGCAGCAACAGCAGCA
GGTTAATCTCATGCAGCAGCAGCAACAAGGTGGACCCGGAGGACCAGGTT
CTGGACTTCCCACGCGCATGCCCAATATGCCCAATGCCTTGGGTATGCTG
CAGAGTCTTCCGCCCAACATGTCGCCAGGCGTTTCTACTCAGGGAGGAAT
GGTGCCCAACCAAAACTGGAACAAGATGCGTTACATGCAAATGAGCCAGT
ACCCGCCACCGTATCCGCAGCGCCAGCGTGGCCCGCACATGGGCGGAGCG
GGACCTGGTCCCGGCCAGCAACAGTTCCCCGGTGGCGGAGGTGGAGCGGG
CAACTTTAATGCGGGTGGTGCTGGTGGTGCAGGCGGCGTTGTCGGTGTGG
GCGGAGTGCCCGGAGGTGCCGGCACGGTGCCCGGTGGCGATCAATACTCG
ATGGCGAATGCCGCGGCTGCCTCCAATATGCTGCAACAGCAGCAGGGCCA
GGTGGGCGTCGGAGTGGGCGTGGGCGTGAAACCAGGACCCGGCCAACAGC
AACAGCAGATGGGCGTTGGCATGCCGCCGGGTATGCAGCAGCAACAGCAG
CAACAGCAACCGCTGCAGCAGCAGCAGATGATGCAGGTAGCAATGCCAAA
TGCGAATGCCCAGAATCCGTCGGCGGTGGTTGGCGGACCCAATGCTCAGG
TGATGGGTCCGCCGACGCCGCACTCTCTGCAGCAGCAGCTGATGCAATCG
GCCCGCTCGTCGCCGCCTATTCGCTCCCCGCAGCCAACGCCATCGCCACG
TTCGGCTCCATCGCCACGTGCTGCTCCATCCGCCTCGCCTAGGGCACAGC
CCTCGCCGCACCATGTGATGAGCAGTCACTCGCCAGCGCCGCAGGGACCA
CCGCATGACGGCATGCACAATCATGGCATGCATCATCAGTCGCCACTGCC
AGGAGTGCCGCAGGATGTTGGCGTCGGAGTCGGTGTCGGCGTTGGCGTTG
GCGTTAACGTTAACGTCGGCAACGTGGGCGTCGGCAATGCCGGAGGAGCC
CTGCCCGACGCCTCCGACCAGCTGACCAAGTTTGTGGAGCGACTCTAGTG
CAGCAACAGCAGCAGCACCAGCACCAGCACCACCACCAGCTACAATGGTT
GGTAGGCGATGTGGCTAGAGGGCTAGGGCTAGACTGAATGAATGAATGAG
TGTCCAGTAGCCGCAGACGGGATGACGACGAAGACCAACCGGCAGGGATA
ACCAGTGTGTGTTAAGCGAATTAACAACTATTACTAACTTAAATCTTTTT
TTTTTTTTAAACGGCACCACAAATAATTGTATATTGTTATAATTAAATC
AACAAATATCGCGCCTAATGTGTACTGTAGATTAAGATGACCCACCATTA
CAACCACTAACAAATACCTTATTATTTAAGTTTAAGACGAAAGTTGGACA
GAGCATTATGATTCGATTTCCATTTTATGTCCGCGATTTAGCAAATATAT
AATATCATATATTTCATATGCCCCCAAAACACACACACACCATGTATTAA
TTAATGCGATTCCTTCGTTTCCACTAAGCAGATATAGAAAAAAAAAAA
 
MMADHLDEPPQKRVKMDPTDISYFLEENLPDELVSSNSGWSDQLTGGAGG
GNGGGGASGVTTNPTSGPNPGGGPNKPAAQGPGSGTGGVGVGVNVGVGGV
VGVGVVPSQMNGAGGGNGSGTGGDDGSGNGSGAGNRISQMQHQQLQHLLQ
QQQQGQKGAMVVPGMQQLGSKSPNLQSPNQGGMQQVVGTQMGMVNSMPMS
ISNNGNNGMNAIPGMNTIAQGNLGNMVLTNSVGGGMGGMVNHLKQQPGGG
GGGMINSVSVPGGPGAGAGGVGAGGGGAVAANQGMHMQNGPMMGRMVGQQ
HMLRGPHLMGASGGAGGPGNGPGGGGPRMQNPNMQMTQLNSLPYGVGQYG
GPGGGNNPQQQQQQQQQLLAQQMAQRGGVVPGMPQGNRPVGTVVPMSTL
GGDGSGPAGQLVSGNPQQQQMLAQQQTGAMGPRPPQPNQLLGHPGQQQQQ
QQQPGTSQQQQQQQGVGIGGAGVVANAGTVAGVPAVAGGGAGGAVQSSGP
GGANRDVPDDRKRQIQQQLMLLLHAHKCNRRENLNPNREVCNVNYCKAMK
SVLAHMGTCKQSKDCTMQHCASSRQILLHYKTCQNSGCVICYPFRQNHSV
FQNANVPPGGGPAGIGGAPPGGGGAGGGAAGAGGNLQQQQQQQQQQQNQ
QPNLTGLVVDGKQGQQVAPGGGQNTAIVLPQQQGAGGAPGAPKTPADMVQ
QLTQQQQQQQQVHQQQVQQQELRRFDGMSQQVVAGGMQQQQQGLPPVI
RIQGAQPAVRVLGPGGPGGPSGPNVLPNDVNSLHQQQQQMLQQQQQQGQN
RRRGGLATMVEQQQQHQQQQQQPNPAQLGGNIPAPLSVNVGGFGNTNFGG
AAAGGAVGANDKQQLKVAQVHPQSHGVGAGGASAGAGASGGQVAAGSSVL
MPADTTGSGNAGNPNQNAGGVAGGAGGGNGGNTGPPGDNEKDWRESVTAD
LRNHLVHKLVQAIFPTSDPTTMQDKRMHNLVSYAEKVEKDMYEMAKSRSE
YYHLLAEKIYKIQKELEEKRLKRKEQHQQMLMQQQGVANPVAGGAAGGAG
SAAGVAGGVVLPQQQQQQQQQQQQQGQQPLQSCIHPSISPMGGVMPPQQL
RPQGPPGILGQQTAAGLGVGVGVTNNMVTMRSHSPGGNMLALQQQQRMQF
PQQQQQQPPGSGAGKMLVGPPGPSPGGMVVNPALSPYQTTNVLTSPVPGQ
QQQQQFINANGGTGANPQLSEIMKQRHIHQQQQQQQQQQQGMLLPQSPF
SNSTPLQQQQQQQQQQQQQQATSNSFSSPMQQQQQGQQQQQQKPGSVLNN
MPPTPTSLEALNAGAGAPGTGGSASNVTVSAPSPSPGFLSNGPSIGTPSN
NNNNSSANNNPPSVSSLMQQPLSNRPGTPPYIPASPVPATSASGLAASST
PASAAATCASSGSGSNSSSGATAAGASSTSSSSSAGSGTPLSSVSTPTSA
TMATSSGGGGGGGGNAGGGSSTTPASNPLLLMSGGTAGGGTGATTTTSTS
SSSRMMSSSSSLSSQMAALEAAARDNDDETPSPSGENTNGSGGSGNAGGM
ASKGKLDSIKQDDDIKKEFMDDSCGGNNDSSQMDCSTGGGKGKNVNNDGT
SMIKMEIKTEDGLDGEVKIKTEAMDVDEAGGSTAGEHHGEGGGGSGVGGG
KDNINGAHDGGATGGAVDIKPKTETKPLVPEPLAPNAGDKKKKCQFNPEE
LRTALLPTLEKLYRQEPESVPFRYPVDPQALGIPDYFEIVKKPMDLGTIR
TNIQNGKYSDPWEYVDDVWLMFDNAWLYNRKTSRVYRYCTKLSEVFEAEI
DPVMQALGYCCGRKYTFNPQVLCCYGKQLCTIPRDAKYYSYQNSLKEYGV
ASNRYTYCQKCFNDIQGDTVTLGDDPLQSQTQIKKDQFKEMKNDHLELEP
FVNCQECGRKQHQICVLWLDSIWPGGFVCDNCLKKKNSKRKENKFNAKRL
PTTKLGVYIETRVNNFLKKKEAGAGEVHIRVVSSSDKCVEVKPGMRRRFV
EQGEMMNEFPYRAKALFAFEEVDGIDVCFFGMHVQEYGSECPAPNTRRVY
IAYLDSVHFFRPRQYRTAVYHEILLGYMDYVKQLGYTMAHIWACPPSEGD
DYIFHCHPTDQKIPKPKRLQEWYKKMLDKGMIERIIQDYKDILKQAMEDK
LGSAAELPYFEGDFWPNVLEESIKELDQEEEEKRKQAEAAEAAAAANLFS
IEENEVSGDGKKKGQKKAKKSNKSKAAQRKNSKKSNEHQSGNDLSTKIYA
TMEKHKEVFFVIRLHSAQSAASLAPIQDPDPLLTCDLMDGRDAFLTLARD
KHFEFSSLRRAQFSTLSMLYELHNQGQDKFVYTCNHCKTAVETRYHCTVC
DDFDLCIVCKEKVGHQHKMEKLGFDIDDGSALADHKQANPQEARKQSIQR
 
CIQSLAHACQCRDANCRLPSCQKMKLVVQHTKNCKRKPNGGCPICKQLIA
LCCYHAKNCEEQKCPVPFCPNIKHKLKQQQSQQKFQQQLLRRRVALMSR
TAAPAALQGPAAVSGPTVVSGGVPVVGMSGVAVSQQVIPGQAGILPPGAG
GMSPSTVAVPSPVSGGAGAGGMGGMTSPHPHQPGIGMKPGGGHSPSPNVL
QVVKQVQEEAARQQVSHGGGFGKGVPMAPPVMNRPMGGAGPNQNVVNQLG
GMGVGVEGVGGVGVGGVGGVGVNQLNSGGGNTPGAPISGPGMNVNHLMSM
DQWGGGGAGGGGANPGGGNPQARYANNTGGMRQPTHVMQTNLIPPQQQQQ
MMGGLGGPNQLGGGQMPVGGQHGGMGMGMGAPPMAGTVGGVRPSPGAGGG
GGSATGGGLNTQQLALIMQKIKNNPTNESNQHILAILKQNPQIMAAIIKQ
RQQSQNNAAAGGGAPGPGGALQQQQAGNGPQNPQQQQQQQQQQVMQQQQ
MQHMMNQQQGGGGPQQMNPNQQQQQQQVNLMQQQQQGGPGGPGSGLPTRM
PNMPNALGMLQSLPPNMSPGVSTQGGMVPNQNWNKMRYMQMSQYPPPYPQ
RQRGPHMGGAGPGPGQQQFPGGGGGAGNFNAGGAGGAGGVVGVGGVPGGA
GTVPGGDQYSMANAAAASNMLQQQGQVGVGVGVGVKPGPGQQQQMGVG
MPPGMQQQQQQQPLQQQMMQVAMPNANAQNPSAVVGGPNAQVMGPPTP
HSLQQQLMQSARSSPPIRSPQPTPSPRSAPSPRAAPSASPRAQPSPHHVM
SSHSPAPQGPPHDGMHNHGMHHQSPLPGVPQDVGVGVGVGVGVNVNVG
NVGVGNAGGALPDASDQLTKFVERL
**Human homologue of Complete Genome candidate** AAC51331- CREB-binding protein 
1 tccgaattcc ttttttttaa ttgaggaatc aacagccgcc atcttgtcgc ggacccgacc
61 ggggcttcga gcgcgatcta ctcggccccg ccggtcccgg gccccacaac cgcccgcgca
121 ccccgctccg cccggccggc ccgctccgcc cggccctcgg cgcccgcccc ggcggccccg
181 ctcgcctctc ggctcggcct cccggagccc ggcggcggcg gcggcggcag cggcggcggc
241 ggcggcggaa cggggggtgg gggggccgcg gcggcggcgg cgaccccgct cggcgcattg
301 tttttcctca cggcggcggc ggcggcgggc cgcgggccgg gagcggagcc cggagccccc
361 tcgtcgtcgg gccgcgagcg aattcattaa gtggggcgcg gggggggagc gaggcggcgg
421 cggcggcggc accatgttct cggggactgc ctgagccgcc cggccgggcg ccgtcgctgc
481 cagccgggcc cgggggggcg gccgggccgc cggggcgccc ccaccgcgga gtgtcgcgct
541 cgggaggcgg gcaggggatg agggggccgc ggccggcggc ggcggcggcg gccgggggcg
601 ggcggtgagc gctgcggggc gctgttgctg tggctgagat ttggccgccg cctcccccac
661 ccggcctgcg ccctccctct ccctcggcgc ccgcccgcgc cgctcgcggc gcccgcgctc
721 gctcctctcc ctcgcagccg gcagggcccc cgacccccgt ccgggccctc gccggcccgg
781 ccgcccgtgc ccggggctgt tttcgcgagc aggtgaaaat ggctgagaac ttgctggacg
841 gaccgcccaa ccccaaaaga gccaaactca gctcgcccgg tttctcggcg aatgacagca
901 cagattttgg atcattgttt gacttggaaa atgatcttcc tgatgagctg atacccaatg
961 gaggagaatt aggcctttta aacagtggga accttgttcc agatgctgct tccaaacata
1021 aacaactgtc ggagcttcta cgaggaggca gcggctctag tatcaaccca ggaataggaa
1081 atgtgagcgc cagcagcccc gtgcagcagg gcctgggtgg ccaggctcaa gggcagccga
1141 acagtgctaa catggccagc ctcagtgcca tgggcaagag ccctctgagc cagggagatt
1201 cttcagcccc cagcctgcct aaacaggcag ccagcacctc tgggcccacc cccgctgcct
1261 cccaagcact gaatccgcaa gcacaaaagc aagtggggct ggcgactagc agccctgcca
1321 cgtcacagac tggacctggt atctgcatga atgctaactt taaccagacc cacccaggcc
1381 tcctcaatag taactctggc catagcttaa ttaatcaggc ttcacaaggg caggcgcaag
1441 tcatgaatgg atctcttggg gctgctggca gaggaagggg agctggaatg ccgtacccta
1501 ctccagccat gcagggcgcc tcgagcagcg tgctggctga gaccctaacg caggtttccc
1561 cgcaaatgac tggtcacgcg ggactgaaca ccgcacaggc aggaggcatg gccaagatgg
1621 gaataactgg gaacacaagt ccatttggac agccctttag tcaagctgga gggcagccaa
 
1681 tgggagccac tggagtgaac ccccagttag ccagcaaaca gagcatggtc aacagtttgc
1741 ccaccttccc tacagatatc aagaatactt cagtcaccaa cgtgccaaat atgtctcaga
1801 tgcaaacatc agtgggaatt gtacccacac aagcaattgc aacaggcccc actgcagatc
1861 ctgaaaaacg caaactgata cagcagcagc tggttctact gcttcatgct cataagtgtc
1921 agagacgaga gcaagcaaac ggagaggttc gggcctgctc gctcccgcat tgtcgaacca
1981 tgaaaaacgt tttgaatcac atgacgcatt gtcaggctgg gaaagcctgc caagttgccc
2041 attgtgcatc ttcacgacaa atcatctctc attggaagaa ctgcacacga catgactgtc
2101 ctgtttgcct ccctttgaaa aatgccagtg acaagcgaaa ccaacaaacc atcctggggt
2161 ctccagctag tggaattcaa aacacaattg gttctgttgg cacagggcaa cagaatgcca
2221 cttctttaag taacccaaat cccatagacc ccagctccat gcagcgagcc tatgctgctc
2281 tcggactccc ctacatgaac cagccccaga cgcagctgca gcctcaggtt cctggccagc
2341 aaccagcaca gcctcaaacc caccagcaga tgaggactct caaccccctg ggaaataatc
2401 caatgaacat tccagcagga ggaataacaa cagatcagca gcccccaaac ttgatttcag
2461 aatcagctct tccgacttcc ctgggggcca caaacccact gatgaacgat ggctccaact
2521 ctggtaacat tggaaccctc agcactatac caacagcagc tcctccttct agcaccggtg
2581 taaggaaagg ctggcacgaa catgtcactc aggacctgcg gagccatcta gtgcataaac
2641 tcgtccaagc catcttccca acacctgatc ccgcagctct aaaggatcgc cgcatggaaa
2701 acctggtagc ctatgctaag aaagtggaag gggacatgta cgagtctgcc aacagcaggg
2761 atgaatatta tcacttatta gcagagaaaa tctacaagat acaaaaagaa ctagaagaaa
2821 aacggaggtc gcgtttacat aaacaaggca tcttggggaa ccagccagcc ttaccagccc
2881 cgggggctca gccccctgtg attccacagg cacaacctgt gagacctcca aatggacccc
2941 tgtccctgcc agtgaatcgc atgcaagttt ctcaagggat gaattcattt aaccccatgt
3001 ccttggggaa cgtccagttg ccacaagcac ccatgggacc tcgtgcagcc tccccaatga
3061 accactctgt ccagatgaac agcatgggct cagtgccagg gatggccatt tctccttccc
3121 gaatgcctca gcctccgaac atgatgggtg cacacaccaa caacatgatg gcccaggcgc
3181 ccgctcagag ccagtttctg ccacagaacc agttcccgtc atccagcggg gcgatgagtg
3241 tgggcatggg gcagccgcca gcccaaacag gcgtgtcaca gggacaggtg cctggtgctg
3301 ctcttcctaa ccctctcaac atgctggggc ctcaggccag ccagctacct tgccctccag
3361 tgacacagtc accactgcac ccaacaccgc ctcctgcttc cacggctgct ggcatgccat
3421 ctctccagca cacgacacca cctgggatga ctcctcccca gccagcagct cccactcagc
3481 catcaactcc tgtgtcgtct tccgggcaga ctcccacccc gactcctggc tcagtgccca
3541 gtgctaccca aacccagagc acccctacag tccaggcagc agcccaggcc caggtgaccc
3601 cgcagcctca aaccccagtt cagcccccgt ctgtggctac ccctcagtca tcgcagcaac
3661 agccgacgcc tgtgcacgcc cagcctcctg gcacaccgct ttcccaggca gcagccagca
3721 ttgataacag agtccctacc ccctcctcgg tggccagcgc agaaaccaat tcccagcagc
3781 caggacctga cgtacctgtg ctggaaatga agacggagac ccaagcagag gacactgagc
3841 ccgatcctgg tgaatccaaa ggggagccca ggtctgagat gatggaggag gatttgcaag
3901 gagcttccca agttaaagaa gaaacagaca tagcagagca gaaatcagaa ccaatggaag
3961 tggatgaaaa gaaacctgaa gtgaaagtag aagttaaaga ggaagaagag agtagcagta
4021 acggcacagc ctctcagtca acatctcctt cgcagccgcg caaaaaaatc tttaaaccag
4081 aggagttacg ccaggccctc atgccaaccc tagaagcact gtatcgacag gacccagagt
4141 cattaccttt ccggcagcct gtagatcccc agctcctcgg aattccagac tattttgaca
4201 tcgtaaagaa tcccatggac ctctccacca tcaagcggaa gctggacaca gggcaatacc
4261 aagagccctg gcagtacgtg gacgacgtct ggctcatgtt caacaatgcc tggctctata
4321 atcgcaagac atcccgagtc tataagtttt gcagtaagct tgcagaggtc tttgagcagg
4381 aaattgaccc tgtcatgcag tcccttggat attgctgtgg acgcaagtat gagttttccc
4441 cacagacttt gtgctgctat gggaagcagc tgtgtaccat tcctcgcgat gctgcctact
4501 acagctatca gaataggtat catttctgtg agaagtgttt cacagagatc cagggcgaga
4561 atgtgaccct gggtgacgac ccttcacagc cccagacgac aatttcaaag gatcagtttg
4621 aaaagaagaa aaatgatacc ttagaccccg aacctttcgt tgattgcaag gagtgtggcc
4681 ggaagatgca tcagatttgc gttctgcact atgacatcat ttggccttca ggttttgtgt
4741 gcgacaactg cttgaagaaa actggcagac ctcgaaaaga aaacaaattc agtgctaaga
4801 ggctgcagac cacaagactg ggaaaccact tggaagaccg agtgaacaaa tttttgcggc
4861 gccagaatca ccctgaagcc ggggaggttt ttgtccgagt ggtggccagc tcagacaaga
4921 cggtggaggt caagcccggg atgaagtcac ggtttgtgga ttctggggaa atgtctgaat
4981 ctttcccata tcgaaccaaa gctctgtttg cttttgagga aattgacggc gtggatgtct
5041 gcttttttgg aatgcacgtc caagaatacg gctctgattg cccccctcca aacacgaggc
5101 gtgtgtacat ttcttatctg gatagtattc atttcttccg gccacgttgc ctccgcacag
 
5161 ccgtttacca tgagatcctt attggatatt tagagtatgt gaagaaatta gggtatgtga
5221 cagggcacat ctgggcctgt cctccaagtg aaggagatga ttacatcttc cattgccacc
5281 cacctgatca aaaaataccc aagccaaaac gactgcagga gtggtacaaa aagatgctgg
5341 acaaggcgtt tgcagagcgg atcatccatg actacaagga tattttcaaa caagcaactg
5401 aagacaggct caccagtgcc aaggaactgc cctattttga aggtgatttc tggcccaatg
5461 tgttagaaga gagcattaag gaactagaac aagaagaaga ggagaggaaa aaggaagaga
5521 gcactgcagc cagtgaaacc actgagggca gtcagggcga cagcaagaat gccaagaaga
5581 agaacaacaa gaaaaccaac aagaacaaaa gcagcatcag ccgcgccaac aagaagaagc
5641 ccagcatgcc caacgtgtcc aatgacctgt cccagaagct gtatgccacc atggagaagc
5701 acaaggaggt cttcttcgtg atccacctgc acgctgggcc tgtcatcaac accctgcccc
5761 ccatcgtcga ccccgacccc ctgctcagct gtgacctcat ggatgggcgc gacgccttcc
5821 tcaccctcgc cagagacaag cactgggagt tctcctcctt gcgccgctcc aagtggtcca
5881 cgctctgcat gctggtggag ctgcacaccc agggccagga ccgctttgtc tacacctgca
5941 acgagtgcaa gcaccacgtg gagacgcgct ggcactgcac tgtgtgcgag gactacgacc
6001 tctgcatcaa ctgctataac acgaagagcc atgcccataa gatggtgaag tgggggctgg
6061 gcctggatga cgagggcagc agccagggcg agccacagtc aaagagcccc caggagtcac
6121 gccggctgag catccagcgc tgcatccagt cgctggtgca cgcgtgccag tgccgcaacg
6181 ccaactgctc gctgccatcc tgccagaaga tgaagcgggt ggtgcagcac accaagggct
6241 gcaaacgcaa gaccaacggg ggctgcccgg tgtgcaagca gctcatcgcc ctctgctgct
6301 accacgccaa gcactgccaa gaaaacaaat gccccgtgcc cttctgcctc aacatcaaac
6361 acaagctccg ccagcagcag atccagcacc gcctgcagca ggcccagctc atgcgccggc
6421 ggatggccac catgaacacc cgcaacgtgc ctcagcagag tctgccttct cctacctcag
6481 caccgcccgg gacccccaca cagcagccca gcacacccca gacgccgcag ccccctgccc
6541 agccccaacc ctcacccgtg agcatgtcac cagctggctt ccccagcgtg gcccggactc
6601 agccccccac cacggtgtcc acagggaagc ctaccagcca ggtgccggcc cccccacccc
6661 cggcccagcc ccctcctgca gcggtggaag cggctcggca gatcgagcgt gaggcccagc
6721 agcagcagca cctgtaccgg gtgaacatca acaacagcat gcccccagga cgcacgggca
6781 tggggacccc ggggagccag atggcccccg tgagcctgaa tgtgccccga cccaaccagg
6841 tgagcgggcc cgtcatgccc agcatgcctc ccgggcagtg gcagcaggcg ccccttcccc
6901 agcagcagcc catgccaggc ttgcccaggc ctgtgatatc catgcaggcc caggcggccg
6961 tggctgggcc ccggatgccc agcgtgcagc cacccaggag catctcaccc agcgctctgc
7021 aagacctgct gcggaccctg aagtcgccca gctcccctca gcagcaacag caggtgctga
7081 acattctcaa atcaaacccg cagctaatgg cagctttcat caaacagcgc acagccaagt
7141 acgtggccaa tcagcccggc atgcagcccc agcctggcct ccagtcccag cccggcatgc
7201 aaccccagcc tggcatgcac cagcagccca gcctgcagaa cctgaatgcc atgcaggctg
7261 gcgtgccgcg gcccggtgtg cctccacagc agcaggcgat gggaggcctg aacccccagg
7321 gccaggcctt gaacatcatg aacccaggac acaaccccaa catggcgagt atgaatccac
7381 agtaccgaga aatgttacgg aggcagctgc tgcagcagca gcagcaacag cagcagcaac
7441 aacagcagca acagcagcag cagcaaggga gtgccggcat ggctgggggc atggcggggc
7501 acggccagtt ccagcagcct caaggacccg gaggctaccc accggccatg cagcagcagc
7561 agcgcatgca gcagcatctc cccctccagg gcagctccat gggccagatg gcggctcaga
7621 tgggacagct tggccagatg gggcagccgg ggctgggggc agacagcacc cccaacatcc
7681 agcaagccct gcagcagcgg attctgcagc aacagcagat gaagcagcag attgggtccc
7741 caggccagcc gaaccccatg agcccccagc aacacatgct ctcaggacag ccacaggcct
7801 cgcatctccc tggccagcag atcgccacgt cccttagtaa ccaggtgcgg tctccagccc
7861 ctgtccagtc tccacggccc cagtcccagc ctccacattc cagcccgtca ccacggatac
7921 agccccagcc ttcgccacac cacgtctcac cccagactgg ttccccccac cccggactcg
7981 cagtcaccat ggccagctcc atagatcagg gacacttggg gaaccccgaa cagagtgcaa
8041 tgctccccca gctgaacacc cccagcagga gtgcgctgtc cagcgaactg tccctggtcg
8101 gggacaccac gggggacacg ctagagaagt ttgtggaggg cttgtag
 
1 maenlldgpp npkraklssp gfsandstdf gslfdlendl pdelipngge lgllnsgnlv
61 pdaaskhkql sellrggsgs sinpgignvs asspvqqglg gqaqgqpnsa nmaslsamgk
121 splsqgdssa pslpkqaast sgptpaasqa lnpqaqkqvg latsspatsq tgpgicmnan
181 fnqthpglln snsghslinq asqgqaqvmn gslgaagrgr gagmpyptpa mqgasssvla
241 etltqvspqm tghaglntaq aggmakmgit gntspfgqpf sqaggqpmga tgvnpqlask
 
301 qsmvnslptf ptdikntsvt nvpnmsqmqt svgivptqai atgptadpek rkliqqqlvl
361 llhahkcqrr eqangevrac slphcrtmkn vlnhmthcqa gkacqvahca ssrqiishwk
421 nctrhdcpvc lplknasdkr nqqtilgspa sgiqntigsv gtgqqnatsl snpnpidpss
481 mqrayaalgl pymnqpqtql qpqvpgqqpa qpqthqqmrt lnplgnnpmn ipaggittdq
541 qppnlisesa lptslgatnp lmndgsnsgn igtlstipta appsstgvrk gwhehvtqdl
601 rshlvhklvq aifptpdpaa lkdrrmenlv ayakkvegdm yesansrdey yhllaekiyk
661 iqkeleekrr srlhkqgilg nqpalpapga qppvipqaqp vrppngplsl pvnrmqvsqg
721 mnsfnpmslg nvqlpqapmg praaspmnhs vqmnsmgsvp gmaispsrmp qppnmmgaht
781 nnmmaqapaq sqflpqnqfp sssgamsvgm gqppaqtgvs qgqvpgaalp nplnmlgpqa
841 sqlpcppvtq splhptpppa staagmpslq httppgmtpp qpaaptqpst pvsssgqtpt
901 ptpgsvpsat qtqstptvqa aaqaqvtpqp qtpvqppsva tpqssqqqpt pvhaqppgtp
961 lsqaaasidn rvptpssvas aetnsqqpgp dvpvlemkte tqaedtepdp geskgeprse
1021 mmeedlqgas qvkeetdiae qksepmevde kkpevkvevk eeeesssngt asqstspsqp
1081 rkkifkpeel rqalmptlea lyrqdpeslp frqpvdpqll gipdyfdivk npmdlstikr
1141 kldtgqyqep wqyvddvwlm fnnawlynrk tsrvykfcsk laevfeqeid pvmqslgycc
1201 grkyefspqt lccygkqlct iprdaayysy qnryhfcekc fteiqgenvt lgddpsqpqt
1261 tiskdqfekk kndtldpepf vdckecgrkm hqicvlhydi iwpsgfvcdn clkktgrprk
1321 enkfsakrlq ttrlgnhled rvnkflrrqn hpeagevfvr vvassdktve vkpgmksrfv
1381 dsgemsesfp yrtkalfafe eidgvdvcff gmhvqeygsd cpppntrrvy isyldsihff
1441 rprclrtavy heiligyley vkklgyvtgh iwacppsegd dyifhchppd qkipkpkrlq
1501 ewykkmldka faeriihdyk difkqatedr ltsakelpyf egdfwpnvle esikeleqee
1561 eerkkeesta asettegsqg dsknakkknn kktnknkssi srankkkpsm pnvsndlsqk
1621 lyatmekhke vffvihlhag pvintlppiv dpdpllscdl mdgrdafltl ardkhwefss
1681 lrrskwstlc mlvelhtqgq drfvytcnec khhvetrwhc tvcedydlci ncyntkshah
1741 kmvkwglgld degssqgepq skspqesrrl siqrciqslv hacqcmanc slpscqkmkr
1801 vvqhtkgckr ktnggcpvck qlialccyha khcqenkcpv pfclnikhkl rqqqiqhrlq
1861 qaqlmrrrma tmntrnvpqq slpsptsapp gtptqqpstp qtpqppaqpq pspvsmspag
1921 fpsvartqpp ttvstgkpts qvpappppaq pppaaveaar qiereaqqqq hlyrvninns
1981 mppgrtgmgt pgsqmapvsl nvprpnqvsg pvmpsmppgq wqqaplpqqq pmpglprpvi
2041 smqaqaavag prmpsvqppr sispsalqdl lrtlkspssp qqqqqvlnil ksnpqlmaaf
2101 ikqrtakyva nqpgmqpqpg lqsqpgmqpq pgmhqqpslq nlnamqagvp rpgvppqqqa
2161 mgglnpqgqa lnimnpghnp nmasmnpqyr emlrrqllqq qqqqqqqqqq qqqqqqgsag
2221 maggmaghgq fqqpqgpggy ppamqqqqrm qqhlplqgss mgqmaaqmgq lgqmgqpglg
2281 adstpniqqa lqqrilqqqq mkqqigspgq pnpmspqqhm lsgqpqashl pgqqiatsls
2341 nqvrspapvq sprpqsqpph sspspriqpq psphhvspqt gsphpglavt massidqghl
2401 gnpeqsamlp qlntpsrsal sselslvgdt tgdtlekfve gl
**Putative function** CREB-binding protein, transcription factor

### Example 2 (Category 1)

**Line ID** - 492
**Phenotype** - Female sterile, few eggs laid, several fully matured eggs in ovarioles
**Annotated *Drosophila* genome genomic segment containing P element insertion site (and map position) -** AE003490 (11B4-14)
**P element insertion site** - 30,773
**Annotated *Drosophila* genome Complete Genome candidate -** CG2028 - CK1 alpha (2 splice variants) 
TAAAGTGCAAGCTGGAAAAGAAAAGCAAAACAAATTCCGGAGAGCAGAAA
GAGAGTTTTTCAAGTGAACGCGTCCAACTGTTTTGAAGCGAAGCGCTTA
GGCGGAGGAGCAGCTAGCCAGGATGGACAAGATGCGGATATTGAAGGAAA
GTCGCCCCGAGATAATCGTCGGTGGCAAATATCGGGTGATCAGGAAGATT
GGAAGCGGATCGTTTGGCGACATTTACCTGGGCATGAGCATCCAGAGCGG
CGAAGAAGTGGCCATCAAGATGGAGAGCGCCCACGCCCGCCATCCGCAGC
TGTTGTACGAGGCCAAGCTGTACCGCATTCTGAGCGGCGGCGTTGGATTC
CCTCGTATACGTCACCATGGCAAGGAAAAGAACTTCAACACCCTGGTCAT
GGACCTGCTGGGACCCTCGCTGGAGGATCTGTTCAATTTCTGTACGCGCC
ATTTCACAATCAAAACGGTTCTGATGCTCGTCGACCAGATGATCGGACGC
TTGGAGTACATCCATCTCAAGTGCTTCATCCATCGCGACATCAAGCCGGA
TAACTTCCTAATGGGCATTGGTCGGCACTGCAATAAGCTGTTCCTGATCG
ATTTCGGTCTGGCCAAGAAGTTCCGCGATCCGCACACGCGCCATCACATC
GTTTACCGCGAGGACAAGAACCTCACCGGCACTGCCCGCTATGCCTCGAT
CAATGCCCATCTGGGCATCGAGCAGTCGCGGCGTGACGACATGGAATCGC
TTGGATACGTGATGATGTACTTCAATCGCGGCGTACTGCCATGGCAAGGC
ATGAAGGCCAACACCAAGCAGCAGAAATACGAGAAGATCTCCGAAAAGAA
GATGTCCACGCCCATCGAGGTCCTCTGCAAGGGCTCGCCGGCCGAGTTCT
CCATGTATCTGAACTATTGTCGTAGCCTGCGCTTCGAGGAGCAGCCAGAT
TACATGTACCTACGTCAATTGTTCCGCATACTGTTCAGAACGCTGAACCA
TCAGTATGACTACATCTACGACTGGACAATGCTGAAGCAGAAGACCCATC
AGGGTCAACCCAATCCAGCTATACTCTTGGAGCAATTGGACAAGGACAAG
GAGAAGCAGAACGGCAAGCCCCTGATCGCGGACTAAGAGCTGCAGCGCAT
TCAGACGAATGGGGGGAGTGCATCAGAGAAGGAGAACGTGGATGCGTGGA
TGTAAATGACGTTGATGTGGGCGAAAGGCCCGGCAAGGAGCGGAGCAAAT
ATGAAACAGACGCAACCGTAAAATTGAGTAACACCAGCGGTCGTCCGAAT
GTTTCTTAATATTAATTTAAATTCAATACTAAACAAATAAGGAACCACAA
ACAAGCAAGCAAC
 
MDKMRILKESRPEIIVGGKYRVIRKIGSGSFGDIYLGMSIQSGEEVAIKM
ESAHARHPQLLYEAKLYRILSGGVGFPRIRHHGKEKNFNTLVMDLLGPSL
EDLFNFCTRHFTIKTVLMLVDQMIGRLEYIHLKCFIHRDIKPDNFLMGIG
RHCNKLFLIDFGLAKKFRDPHTRHHIVYREDKNLTGTARYASINAHLGIE
QSRRDDMESLGYVMMYFNRGVLPWQGMKANTKQQKYEKISEKKMSTPIEV
 
LCKGSPAEFSMYLNYCRSLRFEEQPDYMYLRQLFRILFRTLNHQYDYIYD
WTMLKQKTHQGQPNPAILLEQLDKDKEKQNGKPLIAD
 
TTTGGTTGAACCTATCGGGCCCTATCGATATAAGCAAAAGCATTTTTGCT
GGATCTACCATTTTATTTTAGTTAATAAAATACATATATTTCCTCTCTTT
TTGTTCCGTTTGTGCGCGTACAAAACTAGCTGCGAACTCGTGCAATATTT
CATAAACTGAATGGAAAACAACGATAACGACGAAAGAAAACGAAAACGG
ATCTGCGACGAAATTTTCCCCGTTCCGTTTTTTTCTCCACCAGCAGCA
GAAGCAGCAGAGCAAAAGCAGCGAATATATTTGTAAAAGAGAGCCCCAAC
CTTGAGAAAAAACAACCAGCAGGGCAATAATTAGTTGAATTTATCGTCTG
CTGTTTTTCAAGTGAACGCGTCCAACTGTTTTTGAAGCGAAGCGCTTAGG
CGGAGGAGCAGCTAGCCAGGATGGACAAGATGCGGATATTGAAGGAAAGT
CGCCCCGAGATAATCGTCGGTGGCAAATATCGGGTGATCAGGAAGATTGG
AAGCGGATCGTTTGGCGACATTTACCTGGGCATGAGCATCCAGAGCGGCG
AAGAAGTGGCCATCAAGATGGAGAGCGCCCACGCCCGCCATCCGCAGCTG
TTGTACGAGGCCAAGCTGTACCGCATTCTGAGCGGCGGCGTTGGATTCCC
TCGTATACGTCACCATGGCAAGGAAAAGAACTTCAACACCCTGGTCATGG
ACCTGCTGGGACCCTCGCTGGAGGATCTGTTCAATTTCTGTACGCGCCAT
TTCACAATCAAAACGGTTCTGATGCTCGTCGACCAGATGATCGGACGCTT
GGAGTACATCCATCTCAAGTGCTTCATCCATCGCGACATCAAGCCGGATA
ACTTCCTAATGGGCATTGGTCGGCACTGCAATAAGCTGTTCCTGATCGAT
TTCGGTCTGGCCAAGAAGTTCCGCGATCCGCACACGCGCCATCACATCGT
TTACCGCGAGGACAAGAACCTCACCGGCACTGCCCGCTATGCCTCGATCA
ATGCCCATCTGGGCATCGAGCAGTCGCGGCGTGACGACATGGAATCGCTT
GGATACGTGATGATGTACTTCAATCGCGGCGTACTGCCATGGCAAGGCAT
GAAGGCCAACACCAAGCAGCAGAAATACGAGAAGATCTCCGAAAAGAAGA
TGTCCACGCCCATCGAGGTCCTCTGCAAGGGCTCGCCGGCCGAGTTCTCC
ATGTATCTGAACTATTGTCGTAGCCTGCGCTTCGAGGAGCAGCCAGATTA
CATGTACCTACGTCAATTGTTCCGCATACTGTTCAGAACGCTGAACCATC
AGTATGACTACATCTACGACTGGACAATGCTGAAGCAGAAGACCCATCAG
GGTCAACCCAATCCAGCTATACTCTTGGAGCAATTGGACAAGGACAAGGA
GAAGCAGAACGGCAAGCCCCTGATCGCGGACTAAGAGCTGCAGCGCATTC
AGACGAATGGGGGGAGTGCATCAGAGAAGGAGAACGTGGATGCGTGGATG
TAAATGACGTTGATGTGGGCGAAAGGCCCGGCAAGGAGCGGAGCAAATAT
GAAACAGACGCAACCGTAAAATTGAGTAACACCAGCGGTCGTCCGAATGT
TTCTTAATATTAATTTAAATTCAATACTAAACAAATAAGGAACCACAAAC
AAGCAAGCAAC
 
MDKMRILKESRPEIIVGGKYRVIRKIGSGSFGDIYLGMSIQSGEEVAIKM
ESAHARHPQLLYEAKLYRILSGGVGFPRIRHHGKEKNFNTLVMDLLGPSL
EDLFNFCTRHFTIKTVLMLVDQMIGRLEYIHLKCFIHRDIKPDNFLMGIG
RHCNKLFLIDFGLAKKFRDPHTRHHIVYREDKNLTGTARYASINAHLGIE
QSRRDDMESLGYVMMYFNRGVLPWQGMKANTKQQKYEKISEKKMSTPIEV
LCKGSPAEFSMYLNYCRSLRFEEQPDYMYLRQLFRILFRTLNHQYDYIYD
WTMLKQKTHQGQPNPAILLEQLDKDKEKQNGKPLIAD
**Human homologue of Complete Genome candidate** P48729 Casein kinase I, alpha isoform (cki-alpha) (ck1) 
1 ccgcctccgt gttccgtttc ctgccgccct cctctcgtag ccttgcctag tgtggagccc
61 caggcctccg tcctcttccc agaggtgtcg aggcttggcc ccagcctcca tcttcgtctc
121 tcaggatggc gagtagcagc ggctccaagg ctgaattcat tgtcggtggg aaatataaac
181 tggtacggaa gatcgggtct ggctccttcg gggacatcta tttggcgatc aacatcacca
241 acggcgagga agtggcactg aagctagaat ctcagaaggc caggcatccc cagttgctgt
301 acgagagcaa gctctataag attcttcaag gtggggttgg catcccccac atacggtggt
361 atggtcagga aaaagactac aatgtactag tcatggatct tctgggacct agcctcgaag
421 acctcttcaa tttctgttca agaaggttca caatgaaaac tgtacttatg ttagctgacc
481 agatgatcag tagaattgaa tatgtgcata caaagaattt tatacacaga gacattaaac
541 cagataactt cctaatgggt attgggcgtc actgtaataa gttattcctt attgattttg
601 gtttggccaa aaagtacaga gacaacagga caaggcaaca cataccatac agagaagata
661 aaaacctcac tggcactgcc cgatatgcta gcatcaatgc acatcttggt attgagcaga
721 gtcgccgaga tgacatggaa tcattaggat atgttttgat gtattttaat agaaccagcc
781 tgccatggca agggctaaag gctgcaacaa agaaacaaaa atatgaaaag attagtgaaa
841 agaagatgtc cacgcctgtt gaagttttat gtaaggggtt tcctgcagaa tttgcgatgt
901 acttaaacta ttgtcgtggg ctacgctttg aggaagcccc agattacatg tatctgaggc
961 agctattccg cattcttttc aggaccctga accatcaata tgactacaca tttgattgga
1021 caatgttaaa gcagaaagca gcacagcagg cagcctcttc aagtgggcag ggtcagcagg
1081 cccaaacccc cacaggcaag caaactgaca aatccaagag taacatgaaa ggtttctaat
1141 ttctaagcat gaattgagga acagaagaag cagacgagat gatcggagca gcatttgttt
1201 ctccccaaat ctagaaattt tagttcatat gtacactagc cagtggttgt ggacaacca
 
1 masssgskae fivggkyklv rkigsgsfgd iylainitng eevalklesq karhpqllye
61 sklykilqgg vgiphirwyg qekdynvlvm dllgpsledl fnfcsrrftm ktvlmladqm
121 isrieyvhtk nfihrdikpd nflmgigrhc nklflidfgl akkyrdnrtr qhipyredkn
181 ltgtaryasi nahlgieqsr rddmeslgyv lmyfnrtslp wqglkaatkk qkyekisekk
241 mstpvevlck gfpaefamyl nycrglrfee apdymylrql frilfrtlnh qydytfdwtm
301 lkqkaaqqaa sssgqgqqaq tptgkqtdks ksnmkgf
**Putative function** Casein kinase

### Example 2A (Category 1)

**Line ID** - ccr-a2
**Phenotype** - Female semi-sterile, Lays eggs, but arrest before cortical migration
**Annotated *Drosophila* genome genomic segment containing P element insertion site (and map position) -** AE003435 (5C6)
**P element insertion site sequence** 
GATCAGACGATATTCGGACTCCAAGCAGAGCACTTTGAAGGTGAGTTCGCCG
GAAACCAGGCAAAGCGCCATTCGCCATTCAGGCTGCGCAACTGTTGGGAAGG
GCGATCGGTGCGGGCCTCTTCGCTATTACGCCAGCTGGCGAAAGGGGGATGTG
CTGCAAGGCGATTAAGTTGGGTAACGCCAGGGTTTTCCCAGTCACGACGTTGT
AAAACGACGGCCAGTGCCAAGCTCTGCTGCTCTAAACGACGCATTTCGTACTC
CAAAGTACGAATTTTTTCCCTCAAGCTCTTATTTTCATTAAACAATGAACAGGA
CCTAACGCCACAGTA
**Annotated *Drosophila* genome Complete Genome candidate -** CG3011 -glycine hydroxymethyltransferase 
GTAAATGTTGTTTACCAACGTAACGCGTGTTTCGCTTCGTTGTATTTTC
GGTGTCGAATATTTTGGATGCTGGCCAAGAGATAGCGCAGCGATCGGGTC
GGAACTCTTGGGCGGACTTATCACTGGGTCGGTCAGGGGTCACGGTTAT
CGTTATCGCTTATCAGCCAGCGGCGGCGTCATCTCAGCGCCGGCGACTCT
TCTCACTTTGCGGCAGTTCCGATTCGAACGCAGCCGTTTACAAAGACATG
CAGCGGGCGCGCTCTACACTGACACAAAAGCTTCGGTTTTGCCTTAGTCG
GGACCTGAACACCAAAGTTGGCAACCCGGTTAACTTCGAGACTGGAAAGC
TTAGCGGAGCTTTAACTCGCATCGCCGCCAAAAAACAACCATCACCAACG
CCATTCTTACCGGCGATCAGACGATATTCGGACTCCAAGCAGAGCACTTT
GAAGAATATGGCCGATCAGAAACTGCTGCAAACCCCGCTGGCACAGGGCG
ATCCGGAGCTGGCCGAGCTGATCAAGAAGGAGAAGGAGCGCCAGCGCGAA
GGACTCGAGATGATCGCCAGTGAGAACTTCACCTCGGTGGCGGTTCTCGA
GAGCCTGAGCTCCTGCCTGACCAACAAGTACTCCGAGGGATATCCCGGCA
AGAGGTACTACGGTGGCAACGAGTACATCGACCGCATAGAGCTGCTCGCC
CAGCAACGCGGACGCGAGCTGTTCAACCTGGACGATGAGAAGTGGGGCGT
TAATGTGCAGCCTTATTCCGGATCCCCGGCCAATCTGGCTGTCTACACGG
GCGTCTGCCGGCCCCACGATCGCATCATGGGCCTGGATCTGCCCGATGGC
GGTCACTTGACGCACGGTTTCTTCACGCCCACCAAGAAGATATCGGCCAC
ATCGATCTTCTTCGAGAGCATGCCGTACAAAGTGAACCCGGAGACGGGCA
TCATCGATTACGATAAGTTGGCGGAGGCGGCGAAGAATTTCCGGCCGCAG
ATCATCATTGCTGGCATATCGTGCTACTCCCGTCTGCTGGACTATGCGCG
TTTCCGACAGATTTGCGATGATGTGGGCGCCTACCTGATGGCCGACATGG
CCCATGTGGCGGGCATTGTGGCCGCGGGATTGATACCATCGCCGTTCGAA
TGGGCCGACATTGTGACCACCACCACGCACAAGACACTGCGAGGTCCGCG
CGCCGGCGTGATCTTCTTCCGCAAGGGCGTGCGCAGCACCAAGGCCAATG
GAGACAAGGTACTCTACGATCTGGAGGAGCGCATCAACCAGGCGGTGTTT
CCATCACTCCAGGGTGGTCCGCACAACAACGCCGTGGCTGGCATTGCCAC
CGCCTTCAAGCAGGCCAAGAGTCCCGAATTCAAGGCCTACCAGACGCAGG
TGCTCAAGAATGCCAAGGCCCTGTGCGATGGCCTCATTTCGCGAGGCTAT
 
CAGGTGGCCACCGGCGGCACCGACGTCCATTTGGTGCTGGTCGATGTGCG
TAAGGCTGGCCTGACCGGCGCCAAGGCCGAGTACATCCTCGAGGAGGTGG
GCATCGCGTGCAACAAGAACACTGTGCCCGGCGACAAGTCCGCCATGAAT
CCCTCCGGCATCCGGCTGGGCACACCGGCCCTGACCACTCGCGGCCTTGC
CGAGCAGGACATCGAGCAGGTGGTGGCCTTCATCGATGCTGCCCTAAAGG
TTGGCGTCCAGGCAGCCAAGCTGGCCGGCAGTCCCAAGATAACCGATTAC
CACAAGACGCTGGCCGAGAATGTGGAGCTCAAGGCCCAGGTGGACGAGAT
CCGCAAGAATGTGGCCCAGTTCAGCAGGAAATTCCCGCTGCCCGGCCTGG
AGACCCTGTAG
 
MQRARSTLTQKLRFCLSRDLNTKVGNPVNFETGKLSGALTRIAAKKQPSP
TPFLPAIRRYSDSKQSTLKNMADQKLLQTPLAQGDPELAELIKKEKERQR
EGLEMIASENFTSVAVLESLSSCLTNKYSEGYPGKRYYGGNEYIDRIELL
AQQRGRELFNLDDEKWGVNVQPYSGSPANLAVYTGVCRPHDRIMGLDLPD
GGHLTHGFFTPTKKISATSIFFESMPYKVNPETGIIDYDKLAEAAKNFRP
QIIIAGISCYSRLLDYARFRQICDDVGAYLMADMAHVAGIVAAGLIPSPF
EWADIVTTTTHKTLRGPRAGVIFFRKGVRSTKANGDKVLYDLEERINQAV
FPSLQGGPHNNAVAGIATAFKQAKSPEFKAYQTQVLKNAKALCDGLISRG
YQVATGGTDVHLVLVDVRKAGLTGAKAEYILEEVGIACNKNTVPGDKSAM
NPSGIRLGTPALTTRGLAEQDIEQVVAFIDAALKVGVQAAKLAGSPKITD
YHKTLAENVELKAQVDEIRKNVAQFSRKFPLPGLETL
**Human homologue of Complete Genome candidate** AAA63258 - serine hydroxymethyltransferase 
1 ggcacgaggc ctgcgacttc cgagttgcga tgctgtactt ctctttgttt tgggcggctc
61 ggcctctgca gagatgtggg cagctggtca ggatggccat tcgggctcag cacagcaacg
121 cagcccagac tcagactggg gaagcaaaca ggggctggac aggccaggag agcctgtcgg
181 acagtgatcc tgagatgtgg gagttgctgc agagggagaa ggacaggcag tgtcgtggcc
241 tggagctcat tgcctcagag aacttctgca gccgagctgc gctggaggcc ctggggtcct
301 gtctgaacaa caagtactcg gagggttatc ctggcaagag atactatggg ggagcagagg
361 tggtggatga aattgagctg ctgtgccagc gccgggcctt ggaagccttt gacctggatc
421 ctgcacagtg gggagtcaat gtccagccct actccgggtc cccagccaac ctggccgtct
481 acacagccct tctgcaacct cacgaccgga tcatggggct ggacctgccc gatgggggcc
541 agtgatctca cccacggcta catgtctgac gtcaagcgga tatcagccac gtccatcttc
601 ttcgagtcta tgccctataa gctcaacccc aaaactggcc tcattgacta caaccagctg
661 gcactgactg ctcgactttt ccggccacgg ctcatcatag ctggcaccag cgcctatgct
721 cgcctcattg actacgcccg catgagagag gtgtgtgatg aagtcaaagc acacctgctg
781 gcagacatgg cccacatcag tggcctggtg gctgccaagg tgattccctc gcctttcaag
841 cacgcggaca tcgtcaccac cactactcac aagactcttc gaggggccag gtcagggctc
901 atcttctacc ggaaaggggt gaaggctgtg gaccccaaga ctggccggga gatcccttac
961 acatttgagg accgaatcaa ctttgccgtg ttcccatccc tgcagggggg cccccacaat
1021 catgccattg ctgcagtagc tgtggcccta aagcaggcct gcacccccat gttccgggag
1081 tactccctgc aggttctgaa gaatgctcgg gccatggcag atgccctgct agagcgaggc
1141 tactcactgg tatcaggtgg tactgacaac cacctggtgc tggtggacct gcggcccaag
1201 ggcctggatg gagctcgggc tgagcgggtg ctagagcttg tatccatcac tgccaacaag
1261 aacacctgtc ctggagaccg aagtgccatc acaccgggcg gcctgcggct tggggcccca
 
1321 gccttaactt ctcgacagtt ccgtgaggat gacttccgga gagttgtgga ctttatagat
1381 gaaggggtca acattggctt agaggtgaag agcaagactg ccaagctcca ggatttcaaa
1441 tccttcctgc ttaaggactc agaaacaagt cagcgtctgg ccaacctcag gcaacgggtg
1501 gagcagtttg ccagggcctt ccccatgcct ggttttgatg agcattgaag gcacctggga
1561 aatgaggccc acagactcaa agttactctc cttcccccta cctgggccag tgaaatagaa
1621 agcctttcta ttttttggtg cgggagggaa gacctctcac ttagggcaag agccaggtat
1681 agtctccctt cccagaattt gtaactgaga agatcttttc tttttccttt ttttggtaac
1741 aagacttaga aggagggccc aggcactttc tgtttgaacc cctgtcatga tcacagtgtc
1801 agagacgcgt cctctttctt ggggaagttg aggagtgccc ttcagagcca gtagcaggca
1861 ggggtgggta ggcaccctcc ttcctgtttt tatctaataa aatgctaacc tgcaaaaaaa
1921 aaaaaaaaaa a

1 aaqtqtgean rgwtgqesls dsdpemwell qrekdrqcrg leliasenfc sraalealgs
61 clnnkysegy pgkryyggae vvdeiellcq rraleafdld paqwgvnvqp ysgspanlav
121 ytallqphdr imgldlpdgg hlthgymsdv krisatsiff esmpyklnpk tglidynqla
181 ltarlfrprl iiagtsayar lidyarmrev cdevkahlla dmahisglva akvipspfkh
241 adivtttthk tlrgarsgli fyrkgvkavd pktgreilyt fedrinfavf pslqggphnh
301 aiaavavalk qactpmfrey slqvlknara madallergy slvsggtdnh lvlvdlrpkg
361 ldgaraervl elvsitankn tcpgdrsait pgglrlgapa ltsrqfredd frrvvdfide
421 gvniglevks ktaklqdfks fllkdsetsq rlanlrqrve qfarafpmpg fdeh
**Putative function** hydroxymethyltransferase

### Example 2B (Category 1)

**Line ID** - ewv-b
**Phenotype** - Female sterile, No eggs laid. Fully mature eggs, but "retained eggs" phenotype. Also has a mitotic phenotype: higher mitotic index, uneven chromosome staining, tangled and badly defined chromosomes with frequent bridges
**Annotated *Drosophila* genome genomic segment containing P element insertion site (and map position) -** AE003486 (10D4-6)
**P element insertion site sequence** 
GACAGGAGCAGCTCGGAACGGACAGGAAAAGCAGGAGACTAAACAGTAAGC
AATAAATTGATTTGGCGTATAGTAGCTTACACCAAAGTACATATATTGCCGCA
TATATAGCCAGCCGGTCACTTGCGGATCAGCCAACGTCCTGGGCCCCAAGGCG
ATAGATACCACGATAAGGAGATACAGCGATACCACCAATCATTAGCAGGCGA
CAACGACACATCCGCATCCGCAGAAGATGTCCAACGGCAAGGCGACGGTCTC
GTTCTTCGAGACCGGGAGCACCAAACAGTTCGAGTACTGCTACCAGCTCTATC
CCCAGGTTCTTAAGCTAAAGGCCGAGAAGCGCAACTGTTGGGAAGGGCGATC
GGTGCGGGCCTCTTCGCTATTACGCCAGCTGGCGAAAGGGGGATGTGCTGCAA
GGCGATTAAGTTGGGTAACGCCAGGGTTTTCCCAGNCACGACGNTGNAAAAC
GACGGNCANNGCCANNCTNTGNTGNTNTAAACNACNCATT
**Annotated *Drosophila* genome Complete Genome candidate -** CG2446 (2 transcripts) - encodes a novel protein which may be a glycosylation/membrane protein 
AGATAGAACGACAACTCCTGTTCCCGGTTCGTCGTCGTTCGTCATTCCCA
TATTCGCTTCTCGTATTCCCTCCCATTCCCATTCGCAATCCCAATTCCCA
ATTCCCGTCACACGAGTTAGCAGCACATCGCACAGCTGCATCGCTCCGCT
CCGATCCTTTTTAATTTTTGTTGTGCCTTCGGTGGCGTGCTCATTTCGA
GAACAGAGTAACCCCTTTTTATTTGTCAGTTGTCAACGGCGCCCCTGCAG
GCAGAAAGCAGAAACTGAAACAGCAGAGGAAGAAGAAGCAGCACAGC
ACGGGCACAGCACGAAGCACGCAGCACAGCACAAGCACAGAGGCGAAGCG
AAGCAAAGCAAAGCAGAGGCAACACAGAAAAACAGCAAAGCATTGGAGTA
GTTGTTTGGATGTGGACGGAAAGGAAGACTGGCGGCGACTAACTAAAAGC
AGTACGTTGACAGGAGCAGCTCGGAACGGACAGGAAAAGCAGGAGACTAA
ACACCAGCCGGTCACTTGCGGATCAGCCAACGTCCTGGGCCCCAAGGCGA
TAGATACCACGATAAGGAGATACAGCGATACCACCAATCATTAGCAGGCG
ACAACGACACATCCGCATCCGCAGAAGATGTCCAACGGCAAGGCGACGGT
CTCGTTCTTCGAGACCGGGAGCACCAAACAGTTCGAGTACTGCTACCAGC
TCTATCCCCAGGTTCTTAAGCTAAAGGCCGAGAAGCGCTGCAAGAAGCCG
CAAGAGCTGATCCGCCTGGATCAGTGGTATCAGAATGAACTGCCCAAATT
GATTAAGGCACGCGGCAAGGACGCGCATATGGTATACGATGAGCTCGTCC
AGTCGATGAAGTGGAAGCAGTCGCGCGGCAAATTCTATCCGCAGCTATCC
TACCTGGTCAAGGTCAACACACCGCGCGCCGTCATCCAGGAGACAAAGAA
GGCCTTCCGCAAGCTGCCCAATCTGGAGCAGGCGATCACAGCTTTATCGA
ACCTCAAGGGCGTTGGCACCACAATGGCCAGTGCACTGCTGGCAGCCGCA
GCTCCCGATTCGGCACCATTCATGGCCGACGAGTGCCTGATGGCCATACC
 
AGAGATCGAGGGCATCGATTACACCACCAAGGAGTACCTCAACTTCGTCA
ATCACATTCAGGCCACCGTGGAGCGCCTCAATGCGGAGGTGGGCGGGGAT
ACGCCGCACTGGTCGCCTCATCGCGTGGAGCTGGCCCTCTGGTCACACTA
TGTGGCCAATGATCTCAGTCCCGAGATGCTCGACGATATGCCGCCGCCTG
GATCCGGCGCCTCCACTGGCACCGGTTCACTCAGCACAAACGGCAACAGC
AGCAAGGTGCTCGATGGCGACGATACCAACGATGGTGTGGGTGTTGATTT
GGACGACGAAAGCCAAGGAGCAGGCGGTCGCAACACTGCTACAGAATCGG
AGACAGAGAATGAGAACACCAACCCGGCTGCTCTGACGCCTCTACAGTCG
GGCGAGGCCAAGAACAACGCAGCTGCCGTTGGCGCCGCCCTGCAGGACGG
TGACTCCAACTTTGTTTCGAACGATTCCACCTCCCAGGAGCCGATCATCG
ATGACAACGATGGCACCACACAGACAACGGCCACCACTTCCACAGAGGAC
GGTGAGCCCATCGCCCTAGACATTGGCATTGGCATCGGTTCGAGTGGAAC
ACCGCTCGCCTCGGACTCTGAAAGCAATCAGGAGGCGCCGCCCAAGACCA
ACAGCCTGCCCATCCTGACTCCCACACAGCACTCGAGCCAGAATCAGAAT
CAAAAGCAGTCGCCGAGCCAGCCCCACAAAACTAACAATTCGATCACCAA
CAACGGTCAGCCTGCTCCTTTGGCAGAAGAGGAAGCGGTTACAGCAGCAC
CACAGCCAGCCAGCAAAGCGACTGCAGCACCAGCCAATGGAAATGGTAAC
GGGAACGGCGTCCTGGGCGACGAGGATGAGGATGAGGCGGAGGACGAGGA
GGAAGATGAGCTGGACGAGGAGGAGGATAATGAGGCGGAGCTAGAGGCTG
ACGAGAGCAATAGCAGCAACGGCATTGTGAGGGACAGTAAACTGCAGCAG
CTGGCGGCGAACAAGGCGGTGGATGCGGTTTCACCGGTAGCAGCGGGTGC
AGACTCGGCACCAGCCATTGGACAGAAGCGTACTGCCCTGCACTGCGATA
TGGAGCTGAAGAACGCCGGCGGAGTGGGTGTGGGCGTGGGGGAGAAGTCA
CCGGATCTAAAGAAACTGCGCAGCGAATGA
 
MSNGKATVSFFETGSTKQFEYCYQLYPQVLKLKAEKRCKKPQELIRLDQW
YQNELPKLIKARGKDAHMVYDELVQSMKWKQSRGKFYPQLSYLVKVNTPR
AVIQETKKAFRKLPNLEQAITALSNLKGVGTTMASALLAAAAPDSAPFMA
DECLMAIPEIEGIDYTTKEYLNFVNHIQATVERLNAEVGGDTPHWSPHRV
ELALWSHYVANDLSPEMLDDMPPPGSGASTGTGSLSTNGNSSKVLDGDDT
NDGVGVDLDDESQGAGGRNTATESETENENTNPAALTPLQSGEAKNNAAA
VGAALQDGDSNFVSNDSTSQEPIIDDNDGTTQTTATTSTEDGEPIALDIG
IGIGSSGTPLASDSESNQEAPPKTNSLPILTPTQHSSQNQNQKQSPSQPH
KTNNSITNNGQPAPLAEEEAVTAAPQPASKATAAPANGNGNGNGVLGDED
EDEAEDEEEDELDEEEDNEAELEADESNSSNGIVRDSKLQQLAANKAVDA
VSPVAAGADSAPAIGQKRTALHCDMELKNAGGVGVGVGEKSPDLKKLRSE
 
GCCTGTCAGTTTGACTGTGTGAGTGCATGGCGGACTAAAAAGAACCCGAC
GACAGCACTGTAAAAATTCGATTTGTGTGCTGTGCAAACGGCGGCGGAAG
CGAGCAGATTTTTGGCAAATAGTGAGCGATTATCGGATTGAGTAAATACA
ACAAACAACAGAGACACGGCCGCAGCAGCAGCAGCATTAACACAGTACGT
TGACAGGAGCAGCTCGGAACGGACAGGAAAAGCAGGAGACTAAACACCAG
CCGGTCACTTGCGGATCAGCCAACGTCCTGGGCCCCAAGGCGATAGATAC
CACGATAAGGAGATACAGCGATACCACCAATCATTAGCAGGCGACAACGA
CACATCCGCATCCGCAGAAGATGTCCAACGGCAAGGCGACGGTCTCGTTC
TTCGAGACCGGGAGCACCAAACAGTTCGAGTACTGCTACCAGCTCTATCC
CCAGGTTCTTAAGCTAAAGGCCGAGAAGCGCTGCAAGAAGCCGCAAGAGC
 
TGATCCGCCTGGATCAGTGGTATCAGAATGAACTGCCCAAATTGATTAAG
GCACGCGGCAAGGACGCGCATATGGTATACGATGAGCTCGTCCAGTCGAT
GAAGTGGAAGCAGTCGCGCGGCAAATTCTATCCGCAGCTATCCTACCTGG
TCAAGGTCAACACACCGCGCGCCGTCATCCAGGAGACAAAGAAGGCCTTC
CGCAAGCTGCCCAATCTGGAGCAGGCGATCACAGCTTTATCGAACCTCAA
GGGCGTTGGCACCACAATGGCCAGTGCACTGCTGGCAGCCGCAGCTCCCG
ATTCGGCACCATTCATGGCCGACGAGTGCCTGATGGCCATACCAGAGATC
GAGGGCATCGATTACACCACCAAGGAGTACCTCAACTTCGTCAATCACAT
TCAGGCCACCGTGGAGCGCCTCAATGCGGAGGTGGGCGGGGATACGCCGC
ACTGGTCGCCTCATCGCGTGGAGCTGGCCCTCTGGTCACACTATGTGGCC
AATGATCTCAGTCCCGAGATGCTCGACGATATGCCGCCGCCTGGATCCGG
CGCCTCCACTGGCACCGGTTCACTCAGCACAAACGGCAACAGCAGCAAGG
TGCTCGATGGCGACGATACCAACGATGGTGTGGGTGTTGATTTGGACGAC
GAAAGCCAAGGAGCAGGCGGTCGCAACACTGCTACAGAATCGGAGACAGA
GAATGAGAACACCAACCCGGCTGCTCTGACGCCTCTACAGTCGGGCGAGG
CCAAGAACAACGCAGCTGCCGTTGGCGCCGCCCTGCAGGACGGTGACTCC
AACTTTGTTTCGAACGATTCCACCTCCCAGGAGCCGATCATCGATGACAA
CGATGGCACCACACAGACAACGGCCACCACTTCCACAGAGGACGGTGAGC
CCATCGCCCTAGACATTGGCATTGGCATCGGTTCGAGTGGAACACCGCTC
GCCTCGGACTCTGAAAGCAATCAGGAGGCGCCGCCCAAGACCAACAGCCT
GCCCATCCTGACTCCCACACAGCACTCGAGCCAGAATCAGAATCAAAAGC
AGTCGCCGAGCCAGCCCCACAAAACTAACAATTCGATCACCAACAACGGT
CAGCCTGCTCCTTTGGCAGAAGAGGAAGCGGTTACAGCAGCACCACAGCC
AGCCAGCAAAGCGACTGCAGCACCAGCCAATGGAAATGGTAACGGGAACG
GCGTCCTGGGCGACGAGGATGAGGATGAGGCGGAGGACGAGGAGGAAGAT
GAGCTGGACGAGGAGGAGGATAATGAGGCGGAGCTAGAGGCTGACGAGAG
CAATAGCAGCAACGGCATTGTGAGGGACAGTAAACTGCAGCAGCTGGCGG
CGAACAAGGCGGTGGATGCGGTTTCACCGGTAGCAGCGGGTGCAGACTCG
GCACCAGCCATTGGACAGAAGCGTACTGCCCTGCACTGCGATATGGAGCT
GAAGAACGCCGGCGGAGTGGGTGTGGGCGTGGGGGAGAAGTCACCGGATC
TAAAGAAACTGCGCAGCGAATGA
 
MSNGKATVSFFETGSTKQFEYCYQLYPQVLKLKAEKRCKKPQELIRLDQW
YQNELPKLIKARGKDAHMVYDELVQSMKWKQSRGKFYPQLSYLVKVNTPR
AVIQETKKAFRKLPNLEQAITALSNLKGVGTTMASALLAAAAPDSAPFMA
DECLMAIPEIEGIDYTTKEYLNFVNHIQATVERLNAEVGGDTPHWSPHRV
ELALWSHYVANDLSPEMLDDMPPPGSGASTGTGSLSTNGNSSKVLDGDDT
NDGVGVDLDDESQGAGGRNTATESETENENTNPAALTPLQSGEAKNNAAA
VGAALQDGDSNFVSNDSTSQEPIIDDNDGTTQTTATTSTEDGEPIALDIG
IGIGSSGTPLASDSESNQEAPPKTNSLPILTPTQHSSQNQNQKQSPSQPH
KTNNSITNNGQPAPLAEEEAVTAAPQPASKATAAPANGNGNGNGVLGDED
EDEAEDEEEDELDEEEDNEAELEADESNSSNGIVRDSKLQQLAANKAVDA
VSPVAAGADSAPAIGQKRTALHCDMELKNAGGVGVGVGEKSPDLKKLRSE
**Human homologue of Complete Genome candidate** CG2446 - none
**Putative function** glycosylation/membrane protein

### Example 2C (Category 1)

**Line ID** - fs(l)06
**Phenotype** - Female sterile (semi-sterile), 2-3 fully matured eggs seen in each of the ovarioles
**Annotated *Drosophila* genome genomic segment containing P element insertion site (and map position)** - AE003449 (9B6-7)
**P element insertion site sequence** 
CTNCATGNTGNAGGAGACAAGGCGTTCTATATTATATAGNNGATTTTNNTGTA
TATAAAGGAAGANCTGNGCTAANGNAANAGGCATCTCGATGANTTTNATAAT
NAGGGCAANTGGTANNAANGGTTTATGCCAAAGTATTACACACCAGGGNTGG
GCACAACAGATCTTAACTNANNATAGGNNATTGGNATAANCTTAAATTTGTAA
GATTNTGNAATAATATAGTAGAGANNNTCAATACGCATTANTAATNGTGACG
ATCCCNAGCATAAACTCAAAAAAANCTTATANTTTTATAAAGGCNANNCCNN
ACTAANNAATTAAANGAANNNCNGNCGCCNCNAAANGATGATTGNGCTATAT
AANNANANNATTGATNGAGGCACTTATATTATTATAATTAAAACACTTAATTA
TTNTGTGTGAAATGATTGCACTNNNNATTGGGCNAGAGCCTNNNNCGTATTGA
NANNNNNNNATTTNGGCTNNANCTGTAAATATCNTACAAACTCGTNATTGCTA
AATAACTTTTGTATNCCCCNCTGGTCACTCTGACTTAAACGTNNTTCGNNAAA
ACAGCGGCTGATCACTGANGTTTTCTCCCGNNTTTCGCTNTCAANCCGAANTA
NAAACAGGNGAANNTCCCNGATAATTTGNGGNNTANCCCACTGATCACAGNG
CCCNNGGATNNNCAAGGAANNGCGATCGAAACCCGNCCTGGNGNAACACNN
TTTCCC
**Annotated *Drosophila* genome Complete Genome candidate -** CG2968- hydrogen transporting ATP synthase 
CAAAAACAGCGGCTGATCACTGAAGTTTTCTCGTGTTTTCGCTATCAAA
CCGAAATAAAAACAGCCCAAAATGTCCTTCGTTAAGAACGCCCGTTTGCT
GGCCGCCCGCGGCGCTCGCTTGGCCCAGAACCGCAGCTACTCGGATGAGA
TGAAGCTGACCTTCGCCGCCGCCAACAAAACCTTCTACGATGCCGCTGTG
GTGCGCCAAATCGATGTGCCTTCCTTCTCGGGATCCTTCGGCATCCTGGC
CAAGCACGTGCCCACTCTGGCTGTCCTGAAGCCCGGCGTTGTCCAGGTGG
TGGAAAACGATGGCAAGACCCTCAAGTTCTTCGTCTCCAGCGGTTCCGTC
ACCGTCAACGAGGATTCCTCCGTTCAGGTTCTGGCCGAGGAGGCCCACAA
CATCGAGGACATCGATGCCAATGAGGCGCGCCAGCTGCTCGCGAAATACC
AGTCACAGCTTAGCTCCGCTGGCGACGACAAGGCCAAGGCCCAGGCTGCC
ATTGCCGTGGAGGTCGCCGAAGCGTTAGTCAAGGCTGCCGAATAGACGTA
ATCACCACACAACCGCCACCAATAAACCACAATCGATGCTTTGTGTCTGA
AATAAATAAAAAACATAACGATCACCTTAAAAAGCCAGAGAGTTATGAAA
CAATAAAAAAGCGA
 
MSFVKNARLLAARGARLAQNRSYSDEMKLTFAAANKTFYDAAVVRQIDVP
SFSGSFGILAKHVPTLAVLKPGVVQVVENDGKTLKFFVSSGSVTVNEDSS
VQVLAEEAHNIEDIDANEARQLLAKYQSQLSSAGDDKAKAQAAIAVEVAE
ALVKAAE
**Human homologue of Complete Genome candidate**
   CAA45016 - H(+)-transporting ATP synthase, delta-subunit of the human mitochondrial ATP synthase complex 
1 gtcctcctcg ccctccaggc cgcccgcgcc gcgccggagt ccgctgtccg ccagctaccc
61 gcttcctgcc gcccgccgct gccatgctgc ccgccgcgct gctccgccgc ccgggacttg
121 gccgcctcgt ccgccacgcc cgtgcctatg ccgaggccgc cgccgccccg gctgccgcct
181 ctggccccaa ccagatgtcc ttcaccttcg cctctcccac gcaggtgttc ttcaacggtg
241 ccaacgtccg gcaggtggac gtgcccacgc tgaccggagc cttcggcatc ctggcggccc
301 acgtgcccac gctgcaggtc ctgcggccgg ggctggtcgt ggtgcatgca gaggacggca
361 ccacctccaa atactttgtg agcagcggtt ccatcgcagt gaacgccgac tcttcggtgc
421 agttgttggc cgaagaggcc gtgacgctgg acatgttgga cctgggggca gccaaggcaa
481 acttggagaa ggcccaggcg gagctggtgg ggacagctga cgaggccacg cgggcagaga
541 tccagatccg aatcgaggcc aacgaggccc tggtgaaggc cctggagtag gcggtgcgta
601 cccggtgtcc cgaggcccgg ccaggggctg ggcagggatg ccaggtgggc ccagccagct
661 cctggggtcc cggccacctg gggaagccgc gcctgccaag gaggccacca gagggcagtg
721 caggcttctg cctgggcccc aggccctgcc tgtgttgaaa gctctgggga ctgggccagg
781 gaagctcctc ctcagctttg agctgtggct gccacccatg gggctctcct tccgcctctc
841 aagatccccc cagcctgacg ggccgcttac catcccctct gccctgcaga gccagccgcc
901 aaggttgacc tcagcttcgg agccacctct ggatgaactg cccccagccc ccgccccatt
961 aaagacccgg aagcctgaaa aaaaaaaaaa aaaa
 
1 mlpaallrrp glgrlvrhar ayaeaaaapa aasgpnqmsf tfasptqvff nganvrqvdv
61 ptltgafgil aahvptlqvl rpglvvvhae dgttskyfvs sgsiavnads svqllaeeav
121 tldmldlgaa kanlekaqae lvgtadeatr aeiqiriean ealvkale
**Putative function** hydrogen transporting ATP synthase

### CATEGORY 2 - MALE STERILES

### Example 3 (Category 2)

**Line ID**- 167
**Phenotype** - lethal phase pharate adult, cytokinesis defect. Some onion stage cysts with large nebenkerns
**Annotated *Drosophila* genome genomic segment containing P element insertion site (and map position)** - AE003428 (3F4-5)
**P element insertion site** - 293,654
**Annotated *Drosophila* genome Complete Genome candidate -** CG2829- BcDNA:GH07910 tousled kinase (2 splice variants) 
AGTTTCATTCGGGGATGCTTGGCCTATCGCAAGGAGGATCGCATGGATGT
GTTCGCACTGGCCAGGCACGAGTACATTCAGCCACCGATACCGAAACATG
GGCGCGGTTCGCTCAATCAGCAACAGCAGGCGCAACAACAGCAGCAGCAA
CAACAGCAACAGCAGCAGCAACAGTCGTCGACGTCACAGGCCAATTCTAC
AGGCCAGACATCTTTCTCTGCCCACATGTTTGGCAATATGAATCAGTCGA
GTTCGTCCTAGATGAGAGCGACTGCAAAAAATCGGAATAAACACGGTTA
TAATATATAAGTACAAATAAACCATATATATGTGTTTATGTTATGTATAT
ATACATAAAGGAAAATAACAAGGCAAATGTGAAAATTAGTGCAAACTGAA
CGAAAAGACAAAAATAAAACAAAAGGAAACCCAAATGTGATAATATTGTA
ATATAATGTGAAAAGCAAAACACACACAAATACACAACTCACGCACTTAG
CCACGTATGTGTGTGCAGAAAAATATGCGGCGCTTAAAAAAGATGTCCCC
CGGCGCCCATTTGCAGATGTCCCCGCAGAACACTTCGTCCCTAAGTCAAC
ACCATCCACATCAACAGCAACAGTTACAACCCCCACAGCAGCAACAACAG
CATTTCCCTAACCATCACAGCGCCCAGCAACAGTCGCAGCAGCAGCAGCA
ACAGGAGCAACAGAATCCCCAGCAGCAGGCGCAACAGCAGCAGCAGATAC
TCCCACATCAACATTTGCAGCACCTGCACAAGCATCCGCATCAGCTGCAA
CTGCATCAGCAGCAGCAACAACAACTCCACCAGCAACAGCAGCAACACTT
CCACCAGCAGTCGCTGCAAGGGCTGCATCAGGGTAGCAGCAATCCGGATT
CGAATATGAGCACTGGCTCCTCGCATAGCGAGAAGGATGTCAATGATATG
CTGAGTGGCGGTGCAGCAACGCCAGGAGCTGCAGCAGCAGCGATTCAACA
GCAACATCCCGCCTTTGCGCCCACACTGGGAATGCAGCAACCACCGCCGC
CCCCACCTCAACACTCCAATAATGGAGGCGAGATGGGCTACTTGTCGGCA
GGCACGACCACGACGACGTCGGTGTTAACGGTAGGCAAGCCTCGGACGCC
AGCGGAGCGGAAACGGAAGCGAAAAATGCCTCCATGTGCCACTAGTGCGG
ATGAGGCGGGGAGTGGCGGTGGCTCTGGCGGAGCAGGAGCAACCGTTGTT
AACAACAGCAGCCTGAAGGGCAAATCATTGGCCTTTCGTGATATGCCCAA
GGTAAACATGAGCCTGAATCTGGGCGATCGTCTGGGAGGATCTGCAGGAA
GCGGAGTAGGAGCCGGTGGCGCCGGAAGCGGGGGAGGTGGCGCTGGTTCC
GGTTCTGGAAGCGGTGGCGGCAAAAGCGCCCGCCTGATGCTGCCAGTCAG
CGACAACAAGAAGATCAACGACTATTTCAATAAGCAGCAAACGGGCGTGG
GCGTCGGTGTGCCAGGTGGTGCGGGAGGCAATACCGCTGGCCTTCGAGGA
TCACATACGGGAGGTGGCAGCAAGTCACCCTCATCCGCCCAGCAGCAGCA
 
AACGGCGGCACAGCAGCAGGGAAGCGGTGTTGCGACGGAGGCAGTGCAG
GCGGTTCCGCTGGCAACCAGGTGCAAGTGCAAACGAGCAGCGCTTACGCC
CTTTACCCACCAGCTAGTCCCCAAACCCAGACGTCACAGCAACAGCAGCA
GCAGCAACCGGGATCAGACTTTCACTATGTCAACTCCAGCAAGGCGCAGC
AACAACAGCAGCGTCAACAGCAACAGACTTCCAATCAAATGGTTCCTCCA
CACGTGGTCGTTGGCCTTGGTGGTCATCCACTGAGCCTCGCGTCCATTCA
GCAGCAGACGCCCTTATCCCAGCAGCAACAGCAGCAACAACAGCAGCAGC
AACAGCAGCAACTGGGACCACCGACCACATCGACGGCCTCCGTCGTGCCA
ACGCATCCGCATCAACTCGGATCCCTGGGAGTTGTTGGGATGGTCGGTGT
GGGTGTTGGCGTGGGCGTTGGAGTAAATGTGGGTGTGGGACCACCACTGC
CACCACCACCGCCGATGGCCATGCCAGCGGCCATTATCACTTATAGTAAG
GCCACTCAAACGGAGGTGTCGCTGCATGAATTGCAGGAGCGCGAAGCGGA
GCACGAATCGGGCAAGGTGAAGCTAGACGAGATGACACGGCTGTCCGATG
AACAAAAGTCCCAAATTGTTGGCAACCAGAAGACGATTGACCAGCACAAG
TGCCACATAGCCAAGTGTATTGATGTGGTCAAGAAGCTGTTGAAGGAGAA
GAGCAGCATCGAGAAGAAGGAGGCGCGACAGAAGTGCATGCAGAATCGCC
TCAGGCTCGGACAGTTTGTTACCCAACGAGTGGGCGCCACATTCCAGGAG
AACTGGACGGACGGCTATGCGTTCCAGGAGCTGAGTCGGCGGCAAGAAGA
AATAACCGCTGAGCGTGAAGAGATAGATCGGCAGAAAAAGCAGCTGATGA
AAAAGCGTCCGGCGGAGTCCGGACGCAAGCGCAACAACAACAGTAACCAG
AACAACCAGCAGCAGCAGCAACAGCAACACCAGCAACAGCAGCAGCAACA
AAATTCCAACTCGAACGATTCCACGCAGCTGACGAGCGGAGTTGTTACCG
GTCCAGGCAGTGATCGTGTGAGCGTAAGCGTCGACAGCGGATTGGGTGGC
AATAATGCGGGCGCGATCGGTGGCGGAACCGTTGGTGGTGGCGTTGGAGG
TGGTGGTGTTGGAGGCGGTGGTGTCGGAGGCGGCGGTGGACGTGGACTTT
CTCGCAGCAATTCGACGCAGGCCAATCAGGCTCAATTGCTGCACAACGGC
GGTGGTGGTTCGGGCGGCAATGTCGGCAACTCGGGCGGCGTTGGCGACCG
CTTGTCAGATCGAGGAGGAGGAGGTGGCGGCATCGGCGGAAACGATAGCG
GCAGCTGCTCGGACTCGGGCACTTTCCTGAAGCCAGACCCCGTATCGGGT
GCCTACACAGCGCAGGAGTATTACGAGTACGATGAGATCCTCAAGTTGCG
ACAAAATGCCCTCAAAAAGGAGGACGCCGACCTGCAGCTGGAGATGGAGA
AGCTGGAGCGGGAGCGCAATCTGCACATCCGAGAGCTCAAGCGGATTCTT
AACGAGGATCAGTCCCGCTTTAACAATCATCCCGTGCTGAATGATCGCTA
TCTTCTGTTGATGCTCCTGGGCAAGGGCGGCTTCTCAGAGGTCCACAAGG
CCTTCGACCTGAAGGAGCAACGCTATGTCGCATGTAAGGTGCACCAATTA
AACAAGGATTGGAAGGAGGATAAGAAAGCTAATTATATCAAACACGCTTT
GCGGGAATACAACATTCACAAGGCACTGGATCATCCGCGGGTCGTCAAGC
TATACGATGTCTTCGAGATCGATGCGAATTCCTTTTGCACAGTGCTCGAA
TACTGTGATGGCCACGATCTGGACTTCTATTTGAAGCAACATAAGACTAT
ACCCGAGCGTGAAGCGCGCTCGATAATAATGCAGGTTGTATCTGCACTCA
AGTATCTAAATGAGATTAAGCCTCCAGTTATCCACTACGATCTGAAGCCC
GGCAACATTCTGCTTACCGAGGGCAACGTCTGCGGCGAGATTAAGATCAC
CGACTTCGGTCTGTCAAAGGTGATGGACGACGAGAATTACAATCCCGATC
ACGGCATGGATCTGACCTCTCAGGGGGCGGGAACCTACTGGTATCTGCCA
CCCGAGTGCTTTGTCGTGGGCAAAAATCCGCCGAAAATCTCCTCCAAAGT
GGACGTATGGAGTGTGGGTGTTATCTTCTACCAGTGTCTGTACGGCAAAA
AGCCCTTCGGTCACAATCAGTCGCAGGCCACGATTCTCGAGGAGAATACG
ATCCTGAAGGCCACCGAAGTGCAGTTCTCCAACAAGCCAACCGTTTCTAA
 CGAGGCCAAG 
MCVQKNMRRLKKMSPGAHLQMSPQNTSSLSQHHPHQQQQLQPPQQQQQHF
PNHHSAQQQSQQQQQQEQQNPQQQAQQQQQILPHQHLQHLHKHPHQLQLH
QQQQQQLHQQQQQHFHQQSLQGLHQGSSNPDSNMSTGSSHSEKDVNDMLS
GGAATPGAAAAAIQQQHPAFAPTLGMQQPPPPPPQHSNNGGEMGYLSAGT
TTTTSVLTVGKPRTPAERKRKRKMPPCATSADEAGSGGGSGGAGATVVNN
SSLKGKSLAFRDMPKVNMSLNLGDRLGGSAGSGVGAGGAGSGGGGAGSGS
GSGGGKSARLMLPVSDNKKINDYFNKQQTGVGVGVPGGAGGNTAGLRGSH
TGGGSKSPSSAQQQQTAAQQQGSGVATGGSAGGSAGNQVQVQTSSAYALY
PPASPQTQTSQQQQQQQPGSDFHYVNSSKAQQQQQRQQQQTSNQMVPPHV
VVGLGGHPLSLASIQQQTPLSQQQQQQQQQQQQQQLGPPTTSTASVVPTH
PHQLGSLGVVGMVGVGVGVGVGVNVGVGPPLPPPPPMAMPAAIITYSKAT
QTEVSLHELQEREAEHESGKVKLDEMTRLSDEQKSQIVGNQKTIDQHKCH
IAKCIDVVKKLLKEKSSIEKKEARQKCMQNRLRLGQFVTQRVGATFQENW
TDGYAFQELSRRQEEITAEREEIDRQKKQLMKKRPAESGRKRNNNSNQNN
QQQQQQQHQQQQQQNSNSNDSTQLTSGVVTGPGSDRVSVSVDSGLGGNN
AGAIGGGTVGGGVGGGGVGGGGVGGGGGRGLSRSNSTQANQAQLLHNGGG
GSGGNVGNSGGVGDRLSDRGGGGGGIGGNDSGSCSDSGTFLKPDPVSGAY
TAQEYYEYDEILKLRQNALKKEDADLQLEMEKLERERNLHIRELKRILNE
DQSRFNNHPVLNDRYLLLMLLGKGGFSEVHKAFDLKEQRYVACKVHQLNK
DWKEDKKANYIKHALREYNIHKALDHPRVVKLYDVFEIDANSFCTVLEYC
DGHDLDFYLKQHKTIPEREARSIIMQVVSALKYLNEIKPPVIHYDLKPGN
ILLTEGNVCGEIKITDFGLSKVMDDENYNPDHGMDLTSQGAGTYWYLPPE
CFVVGKNPPKISSKVDVWSVGVIFYQCLYGKKPFGHNQSQATILEENTIL
KATEVQFSNKPTVSNEAK
 
AGTTTCATTCGGGGATGCTTGGCCTATCGCAAGGAGGATCGCATGGATGT
GTTCGCACTGGCCAGGCACGAGTACATTCAGCCACCGATACCGAAACATG
GGCGCGGTTCGCTCAATCAGCAACAGCAGGCGCAACAACAGCAGCAGCAA
CAACAGCAACAGCAGCAGCAACAGTCGTCGACGTCACAGGCCAATTCTAC
AGGCCAGACATCTTTCTCTGCCCACATGTTTGGCAATATGAATCAGTCGA
GTTCGTCCTAGTGGTGTCGGTGTCGTTTTGGTTTTGTCGGCGGTTGCTAA
ACACAATTTAAGTTCACTCGGTTAGCAGACATTACACACTGCCTGCTCTC
ATACATATTTACGCACTTGTATATACATGCAATGTGCCTGTGTGTGCGCA
AGAAACCAGAAAAAACGAAAAGTACAACATTCGTTGAGTCGCGTTCGGCT
TAATTTTTTTTGTGTTACCGTGTGTGTGTTTGTGCTTTGGATTTGCCAA
TTTTAGCCGACTGGCTCTCAGTGTCGAACTTAAACTTAAAGAGCGAGCAA
CGTGACGTGTCGCCCAGTGTCGCTTAAAATTCGCGCACACAACTTCCTAC
TACAAAAAAACGAAAGAAAGAAGGAGAAAAAACGTTAAAGATGTCCCCCG
GCGCCCATTTGCAGATGTCCCCGCAGAACACTTCGTCCCTAAGTCAACAC
CATCCACATCAACAGCAACAGTTACAACCCCCACAGCAGCAACAACAGCA
TTTCCCTAACCATCACAGCGCCCAGCAACAGTCGCAGCAGCAGCAGCAAC
AGGAGCAACAGAATCCCCAGCAGCAGGCGCAACAGCAGCAGCAGATACTC
CCACATCAACATTTGCAGCACCTGCACAAGCATCCGCATCAGCTGCAACT
GCATCAGCAGCAGCAACAACAACTCCACCAGCAACAGCAGCAACACTTCC
ACCAGCAGTCGCTGCAAGGGCTGCATCAGGGTAGCAGCAATCCGGATTCG
 
AATATGAGCACTGGCTCCTCGCATAGCGAGAAGGATGTCAATGATATGCT
GAGTGGCGGTGCAGCAACGCCAGGAGCTGCAGCAGCAGCGATTCAACAGC
AACATCCCGCCTTTGCGCCCACACTGGGAATGCAGCAACCACCGCCGCCC
CCACCTCAACACTCCAATAATGGAGGCGAGATGGGCTACTTGTCGGCAGG
CACGACCACGACGACGTCGGTGTTAACGGTAGGCAAGCCTCGGACGCCAG
CGGAGCGGAAACGGAAGCGAAAAATGCCTCCATGTGCCACTAGTGCGGAT
GAGGCGGGGAGTGGCGGTGGCTCTGGCGGAGCAGGAGCAACCGTTGTTAA
CAACAGCAGCCTGAAGGGCAAATCATTGGCCTTTCGTGATATGCCCAAGG
TAAACATGAGCCTGAATCTGGGCGATCGTCTGGAGGATCTGCAGGAAGC
GGAGTAGGAGCCGGTGGCGCCGGAAGCGGGGGAGGTGGCGCTGGTTCCGG
TTCTGGAAGCGGTGGCGGCAAAAGCGCCCGCCTGATGCTGCCAGTCAGCG
ACAACAAGAAGATCAACGACTATTTCAATAAGCAGCAAACGGGCGTGGGC
GTCGGTGTGCCAGGTGGTGCGGAGGCAATACCGCTGGCCTTCGAGGATC
ACATACGGGAGGTGGCAGCAAGTCACCCTCATCCGCCCAGCAGCAGCAAA
CGGCGGCACAGCAGCAGGGAAGCGGTGTTGCGACGGGAGGCAGTGCAGGC
GGTTCCGCTGGCAACCAGGTGCAAGTGCAAACGAGCAGCGCTTACGCCCT
TTACCCACCAGCTAGTCCCCAAACCCAGACGTCACAGCAACAGCAGCAGC
AGCAACCGGGATCAGACTTTCACTATGTCAACTCCAGCAAGGCGCAGCAA
CAACAGCAGCGTCAACAGCAACAGACTTCCAATCAAATGGTTCCTCCACA
CGTGGTCGTTGGCCTTGGTGGTCATCCACTGAGCCTCGCGTCCATTCAGC
AGCAGACGCCCTTATCCCAGCAGCAACAGCAGCAACAACAGCAGCAGCAA
CAGCAGCAACTGGGACCACCGACCACATCGACGGCCTCCGTCGTGCCAAC
GCATCCGCATCAACTCGGATCCCTGGGAGTTGTTGGGATGGTCGGTGTGG
GTGTTGGCGTGGGCGTTGGAGTAAATGTGGGTGTGGGACCACCACTGCCA
CCACCACCGCCGATGGCCATGCCAGCGGCCATTATCACTTATAGTAAGGC
CACTCAAACGGAGGTGTCGCTGCATGAATTGCAGGAGCGCGAAGCGGAGC
ACGAATCGGGCAAGGTGAAGCTAGACGAGATGACACGGCTGTCCGATGAA
CAAAAGTCCCAAATTGTTGGCAACCAGAAGACGATTGACCAGCACAAGTG
CCACATAGCCAAGTGTATTGATGTGGTCAAGAAGCTGTTGAAGGAGAAGA
GCAGCATCGAGAAGAAGGAGGCGCGACAGAAGTGCATGCAGAATCGCCTC
AGGCTCGGACAGTTTGTTACCCAACGAGTGGGCGCCACATTCCAGGAGAA
CTGGACGGACGGCTATGCGTTCCAGGAGCTGAGTCGGCGGCAAGAAGAAA
TAACCGCTGAGCGTGAAGAGATAGATCGGCAGAAAAAGCAGCTGATGAAA
AAGCGTCCGGCGGAGTCCGGACGCAAGCGCAACAACAACAGTAACCAGAA
CAACCAGCAGCAGCAGCAACAGCAACACCAGCAACAGCAGCAGCAACAAA
ATTCCAACTCGAACGATTCCACGCAGCTGACGAGCGGAGTTGTTACCGGT
CCAGGCAGTGATCGTGTGAGCGTAAGCGTCGACAGCGGATTGGGTGGCAA
TAATGCGGGCGCGATCGGTGGCGGAACCGTTGGTGGTGGCGTTGGAGGTG
GTGGTGTTGGAGGCGGTGGTGTCGGAGGCGGCGGTGGACGTGGACTTTCT
CGCAGCAATTCGACGCAGGCCAATCAGGCTCAATTGCTGCACAACGGCGG
TGGTGGTTCGGGCGGCAATGTCGGCAACTCGGGCGGCGTTGGCGACCGCT
TGTCAGATCGAGGAGGAGGAGGTGGCGGCATCGGCGGAAACGATAGCGGC
AGCTGCTCGGACTCGGGCACTTTCCTGAAGCCAGACCCCGTATCGGGTGC
CTACACAGCGCAGGAGTATTACGAGTACGATGAGATCCTCAAGTTGCGAC
AAAATGCCCTCAAAAAGGAGGACGCCGACCTGCAGCTGGAGATGGAGAAG
CTGGAGCGGGAGCGCAATCTGCACATCCGAGAGCTCAAGCGGATTCTTAA
CGAGGATCAGTCCCGCTTTAACAATCATCCCGTGCTGAATGATCGCTATC
TTCTGTTGATGCTCCTGGGCAAGGGCGGCTTCTCAGAGGTCCACAAGGCC
 
TTCGACCTGAAGGAGCAACGCTATGTCGCATGTAAGGTGCACCAATTAAA
CAAGGATTGGAAGGAGGATAAGAAAGCTAATTATATCAAACACGCTTTGC
GGGAATACAACATTCACAAGGCACTGGATCATCCGCGGGTCGTCAAGCTA
TACGATGTCTTCGAGATCGATGCGAATTCCTTTTGCACAGTGCTCGAATA
CTGTGATGGCCACGATCTGGACTTCTATTTGAAGCAACATAAGACTATAC
CCGAGCGTGAAGCGCGCTCGATAATAATGCAGGTTGTATCTGCACTCAAG
TATCTAAATGAGATTAAGCCTCCAGTTATCCACTACGATCTGAAGCCCGG
CAACATTCTGCTTACCGAGGGCAACGTCTGCGGCGAGATTAAGATCACCG
ACTTCGGTCTGTCAAAGGTGATGGACGACGAGAATTACAATCCCGATCAC
GGCATGGATCTGACCTCTCAGGGGGCGGGAACCTACTGGTATCTGCCACC
CGAGTGCTTTGTCGTGGGCAAAAATCCGCCGAAAATCTCCTCCAAAGTGG
ACGTATGGAGTGTGGGTGTTATCTTCTACCAGTGTCTGTACGGCAAAAAG
CCCTTCGGTCACAATCAGTCGCAGGCCACGATTCTCGAGGAGAATACGAT
CCTGAAGGCCACCGAAGTGCAGTTCTCCAACAAGCCAACCGTTTCTAACG
AGGCCAAG
 
MSPGAHLQMSPQNTSSLSQHHPHQQQQLQPPQQQQQHFPNHHSAQQQSQQ
QQQQEQQNPQQQAQQQQQILPHQHLQHLHKHPHQLQLHQQQQQLHQQQQ
QHFHQQSLQGLHQGSSNPDSNMSTGSSHSEKDVNDMLSGGAATPGAAAAA
IQQQHPAFAPTLGMQQPPPPPPQHSNNGGEMGYLSAGTTTTTSVLTVGKP
RTPAERKRKRKMPPCATSADEAGSGGGSGGAGATVVNNSSLKGKSLAFRD
MPKVNMSLNLGDRLGGSAGSGVGAGGAGSGGGGAGSGSGSGGGKSARLML
PVSDNKKINDYFNKQQTGVGVGVPGGAGGNTAGLRGSHTGGGSKSPSSAQ
QQQTAAQQQGSGVATGGSAGGSAGNQVQVQTSSAYALYPPASPQTQTSQQ
QQQQQPGSDFHYVNSSKAQQQQQRQQQQTSNQMVPPHVVVGLGGHPLSLA
SIQQQTPLSQQQQQQQQQQQQQLGPPTTSTASVVPTHPHQLGSLGVVGM
VGVGVGVGVGVNVGVGPPLPPPPPMAMPAAIITYSKATQTEVSLHELQER
EAEHESGKVKLDEMTRLSDEQKSQIVGNQKTIDQHKCHIAKCIDVVKKLL
KEKSSIEKKEARQKCMQNRLRLGQFVTQRVGATFQENWTDGYAFQELSRR
QEEITAEREEIDRQKKQLMKKRPAESGRKRNNNSNQNNQQQQQQHQQQQ
QQQNSNSNDSTQLTSGVVTGPGSDRVSVSVDSGLGGNNAGAIGGGTVGGG
VGGGGVGGGGVGGGGGRGLSRSNSTQANQAQLLHNGGGGSGGNVGNSGGV
GDRLSDRGGGGGGIGGNDSGSCSDSGTFLKPDPVSGAYTAQEYYEYDEIL
KLRQNALKKEDADLQLEMEKLERERNLHIRELKRILNEDQSRFNNHPVLN
DRYLLLMLLGKGGFSEVHKAFDLKEQRYVACKVHQLNKDWKEDKKANYIK
HALREYNIHKALDHPRVVKLYDVFEIDANSFCTVLEYCDGHDLDFYLKQH
KTIPEREARSIIMQVVSALKYLNEIKPPVIHYDLKPGNILLTEGNVCGEI
KITDFGLSKVMDDENYNPDHGMDLTSQGAGTYWYLPPECFVVGKNPPKIS
SKVDVWSVGVIFYQCLYGKKPFGHNQSQATILEENTILKATEVQFSNKPT
VSNEAK
**Human homologue of Complete Genome candidate** AAF03095 - tousled-like kinase2 
1 ccgggcgggg ggttgcggcg ctcaggagag gccccggctc cgccccgggc ctgcccaggg
61 ggagagcgga gctccgcagc cgggtcgggt cggggcccct cccgggagga gcgtggagcg
121 cggcggcggc ggcggcagca gaaatgatgg aagaattgca tagcctggac ccacgacggc
 
181 aggaattatt ggaggccagg tttactggag taggtgttag taagggacca cttaatagtg
241 agtcttccaa ccagagcttg tgcagcgtcg gatccttgag tgataaagaa gtagagactc
301 ccgagaaaaa gcagaatgac cagcgaaatc ggaaaagaaa agctgaacca tatgaaacta
361 gccaagggaa aggcactcct aggggacata aaattagtga ttactttgag tttgctgggg
421 gaagcgcgcc aggaaccagc cctggcagaa gtgttccacc agttgcacga tcctcaccgc
481 aacattcctt atccaatccc ttaccgcgac gagtagaaca gcccctctat ggtttagatg
541 gcagtgctgc aaaggaggca acggaggagc agtctgctct gccaaccctc atgtcagtga
601 tgctagcaaa acctcggctt gacacagagc agctggcgca aaggggagct ggcctctgct
661 tcacttttgt ttcagctcag caaaacagtc cctcatctac gggatctggc aacacagagc
721 attcctgcag ctcccaaaaa cagatctcca tccagcacag acggacccag tccgacctca
781 caatagaaaa aatatctgca ctagaaaaca gtaagaattc tgacttagag aagaaggagg
841 gaagaataga tgatttatta agagccaact gtgatttgag acggcagatt gatgaacagc
901 aaaagatgct agagaaatac aaggaacgat taaatagatg tgtgacaatg agcaagaaac
961 tccttataga aaagtcaaaa caagagaaga tggcgtgtag agataagagc atgcaagacc
1021 gcttgagact gggccacttt actactgtcc gacacggagc ctcatttact gaacagtgga
1081 cagatggtta tgcttttcag aatcttatca agcaacagga aaggataaat tcacagaggg
1141 aagagataga aagacaacgg aaaatgttag caaagcggaa acctcctgcc atgggtcagg
1201 cccctcctgc aaccaatgag cagaaacagc ggaaaagcaa gaccaatgga gctgaaaatg
1261 aaacgttaac gttagcagaa taccatgaac aagaagaaat cttcaaactc agattaggtc
1321 atcttaaaaa ggaggaagca gagatccagg cagagctgga gagactagaa agggttagaa
1381 atctacatat cagggaacta aaaaggatac ataatgaaga taattcacaa tttaaagatc
1441 atccaacgct aaatgacaga tatttgttgt tacatctttt gggtagagga ggtttcagtg
1501 aagtttacaa ggcatttgat ctaacagagc aaagatacgt agctgtgaaa attcaccagt
1561 taaataaaaa ctggagagat gagaaaaagg agaattacca caagcatgca tgtagggaat
1621 accggattca taaagagctg gatcatccca gaatagttaa gctgtatgat tacttttcac
1681 tggatactga ctcgttttgt acagtattag aatactgtga gggaaatgat ctggacttct
1741 acctgaaaca gcacaaatta atgtcggaga aagaggcccg gtccattatc atgcagattg
1801 tgaatgcttt aaagtactta aatgaaataa aacctcccat catacactat gacctcaaac
1861 caggtaatat tcttttagta aatggtacag cgtgtggaga gataaaaatt acagattttg
1921 gtctttcgaa gatcatggat gatgatagct acaattcagt ggatggcatg gagctaacat
1981 cacaaggtgc tggtacttat tggtatttac caccagagtg ttttgtggtt gggaaagaac
2041 caccaaagat ctcaaataaa gttgatgtgt ggtcggtggg tgtgatcttc tatcagtgtc
2101 tttatggaag gaagcctttt ggccataacc agtctcagca agacatccta caagagaata
2161 cgattcttaa agctactgaa gtgcagttcc cgccaaagcc agtagtaaca cctgaagcaa
2221 aggcgtttat tcgacgatgc ttggcctacc gaaagaggga ccgcattgat gtccagcagc
2281 tggcctgtga tccctacttg ttgcctcaca tccgaaagtc agtctctaca agtagccctg
2341 ctggagctgc tattgcatca acctctgggg cgtccaataa cagttcttct aattgagact
2401 gactccaagg ccacaaactg ttcaacacac acaaagtgga caaatggcgt tcagcagcgg
2461 gtttggaaca tagcgaatcc gaatggatct gatgaaacct gtaccaggtg cttttatttt
2521 cttgcttttt tcccatccat agagcatgac agcatcgatt ctcattgagg agaaaccttg
2581 ggcagctccg gccaggcctt gtaggaaaag gccccgcccg aggttccagc gtcaacggcc
2641 actgtgtgtg gctgctctga gtgaggaaaa aattaaaaag aaaaactggt tccatgtact
2701 gtgaacttga aaacttgcag actcaggggg gtccctgatg cagtgcttca gatgaagaat
2761 gtggacttga aaatacagac tgggctagtc cagtgtctat atttaaactt gttcttttct
2821 tttaataaag tttaggtaac atctcctgaa aagcttgtag cacaaaggct cagctgggga
2881 tggtgtttga cttcggagga aaaaagttgc tattgcccgt taaaggcact agagttagtg
2941 ttttatccct aaataatttc aattttaaaa aacatgcagc ttccctctcc cctttttat
3001 ttttgaaaga atacatttgg tcataaagtg aaacccgtat tagcaagtac gaggcaatgt
 
3061 tcattccaat cagatgcagc tttctcctcc gtctggtctc ctgtttgcaa ttgcttccct
3121 catctcagta gggaaaaaat tgagtgggag tactgagatg tgtgggtttt tgccattgga
3181 caaagaatga ggttagaaga ctgcagcttg gagtctctct aggttttcaa ctatttcttc
3241 acaatttgaa cacttgacgg ttgtcccttt taatttattt gaagtgctat ttttttaaat
3301 aaaggttcat ctgtccatgc aaaaaaa
 
1 meelhsldpr rqellearft gvgvskgpln sessnqslcs vgslsdkeve tpekkqndqr
61 nrkrkaepye tsqgkgtprg hkisdyfefa ggsapgtspg rsvppvarss pqhslsnplp
121 rrveqplygl dgsaakeate eqsalptlms vmlakprldt eqlaqrgagl cftfvsaqqn
181 spsstgsgnt ehscssqkqi siqhrrtqsd ltiekisale nsknsdlekk egriddllra
241 ncdlrrqide qqkmlekyke rlnrcvtmsk klliekskqe kmacrdksmq drlrlghftt
301 vrhgasfteq wtdgyafqnl ikqqerinsq reeierqrkm lakrkppamg qappatneqk
361 qrksktngae netltlaeyh eqeeifklrl ghlkkeeaei qaelerlerv rnlhirelkr
421 ihnednsqfk dhptlndryl llhllgrggf sevykafdlt eqryvavkih qlnknwrdek
481 kenyhkhacr eyrihkeldh privklydyf sldtdsfctv leycegndld fylkqhklms
541 ekearsiimq ivnalkylne ikppiihydl kpgnillvng tacgeikitd fglskimddd
601 synsvdgmel tsqgagtywy lppecfvvgk eppkisnkvd vwsvgvifyq clygrkpfgh
661 nqsqqdilqe ntilkatevq fppkpvvtpe akafirrcla yrkrdridvq qlacdpyllp
721 hirksvstss pagaaiasts gasnnsssn
**Putative function** Serine threonine kinase involved in replication and cell cycle

### Example 4 (Category 2)

**Line ID** - 224
**Phenotype** - Semi-lethal male and female, cytokinesis defect. Onion stage cysts have variable sized Nebenkerns. Also has a mitotic phenotype: Tangled unevenly condensed chromosomes, anaphases with lagging chromosomes and bridges
**Annotated *Drosophila* genome genomic segment containing P element insertion site (and map position) -** AE003450 (9C)
**P element insertion site -** 139,674
**Annotated *Drosophila* genome Complete Genome candidate -** CG2096 - flapwing, phosphatase type 1 
ATCTGTAAGTGAAGTCCACTAACAACCGGTTTACTTGCAGTGCGCAGCTG
CCGAACGGGCAAACAGGTCCAGATGACGGAGGCGGAGGTGCGTGGCCTCT
GTCTCAAGTCGCGCGAGATCTTCTTGCAACAGCCCATCCTGCTGGAACTG
GAGGCACCGCTGATCATCTGCGGCGACATCCACGGCCAGTACACAGACCT
GTTGCGCCTGTTCGAGTACGGCGGATTCCCTCCGGCTGCCAACTACTTGT
TCCTCGGCGACTACGTCGATCGGGGCAAGCAGTCCCTGGAGACCATCTGT
CTGCTGCTGGCCTACAAGATCAAATATCCGGAGAACTTCTTCTTGTTGCG
CGGCAACCACGAGTGCGCCAGTATTAATAGGATTTACGGCTTCTACGATG
AGTGCAAGCGCCGATACAATGTCAAACTGTGGAAGACTTTCACAGATTGC
TTCAACTGTCTGCCGGTAGCCGCCATTATTGACGAAAAGATCTTCTGCTG
CCACGGCGGCCTCAGTCCCGATCTTCAGGGCATGGAGCAGATCCGTCGCC
TAATGCGACCCACAGATGTGCCGGATACCGGGTTACTGTGCGATCTTCTG
TGGAGTGATCCCGACAAGGATGTTCAGGGTTGGGGCGAGAATGATCGCGG
TGTGAGCTTCACCTTCGGTGTGGATGTGGTCTCCAAGTTTTTGAACCGCC
ACGAGCTGGACTTGATCTGCCGTGCACATCAGGTTGTGGAGGATGGCTAT
GAGTTCTTTGCGCGTCGGCAACTGGTCACGTTGTTCTCGGCGCCCAATTA
CTGTGGAGAGTTCGACAATGCCGGCGGAATGATGACCGTGGACGACACGC
TGATGTGCTCATTCCAGATCCTGAAACCATCCGAGAAGAAGGCCAAGTAT
CTGTACAGCGGAATGAACTCGTCGCGACCCACAACACCGCAGCGCAGCGC
CCCAATGCTTGCGACCAACAAGAAGAAATAATATATCCATCCGCTTCCAT
TTCCTTAAAGGTTCAACAAACAACAGAAATAAACTTTTACATAGATACAC
ACATATATACATATAAATATAACGAAACGATAGAAAAGGAGAGCGTTAGG
CGATAGTAGAGAAAGGGCAAATGATAAATTAAATGTGTGAGCTATTAAAG
CAAGCAAAATCGAAGTGCATGAATATCAACATCTATGTGAATCCGTCATT
ATCTGTTATCTGATGTGTCATCTGTATCCAACTTGATTACCTTATCCGTG
TACCTGCTAGTTGCAGCAGCAACATCAGGAGCAACAACACCAGCAGCAGC
AGCAGCAGAAACATCAGTGAAACACTCAGAGGCCCATAGTTAAGTCGATT
CCTGCATTTGATGATTATCTGTTGAATGGAAATTGTGACAACGTCCCCGT
AACAGCAGCTCCCAGATCCAAAACTCCCGAAACATGCAGATAAATAAATA
CATTAAAAGTACAGCGATGTTAAGCAATGAATTTATATATAGGCTTATTA
ATGTAAACT
 
MTEAEVRGLCLKSREIFLQQPILLELEAPLIICGDIHGQYTDLLRLFEYG
GFPPAANYLFLGDYVDRGKQSLETICLLLAYKIKYPENFFLLRGNHECAS
 
INRIYGFYDECKRRYNVKLWKTFTDCFNCLPVAAIIDEKIFCCHGGLSPD
LQGMEQIRRLMRPTDVPDTGLLCDLLWSDPDKDVQGWGENDRGVSFTFGV
DVVSKFLNRHELDLICRAHQVVEDGYEFFARRQLVTLFSAPNYCGEFDNA
GGMMTVDDTLMCSFQILKPSEKKAKYLYSGMNSSRPTTPQRSAPMLATNK
KK
**Human homologue of Complete Genome candidate** NP_002700 protein phosphatase 1, catalytic subunit, beta isoform 
1 cctgggtctg acgcggccct gttcgagggg gcctctcttg tttatttatt tattttccgt
61 gggtgcctcc gagtgtgcgc gcgctctcgc tacccggcgg ggagggggtg gggggagggc
121 ccgggaaaag ggggagttgg agccggggtc gaaacgccgc gtgacttgta ggtgagagaa
181 cgccgagccg tcgccgcagc ctccgccgcc gagaagccct tgttcccgct gctgggaagg
241 agagtctgtg ccgacaagat ggcggacggg gagctgaacg tggacagcct catcacccgg
301 ctgctggagg tacgaggatg tcgtccagga aagattgtgc agatgactga agcagaagtt
361 cgaggcttat gtatcaagtc tcgggagatc tttctcagcc agcctattct tttggaattg
421 gaagcaccgc tgaaaatttg tggagatatt catggacaat atacagattt actgagatta
481 tttgaatatg gaggtttccc accagaagcc aactatcttt tcttaggaga ttatgtggac
541 agaggaaagc agtctttgga aaccatttgt ttgctattgg cttataaaat caaatatcca
601 gagaacttct ttctcttaag aggaaaccat gagtgtgcta gcatcaatcg catttatgga
661 ttctatgatg aatgcaaacg aagatttaat attaaattgt ggaagacctt cactgattgt
721 tttaactgtc tgcctatagc agccattgtg gatgagaaga tcttctgttg tcatggagga
781 ttgtcaccag acctgcaatc tatggagcag attcggagaa ttatgagacc tactgatgtc
841 cctgatacag gtttgctctg tgatttgcta tggtctgatc cagataagga tgtgcaaggc
901 tggggagaaa atgatcgtgg tgtttccttt acttttggag ctgatgtagt cagtaaattt
961 ctgaatcgtc atgatttaga tttgatttgt cgagctcatc aggtggtgga agatggatat
1021 gaattttttg ctaaacgaca gttggtaacc ttattttcag ccccaaatta ctgtggcgag
1081 tttgataatg ctggtggaat gatgagtgtg gatgaaactt tgatgtgttc atttcagata
1141 ttgaaaccat ctgaaaagaa agctaaatac cagtatggtg gactgaattc tggacgtcct
1201 gtcactccac ctcgaacagc taatccgccg aagaaaaggt gaagaaagga attctgtaaa
1261 gaaaccatca gatttgttaa ggacatactt cataatatat aagtgtgcac tgtaaaacca
1321 tccagccatt tgacaccctt tatgatgtca cacctttaac ttaaggagac gggtaaagga
1381 tcttaaattt ttttctaata gaaagatgtg ctacactgta ttgtaataag tatactctgt
1441 tatagtcaac aaagttaaat ccaaattcaa aattatccat taaagttaca tcttcatgta
1501 tcacaatttt taaagttgaa aagcatccca gttaaactag atgtgatagt taaaccagat
1561 gaaagcatga tgatccatct gtgtaatgtg gttttagtgt tgcttggttg tttaattatt
1621 ttgagcttgt tttgtttttg tttgttttca ctagaataat ggcaaatact tctaattttt
1681 ttccctaaac atttttaaaa gtgaaatatg ggaagagctt tacagacatt caccaactat
1741 tattttccct tgtttatcta cttagatatc tgtttaatct tactaagaaa actttcgcct
1801 cattacatta aaaaggaatt ttagagattg attgttttaa aaaaaaatac gcacattgtc
1861 caatccagtg attttaatca tacagtttga ctgggcaaac tttacagctg atagtgaata
1921 ttttgcttta tacaggaatt gacactgatt tggatttgtg cactctaatt tttaacttat
1981 tgatgctcta ttgtgcagta gcatttcatt taagataagg ctcatatagt attacccaac
2041 tagttggtaa tgtgattatg tggtaccttg gctttaggtt ttcattcgca cggaacacct
2101 tttggcatgc ttaacttcct ggtaacacct tcacctgcat tggttttctt tttcttttt
2161 ctttcttttt tttttttttt ttttttttga gttgttgttt gtttttagat ccacagtaca
2221 tgagaatcct tttttgacaa gccttggaaa gctgacactg tctctttttc ctccctctat
 
2281 acgaaggatg tatttaaatg aatgctggtc agtgggacat tttgtcaact atgggtattg
2341 ggtgcttaac tgtctaatat tgccatgtga atgttgtata cgattgtaag gcttatgtca
2401 ctaaagattt ttattctgat tttttcataa tcaaaggtca tatgatactg tatagacaag
2461 ctttgtagtg aagtatagta gcaataattt ctgtacctga tcaagtttat tgcagccttt
2521 cttttcctat ttcttttttt taagggttag tattaacaaa tggcaatgag tagaaaagtt
2581 aacatgaaga ttttagaagg agagaactta caggacacag atttgtgatt ctttgactgt
2641 gacactattg gatgtgattc taaaagcttt tattgagcat tgtcaaattt gtaagcttca
2701 tagggatgga catcatatct ataatgccct tctatatgtg ctaccataga tgtgacattt
2761 ttgaccttaa tatcgtcttt gaaaatgtta aattgagaaa cctgttaact tacattttat
2821 gaattggcac attgtattac ttactgcaag agatatttca ttttcagcac agtgcaaaag
2881 ttctttaaaa tgcatatgtc tttttttcta attccgtttt gttttaaagc acattttaaa
2941 tgtagttttc tcatttagta aaagttgtct aattgatatg aagcctgact gatttttttt
3001 ttccttacag tgagacattt aagcacacat tttattcaca tagatactat gtccttgaca
3061 tattgaaatg attcttttct gaaagtattc atgatctgca tatgatgtat taggttaggt
3121 cacaaaggtt ttatctgagg tgatttaaat aacttcctga ttggagtgtg taagctgagc
3181 gatttctaat aaaattttag ttgtacactt ttagtagtca tagtgaagca ggtctagaaa
3241 ataagccttt ggcagggaaa aagggcaatg ttgattaatc tcagtattaa accacattaa
3301 tctgtatccc attgtctggc ttttgtaaat tcatccaggt caagactaag tatgttggtt
3361 aataggaatc cttttttttt tttaaagact aaatgtgaaa aaataatcac tacttaagct
3421 aattaatatt ggtcattaaa tttaaaggat ggaaatttat catgtttaaa aattattcaa
3481 gcactcttaa aaccacttaa acagcctcca gtcataaaaa tgtgttcttt acaaatattt
3541 gcttggcaac acgacttgaa ataaataaaa ctttgtttct taggagaaaa
 
1 madgelnvds litrllevrg crpgkivqmt eaevrglcik sreiflsqpi lleleaplki
61 cgdihgqytd llrlfeyggf ppeanylflg dyvdrgkqsl eticlllayk ikypenffll
121 rgnhecasin riygfydeck rrfniklwkt ftdcfnclpi aaivdekifc chgglspdlq
181 smeqirrimr ptdvpdtgll cdllwsdpdk dvqgwgendr gvsftfgadv vskflnrhdl
241 dlicrahqvv edgyeffakr qlvtlfsapn ycgefdnagg mmsvdetlmc sfqilkpsek
301 kakyqyggln sgrpvtpprt anppkkr
**Putative function** Protein phosphatase

### Example 5 (Category 2)

**Line ID** - 231
**Phenotype** - Semi-lethal male and female, cytokinesis defect. In some cysts, variable sized Nebenkerns
**Annotated *Drosophila* genome genomic segment containing P element insertion site (and map position)** - AE003429 (3F)
**P element insertion site -** 153,730
**Annotated *Drosophila* genome Complete Genome candidate -** CG5014 - vap-33-1 vesicle associated membrane protein 
CACATCACTAGCTGACAGAATATATGGCTTTTTACATTTTGCGTTTTCA
ACTGAAGTTTGCGAAGAAACCGAAGCGTGGTAAACCACTGAAATCGAAAA
TATCGACAGAAAAGCGACCTAAAGTCGGTGAAGAAGTCGCACGTTGATCG
TTGTGTTTTTTCCCGAAATTTTCTGCAAAAAGCCCGTGCGTGCGTGAGT
TTCTCTGGCTCTTGCTTTTTTTTGTCCATGCGTGTGTGTGTGGTCGCAT
AAATTTACCGATATTTCGCCTGTGAGAGCGAAACGAACGAAAAACGAAAG
AAAAAAAGAGAGACGAGTAAAGTAAAACGAAACAGGCATAAAAACAGCAG
CAGTTTTCTTGATATATTTGGCTAAAAACGCAAACCAAACAGCCAGCAA
GAACAACAAATAGCTGGGCAAAAACAGGACGCACAAAAAATAAAATTAAA
ACGATAAGAGGCGAAAAGCGGAGAGAGTGAAATTCTCGGCAGCAACAACG
ACAAGAACAACACCAGGAGCAGCAGCAACAACAACAAAAGCCAGCCG
CCACAATGAGCAAATCACTCTTTGATCTTCCGTTGACCATTGAACCAGAA
CATGAGTTGCGTTTTGTGGGTCCCTTCACCCGACCCGTTGTCACAATCAT
GACTCTGCGCAACAACTCGGCTCTGCCTCTGGTCTTCAAGATCAAGACAA
CCGCCCCGAAACGCTACTGCGTACGTCCAAACATCGGCAAGATAATTCCC
TTTCGATCAACCCAGGTGGAGATCTGCCTTCAGCCATTCGTCTACGATCA
GCAGGAGAAGAACAAGCACAAGTTCATGGTGCAGAGCGTCCTGGCACCCA
TGGATGCTGATCTAAGCGATTTAAATAAATTGTGGAAGGATCTGGAGCCC
GAGCAGCTGATGGACGCCAAACTGAAGTGCGTTTTCGAGATGCCCACCGC
TGAGGCAAATGCTGAGAACACCAGCGGTGGTGGTGCCGTTGGCGGCGGAA
CCGGAGCTGCCGGAGGCGGAAGCGCGGGTGCCAATACTAGCTCAGCCAGC
GCTGAGGCGCTCGAGAGCAAGCCGAAGCTCTCCAGCGAGGATAAGTTTAA
GCCATCCAATTTGCTCGAAACGTCTGAGAGTCTGGACTTGCTGTCCGGAG
AGATCAAAGCGCTGCGTGAATGCAACATTGAATTGCGAAGAGAGAATCTT
CACTTGAAGGATCAAATCACACGTTTCCGGAGCTCGCCGGCCGTCAAACA
GGTGAATGAGCCCTATGCCCCAGTCCTGGCTGAGAAGCAGATTCCGGTCT
TTTACATTGCAGTTGCCATTGCTGCGGCCATCGTTAGCCTCCTGCTGGGC
AAATTCTTTCTCTGA
 
MSKSLFDLPLTIEPEHELRFVGPFTRPVVTIMTLRNNSALPLVFKIKTTA
PKRYCVRPNIGKIIPFRSTQVEICLQPFVYDQQEKNKHKFMVQSVLAPMD
ADLSDLNKLWKDLEPEQLMDAKLKCVFEMPTAEANAENTSGGGAVGGGTG
AAGGGSAGANTSSASAEALESKPKLSSEDKFKPSNLLETSESLDLLSGEI
KALRECNIELRRENLHLKDQITRFRSSPAVKQVNEPYAPVLAEKQIPVFY
 IAVAIAAAIVSLLLGKFFL
**Human homologue of Complete Genome candidate** AAD13577 VAMP-associated protein B 
1 gcgcgcccac ccggtagagg acccccgccc gtgccccgac cggtccccgc ctttttgtaa
61 aacttaaagc gggcgcagca ttaacgcttc ccgccccggt gacctctcag gggtctcccc
121 gccaaaggtg ctccgccgct aaggaacatg gcgaaggtgg agcaggtcct gagcctcgag
181 ccgcagcacg agctcaaatt ccgaggtccc ttcaccgatg ttgtcaccac caacctaaag
241 cttggcaacc cgacagaccg aaatgtgtgt tttaaggtga agactacagc accacgtagg
301 tactgtgtga ggcccaacag cggaatcatc gatgcagggg cctcaattaa tgtatctgtg
361 atgttacagc ctttcgatta tgatcccaat gagaaaagta aacacaagtt tatggttcag
421 tctatgtttg ctccaactga cacttcagat atggaagcag tatggaagga ggcaaaaccg
481 gaagacctta tggattcaaa acttagatgt gtgtttgaat tgccagcaga gaatgataaa
541 ccacatgatg tagaaataaa taaaattata tccacaactg catcaaagac agaaacacca
601 atagtgtcta agtctctgag ttcttctttg gatgacaccg aagttaagaa ggttatggaa
661 gaatgtaaga ggctgcaagg tgaagttcag aggctacggg aggagaacaa gcagttcaag
721 gaagaagatg gactgcggat gaggaagaca gtgcagagca acagccccat ttcagcatta
781 gccccaactg ggaaggaaga aggccttagc acccggctct tggctctggt ggttttgttc
841 tttatcgttg gtgtaattat tgggaagatt gccttgtaga ggtagcatgc acaggatggt
901 aaattggatt ggtggatcca ccatatcatg ggatttaaat ttatcataac catgtgtaaa
961 aagaaattaa tgtatgatga catctcacag gtcttgcctt taaattaccc ctccctgcac
1021 acacatacac agatacacac acacaaatat aatgtaacga tcttttagaa agttaaaaat
1081 gtatagtaac tgattgaggg ggaaaagaat gatctttatt aatgacaagg gaaaccatga
1141 gtaatgccac aatggcatat tgtaaatgtc attttaaaca ttggtaggcc ttggtacatg
1201 atgctggatt acctctctta aaatgacacc cttcctcgcc tgttggtgct ggcccttggg
1261 gagctggagc ccagcatgct ggggagtgcg gtcagctcca cacagtagtc cccacgtggc
1321 ccactcccgg cccaggctgc tttccgtgtc ttcagttctg tccaagccat cagctccttg
1381 ggactgatga acagagtcag aagcccaaag gaattgcact gtggcagcat cagacgtact
1441 cgtcataagt gagaggcgtg tgttgactga ttgacccagc gctttggaaa taaatggcag
1501 tgctttgttc acttaaaggg accaagctaa atttgtattg gttcatgtag tgaagtcaaa
1561 ctgttattca gagatgttta atgcatattt aacttattta atgtatttca tctcatgttt
1621 tcttattgtc acaagagtac agttaatgct gcgtgctgct gaactctgtt gggtgaactg
1681 gtattgctgc tggagggctg tgggctcctc tgtctctgga gagtctggtc atgtggaggt
1741 ggggtttatt gggatgctgg agaagagctg ccaggaagtg ttttttctgg gtcagtaaat
1801 aacaactgtc ataggcaggg aaattctcag tagtgacagt caactctagg ttaccttttt
1861 taatgaagag tagtcagtct tctagattgt tcttatacca cctctcaacc attactcaca
1921 cttccagcgc ccaggtccaa gtttgagcct gacctcccct tggggaccta gcctggagtc
1981 aggacaaatg gatcgggctg caaagggtta gaagcgaggg caccagcagt tgtgggtggg
2041 gagcaaggga agagagaaac tcttcagcga atccttctag tactagttga gagtttgact
2101 gtgaattaat tttatgccat aaaagaccaa cccagttctg tttgactatg tagcatcttg
2161 aaaagaaaaa ttataataaa gccccaaaat taaga
 
1 
61 
121 
181 
 241 
**Putative function** Membrane associated protein which may be involved in priming synaptic vesicles

### Example 6 (Category 2)

**Line ID** - 248
**Phenotype** - Male sterile, cytokinesis defect. Cytokinesis defect, different meiotic stages within one cyst, variable sized nuclei, 2-4 nuclei. Also has a mitotic phenotype: semi-lethal, rod-like overcondensed chromosomes, high mitotic index, lagging chromosomes and bridges.
**Annotated *Drosophila* genome genomic segment containing P element insertion site (and map position)** - AE003431 (4D1)
**P element insertion site** - 299,078
**Annotated *Drosophila* genome Complete Genome candidate -** CG6998 - cutup (dynein light chain) 
CAAAACGTTCAGTTGTGTTTCAGTTGTCGAGAAGTCAGGGTGTTTCTACC
TTCCATTTACCGTTCCAGTGTAAAATTCAGGCGACACGCTTAGCGTTACC
AAGGAGAACCGCTAAAAAGGGCCACTTTTCAAACGGTTAGATTCCAGTGA
AGTTGTAAGCACACAGGGAACCTAAAAAAAAAAAAAACAGCCAAAATGTC
TGATCGCAAGGCCGTGATTAAAAATGCCGACATGAGCGAGGAGATGCAGC
AGGATGCCGTCGATTGTGCGACACAGGCCCTCGAGAAGTACAACATTGAA
AAGGACATTGCGGCCTACATCAAGAAGGAGTTCGACAAAAAATACAATCC
CACATGGCATTGCATTGTCGGTCGCAACTTTGGATCGTATGTCACACACG
AGACGCGCCACTTTATTTACTTCTATTTGGGCCAGGTGGCTATTTTACTG
TTTAAGAGCGGTTAAAGTATTGTCGAGTCGGATGAAGTGGTGGTGAGGAG
GCTGATGGAGATGCAGCAGCTGCCCCGCCAGCAGCAACAACAGCAGGGGC
AGCAGTCGCATTTCGGAGCATCAGAGGATGAGGATCTAGAGCAGAAACAG
CAACAACCA
 
MSDRKAVIKNADMSEEMQQDAVDCATQALEKYNIEKDIAAYIKKEFDKKY
NPTWHCIVGRNFGSYVTHETRHFIYFYLGQVAILLFKSG
**Human homologue of Complete Genome candidate** AAH10744 Similar to RIKEN cDNA 6720463E02 gene 
1 gctgtgaggc gccagtgcgg agcgggcggg cgggcgggcg ggcgggcggc gcgaggcgga
61 gcgcgggcgg ccggcgaaac tccaagggcg gaccgcggca gggagcgatc ggcctcgggc
121 tgcgggagcc ggagaccgcg gcggcggcgg ctgctgcagc tgcaggagga gcccagggaa
181 caccgcccct gcctgtgctc tgcctcgggc catcgctcct ccccagggcc cagtgcggac
241 tcgcctccgt gaagtgtcac accatgtctg accggaaggc agtgatcaag aacgcagaca
301 tgtctgagga catgcaacag gatgccgttg actgcgccac gcaggccatg gagaagtaca
361 atatagagaa ggacattgct gcctatatca agaaggaatt tgacaagaaa tataacccta
421 cctggcattg tatcgtgggc cgaaattttg gcagctacgt cacacacgag acaaagcact
481 tcatctattt ttacttgggt caagttgcaa tcctcctctt caagtcaggc taggtggcca
541 tggtgaaggt gtcagtggcg gcggcagcga tggcaagcag gcggcgttgc tgggactgtt
601 ttgcactgga gccagcatca ggatgtcctc tccaatggct gtgctactgc atggactgta
661 tactcgattt catgtgtatg tcgcagtaaa caaaaccaaa cctcaaaaaa aaaaaaaaaa
721 aaaaaaaaaa aaaaa
 
1 msdrkavikn admsedmqqd avdcatqame kyniekdiaa yikkefdkky nptwhcivgr
61 nfgsyvthet khfiyfylgq vaillfksg
**Putative function** Dynein light chain, a microtubule motor protein

### Example 7 (Category 2)

**Line ID** - bbl-E1
**Phenotype** - Male sterile. Asynchronous meiotic divisions, cysts with large Nebenkern and 1-2 larger nuclei, testis from 2-3 old males become smaller. High mitotic index, colchicine type overcondensaton, many anaphases and telophases, no decondensation in telophase. Also has a mitotic phenotype: High mitotic index, colchicines-type overcondensed chromosomes, many ana- and relophases, no decondensation in telophase
**Annotated *Drosophila* genome genomic segment containing P element insertion site (and map position)** - AE003431 (4E)
**P element insertion site** - not determined
**Annotated *Drosophila* genome Complete Genome candidate** CG2984 - Pp2C 1 protein phosphatase 
TGTTCGCAAGTCGAGAGCAGAATCGAACGGCAAAAAATGCTGGCGAACAA
CAAATCATCAAGGTAAAACTGCGCGCCTTGGTCATTAAGTCTTTCATCGA
GGATAAAAGACCGATGTCTTTAACGTTATTGCTGTAAGCAAAAGCAGAA
ATCACAATCTACTCATAAATCCTCGATTTGGTGCAAATTAAAGGAAATTC
ATCGGTTTTTGGCGGCCAGTTGCAAACACAAAATACTAAATACGCTAGAT
GGAGCACGCATACACGCAAGCTCGTTGGCGAACGTAAATTACATACATCA
TATAGATAGTCGTCCCGCTTGCACTGCCCGTCACAGCGAGGGCTGCGAGA
GCGAGAGCGGGAGAGAGAAAGGCCTGAGTCGCTTTTTCTTCTTGTACTTT
ATATATTTTTTATTGTTTTTTGTGTTGTGTTGCGTTGTACGTGTGTGTG
AGAGTGCCAAATGTCAACGGAAATTACAACACTGCGAGACGGAGAAGTCT
AAAAGGCAGAAGAAGAAGCAGCAGCAGGCAGCATAAACAAAACTCGG
GGGAAAAATGTTGCCCGCCAATAACAGGAGTAGCACCAGCACCCATACCA
ACACAAATGCCAACACAATCAACGCCACTACCAATACCACCAACAGATGC
CTCATCAATACGGCCATCGAAAAAACGGTAGTCCGTTTGCGAGAGACGGC
AGCGAATAGCGCACCAGCTCCAGCCACAGCCTCCGTTACTCGCCACGGCG
GCAGCAGCAGCGGCAATAACAACAATAACAGTGCATGCCATCCAGCACTG
GATGCCAGCAGTGATGTTGTTGTTGAACCGGCAGCGGTAGGAGTCGC
ACAGGAGGAAGAGGAAGAGCCGGAGCAAAGGCCAGAGAGGATCAGCATAC
CCATTCCCGACCTGGCGTTCACCGAGATGGAAGCATATGCCGAGGATATA
GTCGTCGATATGGAGGGGGGATCACCAGCCAAGCCTTTAAATCCAAAGAA
ACAACGTTTAAACTCAGCAACAACCACAACAATAAATCGCTCGAGGGGCG
GCGGAGCGGCACAGAGTCGATTACGCCGGTCGGCGGCCATCGTTCCACCG
CGATCGATTCCAGAGAGCTGTGCCAGCAGCAGCAATTCCAATTCGAGCAG
CAGTTCCAACAGTAATTCCAGTTCCAGCTCCGCTACAGGAAGTAGCGCAT
CCACCGGCAATCCGTCGCCGTGCTCCTCCCTGGGCGTCAATATGCGCGTA
ACTGGACAATGCTGCCAGGGAGGCCGGAAATACATGGAGGATCAGTTCTC
GGTGGCCTACCAGGAATCACCGATCACCCACGAACTGGAATACGCATTTT
TTGGCATCTACGACGGACACGGCGGTCCCGAGGCCGCGCTCTTCGCCAAG
GAGCACCTTATGCTCGAGATCGTCAAGCAGAAGCAGTTCTGGTCTGATCA
GGATGAGGATGTCCTGCGGGCAATACGCGAGGGATACATCGCCACACATT
TCGCCATGTGGCGGGAACAAGAGAAATGGCCACGCACTGCCAATGGGCAT
 
CTGAGCACCGCCGGCACCACCGCCACAGTGGCCTTTATGCGTCGCGAGAA
GATCTACATTGGTCATGTGGGTGATTCTGGGATCGTTTTGGGTTACCAGA
ACAAGGGCGAACGCAACTGGCGTGCTCGTCCACTGACCACGGACCACAAG
CCGGAGTCACTGGCAGAGAAGACGAGAATCCAGCGTTCCGGCGGCAATGT
TGCCATCAAATCGGGAGTTCCGCGAGTGGTATGGAACCGACCCAGGGACC
CAATGCATCGCGGTCCCATTCGCCGCAGAACTCTGGTAGATGAAATACCC
TTTTTGGCGGTGGCTCGTTCCCTGGGCGATCTCTGGAGCTACAATTCCCG
CTTCAAGGAATTCGTTGTGAGTCCCGATCCGGATGTCAAAGTGGTTAAAA
TAAATCCCAGTACCTTTAGATGCTTAATTTCGGCACCGATGGCCTGTGG
AATGTGGTGACCGCCCAGGAGGCGGTGGACAGTGTGCGCAAGGAGCATCT
AATCGGCGAGATACTCAACGAGCAGGACGTTATGAATCCCAGCAAGGCGC
TGGTGGATCAGGCCCTCAAAACCTGGGCCGCCAAGAAGATGCGTGCGGAC
AACACGTCCGTTGTGACTGTGATACTAACACCAGCGGCCCGCAATAATTC
GCCCACAACGCCAACACGTTCCCCATCCGCGATGGCACGCGACAATGATC
TGGAGGTGGAGCTACTGCTGGAGGAGGACGACGAGGAGCTGCCGACACTG
GATGTGGAGAACAACTACCCTGACTTTCTCATCGAGGAGCATGAGTATGT
GCTGGACCAGCCGTACAGTGCATTGGCCAAGCGACATTCGCCTCCGGAAG
CCTTCCGCAACTTCGACTACTTCGATGTGGACGAGGACGAGTTGGATGAA
GATGAGGAAACAGTGGAAGAAGACGAGGAGGAGGAGGAAGAGGAGGA
AACCAAATCGGTGGAATTCTACAGCAAAGTTTGTTCAACCCCAGAAAAA
CGTGGCGCAAGTCAACCATCAACAATTCCTGGAGTGGCGTCACCGAACCG
GAACCGGAACCCGATCCCGAACCAGATCGAATAGATGTCTTAACACTGGA
CATGTACTCCCACACCAGCATTGACAAGGGCACCAATTATGGCGGCAGCA
TAGCCCAGTCCTCAATAGATCCTGCGGAGACGGCTGAAAATCGTGAGCTG
AGTGAGTTGGAGCAGCATCTGGAGAGTAGCTACAGTTTCGCCGAGTCGTA
CAACTCCCTGTTAAACGAGCAGGAGGAGCAGGAGGCACGCTCACGTTCAG
CAGCAGCAGCAGCCGCCGCCGCAGAAGCAGCAGCAGTAGAAGCACAACAA
ACCACTGCCCATTCCGCATCCGTTGTGCTGGACCGCAGCATGTTGGAGAT
CATCCAGGAGCAGCAGCACTATCAGCAGCAAGAGGGCTATTCGCTAACGC
AACTAGAGACCAGACGTGAAAGGGAGCGGCTGACCGAATCGTGGCCACAG
CAGCCGGCTGAGCTGCTCGAGCTGGATGCTCTACTGCAGCAGGAGCGTGC
CGAGGAGGAGCAGGTAGCCCTGGAGCAGCAGCAGCAGCGCGAACAGCAAA
TGGAGCAAATGGAGGTGGAGGCCATTAGTAGTTCGGGACAGCACGAATTT
GCTTACCCAGTGACCACCGCCACAGCCAGCGAGTGGTGTGCTACATTACA
AGAAGACGAGGAGGAGTTGGACTCCACAGTAATAGACATAGTAATTCAAC
CCGAACAAGAGTTGCAGGACAATGAAGTGAGCTCCACGTTGCCCGCCACA
CCCACTCATGTGGAGCCTGAGCAGATTGTGGACAAGATGGAGCCCCTGAA
GGTTCAGGAGATGCTAACCGCGGTCGAAAAACCTCCATCCAAGCAGGAAA
AGAAGCTGCCGAAGAAGCAAGAGACCAAACAGGTTGCTGTGCTAGATACA
GTGGCCGAGATGCCCAAAGAGGATGCCCATGCCGTGCACTATATATTCCA
GCGCATTCAAAAGGTTCAGGACTCTGAGGCAACACCAGTGGCCGTGACGA
ATTCCACAATGGCTGACGCCCTGCCCACCGAATCTAGTGGACTGGGAGGA
TCTATGACCGCGCCCCGAATCCGACGCTATCGCAACGTGCCCAACGAGAA
CCATCAGCACATGCAGACGCGTCGTCGTCAGATCTTCAAGCATGTCAAGC
CAAAGTCCTTCATACAGTCCAGTGCTGCGGCGATTGTGGCCTATGGAGAC
AGCACCGAAACGGTCGGAGGAACAGCCGGAGCATCTGGCACACCTGCAGC
TGGGCGTGTAGGCGGGGGCGGTGGCGGCGGCGGCAGAGGATCGGCCA
GTGGTGGAGCAGTCCAGCGGTGGCAGCCAATAGTCGGCGGAGCGTCAAT
 
GTGGTGGCCAATGCGAGTGGAAACAGCGCTAGCAAAGTTGTGCCCAGCAG
CAGTTCCATGATGATGACCCGCCGCAGTCACACCTTGACGGCCAGCGGTG
GTGTGAACAAAAGGCAGCTGCGCAGCAGTCTCTGCACCTTGGGCCTGGGT
GTGGGTGTCGGTGTCGGTCTGGGCATGGACCTGGACATGACCAAGCGCAC
GCTAAGGACAAGGAATGTACCCGCTTTGTCGGGCGGTTCAGCCACGCCAT
CTAGCAATTCGTCGCCAGCCAGCGGAGGCAGCAGTCCAGCCGGTTTCACA
AGCCCAGCCAGTCCGGTCATCACGTCCAGGGGAAGCGGATCGCGTACTAC
CGCCTCGCCAGCCAGGCGCCTAAAACGCAGTCATGAGGATCGGGAGCAAA
GAATGAGCTTGCGACGGAGCACTCTGAGTGGCAGTGCCAGCGGCAGTGGG
CTGGTGGGCACTGGTGGGTCGCCCTCGAATGTGAAATCAAATCGCCTGCA
GGCCTGCAATGGAGCCATCTCTGCGCGTCCGCCGCCCTCGCCGAAGAAAC
TGAATGCAGCCGTGCCCACATTGGCAATTGGAACGCGTGCATATACGGCG
GCGTTGGCGGCGGCGGCGGATCACCTGAACAAGCGGTGGTCGTTGCGCAG
CAGCAGTGGCAACTCTGGCAATCTGATAACCGCCATCAGTTGCTACAGTG
ACAGGAGCAGGGCGGCGACTGCGGCGGGATCACCGGGATCTGGAGGCGGG
GCAGCGGGACCACCAGGAGCATCTTTGGCCGCATCCACAGTCGGCACGCG
AAGGCGCTAGGCTAGATTGTAACGAAACATGCGAGCAACTTGCAAGTACA
AATCCTAAGCAACGGAAAATTTTAGATCCTAGTATACTACTTTACTGAAA
ACGCAAAATTGCATAATTTAACCAATTTTTTATGTGCACAACACACACA
C
 
MLPANNRSSTSTHTNTNANTINATTNTTNRCLINTAIEKTVVRLRETAAN
SAPAPATASVTRHGGSSSGNNNNNSACHPALDASSDVVVVEPAAVGVAQE
EEEEPEQRPERISIPIPDLAFTEMEAYAEDIVVDMEGGSPAKPLNPKKQR
LNSATTTTINRSRGGGAAQSRLRRSAAIVPPRSIPESCASSSNSNSSSSS
NSNSSSSSATGSSASTGNPSPCSSLGVNMRVTGQCCQGGRKYMEDQFSVA
YQESPITHELEYAFFGIYDGHGGPEAALFAKEHLMLEIVKQKQFWSDQDE
DVLRAIREGYIATHFAMWREQEKWPRTANGHLSTAGTTATVAFMRREKIY
IGHVGDSGIVLGYQNKGERNWRARPLTTDHKPESLAEKTRIQRSGGNVAI
KSGVPRVVWNRPRDPMHRGPIRRRTLVDEIPFLAVARSLGDLWSYNSRFK
EFVVSPDPDVKVVKINPSTFRCLIFGTDGLWNVVTAQEAVDSVRKEHLIG
EILNEQDVMNPSKALVDQALKTWAAKKMRADNTSVVTVILTPAARNNSPT
TPTRSPSAMARDNDLEVELLLEEDDEELPTLDVENNYPDFLIEEHEYVLD
QPYSALAKRHSPPEAFRNFDYFDVDEDELDEDEETVEEDEEEEEEEEETK
SVGILQQSLFNPRKTWRKSTINNSWSGVTEPEPEPDPEPDRIDVLTLDMY
SHTSIDKGTNYGGSIAQSSIDPAETAENRELSELEQHLESSYSFAESYNS
LLNEQEEQEARSRSAAAAAAAAEAAAVEAQQTTAHSASVVLDRSMLEIIQ
EQQHYQQQEGYSLTQLETRRERERLTESWPQQPAELLELDALLQQERAEE
EQVALEQQQQREQQMEQMEVEAISSSGQHEFAYPVTTATASEWCATLQED
EEELDSTVIDIVIQPEQELQDNEVSSTLPATPTHVEPEQIVDKMEPLKVQ
EMLTAVEKPPSKQEKKLPKKQETKQVAVLDTVAEMPKEDAHAVHYIFQRI
QKVQDSEATPVAVTNSTMADALPTESSGLGGSMTAPRIRRYRNVPNENHQ
HMQTRRRQIFKHVKPKSFIQSSAAAIVAYGDSTETVGGTAGASGTPAAGR
VGGGGGGGGRGSASGGSSPAVAANSRRSVNVVANASGNSASKVVPSSSS
MMMTRRSHTLTASGGVNKRQLRSSLCTLGLGVGVGVGLGMDLDMTKRTLR
TRNVPALSGGSATPSSNSSPASGGSSPAGFTSPASPVITSRGSGSRTTAS
PARRLKRSHEDREQRMSLRRSTLSGSASGSGLVGTGGSPSNVKSNRLQAC
NGAISARPPPSPKKLNAAVPTLAIGTRAYTAALAAAADHLNKRWSLRSSS
 GNSGNLITAISCYSDRSRAATAAGSPGSGGGAAGPPGASLAASTVGTRRR
**Human homologue of Complete Genome candidate** AAB61637 Wip1 
1 ctggctctgc tcgctccggc gctccggccc agctctcgcg gacaagtcca gacatcgcgc
61 gcccccccctt ctccgggtcc gccccctccc ccttctcggc gtcgtcgaag ataaacaata
121 gttggccggc gagcgcctag tgtgtctccc gccgccggat tcggcgggct gcgtgggacc
181 ggcgggatcc cggccagccg gccatggcgg ggctgtactc gctgggagtg agcgtcttct
241 ccgaccaggg cgggaggaag tacatggagg acgttactca aatcgttgtg gagcccgaac
301 cgacggctga agaaaagccc tcgccgcggc ggtcgctgtc tcagccgttg cctccgcggc
361 cgtcgccggc cgcccttccc ggcggcgaag tctcggggaa aggcccagcg gtggcagccc
421 gagaggctcg cgaccctctc ccggacgccg gggcctcgcc ggcacctagc cgctgctgcc
481 gccgccgttc ctccgtggcc tttttcgccg tgtgcgacgg gcacggcggg cgggaggcgg
541 cacagtttgc ccgggagcac ttgtggggtt tcatcaagaa gcagaagggt ttcacctcgt
601 ccgagccggc taaggtttgc gctgccatcc gcaaaggctt tctcgcttgt caccttgcca
661 tgtggaagaa actggcggaa tggccaaaga ctatgacggg tcttcctagc acatcaggga
721 caactgccag tgtggtcatc attcggggca tgaagatgta tgtagctcac gtaggtgact
781 caggggtggt tcttggaatt caggatgacc cgaaggatga ctttgtcaga gctgtggagg
841 tgacacagga ccataagcca gaacttccca aggaaagaga acgaatcgaa ggacttggtg
901 ggagtgtaat gaacaagtct ggggtgaatc gtgtagtttg gaaacgacct cgactcactc
961 acaatggacc tgttagaagg agcacagtta ttgaccagat tccttttctg gcagtagcaa
1021 gagcacttgg tgatttgtgg agctatgatt tcttcagtgg tgaatttgtg gtgtcacctg
1081 aaccagacac aagtgtccac actcttgacc ctcagaagca caagtatatt atattgggga
1141 gtgatggact ttggaatatg attccaccac aagatgccat ctcaatgtgc caggaccaag
1201 aggagaaaaa atacctgatg ggtgagcatg gacaatcttg tgccaaaatg cttgtgaatc
1261 gagcattggg ccgctggagg cagcgtatgc tccgagcaga taacactagt gccatagtaa
1321 tctgcatctc tccagaagtg gacaatcagg gaaactttac caatgaagat gagttatacc
1381 tgaacctgac tgacagccct tcctataata gtcaagaaac ctgtgtgatg actccttccc
1441 catgttctac accaccagtc aagtcactgg aggaggatcc atggccaagg gtgaattcta
1501 aggaccatat acctgccctg gttcgtagca atgccttctc agagaatttt ttagaggttt
1561 cagctgagat agctcgagag aatgtccaag gtgtagtcat accctcaaaa gatccagaac
1621 cacttgaaga aaattgcgct aaagccctga ctttaaggat acatgattct ttgaataata
1681 gccttccaat tggccttgtg cctactaatt caacaaacac tgtcatggac caaaaaaatt
1741 tgaagatgtc aactcctggc caaatgaaag cccaagaaat tgaaagaacc cctccaacaa
1801 actttaaaag gacattagaa gagtccaatt ctggcccccct gatgaagaag catagacgaa
1861 atggcttaag tcgaagtagt ggtgctcagc ctgcaagtct ccccacaacc tcacagcgaa
1921 agaactctgt taaactcacc atgcgacgca gacttagggg ccagaagaaa attggaaatc
1981 ctttacttca tcaacacagg aaaactgttt gtgtttgctg aaatgcatct gggaaatgag
2041 gtttttccaa acttaggata taagagggct ttttaaattt ggtgccgatg ttgaactttt
2101 tttaagggga gaaaattaaa agaaatatac agtttgactt tttggaattc agcagtttta
2161 tcctggcctt gtacttgctt gtattgtaaa tgtggatttt gtagatgtta gggtataagt
2221 tgctgtaaaa tttgtgtaaa tttgtatcca cacaaattca gtctctgaat acacagtatt
2281 cagagtctct gatacacagt aattgtgaca atagggctaa atgtttaaag aaatcaaaag
2341 aatctattag attttagaaa aacatttaaa ctttttaaaa tacttattaa aaaatttgta
2401 taagccactt gtcttgaaaa ctgtgcaact ttttaaagta aattattaag cagactggaa
2461 aagtgatgta ttttcatagt gacctgtgtt tcacttaatg tttcttagag ccaagtgtct
 
2521 tttaaacatt atttttattt tctgatttca taattcagaa ctaaattttt catagaagtg
2581 ttgagccatg ctacagttag tcttgtccca attaaaatac tatgcagtat ctcttacatc
2641 agtagcattt ttctaaaacc ttagtcatca gatatgctta ctaaatcttc agcatagaag
2701 gaagtgtgtt tgcctaaaac aatctaaaac aattcccttc tttttcatcc cagaccaatg
2761 gcattattag gtcttaaagt agttactccc ttctcgtgtt tgcttaaaat atgtgaagtt
2821 ttccttgcta tttcaataac agatggtgct gctaattccc aacatttctt aaattatttt
2881 atatcataca gttttcattg attatatggg tatatattca tctaataaat cagtgaactg
2941 ttcctcatgt tgctgaaaaa aaaaaaaaaa aaa
 
1 
61 
121 
181 
241 
301 
361 
421 
481 
541 
601 
**Putative function** Protein phosphatase, with p53 dependent expression, so may be inhibitory to division

### Example 8 (Category 2)

**Line ID** - ms(1)04
**Phenotype** - Cytokinesis detect, small testis, no meiosis observed, variable
sized Nebenkerns with 2-4N nuclei
**Annotated *Drosophila* genome genomic segment containing P element insertion site (and map position)** - AE003442 (7C-D)
**P element insertion site -** not determined
**Annotated *Drosophila* genome Complete Genome candidate** CG1524 - RpS14A ribosomal protein (2 splice variants) 
GATATCCGGTTAACGCAAGTGTTGCTGATCGACAAACAAACCCAGAATGG
CACCCAGGAAGGCTAAAGTTCAGAAGGAGGAGGTTCAGGTCCAGCTGGGA
CCCCAAGTTCGCGACGGCGAGATCGTGTTCGGAGTGGCTCACATCTACGC
CAGCTTCAACGACACCTTCGTCCATGTCACTGATCTGTCCGGCCGTGAGA
CCATCGCTCGTGTCACCGGAGGCATGAAGGTGAAGGCCGATCGTGATGAG
GCTTCGCCCTACGCCGCTATGTTGGCCGCTCAGGATGTGGCTGAGAAGTG
CAAGACACTGGGCATTACTGCCCTGCATATTAAGCTGCGTGCCACCGGCG
GCAACAAGACCAAGACCCCCGGACCCGGCGCCCAGTCCGCTCTGCGTGCT
TTGGCCCGTTCGTCCATGAAGATTGGCCGCATCGAGGATGTGACGCCCAT
CCCATCGGACTCCACCCGCAGGAAGGGCGGTCGCCGTGGTCGTCGTCTGT
AGATGGCAGTATCTGGAAAGCAGTAGTCTATGTTTGCGGTCGAAATACAA
TACTGC
 
MAPRKAKVQKEEVQVQLGPQVRDGEIVFGVAHIYASFNDTFVHVTDLSGR
ETIARVTGGMKVKADRDEASPYAAMLAAQDVAEKCKTLGITALHIKLRAT
GGNKTKTPGPGAQSALRALARSSMKIGRIEDVTPIPSDSTRRKGGRRGRR
L
 
CAAGTGGTTCGTCTTTAATTTTTCCCTCTTAATTTTTGCGAAAAAAAACC
CGACTTTGAGCCCCTAAACTTAAAAAATGTGCCTTCCTCCAGAGTGTTCA
GAGCGTCGACTGAAAATGACAAACAAGCTGCCCGGCAGCTAATTTTTTT
TACATTTTTGTTTTGTTTGTTCGCACGCATTTGTTTTTATTTGTGAAAC
ACGTGGTATAAATGTGGAAATTCCCTTGCTATTCCCGCAGTTGCTGATCG
ACAAACAAACCCAGAATGGCACCCAGGAAGGCTAAAGTTCAGAAGGAGGA
GGTTCAGGTCCAGCTGGGACCCCAAGTTCGCGACGGCGAGATCGTGTTCG
GAGTGGCTCACATCTACGCCAGCTTCAACGACACCTTCGTCCATGTCACT
GATCTGTCCGGCCGTGAGACCATCGCTCGTGTCACCGGAGGCATGAAGGT
GAAGGCCGATCGTGATGAGGCTTCGCCCTACGCCGCTATGTTGGCCGCTC
AGGATGTGGCTGAGAAGTGCAAGACACTGGGCATTACTGCCCTGCATATT
AAGCTGCGTGCCACCGGCGGCAACAAGACCAAGACCCCCGGACCCGGCGC
CCAGTCCGCTCTGCGTGCTTTGGCCCGTTCGTCCATGAAGATTGGCCGCA
TCGAGGATGTGACGCCCATCCCATCGGACTCCACCCGCAGGAAGGGCGGT
CGCCGTGGTCGTCGTCTGTAGATGGCAGTATCTGGAAAGCAGTAGTCTAT
 GTTTGCGGTCGAAATACAATACTGC 
MAPRKAKVQKEEVQVQLGPQVRDGEIVFGVAHIYASFNDTFVHVTDLSGR
ETIARVTGGMKVKADRDEASPYAAMLAAQDVAEKCKTLGITALHIKLRAT
GGNKTKTPGPGAQSALRALARSSMKIGRIEDVTPIPSDSTRRKGGRRGRR
L
**Human homologue of Complete Genome candidate** A25220 ribosomal protein S14, cytosolic 
1 ctccgccctc tcccactctc tctttccggt gtggagtctg gagacgacgt gcagaaatgg
61 cacctcgaaa ggggaaggaa aagaaggaag aacaggtcat cagcctcgga cctcaggtgg
121 ctgaaggaga gaatgtattt ggtgtctgcc atatctttgc atccttcaat gacacttttg
181 tccatgtcac tgatctttct ggcaaggaaa ccatctgccg tgtgactggt gggatgaagg
241 taaaggcaga ccgagatgaa tcctcaccat atgctgctat gttggctgcc caggatgtgg
301 cccagaggtg caaggagctg ggtatcaccg ccctacacat caaactccgg gccacaggag
361 gaaataggac caagacccct ggacctgggg cccagtcggc cctcagagcc cttgcccgct
421 cgggtatgaa gatcgggcgg attgaggatg tcacccccat cccctctgac agcactcgca
481 ggaagggggg tcgccgtggt cgccgtctgt gaacaagatt cctcaaaata ttttctgtta
541 ataaattgcc ttcatgtaaa aaaaaaaaaa aaaaaaaaaa aaaaaaaaa
 
1 
61 
121 
**Putative function** Ribosomal protein

### Example 9 (Category 2)

**Line ID** - thb-a
**Phenotype** - Male sterile. Cytokinesis defect, larger Nebenkerns with 2-4N nuclei
**Annotated *Drosophila* genome genomic segment containing P element insertion site (and map position)** - (10B1-2)
**P element insertion site** - not determined
**Annotated *Drosophila* genome Complete Genome candidate** 2 candidates: CG1453 - kinesin-like protein KIF2 homolog 
AAACTAAAAAATTGTGTTGCTGACATCTGGTCGCTTGCAAAACTATTTCT
AGCAGATTTTGTGATATTTCGTTGTGATCGGTCGATAAATCCGCCAGTTT
TTTTTTAATGGAAAGTGCTAACACATTGTAGCGGTTGGGAAGATAGCAG
GAAAGAGCCAGCGGGCTGCCGTTTTTCCTTTTTGTTATCCGTTGCCAGAC
GCAACGAAAACGACAGTTGGCATTTGAATTCAGCACAAACACACATACTA
ACGCCGACCCGCAAGCAGCACACACACACACTGGGACACTCGAAAAAA
AAAAAACAGACGCTGTCGGCGACCTCGACAAGCAGTTGGGTTCGATTTAG
TTGTCAATGCCTTGAATTCGGTTCGGGGCTTAGTTTCCACAAGTTTATCG
CTCGTCAAGAAACAACGAAATAAAATTATTTCGACCTAAAAAATCTGAC
TAAATTGTGTTTTTTGTTTATGTATTTATTTAGGCACATTTTGCACACCA
CAACGTAGTTACTACATCTACGACTAACGGAACTCCTCCTGCAAGCAGTG
GAAGTTGCTGTCCATCAAGCAGTACTCGGAGTTAACGCAGGATAAGCCGG
GAGAAAGAGAAAGAGATCGGTGGAGAATAGAGATATACAGGTGGAGTCAA
AGAGGAAGGATCATGGACATGATTACGGTGGGGCAGAGCGTCAAGATCAA
GCGGACGGATGGCCGCGTCCACATGGCCGTGGTGGCGGTGATCAACCAGT
CGGGCAAGTGCATCACAGTCGAATGGTACGAGCGCGGCGAAACGAAGGGC
AAGGAGGTAGAACTGGACGCCATACTCACGCTCAATCCGGAGCTAATGCA
AGATACTGTCGAACAGCACGCCGCCCCGGAGCCCAAGAAACAAGCCACCG
CGCCGATGAACCTCTCGCGTAATCCCACACAATCGGCTATCGGTGGCAAT
CTCACCAGCCGTATGACCATGGCCGGAAACATGCTGAACAAGATCCAGGA
AAGCCAGTCGATTCCCAATCCGATTGTCAGCAGCAATAGCGTGAATACAA
ACAGCAACTCCAACACTACGGCCGGCGGAGGTGGTGGCACCACAACGTCG
ACGACCACTGGATTACAGCGTCCACGGTACTCGCAAGCTGCTACCGGCCA
GCAGCAGACAAGGATCGCCTCGGCGGTGCCTAATAACACATTGCCCAATC
CCAGCGCGGCAGCCAGTGCTGGTCCGGCGGCACAAGGAGTCGCCACTGCG
GCCACAACCCAGGGAGCTGGCGGCGCTAGTACCCGGCGATCGCACGCATT
GAAAGAGGTGGAGCGACTGAAGGAGAATCGCGAGAAGCGACGCGCCCGAC
AGGCCGAGATGAAGGAGGAGAAGGTGGCGCTGATGAACCAGGATCCGGGC
AATCCAAACTGGGAGACGGCGCAAATGATACGCGAATATCAGAGCACGCT
GGAATTTGTGCCGCTGCTCGATGGCCAGGCCGTCGATGACCATCAGATCA
CAGTGTGCGTGCGCAAGCGTCCCATTAGCCGCAAGGAGGTCAATCGCAAG
GAGATCGATGTCATTTCGGTGCCGCGCAAGGACATGCTCATCGTGCACGA
GCCGCGCAGCAAGGTCGACCTCACCAAGTTCCTGGAGAACCACAAGTTTC
GCTTCGACTACGCCTTCAACGACACGTGCGACAATGCCATGGTATACAAA
 
TACACAGCCAAGCCGTTGGTGAAAACCATTTCGAGGGCGGAATGGCGAC
GTGCTTCGCCTACGGCCAGACGGATCGGCAAAACGCACACCATGGGCG
GTGAGTTTAATGGAAAGGTGCAGGACTGCAAGAACGGCATCTACGCCATG
GCGGCCAAGGATGTCTTTGTGACCCTGAATATGCCGCGTTACCGCGCCAT
GAATCTAGTCGTCTCGGCCAGTTTCTTTGAGATTTACAGTGGCAAGGTCT
TCGATCTTCTGTCCGACAAGCAGAAACTGCGCGTCCTGGAGGATGGTAAA
CAGCAAGTGCAGGTGGTGGACTCACCGAGAAGGTGGTCGATGGCGTCGA
GGAGGTACTGAAGCTCATCCAGCACGGCAATGCTGCCCGAACATCCGGCC
AGACGTCGGCCAACTCCAATTCGTCGCGTTCGCACGCCGTTTTCCAGATT
GTGCTGCGGCCGCAGGGCTCGACGAAGATCCATGGCAAGTTCTCGTTCAT
CGATCTGGCGGGCAATGAGCGGGGCGTGGACACTTCCTCGGCCGATCGGC
AGACGCGTATGGAGGGTGCCGAGATTAACAAATCGCTGCTGGCCCTCAAG
GAGTGCATTCGTGCGTTGGGCAAACAGTCGGCCCACTTGCCCTTCCGTGT
CTCCAAACTCACCCAGGTGCTGCGCGACTCGTTCATTGGCGAGAAGAGCA
AGACGTGCATGATAGCCATGATCTCGCCGGGACTTAGCTCCTGCGAGCAC
ACGCTCAACACGCTGCGCTATGCGGATCGTGTCAAGGAGCTGGTGGTCAA
GGATATCGTCGAAGTTTGCCCTGGCGGCGACACCGAGCCCATCGAGATCA
CGGACGACGAGGAGGAGGAGGAGCTCAACATGGTGCATCCGCACTCGCAT
CAGCTGCATCCCAATTCGCATGCACCGGCCAGCCAGTCGAATAATCAGCG
TGCTCCGGCCTCTCATCACTCGGGGGCGGTCATTCACAACAATAATAATA
ACAACAACAAGAACGGAAACGCCGGCAACATGGACCTGGCCATGCTGAGT
TCGCTGAGCGAACACGAGATGTCCGACGAGCTGATTGTGCAGCACCAGGC
CATCGACGACCTGCAGCAGACGGAGGAGATGGTGGTGGAGTATCATCGCA
CCGTTAATGCCACACTGGAGACCTTCCTCGCCGAGTCGAAGGCGCTGTAC
AATCTGACCAACTATGTGGACTACGACCAGGACTCGTACTGCAAACGGGG
CGAGTCGATGTTCTCGCAGCTGCTGGACATCGCCATCCAGTGCCGCGACA
TGATGGCCGAATATCGCGCCAAGTTGGCCAAGGAGGAGATGCTGTCGTGC
AGCTTCAATTCGCCGAATGGCAAGCGTTAGT
 
1 mitvgqsvki krtdgrvhma vvavinqsgk citvewyerg etkgkeveld ailtlnpelm
61 qdtveqhaap epkkqatapm nlsrnptqsa iggnltsrmt magnmlnkiq esqsipnpiv
121 ssnsvntnsn snttaggggg tttstttglq rprysqaatg qqqtriasav pnntlpnpsa
181 aasagpaaqg vataattqga ggastrrsha lkeverlken rekrrarqae mkeekvalmn
241 qdpgnpnwet aqmireyqst lefvplldgq avddhqitvc vrkrpisrke vnrkeidvis
301 vprkdmlivh eprskvdltk flenhkfrfd yafndtcdna mvykytakpl vktifeggma
361 tcfaygqtgs gkthtmggef ngkvqdckng iyamaakdvf vtlnmpryra mnlvvsasff
421 eiysgkvfdl lsdkqklrvl edgkqqvqvv gltekvvdgv eevlkliqhg naartsgqts
481 ansnssrsha vfqivlrpqg stkihgkfsf idlagnergv dtssadrqtr megaeinksl
541 lalkeciral gkqsahlpfr vskltqvlrd sfigeksktc miamispgls scehtlntlr
601 yadrvkelvv kdivevcpgg dtepieitdd eeeeelnmvh phshqlhpns hapasqsnnq
661 rapashhsga vihnnnnnnn kngnagnmdl amlsslsehe msdelivqhq aiddlqqtee
721 mvveyhrtvn atletflaes kalynltnyv dydqdsyckr gesmfsqlld iaiqcrdmma
781 eyraklakee mlscsfnspn gkr
 CG18292-novel 
CGTAATAACGCCTCCTGATATCGATATCGATATCATATCACAAAAAACAA
TAAACCAAAAAAGAAACGCTAAAAACTAGTAGTTTTGTGTGCCAGGAAAA
 
CGGAAAGGTGGACATAGTTAAGTTACCACAACAACCGACGGATATCGACT
CCAGACACCACATCGCCCAGCGCCACCATGGACATCATGGATATCCAGGC
CGTAGAGTCCAAGCTGAGTGACGTCACGGTGACACCGATACCGCGCAGCC
AAGTGCAGAATTTCTACAATTACCAGCAGCAGCGGGAGCAGCGCGAGCAG
CAGCCCCAAATCCAGATATCGGCCATCCACCACTCGCGTGGATCCGTTGG
CGGAGGAGGCGGATCCAACTCATCCAACGCTGCCACCGACTACTCCACGA
GCAGCGGTGGCAAGCGGGAGCGGACCGCTCCTCCGCCAGCGACTACAGC
AGCTCGTCCAGCAAGCAGAGCTCCGCTGCAGCGGCCAATGCAGCAGCAGC
TGCCGCCGCCGTCGCTGCCCTCCAATACTCCCCGCAGTTCCTCCAGGCCC
AGCTGGCGCTACTCCAGCAGCAGTCGAACACGACGGCCACGCCGGCAGCC
GTCGCCGCTGCGGCCCTCTCGCTGGCCAACATGTGCTCCAGCAATGGTGG
TCAGCGGAATTCCGGTGCCGGCGTTTCCTCCACCTCCTCTGGCAGCAATG
GCCAGAGCATGGGCCTGAATCTGAGCTCATCGCAGCTAAAGTACCCGCCA
CCCTCCACCTCGCCCGTGGTGGTGACCACCCAAACTTCGGCCAATATCAC
CACGCCGCTGACCTCCACGGCCAGCCTGCCCTCAGTGGGCCCGGGCAATG
GGCTGACCAAGTACGCCCAGCTGCTGGCCGTCATTGAGGAGATGGGCCGC
GATATCCGGCCCACGTACACGGGCTCGCGCAGCTCCACGGAGCGTCTCAA
GCGGGGCATTGTCCATGCCCGCATCCTGGTGCGCGAATGCCTCATGGAAA
CGGAGCGTGCGGCGCGCCAATGA
 
1 mdiqaveskl sdvtvtpipr sqvqnfynyq qqreqreqqp qiqisaihhs rgsvgggggs
61 nssnaatdys tssggkrerd rssasdysss sskqssaaaa naaaaaaava alqyspqflq
121 aqlallqqqs nttatpaava aaalslanmc ssnggqrnsg agvsstssgs ngqsmglnls
181 ssqlkyppps tspvvvttqt sanittplts taslpsvgpg ngltkyaqll avieemgrdi
241 rptytgsrss terlkrgivh arilvreclm eteraarq
**Human homologue of Complete Genome candidate** (CG1453) - CAA69621 - kinesin-2 
1 ggccgaatac atcaagcaat ggtaacatct ttaaatgaag ataatgaaag tgtaactgtt
61 gaatggatag aaaatggaga tacaaaaggc aaagagattg acctggagag catcttttca
121 cttaaccctg accttgttcc tgatgaagaa attgaaccca gtccagaaac acctccacct
181 ccagcatcct cagccaaagt aaacaaaatt gtaaagaatc gacggactgt agcttctatt
241 aagaatgacc ctccttcaag agataataga gtggttggtt cagcacgtgc acggcccagt
301 caatttcctg aacagtcttc ctctgcacaa cagaatggta gtgtttcaga tatatctcca
361 gttcaagctg caaaaaagga atttggaccc ccttcacgta gaaaatctaa ttgtgtgaaa
421 gaagtagaaa aactgcaaga aaaacgagag aaaaggagat tgcaacagca agaacttaga
481 gaaaaaagag cccaggacgt tgatgctaca aacccaaatt atgaaattat gtgtatgatc
541 agagacttta gaggaagttt ggattataga ccattaacaa cagcagatcc tattgatgaa
601 cataggatat gtgtgtgtgt aagaaaacga ccactcaata aaaaagaaac tcaaatgaaa
661 gatcttgatg taatcacaat tcctagtaaa gatgttgtga tggtacatga accaaaacaa
721 aaagtagatt taacaaggta cctagaaaac caaacatttc gttttgatta tgcctttgat
781 gactcagctc ctaatgaaat ggtttacagg tttactgcta aaccactagt ggaaactata
841 tttgaaaggg gaatggctac atgctttgct tatgggcaga ctggaagtgg aaaaactcat
901 actatgggtg gtgacttttc aggaaagaac caagattgtt ctaaaggaat ttatgcatta
961 gcagctcgag atgtcttttt aatgctaaag aagccaaact ataagaagct agaacttcaa
 
1021 gtatatgcaa ccttctttga aatttatagt ggaaaggtgt ttgacttgct aaacaggaaa
1081 acaaaattaa gagttctaga agatggaaaa cagcaggttc aagtggtggg attacaggaa
1141 cgggaggtca aatgtgttga agatgtactg aaactcattg acataggcaa cagttgcaga
1201 acatccggtc aaacatctgc aaatgcacat tcatctcgga gccatgcagt gtttcagatt
1261 attcttagaa ggaaaggaaa actacatggc aaattttctc tcattgattt ggctggaaat
1321 gaaagaggag ctgatacttc cagtgcggac aggcaaacta ggcttgaagg tgctgaaatt
1381 aataaaagcc ttttagcact caaggagtgc atcagagcct taggtagaaa taaacctcat
1441 actcctttcc gtgcaagtaa actcactcag gtgttaagag attctttcat aggtgaaaac
1501 tctcgtacct gcatgattgc cacaatctct ccaggaatgg catcctgtga aaatactctt
1561 aatacattaa gatatgcaaa tagggtcaaa gaattgactg tagatccaac tgctgctggt
1621 gatgttcgtc caataatgca ccatccacca aaccagattg atgacttaga gacacagtgg
1681 ggtgtgggga gttcccctca gagagatgat ctaaaacttc tttgtgaaca aaatgaagaa
1741 gaagtctctc cacagttgtt tactttccac gaagctgttt cacaaatggt agaaatggaa
1801 gaacaagttg tagaagatca cagggcagtg ttccaggaat ctattcggtg gttagaagat
1861 gaaaaggccc tcttagagat gactgaagaa gtagattatg atgtcgattc atatgctaca
1921 caacttgaag ctattcttga gcaaaaaata gacattttaa ctgaactgcg ggataaagtg
1981 aaatctttcc gtgcagctct acaagaggag gaacaagcca gcaagcaaat caacccgaag
2041 agaccccgtg ccctttaaac cggcatttgc tgctaaagga tacccagaac cctcactact
2101 gtaacataca acggttcagc tgtaagggcc atttgaaagt ttggaatttt aagtgtctgt
2161 ggaaaatgtt ttgtccttca cctgaattac atttcaattt tgtgaaacac tcttttgtct
2221 acaaaatgct tctagtccag gaggcacaac caagaactgg gattaatgaa gcattttgtt
2281 tcatttacac aaatagtgat ttacttttgg agatccttgt cagttttatt ttctatttga
2341 tgaagtaaga ctgtggactc aatccagagc cagatagtag gggaagccac agcatttcct
2401 tttaactcag ttcaattttt gtagtgagac tgagcagttt taaatccttt gcgtgcatgc
2461 atacctcatc agtgattgta cataccttgc ccactcctag agacagctgt gctcactttt
2521 cctgctttgt gccttgatta aggctactga ccctaaattt ctgaagcaca gccaagaaaa
2581 attacattcc ttgtcattgt aaattacctt tgtgtgtaca tttttactgt atttgagaca
2641 ttttttgtgt gtgactagtt aattttgcag gatgtgccat atcattgaac ggaactaaag
2701 tctgtgacag tggatatagc tgctggacca ttccatctta tatgtaaaga aatctggaat
2761 tattatttta aaaccatata acatgtgatt ataatttttc ttagcatttt ctttgtaaag
2821 aactacaata taaactagtt ggtgtataat aaaaagtaat gaaattctga agaaaaaaaa
2881 aaaaaaaaaa aaaaaaaaaa aaaaa
 
1 mvtslnedne svtvewieng dtkgkeidle sifslnpdlv pdeeiepspe tppppassak
61 vnkivknrrt vasikndpps rdnrvvgsar arpsqfpeqs ssaqqngsvs dispvqaakk
121 efgppsrrks ncvkevekl q ekrekrrlqq qelrekraqd vdatnpnyei mcmirdfrgs
181 ldyrplttad pidehricvc vrkrplnkke tqmkdldvit ipskdvvmvh epkqkvdltr
241 ylenqtfrfd yafddsapne mvyrftakpl vetifergma tcfaygqtgs gkthtmggdf
301 sgknqdcskg iyalaardvf lmlkkpnykk lelqvyatff eiysgkvfdl lnrktklrvl
361 edgkqqvqvv glqerevkcv edvlklidig nscrtsgqts anahssrsha vfqiilrrkg
421 klhgkfslid lagnergadt ssadrqtrle gaeinkslla lkeciralgr nkphtpfras
481 kltqvlrdsf igensrtcmi atispgmasc entlntlrya nrvkeltvdp taagdvrpim
541 hhppnqiddl etqwgvgssp qrddlkllce qneeevspql ftfheavsqm vemeeqvved
601 hravfqesir wledekalle mteevdydvd syatqleail eqkidiltel rdkvksfraa
661 lqeeeqaskq inpkrpral
 (CG18292) - BAA22937 - cdk2-associated protein 1; cdk2ap1, deleted in oral cancer 1 (doc-1, alias DORC1) 
1 accgcccggc ctcgccgccg ccgccgccgc cctcgcggcc tggccccgcc gcgcccggcg
61 cgcccgccgc ccggggggat gtcttacaaa ccgaacttgg ccgcgcacat gcccgccgcc
121 gccctcaacg ccgctgggag tgtccactcg ccttccacca gcatggcaac gtcttcacag
181 taccgccagc tgctcagtga ctacgggcca ccgtccctag gctacaccca gggaactggg
241 aacagccagg tgccccaaag caaatacgcg gagctgctgg ccatcattga agagctgggg
301 aaggagatca gacccacgta cgcagggagc aagagtgcca tggagaggct gaagcgcggc
361 atcattcacg ctagaggact ggttcgggag tgcttggcag aaacggaacg gaatgccaga
421 tcctagctgc cttgttggtt ttgaaggatt tccatctttt tacaagatga gaagttacag
481 ttcatctccc ctgttcagat gaaacccttg ttttcaaaat ggttacagtt tcgtttttcc
541 tcccatggtt cacttggctc tgaacctaca gtctcaaaga ttgagaaaag attttgcagt
601 taattaggat ttgcatttta agtagttagg aactgcccag gttttttttg tttttaagc
661 attgatttaa aagatgcacg gaaagttatc ttacagcaaa ctgtagtttg cctccaagac
721 accattgtct ccctttaatc ttctcttttg tatacatttg ttacccatgg tgttctttgt
781 tccttttcat aagctaatac cactgtaggg attttgtttt gaacgcatat tgacagcacg
841 ctttacttag tagccggttc ccatttgcca tacaatgtag gttctgctta atgtaacttc
901 tttttgctt aagcatttgc atgactatta gtgcttcaaa gtcaattttt aaaaatgcac
961 aagttataaa tacagaagaa agagcaaccc accaaaccta acaaggaccc ccgaacactt
1021 tcatactaag actgtaagta gatctcagtt ctgcgtttat tgtaagttga taaaaacatc
1081 tgggaggaaa tgactaaaac tgtttgcatc tttgtatgta tttattactt gatgtaataa
1141 agcttatttt cattaacc
 
1 msykpnlaah mpaaalnaag svhspstsma tssqyrqlls dygppslgyt qgtgnsqvpq
61 skyaellaii eelgkeirpt yagsksamer lkrgiiharg lvreclaete mars
**Putative function** (CG1453) - Motor protein (CG18292) - Cdk2 associated, candidate tumour supressor

### Example 9A (Category 2)

**Line ID** - ms(l)13
**Phenotype** - Male sterile, Cytokinesis defect: variable sized Nebenkerns with 4N nuclei, some nuclei detached from Nebenkern
**Annotated *Drosophila* genome genomic segment containing P element insertion site (and map position) -** AE003436 (5D1)
**P element insertion site sequence** 
CATCATGTATCATACATTGAAGACGGATTAGCACCGTCGACCACGAAAAAAG
AACGCAAGGAAATCGTGCAAAATGTTCAAAAAGTACGTATGGCATGAGTTAG
ATGGGACATCAGACTAACCATAGCAATTCGATCTGTGCAGATTCGAAGAGA
AGGACAGCATTTCCAGCATTCAGCAGCTGAAGTCGTCTGTGCAGAAGGGCATA
CGTGCCAAGTTGCTGGAGGCCTATCCCAAGTTGGAGAGTCACATCGACCTGAT
CCTGCCCAAGAAGGACTCGTACCGCATCGCCAAGTGGTAGGATGGCTCAGTTC
TTGCCACAGCACATAACTCCATTCATATTCCCGATCCCTACTCCTCCACCAGCC
ATGACCACATCGAACTGCTGCTAAACGGAGCCGGCGACCAGGTGTTCTTTCGC
CACCGCGATGGCCCCTGGATGCCTACCCTGCGCAACTGTTGGGAAGGGCGATC
GGTGCGGGCCTCTTCGCTATTACGCCAGCTGGCGAAAGGGGGATGTGCTGCA
AGGCGATTAAGTTGGGTAACGCCAGGGTTTCCCAGNCACGACGTTGNAAAA
CGACGGNCANNGCCAAGCTCTGCTGCT
**Annotated *Drosophila* genome Complete Genome candidate -** CG5941- novel protein with a PUA domain 
CGGATTAGCACCGTCGACCACGAAAAAAGAACGCAAGGAAATCGTGCAAA
ATGTTCAAAAAATTCGAAGAGAAGGACAGCATTTCCAGCATTCAGCAGCT
GAAGTCGTCTGTGCAGAAGGGCATACGTGCCAAGTTGCTGGAGGCCTATC
CCAAGTTGGAGAGTCACATCGACCTGATCCTGCCCAAGAAGGACTCGTAC
CGCATCGCCAAGTGCCATGACCACATCGAACTGCTGCTAAACGGAGCCGG
CGACCAGGTGTTCTTTCGCCACCGCGATGGCCCCTGGATGCCTACCCTGC
GCCTCCTGCACAAGTTCCCCTACTTCGTGACCATGCAGCAAGTGGACAAA
GGCGCCATCCGCTTCGTCCTGAGCGGAGCGAACGTCATGTGTCCCGGCCT
CACATCGCCAGGCGCCTGTATGACGCCGGCCGACAAGGACACCGTGGTGG
CCATCATGGCTGAGGGCAAGGAGCACGCCCTGGCCGTTGGACTCCTCACG
TTATCCACACAGGAAATTCTGGCGAAGAACAAAGGCATCGGTATCGAGAC
GTACCACTTCCTCAACGACGGCCTGTGGAAGTCGAAGCCCGTGAAGTAGG
CGAAATAGGAATCTGCACTTGCACTTTTA
 
MFKKFEEKDSISSIQQLKSSVQKGIRAKLLEAYPKLESHIDLILPKKDSY
RIAKCHDHIELLLNGAGDQVFFRHRDGPWMPTLRLLHKFPYFVTMQQVDK
GAIRFVLSGANVMCPGLTSPGACMTPADKDTVVAIMAEGKEHALAVGLLT
LSTQEILAKNKGIGIETYHFLNDGLWKSKPVK
**Human homologue of Complete Genome candidate** MCT-1(multiple copies in a T-cell malignancies) (BAA86055), a novel candidate oncogene involved in cell cycle which has a domain similar to cyclin H 
1 gctacctcca actgctgagg aaccggttgc ctaaaaggag ccggcaaaag cgcctacgtg
61 gagtccagag gagcggaagt agtcagattt gactgagagc cgtaaagcgc ggctggctct
121 cgttttccgg ataacgacta cagctccgac tgtcagtgcc ggccttcctc gtgtgagggg
181 atctgccgga cccctgcaaa ttcaatttct ttcccattcc gggcccttcc ctatcgtcgc
241 ccccttcacc ttggatcatg ttcaagaaat ttgatgaaaa agaaaatgtg tccaactgca
301 tccagttgaa aacttcagtt attaagggta ttaagaatca attgatagag caatttccag
361 gtattgaacc atggcttaat caaatcatgc ctaagaaaga tcctgtcaaa atagtccgat
421 gccatgaaca tatagaaatc cttacagtaa atggagaatt actctttttt agacaaagag
481 aagggccttt ttatccaacc ctaagattac ttcacaaata tccttttatc ctgccacacc
541 agcaggttga taaaggagcc atcaaatttg tactcagtgg agcaaatatc atgtgtccag
601 gcttaacttc tcctggagct aagctttacc ctgctgcagt agataccatt gttgctatca
661 tggcagaagg aaaacagcat gctctatgtg ttggagtcat gaagatgtct gcagaagaca
721 ttgagaaagt caacaaagga attggcattg aaaatatcca ttatttaaat gatgggctgt
781 ggcatatgaa gacatataaa tgagcctcag aaggaatgca cttgggctaa atatggatat
841 tgtgctgtat ctgtgtttgt gtctgtgtgt gacagcatga agataatgcc tgtggttatg
901 ctgaataaat tcaccagatg ctaaaaaaaa aaaaaaaaaa aaa
 
1 mfkkfdeken vsnciqlkts vikgiknqli eqfpgiepwl nqimpkkdpv kivrchehie
61 iltvngellf frqregpfyp tlrllhkypf ilphqqvdkg aikfvlsgan imcpgltspg
121 aklypaavdt ivaimaegkq halcvgvmkm saediekvnk gigienihyl ndglwhmkty
181 k
**Putative function** Role in cell cycle progression

### CATEGORY 3 - MITOTIC (NEUROBLAST) PHENOTYPES

### Example 10 (Category 3)

**Line ID** - 187
**Phenotype** - lethal phase between pupil and pharate adult (P-pA). High mitotic index, rod-like overcondensed chromosomes, a few circular metaphases, many overcondensed anaphases and telophases, a few tetraploid cells
**Annotated *Drosophila* genome genomic segment containing P element insertion site (and map position)** - AE003445 (8B3-7)
**P element insertion site** - 174,362
**Annotated *Drosophila* genome Complete Genome candidate -** CG10701 moesin, cytoskeletal binding protein (4 splice variants) ACGCCGCATGCACTTTTTATCTATGATATTATGTTTATTATTTCATTAT 
TGAATCGGGAAAACCAAACGTTTTTTTTTTTCGTATACAAATCCATTT
GCAGTTTGTAAACTTTAGCGTGCATTCGCATCTAATAGTGATATGTTTTC
GCTTTTCACAGGTGATGAACCAGGACGTGAAGAAGGAGAATCCCTTGCAG
TTTAGGTTCCGTGCCAAATTCTATCCCGAGGATGTGGCCGAGGAGCTGAT
CCAGGACATTACACTGCGTCTGTTCTACCTGCAGGTGAAGAATGCCATAC
TGACCGACGAGATCTATTGTCCGCCAGAGACATCCGTGCTGCTCGCCTCG
TACGCCGTCCAGGCGCGTCATGGTGACCACAATAAGACCACCCACACAGC
CGGCTTTCTGGCCAACGATCGCCTGCTGCCGCAGCGCGTCATCGACCAGC
ACAAGATGTCCAAGGACGAGTGGGAGCAGTCGATTATGACCTGGTGGCAG
GAGCATCGCAGCATGCTGCGCGAGGATGCCATGATGGAGTATCTGAAGAT
CGCCCAAGACCTGGAGATGTACGGCGTTAACTACTTTGAGATCCGCAACA
AGAAGGGCACGGATCTTTGGCTGGGCGTAGACGCACTGGGTCTGAACATT
TACGAGCAGGACGATAGGTTGACGCCGAAAATTGGTTTCCCATGGTCCGA
GATTCGCAACATTTCGTTCTCGGAGAAGAAGTTCATCATCAAGCCGATCG
ACAAGAAGGCTCCGGACTTTATGTTCTTTGCGCCACGTGTCCGCATCAAC
AAGCGCATTCTGGCCCTCTGCATGGGCAACCACGAGCTGTACATGCGTCG
CCGCAAGCCGGACACCATCGATGTGCAGCAGATGAAGGCGCAGGCGCGCG
AGGAGAAGAATGCCAAACAGCAGGAACGTGAGAAGCTGCAGCTGGCGCTG
GCCGCACGCGAACGCGCTGAAAAGAAGCAGCAGGAGTACGAGGATCGGCT
AAAGCAGATGCAGGAGGACATGGAGCGTTCGCAGCGCGATCTGCTTGAGG
CGCAGGACATGATCCGCCGGCTGGAGGAGCAGCTGAAGCAGCTGCAGGCC
GCCAAGGATGAGCTGGAGCTGCGCCAGAAGGAGCTGCAGGCGATGCTGCA
GCGCCTCGAGGAGGCCAAGAATATGGAGGCCGTCGAGAAGCTCAAGCTCG
AGGAGGAGATCATGGCCAAGCAGATGGAGGTGCAGCGCATTCAGGACGAG
GTCAACGCCAAGGATGAGGAGACAAAGCGTCTGCAGGACGAAGTGGAAGA
CGCCCGACGCAAGCAGGTCATTGCGGCTGAAGCCGCTGCCGCTCTGCTGG
CCGCGTCGACAACGCCGCAGCATCACCACGTGGCCGAGGATGAGAACGAG
AACGAGGAGGAGCTGACGAACGGCGATGCCGGTGGCGATGTGTCGCGCGA
CCTGGACACCGACGAGCATATCAAGGACCCCATCGAGGACAGACGCACGC
TGGCCGAGCGCAACGAACGCTTGCACGATCAGCTCAAGGCTCTGAAACAA
GATTTGGCGCAGTCTCGCGACGAGACGAAAGAGACGGCAAACGATAAGAT
TCATCGCGAGAACGTTCGCCAGGGACGTGACAAGTACAAGACGCTCCGCG
AGATTCGTAAGGGCAACACAAAGCGTCGCGTCGATCAGTTTGAGAACATG
TAAAAGCTATCAAAGATCAGAGATCGATAGTGCGCGGAAAGAGAGAGGG
AGCGGTGAGACTCCAGAAAGA
 
MNQDVKKENPLQFRFRAKFYPEDVAEELIQDITLRLFYLQVKNAILTDEI
YCPPETSVLLASYAVQARHGDHNKTTHTAGFLANDRLLPQRVIDQHKMSK
DEWEQSIMTWWQEHRSMLREDAMMEYLKIAQDLEMYGVNYFEIRNKKGTD
LWLGVDALGLNIYEQDDRLTPKIGFPWSEIRNISFSEKKFIIKPIDKKAP
DFMFFAPRVRINKRILALCMGNHELYMRRRKPDTIDVQQMKAQAREEKNA
KQQEREKLQLALAARERAEKKQQEYEDRLKQMQEDMERSQRDLLEAQDMI
RRLEEQLKQLQAAKDELELRQKELQAMLQRLEEAKNMEAVEKLKLEEEIM
AKQMEVQRIQDEVNAKDEETKRLQDEVEDARRKQVIAAEAAAALLAASTT
PQHHHVAEDENENEEELTNGDAGGDVSRDLDTDEHIKDPIEDRRTLAERN
ERLHDQLKALKQDLAQSRDETKETANDKIHRENVRQGRDKYKTLREIRKG
NTKRRVDQFENM
 
GACAACAGAATCGAATCGTCGCTTTTCCGCTTTTAACCATCGTGTCGCGT
TGGTCGGTTGGTTTTCCCGCGTAGCTTGTGGCTGCTCAAGAATATATATA
TATTTCCCAGACGGAGATTTGCATTGAAAAGGCGTAATAATTCAAAAGCT
ACTGCGCAATCCGTTTTCGGTGCCCAAAATGGTCGTCGTCTCCGACAGCC
GCGTCCGTTTGCCGCGTTACGGCGGAGTCAGCGTCAAACGGAAAACGCTA
AATGTGCGCGTCACGACAATGGACGCGGAACTGGAGTTCGCCATTCAGTC
GACGACGACGGGCAAGCAATTGTTTGACCAGGTGGTGAAGACGATCGGCC
TGCGAGAGGTTTGGTTCTTTGGACTCCAGTACACCGACTCCAAGGGCGAC
TCCACATGGATCAAGCTGTACAAAAAGCCCGAATCGCCGGCCATAAAGAC
AATAAAATATTTAAAGCGTGTAAAGAAGTATGTGGACAAAAAGACAGCCG
ACAGCAATGGAGTAAATCATTTAGAGACGAGCGAAGAGGATGACGACGCC
GATGATATGACTGGATCAATGCCGTTTTCGACATGGGTGATGAACCAGGA
CGTGAAGAAGGAGAATCCCTTGCAGTTTAGGTTCCGTGCCAAATTCTATC
CCGAGGATGTGGCCGAGGAGCTGATCCAGGACATTACACTGCGTCTGTTC
TACCTGCAGGTGAAGAATGCCATACTGACCGACGAGATCTATTGTCCGCC
AGAGACATCCGTGCTGCTCGCCTCGTACGCCGTCCAGGCGCGTCATGGTG
ACCACAATAAGACCACCCACACAGCCGGCTTTCTGGCCAACGATCGCCTG
CTGCCGCAGCGCGTCATCGACCAGCACAAGATGTCCAAGGACGAGTGGGA
GCAGTCGATTATGACCTGGTGGCAGGAGCATCGCAGCATGCTGCGCGAGG
ATGCCATGATGGAGTATCTGAAGATCGCCCAAGACCTGGAGATGTACGGC
GTTAACTACTTTGAGATCCGCAACAAGAAGGGCACGGATCTTTGGCTGGG
CGTAGACGCACTGGGTCTGAACATTTACGAGCAGGACGATAGGTTGACGC
CGAAAATTGGTTTCCCATGGTCCGAGATTCGCAACATTTCGTTCTCGGAG
AAGAAGTTCATCATCAAGCCGATCGACAAGAAGGCTCCGGACTTTATGTT
CTTTGCGCCACGTGTCCGCATCAACAAGCGCATTCTGGCCCTCTGCATGG
GCAACCACGAGCTGTACATGCGTCGCCGCAAGCCGGACACCATCGATGTG
CAGCAGATGAAGGCGCAGGCGCGCGAGGAGAAGAATGCCAAACAGCAGGA
ACGTGAGAAGCTGCAGCTGGCGCTGGCCGCACGCGAACGCGCTGAAAAGA
AGCAGCAGGAGTACGAGGATCGGCTAAAGCAGATGCAGGAGGACATGGAG
CGTTCGCAGCGCGATCTGCTTGAGGCGCAGGACATGATCCGCCGGCTGGA
GGAGCAGCTGAAGCAGCTGCAGGCCGCCAAGGATGAGCTGGAGCTGCGCC
AGAAGGAGCTGCAGGCGATGCTGCAGCGCCTCGAGGAGGCCAAGAATATG
GAGGCCGTCGAGAAGCTCAAGCTCGAGGAGGAGATCATGGCCAAGCAGAT
GGAGGTGCAGCGCATTCAGGACGAGGTCAACGCCAAGGATGAGGAGACAA
AGCGTCTGCAGGACGAAGTGGAAGACGCCCGACGCAAGCAGGTCATTGCG
GCTGAAGCCGCTGCCGCTCTGCTGGCCGCGTCGACAACGCCGCAGCATCA
CCACGTGGCCGAGGATGAGAACGAGAACGAGGAGGAGCTGACGAACGGCG
ATGCCGGTGGCGATGTGTCGCGCGACCTGGACACCGACGAGCATATCAAG
GACCCCATCGAGGACAGACGCACGCTGGCCGAGCGCAACGAACGCTTGCA
CGATCAGCTCAAGGCTCTGAAACAAGATTTGGCGCAGTCTCGCGACGAGA
CGAAAGAGACGGCAAACGATAAGATTCATCGCGAGAACGTTCGCCAGGGA
CGTGACAAGTACAAGACGCTCCGCGAGATTCGTAAGGGCAACACAAAGCG
TCGCGTCGATCAGTTTGAGAACATGTAAAAGCTATCAAAGATCAGAGATC
GATAGTGCGCGGGAAAGAGAGAGGGAGCGGTGAGACTCCAGAAAGA
 
MVVVSDSRVRLPRYGGVSVKRKTLNVRVTTMDAELEFAIQSTTTGKQLFD
QVVKTIGLREVWFFGLQYTDSKGDSTWIKLYKKPESPAIKTIKYLKRVKK
YVDKKTADSNGVNHLETSEEDDDADDMTGSMPFSTWVMNQDVKKENPLQF
RFRAKFYPEDVAEELIQDITLRLFYLQVKNAILTDEIYCPPETSVLLASY
AVQARHGDHNKTTHTAGFLANDRLLPQRVIDQHKMSKDEWEQSIMTWWQE
HRSMLREDAMMEYLKIAQDLEMYGVNYFEIRNKKGTDLWLGVDALGLNIY
EQDDRLTPKIGFPWSEIRNISFSEKKFIIKPIDKKAPDFMFFAPRVRINK
RILALCMGNHELYMRRRKPDTIDVQQMKAQAREEKNAKQQEREKLQLALA
ARERAEKKQQEYEDRLKQMQEDMERSQRDLLEAQDMIRRLEEQLKQLQAA
KDELELRQKELQAMLQRLEEAKNMEAVEKLKLEEEIMAKQMEVQRIQDEV
NAKDEETKRLQDEVEDARRKQVIAAEAAAALLAASTTPQHHHVAEDENEN
EEELTNGDAGGDVSRDLDTDEHIKDPIEDRRTLAERNERLHDQLKALKQD
LAQSRDETKETANDKIHRENVRQGRDKYKTLREIRKGNTKRRVDQFENM
 
CCAAAGCGAAACGGGAGCTCTTGGCACGTGCCCTGCTCACATCCCGTTAA
TCCATCGACCCCTAAACAAATCGTGGGGGATTCTCCTCTGCACGCCACCT
TCATCGATGGGTGTCAATTTTTACTCTTTTTTTTTCTATTTGGCTTCT
AAATGTGCGCGTCACGACAATGGACGCGGAACTGGAGTTCGCCATTCAGT
CGACGACGACGGGCAAGCAATTGTTTGACCAGGTGGTGAAGACGATCGGC
CTGCGAGAGGTTTGGTTCTTTGGACTCCAGTACACCGACTCCAAGGGCGA
CTCCACATGGATCAAGCTGTACAAAAAGCCCGAATCGCCGGCCATAAAGA
CAATAAAATATTTAAAGCGTGTAAAGAAGTATGTGGACAAAAAGACAGCC
GACAGCAATGGAGTAAATCATTTAGAGACGAGCGAAGAGGATGACGACGC
CGATGATATGACTGGATCAATGCCGTTTTCGACATGGGTGATGAACCAGG
ACGTGAAGAAGGAGAATCCCTTGCAGTTTAGGTTCCGTGCCAAATTCTAT
CCCGAGGATGTGGCCGAGGAGCTGATCCAGGACATTACACTGCGTCTGTT
CTACCTGCAGGTGAAGAATGCCATACTGACCGACGAGATCTATTGTCCGC
CAGAGACATCCGTGCTGCTCGCCTCGTACGCCGTCCAGGCGCGTCATGGT
GACCACAATAAGACCACCCACACAGCCGGCTTTCTGGCCAACGATCGCCT
GCTGCCGCAGCGCGTCATCGACCAGCACAAGATGTCCAAGGACGAGTGGG
AGCAGTCGATTATGACCTGGTGGCAGGAGCATCGCAGCATGCTGCGCGAG
GATGCCATGATGGAGTATCTGAAGATCGCCCAAGACCTGGAGATGTACGG
CGTTAACTACTTTGAGATCCGCAACAAGAAGGGCACGGATCTTTGGCTGG
GCGTAGACGCACTGGGTCTGAACATTTACGAGCAGGACGATAGGTTGACG
CCGAAAATTGGTTTCCCATGGTCCGAGATTCGCAACATTTCGTTCTCGGA
GAAGAAGTTCATCATCAAGCCGATCGACAAGAAGGCTCCGGACTTTATGT
TCTTTGCGCCACGTGTCCGCATCAACAAGCGCATTCTGGCCCTCTGCATG
GGCAACCACGAGCTGTACATGCGTCGCCGCAAGCCGGACACCATCGATGT
GCAGCAGATGAAGGCGCAGGCGCGCGAGGAGAAGAATGCCAAACAGCAGG
AACGTGAGAAGCTGCAGCTGGCGCTGGCCGCACGCGAACGCGCTGAAAAG
AAGCAGCAGGAGTACGAGGATCGGCTAAAGCAGATGCAGGAGGACATGGA
GCGTTCGCAGCGCGATCTGCTTGAGGCGCAGGACATGATCCGCCGGCTGG
AGGAGCAGCTGAAGCAGCTGCAGGCCGCCAAGGATGAGCTGGAGCTGCGC
CAGAAGGAGCTGCAGGCGATGCTGCAGCGCCTCGAGGAGGCCAAGAATAT
GGAGGCCGTCGAGAAGCTCAAGCTCGAGGAGGAGATCATGGCCAAGCAGA
TGGAGGTGCAGCGCATTCAGGACGAGGTCAACGCCAAGGATGAGGAGACA
 
AAGCGTCTGCAGGACGAAGTGGAAGACGCCCGACGCAAGCAGGTCATTGC
GGCTGAAGCCGCTGCCGCTCTGCTGGCCGCGTCGACAACGCCGCAGCATC
ACCACGTGGCCGAGGATGAGAACGAGAACGAGGAGGAGCTGACGAACGGC
GATGCCGGTGGCGATGTGTCGCGCGACCTGGACACCGACGAGCATATCAA
GGACCCCATCGAGGACAGACGCACGCTGGCCGAGCGCAACGAACGCTTGC
ACGATCAGCTCAAGGCTCTGAAACAAGATTTGGCGCAGTCTCGCGACGAG
ACGAAAGAGACGGCAAACGATAAGATTCATCGCGAGAACGTTCGCCAGGG
ACGTGACAAGTACAAGACGCTCCGCGAGATTCGTAAGGGCAACACAAAGC
GTCGCGTCGATCAGTTTGAGAACATGTAAAAGCTATCAAAGATCAGAGAT
CGATAGTGCGCGGAAAGAGAGAGGAGCGGTGAGACTCCAGAAAGA
 
MGVNFLLFFFSIWLLNVRVTTMDAELEFAIQSTTTGKQLFDQVVKTIGLR
EVWFFGLQYTDSKGDSTWIKLYKKPESPAIKTIKYLKRVKKYVDKKTADS
NGVNHLETSEEDDDADDMTGSMPFSTWVMNQDVKKENPLQFRFRAKFYPE
DVAEELIQDITLRLFYLQVKNAILTDEIYCPPETSVLLASYAVQARHGDH
NKTTHTAGFLANDRLLPQRVIDQHKMSKDEWEQSIMTWWQEHRSMLREDA
MMEYLKIAQDLEMYGVNYFEIRNKKGTDLWLGVDALGLNIYEQDDRLTPK
IGFPWSEIRNISFSEKKFIIKPIDKKAPDFMFFAPRVRINKRILALCMGN
HELYMRRRKPDTIDVQQMKAQAREEKNAKQQEREKLQLALAARERAEKKQ
QEYEDRLKQMQEDMERSQRDLLEAQDMIRRLEEQLKQLQAAKDELELRQK
ELQAMLQRLEEAKNMEAVEKLKLEEEIMAKQMEVQRIQDEVNAKDEETKR
LQDEVEDARRKQVIAAEAAAALLAASTTPQHHHVAEDENENEEELTNGDA
GGDVSRDLDTDEHIKDPIEDRRTLAERNERLHDQLKALKQDLAQSRDETK
ETANDKIHRENVRQGRDKYKTLREIRKGNTKRRVDQFENM
 
AAAGCTCACGAAAAACACGCGGCAATTGGATAAGAAACGAAATTGTTGAT
CCAACGCGAGGAAGAAGAAGAATTGTGAAGCAAGAAGAAGCGAAAACAAA
CTGCGATTGCAGCACAAAAACAATAAAGAGTTCAGACGATAATATCCTGG
AAAGAAAACATTTCGTTTCGATAAGTACGACAAGACACGAAACAACAAAA
TGTCTCCAAAAGCGCTAAATGTGCGCGTCACGACAATGGACGCGGAACTG
GAGTTCGCCATTCAGTCGACGACGACGGGCAAGCAATTGTTTGACCAGGT
GGTGAAGACGATCGGCCTGCGAGAGGTTTGGTTCTTTGGACTCCAGTACA
CCGACTCCAAGGGCGACTCCACATGGATCAAGCTGTACAAAAAGGTGATG
AACCAGGACGTGAAGAAGGAGAATCCCTTGCAGTTTAGGTTCCGTGCCAA
ATTCTATCCCGAGGATGTGGCCGAGGAGCTGATCCAGGACATTACACTGC
GTCTGTTCTACCTGCAGGTGAAGAATGCCATACTGACCGACGAGATCTAT
TGTCCGCCAGAGACATCCGTGCTGCTCGCCTCGTACGCCGTCCAGGCGCG
TCATGGTGACCACAATAAGACCACCCACACAGCCGGCTTTCTGGCCAACG
ATCGCCTGCTGCCGCAGCGCGTCATCGACCAGCACAAGATGTCCAAGGAC
GAGTGGGAGCAGTCGATTATGACCTGGTGGCAGGAGCATCGCAGCATGCT
GCGCGAGGATGCCATGATGGAGTATCTGAAGATCGCCCAAGACCTGGAGA
TGTACGGCGTTAACTACTTTGAGATCCGCAACAAGAAGGGCACGGATCTT
TGGCTGGGCGTAGACGCACTGGGTCTGAACATTTACGAGCAGGACGATAG
GTTGACGCCGAAAATTGGTTTCCCATGGTCCGAGATTCGCAACATTTCGT
TCTCGGAGAAGAAGTTCATCATCAAGCCGATCGACAAGAAGGCTCCGGAC
 
TTTATGTTCTTTGCGCCACGTGTCCGCATCAACAAGCGCATTCTGGCCCT
CTGCATGGGCAACCACGAGCTGTACATGCGTCGCCGCAAGCCGGACACCA
TCGATGTGCAGCAGATGAAGGCGCAGGCGCGCGAGGAGAAGAATGCCAAA
CAGCAGGAACGTGAGAAGCTGCAGCTGGCGCTGGCCGCACGCGAACGCGC
TGAAAAGAAGCAGCAGGAGTACGAGGATCGGCTAAAGCAGATGCAGGAGG
ACATGGAGCGTTCGCAGCGCGATCTGCTTGAGGCGCAGGACATGATCCGC
CGGCTGGAGGAGCAGCTGAAGCAGCTGCAGGCCGCCAAGGATGAGCTGGA
GCTGCGCCAGAAGGAGCTGCAGGCGATGCTGCAGCGCCTCGAGGAGGCCA
AGAATATGGAGGCCGTCGAGAAGCTCAAGCTCGAGGAGGAGATCATGGCC
AAGCAGATGGAGGTGCAGCGCATTCAGGACGAGGTCAACGCCAAGGATGA
GGAGACAAAGCGTCTGCAGGACGAAGTGGAAGACGCCCGACGCAAGCAGG
TCATTGCGGCTGAAGCCGCTGCCGCTCTGCTGGCCGCGTCGACAACGCCG
CAGCATCACCACGTGGCCGAGGATGAGAACGAGAACGAGGAGGAGCTGAC
GAACGGCGATGCCGGTGGCGATGTGTCGCGCGACCTGGACACCGACGAGC
ATATCAAGGACCCCATCGAGGACAGACGCACGCTGGCCGAGCGCAACGAA
CGCTTGCACGATCAGCTCAAGGCTCTGAAACAAGATTTGGCGCAGTCTCG
CGACGAGACGAAAGAGACGGCAAACGATAAGATTCATCGCGAGAACGTTC
GCCAGGGACGTGACAAGTACAAGACGCTCCGCGAGATTCGTAAGGGCAAC
ACAAAGCGTCGCGTCGATCAGTTTGAGAACATGTAAAAGCTATCAAAGAT
CAGAGATCGATAGTGCGCGGGAAAGAGAGAGGGAGCGGTGAGACTCCAGA
AAGA
 
MSPKALNVRVTTMDAELEFAIQSTTTGKQLFDQVVKTIGLREVWFFGLQY
TDSKGDSTWIKLYKKVMNQDVKKENPLQFRFRAKFYPEDVAEELIQDITL
RLFYLQVKNAILTDEIYCPPETSVLLASYAVQARHGDHNKTTHTAGFLAN
DRLLPQRVIDQHKMSKDEWEQSIMTWWQEHRSMLREDAMMEYLKIAQDLE
MYGVNYFEIRNKKGTDLWLGVDALGLNIYEQDDRLTPKIGFPWSEIRNIS
FSEKKFIIKPIDKKAPDFMFFAPRVRINKRILALCMGNHELYMRRRKPDT
IDVQQMKAQAREEKNAKQQEREKLQLALAARERAEKKQQEYEDRLKQMQE
DMERSQRDLLEAQDMIRRLEEQLKQLQAAKDELELRQKELQAMLQRLEEA
KNMEAVEKLKLEEEIMAKQMEVQRIQDEVNAKDEETKRLQDEVEDARRKQ
VIAAEAAAALLAASTTPQHHHVAEDENENEEELTNGDAGGDVSRDLDTDE
HIKDPIEDRRTLAERNERLHDQLKALKQDLAQSRDETKETANDKIHRENV
RQGRDKYKTLREIRKGNTKRRVDQFENM
**Human homologue of Complete Genome candidate** A41289 human moesin 
1 ggcacgaggc cagccgaatc caagccgtgt gtactgcgtg ctcagcactg cccgacagtc
61 ctagctaaac ttcgccaact ccgctgcctt tgccgccacc atgcccaaaa cgatcagtgt
121 gcgtgtgacc accatggatg cagagctgga gtttgccatc cagcccaaca ccaccgggaa
181 gcagctattt gaccaggtgg tgaaaactat tggcttgagg gaagtttggt tctttggtct
241 gcagtaccag gacactaaag gtttctccac ctggctgaaa ctcaataaga aggtgactgc
301 ccaggatgtg cggaaggaaa gccccctgct ctttaagttc cgtgccaagt tctaccctga
361 ggatgtgtcc gaggaattga ttcaggacat cactcagcgc ctgttctttc tgcaagtgaa
 
421 agagggcatt ctcaatgatg atatttactg cccgcctgag accgctgtgc tgctggcctc
481 gtatgctgtc cagtctaagt atggcgactt caataaggaa gtgcataagt ctggctacct
541 ggccggagac aagttgctcc cgcagagagt cctggaacag cacaaactca acaaggacca
601 gtgggaggag cggatccagg tgtggcatga ggaacaccgt ggcatgctca gggaggatgc
661 tgtcctggaa tatctgaaga ttgctcaaga tctggagatg tatggtgtga actacttcag
721 catcaagaac aagaaaggct cagagctgtg gctgggggtg gatgccctgg gtctcaacat
781 ctatgagcag aatgacagac taactcccaa gataggcttc ccctggagtg aaatcaggaa
841 catctctttc aatgataaga aatttgtcat caagcccatt gacaaaaaag ccccggactt
901 cgtcttctat gctccccggc tgcggattaa caagcggatc ttggccttgt gcatggggaa
961 ccatgaacta tacatgcgcc gtcgcaagcc tgataccatt gaggtgcagc agatgaaggc
1021 acaggcccgg gaggagaagc accagaagca gatggagcgt gctatgctgg aaaatgagaa
1081 gaagaagcgt gaaatggcag agaaggagaa agagaagatt gaacgggaga aggaggagct
1141 gatggagagg ctgaagcaga tcgaggaaca gactaagaag gctcagcaag aactggaaga
1201 acagacccgt agggctctgg aacttgagca ggaacggaag cgtgcccaga gcgaggctga
1261 aaagctggcc aaggagcgtc aagaagctga agaggccaag gaggccttgc tgcaggcctc
1321 ccgggaccag aaaaagactc aggaacagct ggccttggaa atggcagagc tgacagctcg
1381 aatctcccag ctggagatgg cccgacagaa gaaggagagt gaggctgtgg agtggcagca
1441 gaaggcccag atggtacagg aagacttgga gaagacccgt gctgagctga agactgccat
1501 gagtacacct catgtggcag agcctgctga gaatgagcag gatgagcagg atgagaatgg
1561 ggcagaggct agtgctgacc tacgggctga tgctatggcc aaggaccgca gtgaggagga
1621 acgtaccact gaggcagaga agaatgagcg tgtgcagaag cacctgaagg ccctcacttc
1681 ggagctggcc aatgccagag atgagtccaa gaagactgcc aatgacatga tccatgctga
1741 gaacatgcga ctgggccgag acaaatacaa gaccctgcgc cagatccggc agggcaacac
1801 caagcagcgc attgacgaat ttgagtctat gtaatgggca cccagcctct agggacccct
1861 cctccctttt tccttgtccc cacactccta cacctaactc acctaactca tactgtgctg
1921 gagccactaa ctagagcagc cctggagtca tgccaagcat ttaatgtagc catgggacca
1981 aacctagccc cttagccccc acccacttcc ctgggcaaat gaatggctca ctatggtgcc
2041 aatggaacct cctttctctt ctctgttcca ttgaatctgt atggctagaa tatcctactt
2101 ctccagccta gaggtacttt ccacttgatt ttgcaaatgc ccttacactt actgttgtcc
2161 tatgggagtc aagtgtggag taggttggaa gctagctccc ctcctctccc ctccactgtc
2221 ttcttcaggt cctgagatta cacggtggag tgtatgcggt ctaggaatga gacaggacct
2281 agatatcttc tccagggatg tcaactgacc taaaatttgc cctcccatcc cgtttagagt
2341 tatttaggct ttgtaacgat tgggggaata aaaagatgtt cagtcatttt tgtttctacc
2401 tcccagatcg gatctgttgc aaactcagcc tcaataagcc ttgtcgttga ctttagggac
2461 tcaatttctc cccagggtgg atgggggaaa tggtgccttc aagaccttca ccaaacatac
2521 tagaagggca ttggccattc tattgtggca aggctgagta gaagatccta ccccaattcc
2581 ttgtaggagt ataggccggt ctaaagtgag ctctatgggc agatctaccc cttacttatt
2641 attccagatc tgcagtcact tcgtgggatc tgcccctccc tgcttcaata cccaaatcct
2701 ctccagctat aacagtaggg atgagtaccc aaaagctcag ccagccccat caggactctt
2761 gtgaaaagag aggatatgtt cacacctagc gtcagtattt tccctgctag ggttttagg
2821 tctcttcccc tctcagagct acttgggcca tagctcctgc tccacagcca tcccagcctt
2881 ggcatctaga gcttgatgcc agtaggctca actagggagt gagtgcaaaa agctgagtat
2941 ggtgagagaa gcctgtgccc tgatccaagt ttactcaacc ctctcaggtg accaaaatcc
3001 ccttctcatc actcccctca aagaggtgac tgggccctgc ctctgtttga caaacctcta
3061 acccaggtct tgacaccagc tgttctgtcc cttggagctg taaaccagag agctgctggg
3121 ggattctggc ctagtccctt ccacaccccc accccttgct ctcaacccag gagcatccac
3181 ctccttctct gtctcatgtg tgctcttctt ctttctacag tattatgtac tctactgata
3241 tctaaatatt gatttctgcc ttccttgcta atgcaccatt agaagatatt agtcttgggg
 
3301 caggatgatt ttggcctcat tactttacca cccccacacc tggaaagcat atactatatt
3361 acaaaatgac attttgccaa aattattaat ataagaagct ttcagtatta gtgatgtcat
3421 ctgtcactat aggtcataca atccattctt aaagtacttg ttatttgttt ttattattac
3481 tgtttgtctt ctccccaggg ttcagtccct caaggggcca tcctgtccca ccatgcagtg
3541 ccccctagct tagagcctcc ctcaattccc cctggccacc accccccact ctgtgcctga
3601 ccttgaggag tcttgtgtgc attgctgtga attagctcac ttggtgatat gtcctatatt
3661 ggctaaattg aaacctggaa ttgtggggca atctattaat agctgcctta aagtcagtaa
3721 cttaccctta gggaggctgg gggaaaaggt tagattttgt attcaggggt ttttgtgta
3781 ctttttgggt ttttaaaaaa ttgtttttgg aggggtttat gctcaatcca tgttctattt
3841 cagtgccaat aaaatttagg tgacttcaaa aaaaaaaa
 
1 
61 
121 
181 
241 
301 
361 
421 
481 
541 
**Putative function** Cytoskeletal binding protein linking to plama membrane, involved in cytokinesis and cell shape

### Example 11 (Category 3)

**Line ID** - 226
**Phenotype** - Lethal phase pharate adult. High mitotic index, rod-like overcondensed chromosomes, lagging chromosomes and bridges in anaphase, highly condensed
**Annotated *Drosophila* genome genomic segment containing P element insertion site (and map position) -** AE003423 (2F1-2)
**P element insertion site** - 226,527
**Annotated *Drosophila* genome Complete Genome candidate -** CG2865 - EG:25E8.4 
AGAAAACCACATAAACAAGCCAGCAAACAAGGCACACACTTGCTTGAAAA
ACGCACAATGACCTTGCCCACAAACACACACGCATCTGCAAACGACGGCG
GCAGCGGCAACAACAACCACAGCAATATCAGCAGTAACAACAGCAGCAGC
AGCGACGAAGACTCAGACATGTTTGGACCACCCGCTGCTCCCCGCCCAT
CGGCTATCACCATCACCGTTCCCGTGTGCCCATGATCTCGCCAAAGCTGC
GGCAGCGCGAGGAGCGCAAGCGGATCCTCCAGCTCTGCGCCCACAAGATG
GAGAGGATCAAGGACTCGGAGGCGAACCTGCGGCGCAGCGTCTGCATCAA
CAACACCTACTGCCGCCTGAATGACGAACTGCGGCGCGAGAAGCAGATGC
GCTACCTCCAGAATCTGCCCAGAACCAGCGACAGCGGCGCAAGCACCGAA
CTGGCGCGTGAGAATCTCTTCCAGCCGAACATGGACGACGCCAAGCCGGC
CGGCAATAGCACTAGCAATAATATCAACGCCAACGGCAAGCCTTCATCCT
CTTTTGGCGATGCCTTTGGCTCCTCAAACGGATCATCGTCGGGTCGCGGC
GGAATTTGCTCCCTGGAGAATCAACCGCCCGAGCGTCAGCAGTTGGGGAC
GCCCGCTGGTGCCTCCGCTCCCGAGGCGGCCAATTCGGCGCCCCTTTCCG
TTTCGGGCTCGGCATCGGAACGCGTGAATAACCGAAAACGCCACCTGTCC
AGCTGCAACTTGGTCAACGATCTGGAAATACTGGACAGGGAGCTGAGCGC
CATCAATGCACCCATGCTGCTAATCGATCCAGAGATTACCCAAGGAGCCG
AACAGCTGGAGAAGGCCGCCTTGTCCGCCAGCAGGAAGAGATTGAGGAGC
AATAGCGGCAGCGAGGACGAAAGTGATCGCCTGGTGCGCGAGGCTCTGTC
CCAGTTCTACATACCGCCACAGCGCCTCATCTCCGCCATTGAGGAGTGTC
CCCTGGATGTGGTTGGCTTGGGTATGGGAATGAATGTGAATGTGAATGTG
GGAGGAATTAGTGGAATCGGTGGCATCGGAGGAGCTGCAGGCGCTGGCGT
CGAAATGCCCGGAGGCAAACGGATGAAGCTGAATGACCATCACCATCTCA
ATCACCATCACCATTTGCACCATCATCTGGAGCTGGTCGATTTCGACATG
AACCAAAACCAAAAGGATTTCGAGGTGATCATGGACGCCTTGAGGCTGGG
AACGGCGACACCGCCGAGCGGCGCCAGCAGCGATTCTTGCGGACAGGCGG
CGATGATGAGCGAGTCGGCCAGCGTGTTCCACAATCTGGTGGTCACCTCG
TTGGAGACATGA
 
MTLPTNTHASANDGGSGNNNHSNISSNNSSSSDEDSDMFGPPRCSPPIGY
HHHRSRVPMISPKLRQREERKRILQLCAHKMERIKDSEANLRRSVCINNT
YCRLNDELRREKQMRYLQNLPRTSDSGASTELARENLFQPNMDDAKPAGN
STSNNINANGKPSSSFGDAFGSSNGSSSGRGGICSLENQPPERQQLGTPA
 
GASAPEAANSAPLSVSGSASERVNNRKRHLSSCNLVNDLEILDRELSAIN
APMLLIDPEITQGAEQLEKAALSASRKRLRSNSGSEDESDRLVREALSQF
YIPPQRLISAIEECPLDVVGLGMGMNVNVNVGGISGIGGIGGAAGAGVEM
PGGKRMKLNDHHHLNHHHHLHHHLELVDFDMNQNQKDFEVIMDALRLGTA
TPPSGASSDSCGQAAMMSESASVFHNLVVTSLET
**Human homologue of Complete Genome candidate** CG2865 - none
**Putative function** Putative phosphatidylinositol 3-kinase

### Example 12 (Category 3)

**Line ID** - 269
**Phenotype** -Lethal phase pupal - pharate adult. High mitotic index, colchicines-type overcondensation, high frequency of polyploids
**Annotated *Drosophila* genome genomic segment containing P element insertion site (and map position)** - AE003568 (19F)
**P element insertion site -** 197,805
**Annotated *Drosophila* genome Complete Genome candidate -** CG 1696 - novel protein 
AAAACTCATCGATGCTGCGAAAGTGCGATAGTATCGAATAAACATGAGTG
TGTGCATGAGTGTGGGAATTTATTAAACAAAAACGAAACGCGGACAAACT
ATATTTATGTAATAAACACTAAGCCGCAGCGCCAACGAGTAATGAACAGT
CCACGGCCAGGTCGTACTATTCAGGCGAACGCACCTCGCAATCGACTGCA
ATCAAAGTGCAATAGCTCAATCAATTGATTCGTTTTGCTCAACCAAAAAC
AAAATCTATTCCCAAATCGGTGCGATAGTTGCCAAAATATAAAAACTACA
CTACGCTAAAAAAAAAACAATACACTCACACACTGGCGTACAAGACAACA
AAAGAGAAGAAGAAGAGCAGACGCCAGATATAAAAAGCCCCCAAAAGAAT
TGGAAATAAGACCATACCCCTCCTTCTCCCTTGAAAAGGGACCTTAAAAC
TAGGCGACACCGAATAATTGAACTCAAGTAAAAAACCGGGAAAAGAGAAA
AACACTTTCAACAAAATATCTAGAAGCCTTGTTATCGATTTTGTTCCGGG
TTTTTTTGTGTGAGTGTGTGTTGTGTGAAGCGCGCCCGCGGGTGTGTGG
GTGAGTGTGCGTGTGGCTCTCGGCGCGTTATCAAAAACAACAACAATTCG
TTGCAAAAGAAAAAATAAAGTAGAGGAGGCGGAAGAAGAAGAGGAATCTG
CTCGCACCGCGGTCAATCGCGGATCGTGGTCGATTTATCGAATTAATCGC
CCCGAACAAAAAAACACCGTACAAGGACTTGCACTATTTCCAATGATTT
CGCTGCTGCAAATGAAATTCCGTGCGCTTTTGTTGTTGCTATCAAAAGTA
TGGACATGCATTTGTTTCATGTTCAATCGCCAAGTGCGAGCTTTTATCCA
GTATCAACCGGTTAAATACGAACTCTTCCCGTTGTCACCCGTCTCGCGGC
ACCGCCTGAGCCTGGTGCAGCGCAAGACCCTCGTTCTGGACCTGGACGAA
ACGCTAATCCACTCCCATCACAATGCGATGCCCCGGAATACGGTGAAGCC
GGGCACGCCGCACGATTTCACTGTCAAAGTGACCATCGATCGGAATCCAG
TGCGCTTTTTCGTGCACAAGCGACCGCATGTGGACTACTTCCTGGACGTG
GTCTCGCAGTGGTACGATCTGGTGGTCTTCACGGCCAGCATGGAGATTTA
CGGAGCGGCGGTGGCAGACAAGCTGGACAACGGACGAAACATCCTCCGGA
GGCGATACTACAGACAGCACTGCACGCCCGACTACGGATCCTACACCAAA
GACCTGTCGGCCATCTGCAGTGACCTAAATAGGATATTTATCATCGACAA
TTCGCCCGGCGCCTATCGCTGTTTTCCCAACAACGCCATACCCATCAAGA
GTTGGTTCTCGGACCCGATGGACACGGCGCTGCTGTCGCTGCTGCCCATG
CTGGATGCGCTGAGGTTCACGAACGACGTGAGATCGGTGCTGTCGAGGAA
CTTGCACCTGCACCGCCTCTGGTAGCAGGTGGGCCGCCTGTCGCTAGTTT
AGTTTA
 MISLLQMKFRALLLLLSKVWTCICFMFNRQVRAFIQYQPVKYELFPLSPV 
SRHRLSLVQRKTLVLDLDETLIHSHHNAMPRNTVKPGTPHDFTVKVTIDR
NPVRFFVHKRPHVDYFLDVVSQWYDLVVFTASMEIYGAAVADKLDNGRNI
LRRRYYRQHCTPDYGSYTKDLSAICSDLNRIFIIDNSPGAYRCFPNNAIP
IKSWFSDPMDTALLSLLPMLDALRFTNDVRSVLSRNLHLHRLW
**Human homologue of Complete Genome candidate** NP_056158 hypothetical protein 
1 gccggggccg gcggtgccgg ggtcatcggg atgatgcgga cgcagtgtct gctggggctg
61 cgcgcgttcg tggccttcgc cgccaagctc tggagcttct tcatttacct tttgcggagg
121 cagatccgca cggtaattca gtaccaaact gttcgatatg atatcctccc cttatctcct
181 gtgtcccgga atcggctagc ccaggtgaag aggaagatcc tggtgctgga tctggatgag
241 acacttattc actcccacca tgatggggtc ctgaggccca cagtccggcc tggtacgcct
301 cctgacttca tcctcaaggt ggtaatagac aaacatcctg tccggttttt tgtacataag
361 aggccccatg tggatttctt cctggaagtg gtgagccagt ggtacgagct ggtggtgttt
421 acagcaagca tggagatcta tggctctgct gtggcagata aactggacaa tagcagaagc
481 attcttaaga ggagatatta cagacagcac tgcactttgg agttgggcag ctacatcaag
541 gacctctctg tggtccacag tgacctctcc agcattgtga tcctggataa ctccccaggg
601 gcttacagga gccatccaga caatgccatc cccatcaaat cctggttcag tgaccccagc
661 gacacagccc ttctcaacct gctcccaatg ctggatgccc tcaggttcac cgctgatgtt
721 cgttccgtgc tgagccgaaa ccttcaccaa catcggctct ggtgacagct gctccccctc
781 cacctgagtt ggggtggggg ggaaagggag ggcgagccct tgggatgccg tctgatgccc
841 tgtccaatgt gaggactgcc tgggcagggt ctgcccctcc cacccctctc tgccctggga
901 gccctacact ccacttggag tctggatgga cacatgggcc aggggctctg aagcagcctc
961 actcttaact tcgtgttcac actccatgga aaccccagac tgggacacag gcggaagcct
1021 aggagagccg aatcagtgtt tgtgaagagg caggactggc cagagtgaca gacatacggt
1081 gatccaggag gctcaaagag aagccaagtc agctttgttg tgatttgatt tttttaaaa
1141 aactcttgta caaaactgat ctaattcttc actcctgctc caagggctgg gctgtgggtg
1201 ggatactggg attttgggcc actggatttt ccctaaattt gtcccccctt tactctccct
1261 ctatttttct ctccttagac tccctcagac ctgtaaccag ctttgtgtct tttttccttt
1321 tctctctttt aaaccatgca ttataacttt gaaacc
 
1 mmrtqcllgl rafvafaakl wsffiyllrr qirtviqyqt vrydilplsp vsmrlaqvk
61 rkilvldlde tlihshhdgv lrptvrpgtp pdfilkvvid khpvrffvhk rphvdfflev
121 vsqwyelvvf tasmeiygsa vadkldnsrs ilkrryyrqh ctlelgsyik dlsvvhsdls
181 sivildnspg ayrshpdnai pikswfsdps dtallnllpm ldalrftadv rsvlsrnlhq
241 hrlw
**Putative function** unknown

### Example 13 (Category 3)

**Line ID** - 291
**Phenotype** - Lethal phase pupal - pharate adult. High mitotic index, colchicines-type overcondensed chromosomes, many strongly stained nuclei
**Annotated *Drosophila* genome genomic segment containing P element insertion site (and map position)** - AE003427 (3D5)
**P element insertion site -** 131,166
**Annotated *Drosophila* genome Complete Genome candidate -** CG10798 - dm diminutive, dMyc1 
GTCGCGTGTTCAGTTCACCGCGGGTAATTCAGAGAATCGCTTTGTGGATT
GGATTTTGCCTGTTTTCCGCCCGATACAAAAAAAAAAACCAAACGCTA
TATAAATAGTTCTGTAGTAAAACCTGAAGCAACACGTTTAAAATATACA
ACTACTACTAACAACTGTCACAGCCAAGTTACAAAAGTGCTAAATCCCAG
AAATAACCTAAGAGCCGACTTAAAACCGCGCAAATACATAAAAAAAAATC
TTCTCCAAAGCAGAAACAAAAACTTGTGAAAAACTAGAATTAAAAAAAGA
TTTTTTAAAAAAAATCAGCTAGTGCAAAATAAACGGAAGAATTTTTTT
TGTGTCCCTTTTTTTGGTGTTTTTCTCCGTCTTTCCCCTTCTTTGACGC
AAAAAAAAAAGTGCCCAACTTGCTGGCGGCACGGGAACGGGATAGAAATA
GATATAGCCGAAAGCGACTGGAAAGCAAAGGAAGCTAACTAAATTGGATT
ACAATCAATTAAATAGAGACGGATACGGAAACTATGTTCAGCGAGACAGG
CATATAACTCAGGAACTTAAGATATATAGAAAGAAAAAAAAACCCAGACA
ACATAATCGCAATGGCCCTTTACCGCTCTGATCCGTATTCCATAATGGAC
GACCAACTTTTTTCAAATATTTCAATATTCGATATGGATAATGATCTGTA
CGATATGGACAAACTCCTTTCGTCGTCCACCATTCAGAGTGATCTCGAGA
AGATCGAGGACATGGAAAGTGTATTTCAAGACTATGACTTAGAGGAGGAT
ATGAAGCCAGAGATCCGCAACATCGACTGCATGTGGCCGGCGATGTCCAG
CTGTTTGACCAGCGGTAACGGTAATGGAATAGAGAGCGGAAACAGTGCAG
CCTCGTCGTACAGCGAAACCGGTGCCGTATCCCTGGCGATGGTTTCCGGC
TCTACGAATCTCTACAGCGCGTATCAACGATCGCAGACGACAGATAACAC
CCAGTCAAATCAACAGCATGTCGTCAACAGTGCCGAGAACATGCCGGTGA
TCATCAAGAAGGAGCTCGCAGATCTGGACTACACGGTCTGTCAGAAGCGC
CTCCGTTTGAGCGGCGGTGACAAGAAGTCACAGATCCAGGACGAGGTCCA
TTTAATACCGCCCGGCGGAAGTTTGCTCCGCAAGCGGAACAACCAGGACA
TTATCCGCAAATCGGGCGAATTGAGCGGCAGCGATAGCATAAAATACCAG
AGACCAGACACACCTCACAGTCTTACCGACGAGGTGGCCGCCTCAGAGTT
TAGACATAACGTCGACTTGCGTGCCTGCGTGATGGGCAGCAATAATATCT
CGCTGACCGGCAATGATAGCGATGTCAACTACATTAAGCAAATCAGCAGG
GAGCTTCAGAATACCGGCAAGGATCCGTTGCCGGTGCGTTACATCCCGCC
GATCAACGATGTCCTCGATGTGCTCAACCAGCATTCCAATTCGACGGGTG
GCCAACAGCAGTTGAACCAACAGCAACTGGACGAGCAACAACAGGCCATC
GATATAGCCACTGGACGCAACACAGTGGATTCTCCGCCGACGACCGGCTC
TGATAGTGACTCCGATGACGGTGAACCCCTCAACTTTGACCTGCGCCATC
ATCGCACTAGCAAAAGCGGCAGCAATGCCAGCATCACCACCAACAACAAC
AACAGCAACAACAAAAACAACAAATTGAAGAACAACAGCAACGGCATGCT
 
GCACATGATGCACATCACCGATCACAGCTACACGCGCTGCAACGATATGG
TGGACGATGGTCCCAATTTGGAGACCCCCTCAGATTCCGATGAGGAAATC
GATGTCGTTTCATATACGGACAAGAAGCTACCCACAAATCCCTCGTGCCA
CTTGATGGGCGCCCTACAGTTCCAGATGGCCCATAAGATCTCGATTGATC
ACATGAAGCAAAAACCGCGCTACAATAACTTCAATCTGCCGTACACACCG
GCCAGCAGCAGTCCAGTGAAATCGGTGGCCAACTCGCGTTATCCATCACC
GTCGAGCACACCGTATCAGAACTGCTCCTCCGCTTCGCCGTCCTACTCGC
CGCTATCCGTGGACTCTTCAAATGTCAGCTCGAGCAGCTCCAGTTCCAGT
TCGCAGTCAAGCTTCACCACCTCCAGTTCGAACAAGGGACGCAAACGATC
CAGTCTGAAGGATCCAGGCTTGTTGATCTCCTCCAGCAGCGTTTATCTGC
CGGGAGTCAATAACAAAGTGACGCATAGCTCCATGATGAGCAAAAAGAGT
CGTGGCAAGAAGGTGGTTGGCACCTCGTCTGGCAATACATCTCCGATATC
GTCTGGCCAGGATGTGGATGCCATGGATCGTAATTGGCAGCGGCGCAGTG
GTGGAATTGCCACTAGCACAAGCTCCAACAGCAGTGTCCATCGGAAGGAC
TTTGTTTTGGGCTTTGATGAGGCCGATACGATCGAGAAGCGCAATCAGCA
CAATGATATGGAGCGTCAGCGACGCATTGGACTCAAGAACCTCTTTGAGG
CTCTAAAGAAACAGATTCCCACAATTAGGGACAAGGAGCGGGCTCCCAAG
GTAAATATCCTGCGAGAGGCGGCCAAGCTATGCATCCAGCTGACCCAGGA
GGAGAAGGAGCTTAGTATGCAGCGCCAGCTTTTGTCGCTGCAGCTGAAGC
AACGTCAGGACACTCTGGCCAGTTACCAAATGGAGTTGAACGAATCGCGC
TCGGTTAGTGGATAGTGTTGTCTCATACTATCGGCTTAAAGCGGCGGCGT
AGGGCTAGGATAACCCCCAATGTATATGCAAGATTTGTATATCCTCCTAC
TTTTTTTTTTTTGCAATTTACTTTGATTTAGCTTCGATCCTTTCTTGACA
TTAAGCCCTAAATATGATTTTTTTCTGGAGAACTTCAATATCAGTTAGTA
GGTTATGTTTAACGATTTGCTTGCGCTTTTCCGCTTTTTTTTTGTTTT
TTTACCATACCATACCATAC
 
MDDQLFSNISIFDMDNDLYDMDKLLSSSTIQSDLEKIEDMESVFQDYDLE
EDMKPEIRNIDCMWPAMSSCLTSGNGNGIESGNSAASSYSETGAVSLAMV
SGSTNLYSAYQRSQTTDNTQSNQQHVVNSAENMPVIIKKELADLDYTVCQ
KRLRLSGGDKKSQIQDEVHLIPPGGSLLRKRNNQDIIRKSGELSGSDSIK
YQRPDTPHSLTDEVAASEFRHNVDLRACVMGSNNISLTGNDSDVNYIKQI
SRELQNTGKDPLPVRYIPPINDVLDVLNQHSNSTGGQQQLNQQQLDEQQQ
AIDIATGRNTVDSPPTTGSDSDSDDGEPLNFDLRHHRTSKSGSNASITTN
NNNSNNKNNKLKNNSNGMLHMMHITDHSYTRCNDMVDDGPNLETPSDSDE
EIDVVSYTDKKLPTNPSCHLMGALQFQMAHKISIDHMKQKPRYNNFNLPY
TPASSSPVKSVANSRYPSPSSTPYQNCSSASPSYSPLSVDSSNVSSSSSS
SSSQSSFTTSSSNKGRKRSSLKDPGLLISSSSVYLPGVNNKVTHSSMMSK
KSRGKKVVGTSSGNTSPISSGQDVDAMDRNWQRRSGGIATSTSSNSSVHR
KDFVLGFDEADTIEKRNQHNDMERQRRIGLKNLFEALKKQIPTIRDKERA
PKVNILREAAKLCIQLTQEEKELSMQRQLLSLQLKQRQDTLASYQMELNE
SRSVSG
**Human homologue of Complete Genome candidate** CAA23831 c-myc oncogene 1 ctgctcgcgg ccgccaccgc cgggccccgg ccgtccctgg ctcccctcct gcctcgagaa 
61 gggcagggct tctcagaggc ttggcgggaa aaaagaacgg agggagggat cgcgctgagt
121 ataaaagccg gttttcgggg ctttatctaa ctcgctgtag taattccagc gagaggcaga
181 gggagcgagc gggcggccgg ctagggtgga agagccgggc gagcagagct gcgctgcggg
241 cgtcctggga agggagatcc ggagcgaata gggggcttcg cctctggccc agccctcccg
301 cttgatcccc caggccagcg gtccgcaacc cttgccgcat ccacgaaact ttgcccatag
361 cagcgggcgg gcactttgca ctggaactta caacacccga gcaaggacgc gactctcccg
421 acgcggggag gctattctgc ccatttgggg acacttcccc gccgctgcca ggacccgctt
481 ctctgaaagg ctctccttgc agctgcttag acgctggatt tttttcgggt agtggaaaac
541 cagcagcctc ccgcgacgat gcccctcaac gttagcttca ccaacaggaa ctatgacctc
601 gactacgact cggtgcagcc gtatttctac tgcgacgagg aggagaactt ctaccagcag
661 cagcagcaga gcgagctgca gcccccggcg cccagcgagg atatctggaa gaaattcgag
721 ctgctgccca ccccgcccct gtcccctagc cgccgctccg ggctctgctc gccctcctac
781 gttgcggtca cacccttctc ccttcgggga gacaacgacg gcggtggcgg gagcttctcc
841 acggccgacc agctggagat ggtgaccgag ctgctgggag gagacatggt gaaccagagt
901 ttcatctgcg acccggacga cgagaccttc atcaaaaaca tcatcatcca ggactgtatg
961 tggagcggct tctcggccgc cgccaagctc gtctcagaga agctggcctc ctaccaggct
1021 gcgcgcaaag acagcggcag cccgaacccc gcccgcggcc acagcgtctg ctccacctcc
1081 agcttgtacc tgcaggatct gagcgccgcc gcctcagagt gcatcgaccc ctcggtggtc
1141 ttcccctacc ctctcaacga cagcagctcg cccaagtcct gcgcctcgca agactccagc
1201 gccttctctc cgtcctcgga ttctctgctc tcctcgacgg agtcctcccc gcagggcagc
1261 cccgagcccc tggtgctcca tgaggagaca ccgcccacca ccagcagcga ctctgaggag
1321 gaacaagaag atgaggaaga aatcgatgtt gtttctgtgg aaaagaggca ggctcctggc
1381 aaaaggtcag agtctggatc accttctgct ggaggccaca gcaaacctcc tcacagccca
1441 ctggtcctca agaggtgcca cgtctccaca catcagcaca actacgcagc gcctccctcc
1501 actcggaagg actatcctgc tgccaagagg gtcaagttgg acagtgtcag agtcctgaga
1561 cagatcagca acaaccgaaa atgcaccagc cccaggtcct cggacaccga ggagaatgtc
1621 aagaggcgaa cacacaacgt cttggagcgc cagaggagga acgagctaaa acggagcttt
1681 tttgccctgc gtgaccagat cccggagttg gaaaacaatg aaaaggcccc caaggtagtt
1741 atccttaaaa aagccacagc atacatcctg tccgtccaag cagaggagca aaagctcatt
1801 tctgaagagg acttgttgcg gaaacgacga gaacagttga aacacaaact tgaacagcta
1861 cggaactctt gtgcgtaagg aaaagtaagg aaaacgattc cttctaacag aaatgtcctg
1921 agcaatcacc tatgaacttg tttcaaatgc atgatcaaat gcaacctcac aaccttggct
1981 gagtcttgag actgaaagat ttagccataa tgtaaactgc ctcaaattgg actttgggca
2041 taaaagaact tttttatgct taccatcttt tttttttctt taacagattt gtatttaaga
2101 attgttttta aaaaatttta a
 
1 mplnvsftnr nydldydsvq pyfycdeeen fyqqqqqsel qppapsediw kkfellptpp
61 lspsrrsglc spsyvavtpf slrgdndggg gsfstadqle mvtellggdm vnqsficdpd
121 detfikniii qdcmwsgfsa aaklvsekla syqaarkdsg spnparghsv cstsslylqd
181 lsaaasecid psvvfpypln dssspkscas qdssafspss dsllsstess pqgspeplvl
241 heetppttss dseeeqedee eidvvsvekr qapgkrsesg spsagghskp phsplvlkrc
301 hvsthqhnya appstrkdyp aakrvkldsv rvlrqisnnr kctsprssdt eenvkrrthn
361 vlerqrrnel krsffalrdq ipelenneka pkvvilkkat ayilsvqaee qkliseedll
421 rkrreqlkhk leqlrnsca
**Putative function** C-myc oncogene, transcription factor

### Example 14 (Category 3)

**Line ID** - 316
**Phenotype** - Lethal phase larval stage 3 - Pre-pupal-pupal. Small optic lobes, missing or small imaginal discs, badly defined chromosomes.
**Annotated *Drosophila* genome genomic segment containing P element insertion site (and map position)** - AE003506 (16B-C)
**P element insertion site** - 27,868
**Annotated *Drosophila* genome Complete Genome candidate -** CG8465 - novel protein (3 splice variants) 
TGACAGTCCGCCTCTAATTTAATTTCGTTTGTGCACATTTTGTTTGAAAG
ACGCTTAAGATTATTGGGTTTTGTTTCATGTATTGTGCCCTTTGTGCTAA
AAGTGCATCCGCCATTTTACGCAGAGATGTCGACCTATTTCGGGGTCTAT
ATCCCGACCTCCAAAGCGGGCTGTTTTGAGGGATCGGTGTCGCAGTGCAT
CGGCTCCATAGCCGCGGTGAACATAAAGCCATCCAATCCGGCGTCTGGAT
CGGCATCAGTAGCATCGGGATCGCCATCCGGCTCGGCGGCATCCGTGCAA
ACGGGCAACGCAGACGATGGCAGTGCTGCCACCAAGTACGAGGATCCCGA
CTATCCACCGGACTCGCCACTGTGGCTGATCTTCACGGAGAAATCCAAGG
CGCTGGACATCCTGCGACACTACAAGGAGGCGCGCCTCCGCGAGTTTCCC
AATCTGGAGCAGGCGGAGAGTTACGTTCAGTTTGGGTTCGAGAGCATCGA
GGCGCTCAAGAGATTTTGCAAGGCAAAGCCCGAAAGCAAGCCCATTCCGA
TAATCAGCGGTAGCGGTTACAAGAGCTCACCGACCTCGACGGACAATTCG
TGCTCCTCCTCGCCGACGGGTAACGGCAGTGGCTTCATCATTCCCCTGGG
AAGCAATTCCTCAATGTCGAATTTACTGCTCAGTGACTCACCGACTTCCT
CGCCGAGCAGCTCCAGCAACGTCATTGCCAATGGGCGACAGCAGCAGATG
CAGCAGCAACAGCAGCAGCAGCCGCAGCAGCCGGATGTGTCCGGAGAAGG
CCCTCCTTTCCGGGCGCCCACCAAACAGGAACTGGTAGAGTTTCGCAAGC
AAATCGAAGGTGGTCACATAGACCGGGTGAAGAGGATTATATGGGAGAAT
CCACGATTTTTGATCAGCAGCGGTGATACGCCCACCAGTTTGAAGGAGGG
CTGTCGCTATAATGCCATGCACATCTGCGCCCAGGTCAATAAGGCCAGGA
TCGCTCAGTTGCTGTTAAAGACCATTTCGGATCGGGAGTTCACTCAGCTT
TACGTTGGCAAGAAGGGCAGTGGCAAGATGTGTGCTGCCCTCAACATCAG
TCTCCTGGACTATTACCTGAACATGCCGGACAAGGGGCGCGGCGAAACAC
CGCTCCACTTTGCCGCAAAGAACGGTCATGTGGCCATGGTCGAGGTTCTC
GTTTCCTATCCGGAGTGCAAATCGCTGCGGAATCATGAGGGCAAGGAGCC
CAAGGAAATCATCTGCCTGCGTAATGCTAATGCTACACATGTGACCATCA
AGAAGCTGGAGCTGCTCTTGTACGATCCGCATTTTGTGCCCGTACTAAGA
TCCCAGTCAAATACACTGCCGCCAAAAGTGGGTCAACCGTTCTCGCCCAA
AGATCCACCGAACCTGCAACACAAAGCGGACGATTACGAGGGCCTCAGCG
TGGACCTGGCAATCAGTGCGCTGGCGGACCCATGTCCCGCGAAAAGGCC
ATGAACTTCTATCGCCGTTGGAAGACACCACCGCGGGTCAGCAACAATGT
GATGTCGCCGCTGGCTGGTTCACCATTTAGCTCGCCGGTGAAAGTAACCC
CAAGCAAGTCGATCTTTGACCGAAGTGCTGGAAACTCGAGTCCAGTCCAC
TCAGGACGCAGAGTGCTCTTTAGTCCATTGGCGGAGGCGACCAGCTCACC
 
AAAACCGACGAAAAACGTGCCCAATGGCACCAATGAGTGCGAGCACAACA
ATAATAATGTGAAGCCAGTGTATCCGTTGGAGTTCCCGGCGACACCCATT
CGAAAAATGAAACCGGATTTATTCATGGCCTATCGCAATAACAATAGCTT
TGATTCGCCATCTTTGGCCGATGACTCCCAAATCCTGGACATGAGCCTAA
GCCGCAGCCTGAATGCGTCGCTAAATGACAGCTTCCGTGAGCGGCACATC
AAGAACACTGATATCGAGAAGGGTCTGGAGGTGGTCGGCCGCCAACTGGC
ACGACAGGAGCAGTTAGAGTGGCGCGAGTACTGGGATTTTCTCGATTCAT
TTTTGGACATTGGTACGACCGAAGGCCTGGCCCGTCTTGAAGCGTATTTC
CTGGAAAAGACCGAACAGCAGGCGGATAAATCAGAAACGGTCTGGAACTT
TGCCCATCTGCATCAGTATTTCGATTCGATGGCCGGCGAGCAACAGCAGC
AACTCCGAAAGGATAAAAATGAGGCTGCGGAGCAACTTCGCCATCCGCC
GGAGTCATGACTCCGTACACATGCGTAGAGAAGTCGCTGCAAGTGTTCGC
CAAGCGCATCACTAAAACGTTGATCAACAAAATCGGCAACATGGTGTCCA
TCAACGACACGCTGCTCTGTGAGCTCAAAAGACTGAAATCGCTGATTGTC
AGCTTCAAGGATGATGCCCGCTTCATTAGCGTGGACTTTAGCAAGGTGCA
TTCACGTATCGCCCACCTGGTGGCCAGCTATGTGACCCACTCGCAGGAGG
TCAGCGTAGCCATGCGTCTACAATTGTTGCAGATGCTCCGAAGTTTGCGG
CAACTGCTGGCCGACGAGCGTGGTCGAGAACAGCATTTGGGCTGCGTGTG
CGCTAGTCTATTGCTGATGCTGGAACAGGCGCCGACATCCGCCGTGCATC
TACCAGACACTCTGAAGACCGAGGAGCTATGTTGCGCCGCCTGGGAGACG
GAGCAGTGTTGCGCCTGTCTGTGGGACGCAAATCTCAGCCGTAAGACCAG
TCGTCGAAAGCGCACTAAGTCGCTGCGGGCAGCTGCTGTTGTTCAGTCTC
AGGGTCAGCTTCAGGATACTTCGGGATCGACAGGGTCGTCCGCCTTGCAC
GCTTCGCTTGGTGTGGGATCGACCAGTTTGGGAGCATCGAGGGTCGTGGC
GTCCGCTTCGAAAGATGCTTGGCGCCGTCAACAAAGCGACGACGAGGACT
ACGACAGCGATGAGCAAGTAATCTTTTTCGACTGCACTAATGTTACGCTG
CCTTATGGAAGCAGCAGCGAGGACGAGGAAAACTTCCGTACGCCGCCGCA
AAGCTTGTCGCCAGGTATTTCCATGGATTTGGAGCCGCGTTACGAGTTGT
TTATTTTTGGAAACGAGCCAACCAAGCGAGATTTGGATGTGCTGAATGCC
CTTTCCAATGTCGACATTGATAAGGAAACACTGCCGCATGTCTACGCCTG
GAAGACTGCCATGGAGAGCTACTCCTGTGCTGAAATGAATCTGAACGTCA
AGGTTCAAAAGCCGGAGCCTTGGTATTCTGGAACCAGTTCTAGCCACAAC
AGCCAACCATTGTTGCATCCCAAGCGTCTGCTTGCCACGCCAAAGCTGAA
TGCCGTGGTCAGCGGCAGACGCGGATCCGGACCATTGACGGCGCCAGTTA
CACCGCGTCTGGCGCGAACTCCGTCCGCCGCCAGTATTCAAGTTGCATCC
GAGACGAATGGCGAGTCGGTCGGAACTGCTGTGACTCCGGCATCGCCGAT
TTTGAGTTTTGCCGCCTTGACGGCAGCGACGCAGTCATTCCAAACACCAT
TGAACAAGGTGCGCGGCTTGTTCAGCCAATATCGGGATCAACGGTCCTAT
AACGAGGGGGACACGCCGCTGGGCAATCGGAACTGAAACGGAATCGGCCC
GGAAACAGAAACAGAAACAGCGACTGATTGATGAAAGGCCGACTGCATAC
TTACCCCCCTGAATAGCCGGTGTCGTCCATTGTCCCTTTTAATGTTAATC
GCATGTATATTA
 
MSTYFGVYIPTSKAGCFEGSVSQCIGSIAAVNIKPSNPASGSASVASGSP
SGSAASVQTGNADDGSAATKYEDPDYPPDSPLWLIFTEKSKALDILRHYK
EARLREFPNLEQAESYVQFGFESIEALKRFCKAKPESKPIPIISGSGYKS
SPTSTDNSCSSSPTGNGSGFIIPLGSNSSMSNLLLSDSPTSSPSSSSNVI
ANGRQQQMQQQQQQPQQPDVSGEGPPFRAPTKQELVEFRKQIEGGHIDR
 
VKRIIWENPRFLISSGDTPTSLKEGCRYNAMHICAQVNKARIAQLLLKTI
SDREFTQLYVGKKGSGKMCAALNISLLDYYLNMPDKGRGETPLHFAAKNG
HVAMVEVLVSYPECKSLRNHEGKEPKEIICLRNANATHVTIKKLELLLYD
PHFVPVLRSQSNTLPPKVGQPFSPKDPPNLQHKADDYEGLSVDLAISALA
GPMSREKAMNFYRRWKTPPRVSNNVMSPLAGSPFSSPVKVTPSKSIFDRS
AGNSSPVHSGRRVLFSPLAEATSSPKPTKNVPNGTNECEHNNNNVKPVYP
LEFPATPIRKMKPDLFMAYRNNNSFDSPSLADDSQILDMSLSRSLNASLN
DSFRERHIKNTDIEKGLEVVGRQLARQEQLEWREYWDFLDSFLDIGTTEG
LARLEAYFLEKTEQQADKSETVWNFAHLHQYFDSMAGEQQQQLRKDKNEA
AGATSPSAGVMTPYTCVEKSLQVFAKRITKTLINKIGNMVSINDTLLCEL
KRLKSLIVSFKDDARFISVDFSKVHSRIAHLVASYVTHSQEVSVAMRLQL
LQMLRSLRQLLADERGREQHLGCVCASLLLMLEQAPTSAVHLPDTLKTEE
LCCAAWETEQCCACLWDANLSRKTSRRKRTKSLRAAAVVQSQGQLQDTSG
STGSSALHASLGVGSTSLGASRVVASASKDAWRRQQSDDEDYDSDEQVIF
FDCTNVTLPYGSSSEDEENFRTPPQSLSPGISMDLEPRYELFIFGNEPTK
RDLDVLNALSNVDIDKETLPHVYAWKTAMESYSCAEMNLNVKVQKPEPWY
SGTSSSHNSQPLLHPKRLLATPKLNAVVSGRRGSGPLTAPVTPRLARTPS
AASIQVASETNGESVGTAVTPASPILSFAALTAATQSFQTPLNKVRGLFS
QYRDQRSYNEGDTPLGNRN
 
TTGATGTTACCCTATTTTACCGTTGCCTTCGCTTGCCATCAGCGGAACT
TTACATTTTTCACGGAGTTGTGAAGAAGTTGCCTGTTATTGGTGTTGA
TGTCAAACCATTTTAACCGCTTACCTTGCAGTGCATCCGCCATTTTACGC
AGAGATGTCGACCTATTTCGGGGTCTATATCCCGACCTCCAAAGCGGGCT
GTTTTGAGGGATCGGTGTCGCAGTGCATCGGCTCCATAGCCGCGGTGAAC
ATAAAGCCATCCAATCCGGCGTCTGGATCGGCATCAGTAGCATCGGGATC
GCCATCCGGCTCGGCGGCATCCGTGCAAACGGGCAACGCAGACGATGGCA
GTGCTGCCACCAAGTACGAGGATCCCGACTATCCACCGGACTCGCCACTG
TGGCTGATCTTCACGGAGAAATCCAAGGCGCTGGACATCCTGCGACACTA
CAAGGAGGCGCGCCTCCGCGAGTTTCCCAATCTGGAGCAGGCGGAGAGTT
ACGTTCAGTTTGGGTTCGAGAGCATCGAGGCGCTCAAGAGATTTTGCAAG
GCAAAGCCCGAAAGCAAGCCCATTCCGATAATCAGCGGTAGCGGTTACAA
GAGCTCACCGACCTCGACGGACAATTCGTGCTCCTCCTCGCCGACGGGTA
ACGGCAGTGGCTTCATCATTCCCCTGGGAAGCAATTCCTCAATGTCGAAT
TTACTGCTCAGTGACTCACCGACTTCCTCGCCGAGCAGCTCCAGCAACGT
CATTGCCAATGGGCGACAGCAGCAGATGCAGCAGCAACAGCAGCAGCAGC
CGCAGCAGCCGGATGTGTCCGGAGAAGGCCCTCCTTCCGGGCGCCCACC
AAACAGGAACTGGTAGAGTTTCGCAAGCAAATCGAAGGTGGTCACATAGA
CCGGGTGAAGAGGATTATATGGGAGAATCCACGATTTTTGATCAGCAGCG
GTGATACGCCCACCAGTTTGAAGGAGGGCTGTCGCTATAATGCCATGCAC
ATCTGCGCCCAGGTCAATAAGGCCAGGATCGCTCAGTTGCTGTTAAAGAC
CATTTCGGATCGGGAGTTCACTCAGCTTTACGTTGGCAAGAAGGGCAGTG
GCAAGATGTGTGCTGCCCTCAACATCAGTCTCCTGGACTATTACCTGAAC
ATGCCGGACAAGGGGCGCGGCGAAACACCGCTCCACTTTGCCGCAAAGAA
CGGTCATGTGGCCATGGTCGAGGTTCTCGTTTCCTATCCGGAGTGCAAAT
CGCTGCGGAATCATGAGGGCAAGGAGCCCAAGGAAATCATCTGCCTGCGT
 
AATGCTAATGCTACACATGTGACCATCAAGAAGCTGGAGCTGCTCTTGTA
CGATCCGCATTTTGTGCCCGTACTAAGATCCCAGTCAAATACACTGCCGC
CAAAAGTGGGTCAACCGTTCTCGCCCAAAGATCCACCGAACCTGCAACAC
AAAGCGGACGATTACGAGGGCCTCAGCGTGGACCTGGCAATCAGTGCGCT
GGCGGGACCCATGTCCCGCGAAAAGGCCATGAACTTCTATCGCCGTTGGA
AGACACCACCGCGGGTCAGCAACAATGTGATGTCGCCGCTGGCTGGTTCA
CCATTTAGCTCGCCGGTGAAAGTAACCCCAAGCAAGTCGATCTTTGACCG
AAGTGCTGGAAACTCGAGTCCAGTCCACTCAGGACGCAGAGTGCTCTTTA
GTCCATTGGCGGAGGCGACCAGCTCACCAAAACCGACGAAAAACGTGCCC
AATGGCACCAATGAGTGCGAGCACAACAATAATAATGTGAAGCCAGTGTA
TCCGTTGGAGTTCCCGGCGACACCCATTCGAAAAATGAAACCGGATTTAT
TCATGGCCTATCGCAATAACAATAGCTTTGATTCGCCATCTTTGGCCGAT
GACTCCCAAATCCTGGACATGAGCCTAAGCCGCAGCCTGAATGCGTCGCT
AAATGACAGCTTCCGTGAGCGGCACATCAAGAACACTGATATCGAGAAGG
GTCTGGAGGTGGTCGGCCGCCAACTGGCACGACAGGAGCAGTTAGAGTGG
CGCGAGTACTGGGATTTTCTCGATTCATTTTTGGACATTGGTACGACCGA
AGGCCTGGCCCGTCTTGAAGCGTATTTCCTGGAAAAGACCGAACAGCAGG
CGGATAAATCAGAAACGGTCTGGAACTTTGCCCATCTGCATCAGTATTTC
GATTCGATGGCCGGCGAGCAACAGCAGCAACTCCGAAAGGATAAAAATGA
GGCTGCGGGAGCAACTTCGCCATCCGCCGGAGTCATGACTCCGTACACAT
GCGTAGAGAAGTCGCTGCAAGTGTTCGCCAAGCGCATCACTAAAACGTTG
ATCAACAAAATCGGCAACATGGTGTCCATCAACGACACGCTGCTCTGTGA
GCTCAAAAGACTGAAATCGCTGATTGTCAGCTTCAAGGATGATGCCCGCT
TCATTAGCGTGGACTTTAGCAAGGTGCATTCACGTATCGCCCACCTGGTG
GCCAGCTATGTGACCCACTCGCAGGAGGTCAGCGTAGCCATGCGTCTACA
ATTGTTGCAGATGCTCCGAAGTTTGCGGCAACTGCTGGCCGACGAGCGTG
GTCGAGAACAGCATTTGGGCTGCGTGTGCGCTAGTCTATTGCTGATGCTG
GAACAGGCGCCGACATCCGCCGTGCATCTACCAGACACTCTGAAGACCGA
GGAGCTATGTTGCGCCGCCTGGGAGACGGAGCAGTGTTGCGCCTGTCTGT
GGGACGCAAATCTCAGCCGTAAGACCAGTCGTCGAAAGCGCACTAAGTCG
CTGCGGGCAGCTGCTGTTGTTCAGTCTCAGGGTCAGCTTCAGGATACTTC
GGGATCGACAGGGTCGTCCGCCTTGCACGCTTCGCTTGGTGTGGGATCGA
CCAGTTTGGGAGCATCGAGGGTCGTGGCGTCCGCTTCGAAAGATGCTTGG
CGCCGTCAACAAAGCGACGACGAGGACTACGACAGCGATGAGCAAGTAAT
CTTTTTCGACTGCACTAATGTTACGCTGCCTTATGGAAGCAGCAGCGAGG
ACGAGGAAAACTTCCGTACGCCGCCGCAAAGCTTGTCGCCAGGTATTTCC
ATGGATTTGGAGCCGCGTTACGAGTTGTTTATTTTTGGAAACGAGCCAAC
CAAGCGAGATTTGGATGTGCTGAATGCCCTTTCCAATGTCGACATTGATA
AGGAAACACTGCCGCATGTCTACGCCTGGAAGACTGCCATGGAGAGCTAC
TCCTGTGCTGAAATGAATCTGAACGTCAAGGTTCAAAAGCCGGAGCCTTG
GTATTCTGGAACCAGTTCTAGCCACAACAGCCAACCATTGTTGCATCCCA
AGCGTCTGCTTGCCACGCCAAAGCTGAATGCCGTGGTCAGCGGCAGACGC
GGATCCGGACCATTGACGGCGCCAGTTACACCGCGTCTGGCGCGAACTCC
GTCCGCCGCCAGTATTCAAGTTGCATCCGAGACGAATGGCGAGTCGGTCG
GAACTGCTGTGACTCCGGCATCGCCGATTTTGAGTTTTGCCGCCTTGACG
GCAGCGACGCAGTCATTCCAAACACCATTGAACAAGGTGCGCGGCTTGTT
CAGCCAATATCGGGATCAACGGTCCTATAACGAGGGGGACACGCCGCTGG
GCAATCGGAACTGAAACGGAATCGGCCCGGAAACAGAAACAGAAACAGCG
 
ACTGATTGATGAAAGGCCGACTGCATACTTACCCCCCTGAATAGCCGGTG
TCGTCCATTGTCCCTTTTAATGTTAATCGCATGTATATTA
 
MSTYFGVYIPTSKAGCFEGSVSQCIGSIAAVNIKPSNPASGSASVASGSP
SGSAASVQTGNADDGSAATKYEDPDYPPDSPLWLIFTEKSKALDILRHYK
EARLREFPNLEQAESYVQFGFESIEALKRFCKAKPESKPIPIISGSGYKS
SPTSTDNSCSSSPTGNGSGFIIPLGSNSSMSNLLLSDSPTSSPSSSSNVI
ANGRQQQMQQQQQQPQQPDVSGEGPPFRAPTKQELVEFRKQIEGGHIDR
VKRIIWENPRFLISSGDTPTSLKEGCRYNAMHICAQVNKARIAQLLLKTI
SDREFTQLYVGKKGSGKMCAALNISLLDYYLNMPDKGRGETPLHFAAKNG
HVAMVEVLVSYPECKSLRNHEGKEPKEIICLRNANATHVTIKKLELLLYD
PHFVPVLRSQSNTLPPKVGQPFSPKDPPNLQHKADDYEGLSVDLAISALA
GPMSREKAMNFYRRWKTPPRVSNNVMSPLAGSPFSSPVKVTPSKSIFDRS
AGNSSPVHSGRRVLFSPLAEATSSPKPTKNVPNGTNECEHNNNNVKPVYP
LEFPATPIRKMKPDLFMAYRNNNSFDSPSLADDSQILDMSLSRSLNASLN
DSFRERHIKNTDIEKGLEVVGRQLARQEQLEWREYWDFLDSFLDIGTTEG
LARLEAYFLEKTEQQADKSETVWNFAHLHQYFDSMAGEQQQLRKDKNEA
AGATSPSAGVMTPYTCVEKSLQVFAKRITKTLINKIGNMVSINDTLLCEL
KRLKSLIVSFKDDARFISVDFSKVHSRIAHLVASYVTHSQEVSVAMRLQL
LQMLRSLRQLLADERGREQHLGCVCASLLLMLEQAPTSAVHLPDTLKTEE
LCCAAWETEQCCACLWDANLSRKTSRRKRTKSLRAAAVVQSQGQLQDTSG
STGSSALHASLGVGSTSLGASRVVASASKDAWRRQQSDDEDYDSDEQVIF
FDCTNVTLPYGSSSEDEENFRTPPQSLSPGISMDLEPRYELFIFGNEPTK
RDLDVLNALSNVDIDKETLPHVYAWKTAMESYSCAEMNLNVKVQKPEPWY
SGTSSSHNSQPLLHPKRLLATPKLNAVVSGRRGSGPLTAPVTPRLARTPS
AASIQVASETNGESVGTAVTPASPILSFAALTAATQSFQTPLNKVRGLFS
QYRDQRSYNEGDTPLGNRN
 
AAAACAGCCAGCTCATTTATTAATGGTTTATCCCTCTCGATGCCCACACA
TCAACATTGCCATCGCCACGACGGAGCAGCGGACTCGCCACTGTGGCTGA
TCTTCACGGAGAAATCCAAGGCGCTGGACATCCTGCGACACTACAAGGAG
GCGCGCCTCCGCGAGTTTCCCAATCTGGAGCAGGCGGAGAGTTACGTTCA
GTTTGGGTTCGAGAGCATCGAGGCGCTCAAGAGATTTGCAAGGCAAAGC
CCGAAAGCAAGCCCATTCCGATAATCAGCGGTAGCGGTTACAAGAGCTCA
CCGACCTCGACGGACAATTCGTGCTCCTCCTCGCCGACGGGTAACGGCAG
TGGCTTCATCATTCCCCTGGGAAGCAATTCCTCAATGTCGAATTTACTGC
TCAGTGACTCACCGACTTCCTCGCCGAGCAGCTCCAGCAACGTCATTGCC
AATGGGCGACAGCAGCAGATGCAGCAGCAACAGCAGCAGCAGCCGCAGCA
GCCGGATGTGTCCGGAGAAGGCCCTCCTTTCCGGGCGCCCACCAAACAGG
AACTGGTAGAGTTTCGCAAGCAAATCGAAGGTGGTCACATAGACCGGGTG
AAGAGGATTATATGGGAGAATCCACGATTTTTGATCAGCAGCGGTGATAC
GCCCACCAGTTTGAAGGAGGGCTGTCGCTATAATGCCATGCACATCTGCG
CCCAGGTCAATAAGGCCAGGATCGCTCAGTTGCTGTTAAAGACCATTTCG
GATCGGGAGTTCACTCAGCTTTACGTTGGCAAGAAGGGCAGTGGCAAGAT
GTGTGCTGCCCTCAACATCAGTCTCCTGGACTATTACCTGAACATGCCGG
ACAAGGGGCGCGGCGAAACACCGCTCCACTTTGCCGCAAAGAACGGTCAT
 
GTGGCCATGGTCGAGGTTCTCGTTTCCTATCCGGAGTGCAAATCGCTGCG
GAATCATGAGGGCAAGGAGCCCAAGGAAATCATCTGCCTGCGTAATGCTA
ATGCTACACATGTGACCATCAAGAAGCTGGAGCTGCTCTTGTACGATCCG
CATTTTGTGCCCGTACTAAGATCCCAGTCAAATACACTGCCGCCAAAAGT
GGGTCAACCGTTCTCGCCCAAAGATCCACCGAACCTGCAACACAAAGCGG
ACGATTACGAGGGCCTCAGCGTGGACCTGGCAATCAGTGCGCTGGCGGGA
CCCATGTCCCGCGAAAAGGCCATGAACTTCTATCGCCGTTGGAAGACACC
ACCGCGGGTCAGCAACAATGTGATGTCGCCGCTGGCTGGTTCACCATTTA
GCTCGCCGGTGAAAGTAACCCCAAGCAAGTCGATCTTTGACCGAAGTGCT
GGAAACTCGAGTCCAGTCCACTCAGGACGCAGAGTGCTCTTTAGTCCATT
GGCGGAGGCGACCAGCTCACCAAAACCGACGAAAAACGTGCCCAATGGCA
CCAATGAGTGCGAGCACAACAATAATAATGTGAAGCCAGTGTATCCGTTG
GAGTTCCCGGCGACACCCATTCGAAAAATGAAACCGGATTTATTCATGGC
CTATCGCAATAACAATAGCTTTGATTCGCCATCTTTGGCCGATGACTCCC
AAATCCTGGACATGAGCCTAAGCCGCAGCCTGAATGCGTCGCTAAATGAC
AGCTTCCGTGAGCGGCACATCAAGAACACTGATATCGAGAAGGGTCTGGA
GGTGGTCGGCCGCCAACTGGCACGACAGGAGCAGTTAGAGTGGCGCGAGT
ACTGGGATTTTCTCGATTCATTTTTGGACATTGGTACGACCGAAGGCCTG
GCCCGTCTTGAAGCGTATTTCCTGGAAAAGACCGAACAGCAGGCGGATAA
ATCAGAAACGGTCTGGAACTTTGCCCATCTGCATCAGTATTTCGATTCGA
TGGCCGGCGAGCAACAGCAGCAACTCCGAAAGGATAAAAATGAGGCTGCG
GGAGCAACTTCGCCATCCGCCGGAGTCATGACTCCGTACACATGCGTAGA
GAAGTCGCTGCAAGTGTTCGCCAAGCGCATCACTAAAACGTTGATCAACA
AAATCGGCAACATGGTGTCCATCAACGACACGCTGCTCTGTGAGCTCAAA
AGACTGAAATCGCTGATTGTCAGCTTCAAGGATGATGCCCGCTTCATTAG
CGTGGACTTTAGCAAGGTGCATTCACGTATCGCCCACCTGGTGGCCAGCT
ATGTGACCCACTCGCAGGAGGTCAGCGTAGCCATGCGTCTACAATTGTTG
CAGATGCTCCGAAGTTTGCGGCAACTGCTGGCCGACGAGCGTGGTCGAGA
ACAGCATTTGGGCTGCGTGTGCGCTAGTCTATTGCTGATGCTGGAACAGG
CGCCGACATCCGCCGTGCATCTACCAGACACTCTGAAGACCGAGGAGCTA
TGTTGCGCCGCCTGGGAGACGGAGCAGTGTTGCGCCTGTCTGTGGGACGC
AAATCTCAGCCGTAAGACCAGTCGTCGAAAGCGCACTAAGTCGCTGCGGG
CAGCTGCTGTTGTTCAGTCTCAGGGTCAGCTTCAGGATACTTCGGGATCG
ACAGGGTCGTCCGCCTTGCACGCTTCGCTTGGTGTGGGATCGACCAGTTT
GGGAGCATCGAGGGTCGTGGCGTCCGCTTCGAAAGATGCTTGGCGCCGTC
AACAAAGCGACGACGAGGACTACGACAGCGATGAGCAAGTAATCTTTTTC
GACTGCACTAATGTTACGCTGCCTTATGGAAGCAGCAGCGAGGACGAGGA
AAACTTCCGTACGCCGCCGCAAAGCTTGTCGCCAGGTATTTCCATGGATT
TGGAGCCGCGTTACGAGTTGTTTATTTTGGAAACGAGCCAACCAAGCGA
GATTTGGATGTGCTGAATGCCCTTTCCAATGTCGACATTGATAAGGAAAC
ACTGCCGCATGTCTACGCCTGGAAGACTGCCATGGAGAGCTACTCCTGTG
CTGAAATGAATCTGAACGTCAAGGTTCAAAAGCCGGAGCCTTGGTATTCT
GGAACCAGTTCTAGCCACAACAGCCAACCATTGTTGCATCCCAAGCGTCT
GCTTGCCACGCCAAAGCTGAATGCCGTGGTCAGCGGCAGACGCGGATCCG
GACCATTGACGGCGCCAGTTACACCGCGTCTGGCGCGAACTCCGTCCGCC
GCCAGTATTCAAGTTGCATCCGAGACGAATGGCGAGTCGGTCGGAACTGC
TGTGACTCCGGCATCGCCGATTTTGAGTTTTGCCGCCTTGACGGCAGCGA
CGCAGTCATTCCAAACACCATTGAACAAGGTGCGCGGCTTGTTCAGCCAA
 
TATCGGGATCAACGGTCCTATAACGAGGGGGACACGCCGCTGGGCAATCG
GAACTGAAACGGAATCGGCCCGGAAACAGAAACAGAAACAGCGACTGATT
GATGAAAGGCCGACTGCATACTTACCCCCCTGAATAGCCGGTGTCGTCCA
TTGTCCCTTTTAATGTTAATCGCATGTATATTA
 
MPTHQHCHRHDGAADSPLWLIFTEKSKALDILRHYKEARLREFPNLEQAE
SYVQFGFESIEALKRFCKAKPESKPIPIISGSGYKSSPTSTDNSCSSSPT
GNGSGFIIPLGSNSSMSNLLLSDSPTSSPSSSSNVIANGRQQQMQQQQQQ
QPQQPDVSGEGPPFRAPTKQELVEFRKQIEGGHIDRVKRIIWENPRFLIS
SGDTPTSLKEGCRYNAMHICAQVNKARIAQLLLKTISDREFTQLYVGKKG
SGKMCAALNISLLDYYLNMPDKGRGETPLHFAAKNGHVAMVEVLVSYPEC
KSLRNHEGKEPKEIICLRNANATHVTIKKLELLLYDPHFVPVLRSQSNTL
PPKVGQPFSPKDPPNLQHKADDYEGLSVDLAISALAGPMSREKAMNFYRR
WKTPPRVSNNVMSPLAGSPFSSPVKVTPSKSIFDRSAGNSSPVHSGRRVL
FSPLAEATSSPKPTKNVPNGTNECEHNNNNVKPVYPLEFPATPIRKMKPD
LFMAYRNNNSFDSPSLADDSQILDMSLSRSLNASLNDSFRERHIKNTDIE
KGLEVVGRQLARQEQLEWREYWDFLDSFLDIGTTEGLARLEAYFLEKTEQ
QADKSETVWNFAHLHQYFDSMAGEQQQQLRKDKNEAAGATSPSAGVMTPY
TCVEKSLQVFAKRITKTLINKIGNMVSINDTLLCELKRLKSLIVSFKDDA
RFISVDFSKVHSRIAHLVASYVTHSQEVSVAMRLQLLQMLRSLRQLLADE
RGREQHLGCVCASLLLMLEQAPTSAVHLPDTLKTEELCCAAWETEQCCAC
LWDANLSRKTSRRKRTKSLRAAAVVQSQGQLQDTSGSTGSSALHASLGVG
STSLGASRVVASASKDAWRRQQSDDEDYDSDEQVIFFDCTNVTLPYGSSS
EDEENFRTPPQSLSPGISMDLEPRYELFIFGNEPTKRDLDVLNALSNVDI
DKETLPHVYAWKTAMESYSCAEMNLNVKVQKPEPWYSGTSSSHNSQPLLH
PKRLLATPKLNAVVSGRRGSGPLTAPVTPRLARTPSAASIQVASETNGES
VGTAVTPASPILSFAALTAATQSFQTPLNKVRGLFSQYRDQRSYNEGDTP
LGNRN
**Human homologue of Complete Genome candidate** BAA31667 KIAA0692 protein 
1 gagattttgg ttacagtgtg ggcctgaatc ctccagagga ggaagctgtg acatccaaga
61 cctgctcggt gccccctagt gacaccgaca cctacagagc tggagcgact gcgtctaagg
121 agccgcccct gtactatggg gtgtgtccag tgtatgagga cgtcccagcg agaaatgaaa
181 ggatctatgt ttatgaaaat aaaaaggaag cattgcaagc tgtcaagatg atcaaagggt
241 cccgatttaa agctttttct accagagaag acgctgagaa atttgctaga ggaatttgtg
301 attatttccc ttctccaagc aaaacgtcct taccactgtc tcctgtgaaa acagctccac
361 tctttagcaa tgacaggttg aaagatggtt tgtgcttgtc ggaatcagaa acagtcaaca
421 aagagcgagc gaacagttac aaaaatcccc gcacgcagga cctcaccgcc aagcttcgga
481 aagctgtgga gaagggagag gaggacacct tttctgacct tatctggagc aacccccggt
541 atctgatagg ctcaggagac aaccccacta tcgtgcagga agggtgcagg tacaacgtga
601 tgcatgttgc tgccaaagag aaccaggctt ccatctgcca gctgactctg gacgtcctgg
661 agaaccctga cttcatgagg ctgatgtacc ctgatgacga cgaggccatg ctgcagaagc
721 gtatccgtta cgtggtggac ctgtacctca acacccccga caagatgggc tatgacacac
781 cgttgcattt tgcttgtaag tttggaaatg cagatgtagt caacgtgctt tcgtcacacc
 
841 atttgattgt aaaaaactca aggaataaat atgataaaac acctgaagat gtaatttgtg
901 aaagaagcaa aaataaatct gtggaactga aggagcggat cagagagtat ttaaagggcc
961 actactacgt gcccctcctg agagcggaag agacttcttc tccagtcatc ggggagctgt
1021 ggtccccaga ccagacggct gaggcctctc acgtcagccg ctatggaggc agccccagag
1081 acccggtact gaccctgaga gccttcgcag ggcccctgag tccagccaag gcagaagatt
1141 ttcgcaagct ctggaaaact ccacctcgag agaaagcagg cttccttcac cacgtcaaga
1201 agtcggaccc ggaaagaggc tttgagagag tgggaaggga gctagctcat gagctggggt
1261 atccctgggt tgaatactgg gaatttctgg gctgttttgt tgatctgtct tcccaggaag
1321 gcctgcaaag actagaagaa tatctcacac agcaggaaat aggcaaaaag gctcaacaag
1381 aaacaggaga acgggaagcc tcctgccgag ataaagccac cacgtctggc agcaattcca
1441 tttccgtgag ggcgtttcta gatgaagatg acatgagctt ggaagaaata aaaaatcggc
1501 aaaatgcagc tcgaaataac agcccgccca cagtcggtgc ttttggacat acgaggtgca
1561 gcgccttccc cttggagcag gaggcagacc tcatagaagc cgccgagccg ggaggtccac
1621 acagcagcag aaatgggctc tgccatcctc tgaatcacag caggaccctg gcgggcaaga
1681 gaccaaaggc cccccatggg gaggaagccc atctgccacc tgtctcggat ttgactgttg
1741 agtttgataa actgaatttg caaaatatag gacgtagcgt ttccaagaca ccagatgaaa
1801 gtacaaaaac taaagatcag atcctgactt caagaatcaa tgcagtagaa agagacttgt
1861 tagagccttc tcccgcagac caactcggga atggccacag gaggacagaa agtgaaatgt
1921 cagccaggat cgctaaaatg tccttgagtc ccagcagccc caggcacgag gatcagctcg
1981 aggtcaccag ggaaccggcc aggcggctct tcctttttgg agaggagcca tcaaaactcg
2041 atcaggatgt tttggccgct cttgaatgtg cagacgtcga cccccatcag ttcccggccg
2101 tgcacagatg gaagagtgct gtcctgtgct actcaccctc ggacagacag agttggccca
2161 gtcccgcggt gaaaggaagg ttcaagtctc agctgccaga tctcagtggc cctcacagct
2221 acagtccggg gagaaacagc gtggctggaa gcaaccccgc aaagccaggc ctgggcagtc
2281 ctgggcgcta cagccccgtg cacgggagcc agctccgcag gatggcgcgc ctggctgagc
2341 ttgccgccct gtaggcttgg cgctgggctc tcggtttgtt cttcattttt aaagaaggaa
2401 gggtcatatg tttattgcta aactgtcaaa aaggaatata ttctgattaa attattactc
2461 ctcactttga gggtgtgaga attttagaag atttaaatgt tctatataac acttagattt
2521 ctgatatttt ggaagaagtt agaagttaat gaaagcaaac tcagttacca attttctgga
2581 aaatatccat gtggtaatgt agacttttta ggtggcaatt tctaggtctg aaatatagca
2641 gaggaaaggg cgctgaggca gttgcaggca ggcagccctg tacttaccct gtactcacct
2701 catccgacag acgctgtgga tgaggagggg cttggcggag gcgtgagcac cgatgtccct
2761 ttgataacct gcactcacca agatgaacta tttgccgccc tgtcttttcc tgggttgggg
2821 ggtggcatct gatggtggca gagtgcctgt tggttcgccc gtgggtctca tggttcagac
2881 agagggaggt ggacggcagg gatcagggag ccaggagcgc gcctcagact tgcagcaacc
2941 attgtgattt gggttgttcg gaatatttaa attactgatc agaagatgaa agtagctttt
3001 ctcttgggaa gtcttgcagc ccgtgggagt gataccagga gcaacacaga gctcagcagc
3061 ggcgccaagg tgttccctgt ttcctcagca cgtgagcctt caccgcctgc ttcattcagg
3121 agccagtgca gcagtaatac agtctataca ttgttctgtt ttcaaattta tcctgaggct
3181 ttgttgagca taaatgatta tacgataaag gtatccgtta ttttggaact catttcagtt
3241 gggatctcct gtatgcagag tgttgcattt agaggtttga gtcccatctt ggtttcttgc
3301 cgtgctgact gtagccttca ccttgacttg aatgaaggtc tgtggttgga atgtgtgagg
3361 agccgctgag gtgttcagga ggtgctgcct ggaggtcggt ttcttcctgg gtgttacggg
3421 caactgctca cacagttgtt tctctgtgaa catttccagt gtttaatcca aaatgaaaac
3481 ccaccaatgc ttttgctaac ttcagtgcct tttataaatc atttttaaat ttcctgaact
3541 tgctttttga ggatatacag ggatattaag tagacgcagg attgtttttg tttgtaaaaa
3601 ttctgaattg aaactttgtt ttaaaaaaag gcttctttct ttcatatgac aagagatagg
3661 tcaggaatat tggaatcaag atttaaatgt taaaattcga ttttgttaca cagggtgtgt
 
3721 tcatttgttt tgtagcagac aagatctaga tcccagacag aaacaacaca tgctattcta
3781 aaaagccgca ttttaaaagg caccttggtt ctcaaaagaa atcagaatat ggatattcgt
3841 agtgatgatc tgttttctct aaaatcttac catattgtct gtatatggtt gtaaattcaa
3901 atggaaagta aaacgttttg gccctgattt tgtatgtgga ccactgctcc tgatttccca
3961 ggtcttaggc cacctttgac tgtttctccg tttgtttgtg ggcagcgatt ccagtcccaa
4021 cggaggcatt ctcgtgtgtc ccggggggtt atgtccttca caaaacactt aatgaaatga
4081 attacttc
 
1 dfgysvglnp peeeavtskt csvppsdtdt yragataske pplyygvcpv yedvparner
61 iyvyenkkea lqavkmikgs rfkafstred aekfargicd yfpspsktsl plspvktapl
121 fsndrlkdgl clsesetvnk eransyknpr tqdltaklrk avekgeedtf sdliwsnpry
181 ligsgdnpti vqegcrynvm hvaakenqas icqltldvle npdfmrlmyp dddeamlqkr
241 iryvvdlyln tpdkmgydtp lhfackfgna dvvnvlsshh livknsrnky dktpedvice
301 rsknksvelk erireylkgh yyvpllraee tsspvigelw spdqtaeash vsryggsprd
361 pvltlrafag plspakaedf rklwktppre kagflhhvkk sdpergferv grelahelgy
421 pwveyweflg cfvdlssqeg lqrleeyltq qeigkkaqqe tgereascrd kattsgsnsi
481 svrafldedd msleeiknrq naarnnsppt vgafghtrcs afpleqeadl ieaaepggph
541 ssrnglchpl nhsrtlagkr pkaphgeeah lppvsdltve fdklnlqnig rsvsktpdes
601 tktkdqilts rinaverdll epspadqlgn ghrrtesems ariakmslsp ssprhedqle
661 vtreparrlf lfgeepskld qdvlaaleca dvdphqfpav hrwksavlcy spsdrqswps
721 pavkgrfksq lpdlsgphsy spgrnsvags npakpglgsp gryspvhgsq lrrmarlael
781 aal
**Putative function** Unknown

### Example 15 (Category 3)

**Line ID** - 379
**Category** - Lethal phase pharate adult, Dot and rod-like overcondensed chromosomes, high mitotic index, overcondensed anaphases some with lagging chromosomes, a few tetraploid cells with overcondensed chromosomes, XYY males.
**Annotated *Drosophila* genome genomic segment containing P element insertion site (and map position)** - AE003443 (7D14-E2)
**P element insertion site** - 130,532
**Annotated *Drosophila* genome Complete Genome candidate -** 2 candidates: CG10964 - novel, similarity to dehydrogenases 
AACGAAACAGCCGGCCGTCAAAATTTTCCTAACATTTCACTATTTCAC
GCTTGTGTTACGGCAATAAAGTCGATTGATAAGCACGGAAAGATCTGGCT
GCGGGTCTGGTGAAATCCACAGAACACACGGAACCCGTATAGTAGTGCCG
CCCTTTATTGGTTTATCTCAAGTACGACGCGATAAGATTTCGAGCAACT
CGATCGCGGATCTTCGGAAAAAAAAACATGAACTCCATCCTGATAACCG
GCTGCAATCGAGGATTGGGTCTGGGCCTGGTCAAGGCGCTGCTCAATCTT
CCCCAGCCGCCGCAGCATCTATTTACCACCTGCCGGAATCGCGAGCAGGC
AAAGGAGCTGGAGGATCTAGCCAAGAACCACTCGAACATACACATACTTG
AGATTGATTTGAGAAATTTCGATGCCTATGACAAGCTAGTCGCCGACATC
GAGGGCGTGACCAAGGACCAAGGCCTCAATGTGCTCTTCAACAATGCCGG
CATAGCGCCCAAATCGGCCAGGATAACGGCCGTTCGATCGCAGGAGCTGC
TCGACACCTTGCAGACCAACACGGTTGTGCCCATCATGCTGGCCAAGGCG
TGTCTGCCGCTCCTTAAGAAGGCAGCCAAAGCGAACGAATCCCAGCCGAT
GGGCGTGGGCCGTGCCGCCATTATTAACATGTCCTCGATCCTTGGCTCCA
TCCAGGGCAACACGGACGGCGGAATGTACGCCTATCGCACCTCTAAGTCG
GCCTTGAATGCGGCCACCAAGTCGTTGAGCGTGGATCTGTATCCGCAACG
CATCATGTGCGTCAGTCTGCATCCTGGCTGGGTGAAAACCGACATGGGTG
GCTCCAGTGCCCCCTTGGACGTGCCCACCAGCACGGACAAATTGTGCAG
ACCATCAGCAAGCTGGGCGAGAAACAGAACGGCGGTTTTGTCAACTACGA
CGGCACTCCGCTGGCCTGGTAA
 
MNSILITGCNRGLGLGLVKALLNLPQPPQHLFTTCRNREQAKELEDLAKN
HSNIHILEIDLRNFDAYDKLVADIEGVTKDQGLNVLFNNAGIAPKSARIT
AVRSQELLDTLQTNTVVPIMLAKACLPLLKKAAKANESQPMGVGRAAIIN
MSSILGSIQGNTDGGMYAYRTSKSALNAATKSLSVDLYPQRIMCVSLHPG
WVKTDMGGSSAPLDVPTSTGQIVQTISKLGEKQNGGFVNYDGTPLAW
 CG2151 -Trxr-1 thoredoxin reductase -1 (2 splice variants) 
CGACAAGCCAATCGACGTCTCCCTTTCGCACGCTCGTACGAAAGTACAAA
AGCTATTGCAAAAGTTGGCTCCGCTTATTCGTTTCGTGCTTTCGCGAGTG
 
CCGAGAGCCGCTACAATACACGCTTAGCAGTTTTACATTTCCGCTTCGA
CTACAACAACATTCACTACCCGCCGTTGATCCTTGTTTTCTGTCTGATTT
ACGTGGAGCACCTACCAACAAGCAACAAAATAATGGCGCCCGTGCAAGGA
TCCTACGACTACGACCTTATTGTGATTGGAGGCGGCTCAGCTGGCCTGGC
CTGCGCCAAGGAGGCAGTCCTCAATGGAGCCCGTGTGGCCTGTCTGGATT
TCGTTAAGCCCACGCCCACTCTGGGCACCAAGTGGGGCGTTGGCGGCACC
TGCGTGAACGTGGGCTGCATTCCCAAGAAGCTGATGCACCAGGCCTCCCT
TCTGGGCGAGGCTGTCCATGAGGCGGCCGCCTACGGCTGGAACGTGGACG
AAAAGATCAAGCCAGACTGGCACAAGCTGGTGCAGTCCGTACAGAACCAC
ATCAAGTCCGTCAACTGGGTGACCCGTGTGGATCTGCGCGACAAGAAAGT
GGAGTACATCAATGGACTGGGCTCCTTCGTGGACTCGCACACACTGCTGG
CCAAGCTGAAGAGCGGCGAGCGCACAATCACCGCCCAGACCTTCGTCATT
GCCGTTGGCGGCCGACCACGTTATCCGGATATTCCCGGTGCTGTCGAGTA
TGGCATCACCAGCGATGATCTGTTCAGTTTGGACCGCGAGCCCGGCAAGA
CCCTGGTGGTGGGAGCTGGCTACATTGGCTTGGAGTGCGCTGGATTCCTG
AAGGGTCTCGGCTACGAGCCCACTGTGATGGTGCGTTCTATTGTGCTGCG
TGGCTTCGACCAGCAGATGGCCGAGCTGGTGGCAGCCTCGATGGAGGAGC
GTGGCATTCCCTTCCTCCGCAAGACGGTGCCGCTGTCCGTGGAAAAGCAG
GATGATGGCAAGCTGCTCGTGAAGTACAAGAACGTGGAGACCGGCGAGGA
GGCCGAGGATGTTTACGACACCGTTCTGTGGGCCATCGGCCGCAAGGGTC
TGGTGGACGATCTGAACCTGCCCAATGCCGGCGTGACTGTGCAGAAGGAC
AAGATTCCAGTGGACTCCCAGGAGGCTACCAATGTGGCAAACATCTACGC
TGTCGGCGATATCATCTATGGCAAGCCAGAGCTGACGCCCGTCGCCGTTT
TGGCTGGCCGTTTGCTGGCCCGCCGCCTGTACGGAGGATCTACCCAGCGC
ATGGACTACAAGGATGTGGCCACCACCGTTTTCACGCCCCTGGAGTACGC
CTGCGTCGGCCTGAGCGAGGAGGATGCCGTCAAGCAGTTCGGAGCCGATG
AGATCGAGGTGTTCCACGGCTACTACAAGCCCACGGAGTTCTTCATTCCC
CAGAAGAGCGTGCGCTACTGCTACTTGAAGGCTGTGGCCGAGCGCCATGG
TGACCAGCGCGTCTATGGACTGCACTATATTGGCCCGGTGGCCGGTGAGG
TTATCCAGGGATTCGCTGCCGCTTTGAAGTCTGGCCTGACTATTAACACG
CTGATCAACACCGTGGGCATCCATCCCACTACCGCCGAAGAATTCACCCG
GCTGGCCATCACCAAGCGCTCCGGACTGGACCCCACGCCGGCCAGCTGCT
GCAGCTAAAGCGGGAACGCAGCTCAGCCGCCTGGGACGTGTCGAAGCCGC
TTGCTCCACCCGAAATCCCGTAGATGAATGGTTGTTGTCGCGGCCCAGCG
ATCGATGAGTTCAATAGTTCCGTTTCGTTTCCACAATTAACACCCAACAC
AATAGCTCTGCGCAAGGGAGGGGCACTGGGCAGCGATGGCGGGTGGAACG
ACACCAGTGGAACTACCCGCGCGACCAGCCCAACCCACGACTGCTGCGCC
GCCGACATGCACTCAAAATTTGAATTTGTTTGAACCTATGAAATTAACT
ATGAAATCCCCTAAATGTACGGTTGAAGAATATAATTTTTCACC
 
MAPVQGSYDYDLIVIGGGSAGLACAKEAVLNGARVACLDFVKPTPTLGTK
WGVGGTCVNVGCIPKKLMHQASLLGEAVHEAAAYGWNVDEKIKPDWHKLV
QSVQNHIKSVNWVTRVDLRDKKVEYINGLGSFVDSHTLLAKLKSGERTIT
AQTFVIAVGGRPRYPDIPGAVEYGITSDDLFSLDREPGKTLVVGAGYIGL
ECAGFLKGLGYEPTVMVRSIVLRGFDQQMAELVAASMEERGIPFLRKTVP
LSVEKQDDGKLLVKYKNVETGEEAEDVYDTVLWAIGRKGLVDDLNLPNAG
VTVQKDKIPVDSQEATNVANIYAVGDIIYGKPELTPVAVLAGRLLARRLY
 
GGSTQRMDYKDVATTVFTPLEYACVGLSEEDAVKQFGADEIEVFHGYYKP
TEFFIPQKSVRYCYLKAVAERHGDQRVYGLHYIGPVAGEVIQGFAAALKS
GLTINTLINTVGIHPTTAEEFTRLAITKRSGLDPTPASCCS
 
CCCGGCCGAACCAGCGAACGTGTTTGTGTTGTGTGTTCCGCCGTCATTTT
TCTGCACCCTTTTCGCGAATAGTTTCGTTTCGCCTCCAGCTGGTAGAGTG
AAACGCCAAACGTTGAAGAAGGGGAAAGGCCAACAAGATGAACTTGTGCA
ATTCGAGATTCTCCGTTACGTTCGTGCGGCAGTGCTCGACGATTTTAACG
TCTCCTTCGGCTGGCATTATACAAAACAGAGGCTCACTGACAACAAAGGT
TCCCCATTGGATTTCCAGTAGTCTCAGCTGTGCCCATCACACGTTTCAGC
GAACTATGAACTTGACGGGACAGCGAGGATCACGCGACAGTACTGGAGCT
ACCGGTGGGAATGCTCCAGCCGGATCCGGTGCCGGCGCACCACCACCCTT
CCAGCATCCACATTGCGACAGGGCGGCCATGTACGCGCAACCGGTGCGAA
AGATGAGCACCAAAGGAGGATCCTACGACTACGACCTTATTGTGATTGGA
GGCGGCTCAGCTGGCCTGGCCTGCGCCAAGGAGGCAGTCCTCAATGGAGC
CCGTGTGGCCTGTCTGGATTTCGTTAAGCCCACGCCCACTCTGGGCACCA
AGTGGGGCGTTGGCGGCACCTGCGTGAACGTGGGCTGCATTCCCAAGAAG
CTGATGCACCAGGCCTCCCTTCTGGGCGAGGCTGTCCATGAGGCGGCCGC
CTACGGCTGGAACGTGGACGAAAAGATCAAGCCAGACTGGCACAAGCTGG
TGCAGTCCGTACAGAACCACATCAAGTCCGTCAACTGGGTGACCCGTGTG
GATCTGCGCGACAAGAAAGTGGAGTACATCAATGGACTGGGCTCCTTCGT
GGACTCGCACACACTGCTGGCCAAGCTGAAGAGCGGCGAGCGCACAATCA
CCGCCCAGACCTTCGTCATTGCCGTTGGCGGCCGACCACGTTATCCGGAT
ATTCCCGGTGCTGTCGAGTATGGCATCACCAGCGATGATCTGTTCAGTTT
GGACCGCGAGCCCGGCAAGACCCTGGTGGTGGGAGCTGGCTACATTGGCT
TGGAGTGCGCTGGATTCCTGAAGGGTCTCGGCTACGAGCCCACTGTGATG
GTGCGTTCTATTGTGCTGCGTGGCTTCGACCAGCAGATGGCCGAGCTGGT
GGCAGCCTCGATGGAGGAGCGTGGCATTCCCTTCCTCCGCAAGACGGTGC
CGCTGTCCGTGGAAAAGCAGGATGATGGCAAGCTGCTCGTGAAGTACAAG
AACGTGGAGACCGGCGAGGAGGCCGAGGATGTTTACGACACCGTTCTGTG
GGCCATCGGCCGCAAGGGTCTGGTGGACGATCTGAACCTGCCCAATGCCG
GCGTGACTGTGCAGAAGGACAAGATTCCAGTGGACTCCCAGGAGGCTACC
AATGTGGCAAACATCTACGCTGTCGGCGATATCATCTATGGCAAGCCAGA
GCTGACGCCCGTCGCCGTTTTGGCTGGCCGTTTGCTGGCCCGCCGCCTGT
ACGGAGGATCTACCCAGCGCATGGACTACAAGGATGTGGCCACCACCGTT
TTCACGCCCCTGGAGTACGCCTGCGTCGGCCTGAGCGAGGAGGATGCCGT
CAAGCAGTTCGGAGCCGATGAGATCGAGGTGTTCCACGGCTACTACAAGC
CCACGGAGTTCTTCATTCCCCAGAAGAGCGTGCGCTACTGCTACTTGAAG
GCTGTGGCCGAGCGCCATGGTGACCAGCGCGTCTATGGACTGCACTATAT
TGGCCCGGTGGCCGGTGAGGTTATCCAGGGATTCGCTGCCGCTTTGAAGT
CTGGCCTGACTATTAACACGCTGATCAACACCGTGGGCATCCATCCCACT
ACCGCCGAAGAATTCACCCGGCTGGCCATCACCAAGCGCTCCGGACTGGA
CCCCACGCCGGCCAGCTGCTGCAGCTAAAGCGGAACGCAGCTCAGCCGC
CTGGGACGTGTCGAAGCCGCTTGCTCCACCCGAAATCCCGTAGATGAATG
GTTGTTGTCGCGGCCCAGCGATCGATGAGTTCAATAGTTCCGTTTCGTTT
CCACAATTAACACCCAACACAATAGCTCTGCGCAAGGGAGGGGCACTGGG
 
CAGCGATGGCGGGTGGAACGACACCAGTGGAACTACCCGCGCGACCAGCC
CAACCCACGACTGCTGCGCCGCCGACATGCACTCAAAATTTTGAATTTGT
TTGAACCTATGAAATTAACTATGAAATCCCCTAAATGTACGGTTGAAGAA
TATAATTTTTCACC
 
MSTKGGSYDYDLIVIGGGSAGLACAKEAVLNGARVACLDFVKPTPTLGTK
WGVGGTCVNVGCIPKKLMHQASLLGEAVHEAAAYGWNVDEKIKPDWHKLV
QSVQNHIKSVNWVTRVDLRDKKVEYINGLGSFVDSHTLLAKLKSGERTIT
AQTFVIAVGGRPRYPDIPGAVEYGITSDDLFSLDREPGKTLVVGAGYIGL
ECAGFLKGLGYEPTVMVRSIVLRGFDQQMAELVAASMEERGIPFLRKTVP
LSVEKQDDGKLLVKYKNVETGEEAEDVYDTVLWAIGRKGLVDDLNLPNAG
VTVQKDKIPVDSQEATNVANIYAVGDIIYGKPELTPVAVLAGRLLARRLY
GGSTQRMDYKDVATTVFTPLEYACVGLSEEDAVKQFGADEIEVFHGYYKP
TEFFIPQKSVRYCYLKAVAERHGDQRVYGLHYIGPVAGEVIQGFAAALKS
GLTINTLINTVGIHPTTAEEFTRLAITKRSGLDPTPASCCS
**Human homologue of Complete Genome candidate** (CG10965) - AAC50725 11-cis retinol dehydrogenase 
1 taagcttcgg gcgctgtagt acctgccagc tttcgccaca ggaggctgcc acctgtaggt
61 cacttgggct ccagctatgt ggctgcctct tctgctgggt gccttactct gggcagtgct
121 gtggttgctc agggaccggc agagcctgcc cgccagcaat gcctttgtct tcatcaccgg
181 ctgtgactca ggctttgggc gccttctggc actgcagctg gaccagagag gcttccgagt
241 cctggccagc tgcctgaccc cctccggggc cgaggacctg cagcgggtgg cctcctcccg
301 cctccacacc accctgttgg atatcactga tccccagagc gtccagcagg cagccaagtg
361 ggtggagatg cacgttaagg aagcagggct ttttggtctg gtgaataatg ctggtgtggc
421 tggtatcatc ggacccacac catggctgac ccgggacgat ttccagcggg tgctgaatgt
481 gaacacaatg ggtcccatcg gggtcaccct tgccctgctg cctctgctgc agcaagcccg
541 gggccgggtg atcaacatca ccagcgtcct gggtcgcctg gcagccaatg gtgggggcta
601 ctgtgtctcc aaatttggcc tggaggcctt ctctgacagc ctgaggcggg atgtagctca
661 ttttgggata cgagtctcca tcgtggagcc tggcttcttc cgaacccctg tgaccaacct
721 ggagagtctg gagaaaaccc tgcaggcctg ctgggcacgg ctgcctcctg ccacacaggc
781 ccactatggg ggggccttcc tcaccaagta cctgaaaatg caacagcgca tcatgaacct
841 gatctgtgac ccggacctaa ccaaggtgag ccgatgcctg gagcatgccc tgactgctcg
901 acacccccga acccgctaca gcccaggttg ggatgccaag ctgctctggc tgcctgcctc
961 ctacctgcca gccagcctgg tggatgctgt gctcacctgg gtccttccca agcctgccca
1021 agcagtctac tgaatccagc cttccagcaa gagattgttt ttcaaggaca aggactttga
1081 tttatttctg cccccaccct ggtactgcct ggtgcctgcc acaaaata
 
1 
61 
121 
181 
241 
301 
 (CG2151) - XP_033135 thioredoxin reductase beta 
1 ccggacctca ggcccagttc agtgtacttc ccctctctac ttcctccctc cagtcccttc
61 tccatccctc ccttttttgg ctgccccttg cctgccttcc tcgccagtag cttgcagagt
121 agacacgatg acaccttttg caggctaaaa aggctgagag tggcactatg tgcagtgagc
181 caccatggag gaccaagcag gtcagcggga ctatgatctc ctggtggtcg gcgggggatc
241 tggtggcctg gcttgtgcca aggaggccgc ccagctggga aggaaggtgg ccgtggtgga
301 ctacgtggaa ccttctcccc aaggcacccg gtggggcctc ggcggcacct gcgtcaacgt
361 gggctgcatc cccaagaagc tgatgcacca ggcggcactg ctgggaggcc tgatccaaga
421 tgcccccaac tatggctggg aggtggccca gcccgtgccg catgactgga ggaagatggc
481 agaagctgtt caaaatcacg tgaaatcctt gaactggggc caccgtgtcc agcttcagga
541 cagaaaagtc aagtacttta acatcaaagc cagctttgtt gacgagcaca cggtttgcgg
601 cgttgccaaa ggtgggaaag agattctgct gtcagccgat cacatcatca ttgctactgg
661 agggcggccg agatacccca cgcacatcga aggtgccttg gaatatggaa tcacaagtga
721 tgacatcttc tggctgaagg aatcccctgg aaaaacgttg gtggtcgggg ccagctatgt
781 ggccctggag tgtgctggct tcctcaccgg gattgggctg gacaccacca tcatgatgcg
841 cagcatcccc ctccgcggct tcgaccagca aatgtcctcc atggtcatag agcacatggc
901 atctcatggc acccggttcc tgaggggctg tgccccctcg cgggtcagga ggctccctga
961 tggccagctg caggtcacct gggaggacag caccaccggc aaggaggaca cgggcacctt
1021 tgacaccgtc ctgtgggcca taggtcgagt cccagacacc agaagtctga atttggagaa
1081 ggctggggta gatactagcc ccgacactca gaagatcctg gtggactccc gggaagccac
1141 ctctgtgccc cacatctacg ccattggtga cgtggtggag gggcggcctg agctgacacc
1201 catagcgatc atggccggga ggctcctggt gcagcggctc ttcggcgggt cctcagatct
1261 gatggactac gacaatgttc ccacgaccgt cttcaccccg ctggagtatg gctgtgtggg
1321 gctgtccgag gaggaggcag tggctcgcca cgggcaggag catgttgagg tctatcacgc
1381 ccattataaa ccactggagt tcacggtggc tggacgagat gcatcccagt gttatgtaaa
1441 gatggtgtgc ctgagggagc ccccacagct ggtgctgggc ctgcatttcc ttggccccaa
1501 cgcaggcgaa gttactcaag gatttgctct ggggatcaag tgtggggctt cctatgcgca
1561 ggtgatgcgg accgtgggta tccatcccac atgctctgag gaggtagtca agctgcgcat
1621 ctccaagcgc tcaggcctgg accccacggt gacaggctgc tgagggtaag cgccatccct
1681 gcaggccagg gcacacggtg cgcccgccgc cagctcctcg gaggccagac ccaggatggc
1741 tgcaggccag gtttgggggg cctcaaccct ctcctggagc gcctgtgaga tggtcagcgt
1801 ggagcgcaag tgctggacag gtggcccgtg tgccccacag ggatggctca ggggactgtc
1861 cacctcaccc ctgcacctct cagcctctgc cgccgggcac ccccccccag gctcctggtg
1921 ccagatgatg acgacctggg tggaaaccta ccctgtgggc acccatgtcc gagcccctg
1981 gcatttctgc aatgcaaata aagagggtac tttttctgaa gtgtg
 
1 medqagqrdy dllvvgggsg glacakeaaq lgrkvavvdy vepspqgtrw glggtcvnvg
61 cipkklmhqa allggliqda pnygwevaqp vphdwrkmae avqnhvksln wghrvqlqdr
121 kvkyfnikas fvdehtvcgv akggkeills adhiiiatgg rprypthieg aleygitsdd
181 ifwlkespgk tlvvgasyva lecagfltgi gldttimmrs iplrgfdqqm ssmviehmas
241 hgtrflrgca psrvrrlpdg qlqvtwedst tgkedtgtfd tvlwaigrvp dtrslnleka
301 gvdtspdtqk ilvdsreats vphiyaigdv vegrpeltpi aimagrllvq rlfggssdlm
361 dydnvpttvf tpleygcvgl seeeavarhg qehvevyhah ykpleftvag rdasqcyvkm
421 vclreppqlv lglhflgpna gevtqgfalg ikcgasyaqv mrtvgihptc seevvklris
481 krsgldptvt gcxg
**Putative function** (CG10964) - unknown, similarity to dehydrogenases (CG2151) - thioredoxin reductase

### Example 16 (Category 3)

**Line ID** - 418
**Phenotype** - Lethal phase embryonic larval phase3-pre-pupal-pupal. High mitotic index, dot-like chromosomes, strong metaphase arrest
**Annotated *Drosophila* genome genomic segment containing P element insertion site (and map position) -** AE003431 (4C11-16)
**P element insertion site** - 289,752
**Annotated *Drosophila* genome Complete Genome candidate** CG3000- rap, fizzy related 
CTTTGGCTTGTTTGCTTGAAAAAACGTAACTTTTTTGTTGTAATGAAGG
AAGCAGCACGGGCAGTAGACCAACTCGAAATCGCGCATTGCCAACACGTA
ACGTACCAGCCCGTGTAATAACAGAAGAAACCCCGAGCCGCAACAACAAC
CCCCGAAAAGCGGTAGTTGTAAGAGTTTCCCAAAGTGGCAGCGGCAATT
ACACGGCGAGAAACGAGTTCGCGTCGCGTCCAGCTGTTTGAAAATCAAAA
TTAACCGTTTTTAGCGCGTGAAACAAGACGTTTAGAACCGTGTTCAAAAT
CCCTCGTACATAAATTGTGTGTACATTTATATATATATATATTTTCTACG
CCACGTTAACCAGACTTTTTAAGTTTTAAATTAAAACTAAAGACGTATTA
TTTTTTTTTTTTTGAGTGTTTATATTTTTTTTTTGCAAGTTTTGTTTGG
TTACATTTGAGTTTGTGTTGAGTTTTGCCAGCCAAAGGCGCTTAAGATG
TTTAGTCCCGAGTACGAGAAGCGCATCCTGAAGCACTACAGTCCTGTGGC
ACGGAATCTGTTCAACAACTTCGAGTCGTCCACTACGCCCACATCTCTCG
ACCGCTTCATACCCTGCAGAGCGTACAACAACTGGCAGACGAACTTTGCG
TCAATCAACAAGTCCAATGACAACTCGCCGCAGACGAGTAAGAAGCAGCG
GGACTGCGGGGAAACGGCACGCGATAGTCTCGCCTACTCCTGCCTACTGA
AGAACGAGCTCCTCGGATCGGCAATCGACGACGTGAAGACCGCCGGCGAG
GAGCGGAATGAGAATGCCTACACGCCGGCCGCAAAGCGGAGTCTCTTCAA
GTACCAGTCACCCACCAAGCAGGACTACAATGGCGAGTGTCCGTACTCGT
TGTCACCCGTCAGCGCCAAAAGTCAGAAGCTGTTGCGATCGCCGCGCAAG
GCTACGCGCAAAATCTCTCGCATTCCCTTCAAGGTGCTAGACGCGCCCGA
GTTGCAGGACGACTTCTATCTGAACCTGGTCGACTGGTCGTCGCAGAACG
TACTGGCTGTAGGCCTGGGCAGCTGTGTCTATCTGTGGAGCGCGTGCACC
AGTCAGGTTACCCGCCTGTGTGATCTCAGTCCGGATGCGAATACGGTGAC
CTCGGTGTCGTGGAACGAGCGTGGCAACACCGTGGCCGTGGGCACACATC
ACGGCTACGTGACCGTCTGGGATGTGGCGGCCAATAAGCAGATCAACAAA
CTGAATGGCCATTCGGCGCGTGTGGGCGCCTTGGCATGGAACAGTGACAT
CCTGTCGAGCGGGTCGCGAGACCGTTGGATCATACAGCGGGATACGAGAA
CGCCGCAACTGCAATCGGAGCGCAGATTGGCCGGACATCGGCAGGAGGTG
TGCGGACTGAAATGGTCACCGGATAATCAATACTTGGCCAGTGGCGGCAA
CGATAATCGGTTGTATGTGTGGAATCAGCATTCCGTGAATCCCGTACAAT
CATACACGGAGCATATGGCGGCTGTAAAGGCGATCGCGTGGTCGCCGCAT
CACCACGGACTCCTGGCCAGCGGCGGTGGAACGGCGGATAGGTGTATCCG
TTTCTGGAATACGCTGACGGGCCAGCCCATGCAGTGCGTGGACACGGGCT
CGCAGGTTTGCAATCTGGCCTGGTCCAAGCACTCCTCGGAGCTGGTCTCC
ACGCACGGCTACTCGCAGAACCAGATACTCGTGTGGAAATATCCCTCCCT
 
GACGCAAGTGGCCAAGCTGACGGGCCATTCGTATCGTGTGCTCTATCTGG
CGCTGAGTCCCGATGGTGAGGCTATTGTTACGGCGCCGGCGACGAGACG
CTGCGATTTTGGAACGTATTCAGCAAGGCGCGCAGTCAGAAGGAGAACAA
GTCCGTTCTGAATCTGTTTGCCAATATCAGATAAGGACAATAACTCCAAG
CGAGCGAAGACTGAGCGAGCGCCAAAGGCAAACACAACACAACACAAAAC
AAAACAAAACAAAGCAAAGTATAATATAAATAAAATGGATACTTGAAACC
GAAAAACAAAGCCAACCAACCAATCAGCAAAAACCAAGCTGAAGCTAACA
AACTAATCGAGCCTATATGCTATATATATACAAACGATTCTTGTTCAGCA
GTCGTTTTGTAAATTGTTGTGTGACCCCACAGCAGCAATAGATTAAATAA
ATTTAAGTTAAGCAATCTGTATAGAACGGTAATTAGCAACATTTACGTAG
GTAAACACATGCAATTTATGAAGGAATAACATCAAGAGAGATGGCTGAAA
CAAGAACTGAAAATGAAACTAAGTCTATGGAAATTGTAAGTAATTGGAAA
ATCAACAACACCACACTCACACACTATCTTTAATCGACATTTTTTGTTGC
TGCTTTTTTAAATGTATTGTTTTTTTTTTGTGGTACACCTACACTACACC
TAAGAAAATTGGATACCCCTACATATACATTTATACGTTTATATATATAT
ATTTTTTTGCTAGCCTCTAAGTAACTAACTTTATTTCAAGCAAACATTTA
TACACATATTTCGCTCACTAGAAACACTCATACCCCCGAAAACACAATGT
ATATTAAATAAACTTATACAATTTCAAAATGTGCCCCAAAAAGTA
 
MFSPEYEKRILKHYSPVARNLFNNFESSTTPTSLDRFIPCRAYNNWQTNF
ASINKSNDNSPQTSKKQRDCGETARDSLAYSCLLKNELLGSAIDDVKTAG
EERNENAYTPAAKRSLFKYQSPTKQDYNGECPYSLSPVSAKSQKLLRSPR
KATRKISRIPFKVLDAPELQDDFYLNLVDWSSQNVLAVGLGSCVYLWSAC
TSQVTRLCDLSPDANTVTSVSWNERGNTVAVGTHHGYVTVWDVAANKQIN
KLNGHSARVGALAWNSDILSSGSRDRWIIQRDTRTPQLQSERRLAGHRQE
VCGLKWSPDNQYLASGGNDNRLYVWNQHSVNPVQSYTEHMAAVKAIAWSP
HHHGLLASGGGTADRCIRFWNTLTGQPMQCVDTGSQVCNLAWSKHSSELV
STHGYSQNQILVWKYPSLTQVAKLTGHSYRVLYLALSPDGEAIVTGAGDE
TLRFWNVFSKARSQKENKSVLNLFANIR
**Human homologue of Complete Genome candidate** XP_009259 Fzr1 protein 
1 ggccgcggcc gggcctgcgg gagctgcgga ggccggaggc gggcgctgtg cggtgccagg
61 agaggcgggg tcggcgggag ccagcgagcc acgggagcga gccaggctaa ccttgccgcg
121 ggccgagccc tgcctcgcca tggaccagga ctatgagcgg cgcctgcttc gccagatcgt
181 catccagaat gagaacacga tgccacgcgt cacagagatg cggcggaccc tgacgcctgc
241 cagctcccca gtgtcctcgc ccagcaagca cggagaccgc ttcatcccct ccagagccgg
301 agccaactgg agcgtgaact tccacaggat taacgagaat gagaagtctc ccagtcagaa
361 ccggaaagcc aaggacgcca cctcagacaa cggcaaagac ggcctggcct actctgccct
421 gctcaagaat gagctgctgg gtgccggcat cgagaaggtg caggacccgc agactgagga
481 ccgcaggctg cagccctcca cgcctgagaa gaagggtctg ttcacgtatt cccttagcac
541 caagcgctcc agccccgatg acggcaacga tgtgtctccc tactccctgt ctcccgtcag
601 caacaagagc cagaagctgc tccggtcccc ccggaaaccc acccgcaaga tctccaagat
661 ccccttcaag gtgctggacg cgcccgagct gcaggacgac ttctacctca atctggtgga
721 ctggtcgtcc ctcaatgtgc tcagcgtggg gctaggcacc tgcgtgtacc tgtggagtgc
781 ctgtaccagc caggtgacgc ggctctgtga cctctcagtg gaaggggact cagtgacctc
 
841 cgtgggctgg tctgagcggg ggaacctggt ggcggtgggc acacacaagg gcttcgtgca
901 gatctgggac gcagccgcag ggaagaagct gtccatgttg gagggccaca cggcacgcgt
961 cggggcgctg gcctggaatg ctgagcagct gtcgtccggg agccgcgacc gcatgatcct
1021 gcagagggac atccgcaccc cgccactgca gtcggagcgg cggctgcagg gccaccggca
1081 ggaggtgtgc gggctcaagt ggtccacaga ccaccagctc ctcgcctcgg ggggcaacga
1141 caacaagctg ctggtctgga atcactcgag cctgagcccc gtgcagcagt acacggagca
1201 cctggcggcc gtgaaggcca tcgcctggtc cccacatcag cacgggctgc tggcctcggg
1261 gggcggcaca gctgaccgct gtatccgctt ctggaacacg ctgacaggac aaccactgca
1321 gtgtatcgac acgggctccc aagtgtgcaa tctggcctgg tccaagcacg ccaacgagct
1381 ggtgagcacg cacggctact cacagaacca gatccttgtc tggaagtacc cctccctgac
1441 ccaggtggcc aagctgaccg ggcactccta ccgcgtgctg tacctggcaa tgtcccctga
1501 tggggaggcc atcgtcactg gtgctggaga cgagaccctg aggttctgga acgtctttag
1561 caaaacccgt tcgacaaagg agtctgtgtc tgtgctcaac ctcttcacca ggatccggta
1621 aacctgccgg gcaggaccgt gccacaccag ctgtccagag tcggaggacc ccagctcctc
1681 agcttgcatg gactctgcct tcccagcgct tgtcccccga ggaaggcggc tgggcgggcg
1741 gggagctggg cctggaggat cctggagtct cattaaatgc ctgattgtga accatgtcca
1801 ccagtatctg gggtgggcac gtggtcgggg accctcagca gcaggggctc tgtctccctt
1861 cccaaagggc gagaaccaca ttggacggtc ccggctcaga ccgtctgtac tcagagcgac
1921 ggatgccccc tgggaccctc actgcctccg tctgttcatc acctgcccac cggagccgca
1981 tgctcttcct ggaactgccc acgtctgcac agaacagacc accagacgcc agggctgatt
2041 ggtgggggcc tgagaccccg gttgcccatt catggctgca ccccaccatg tcaaacccaa
2101 gaccagcccc aaggccagac caaggcatgt aggcctgggc aggtggctcg gggccactgg
2161 cggagccagc ctgtggatcc aagagacagt ccccacctgg gcttcacggc atccttgcag
2221 ccacctctgc tgtcactgct cgaagcagca gtctctctgg aagcatctgt gtcatggcca
2281 tcgcccggcg gtcagtgggc ttcagatggg cctgtgcatc ctggccaagc gtcaccctca
2341 cactggagga ggatgtctgc tctggactta tcaccccagg agaactgaac ccggacctgc
2401 tcactgccct ggctggagag gagcacaaca gatgccacgt cttcgtgcat tcgccaacac
2461 gtgccctcac agggccagcg tcctccttcc ctgcgcaaga cttgcgtccc ccatgcctgc
2521 tgggtggctg ggtcctgtgg aggccagcag cggtgtggcc cccgccccca ggctgcctgt
2581 gtcttcacct gtcctgtcca ccagcgccaa cagccgtggg gaagccaagg agacccaagg
2641 ggtccaggag gtgggcgccc tccatccttc gagaagcttc ccaggctcct ctgcttctct
2701 gtctcatgct cccaggctgc acagcaggca gggagggagg caaggcaggg gagtggggcc
2761 tgagctgagc actgccccct caccccccca ccaccccttc ccatttcatc ggtggggacg
2821 tggagagggt ggggcgggct ggggttggag ggtcccaccc accaccctgc tgtgcttggg
2881 aacccccact ccccactccc cacatcccaa catcctggtg tctgtcccca gtggggttgg
2941 cgtgcatgtg tacatatgta tttgtgactt ttctttgg
 
1 mdqdyerrll rqiviqnent mprvtemrrt ltpasspvss pskhgdrfip sraganwsvn
61 fhrineneks psqnrkakda tsdngkdgla ysallknell gagiekvqdp qtedrrlqps
121 tpekkglfty slstkrsspd dgndvspysl spvsnksqkl lrsprkptrk iskipfkvld
181 apelqddfyl nlvdwsslnv lsvglgtcvy lwsactsqvt rlcdlsvegd svtsvgwser
241 gnlvavgthk gfvqiwdaaa gkklsmlegh tarvgalawn aeqlssgsrd rmilqrdirt
301 pplqserrlq ghrqevcglk wstdhqllas ggndnkllvw nhsslspvqq ytehlaavka
361 iawsphqhgl lasgggtadr cirfwntltg qplqcidtgs qvcnlawskh anelvsthgy
421 sqnqilvwky psltqvaklt ghsyrvlyla mspdgeaivt gagdetlrfw nvfsktrstk
481 esvsvlnlft rir
**Putative function** Cell cycle regulator involved in cyclin degradation

### Example 17 (Category 3)

**Line ID** - 121
**Phenotype** - Lethal phase larval phase 3 - prepupal - pupal - pharate adult-adult. High mitotic index, dot and rod-like overcondensed chromosomes, high frequency of polyploids
**Annotated *Drosophila* genome genomic segment containing P element insertion site (and map position)** - AE003493 (12B7)
**P element insertion site** - not determined
**Annotated *Drosophila* genome Complete Genome candidate** CG10988 -1(1)dd4 gamma tubulin ring complex 
TAACACTGCACTAAATAATTTAATAAATTATTGTATGAAGTACGCGCC
AATTGGATGCGTTTTGTCCTATCTGTCGAAGATTTCACGCATCCCGAAC
AATTGCCAGTGACTGCACGCCGTATTATAGCCAGGGAACAGCTGTGCGTT
TGCCATTGGCCAACAGTTGTTGTCCACTTCGCAATTACCAAGCCATCCAA
AATCGGCTGTTTAACGCGCGCTTGATTGGATATTTATGAACAATTCAGTG
CACCAGGATGTCGCAGGACAGGATCGCCGGCATCGATGTGGCAACCAATT
CCACTGATATATCGAATATCATTAACGAGATGATCATCTGCATCAAGGGC
AAGCAGATGCCCGAAGTTCACGAAAAAGCAATGGATCATTTAAGCAAAAT
GATTGCCGCCAATAGTCGGGTCATTCGGGACTCAAATATGTTGACTGAGC
GCGAATGTGTCCAGAAGATAATGAAACTGCTGAGCGCCCGGAATAAGAAG
GAGGAGGGCAAAACTGTGTCGGATCACTTCAATGAGCTGTACAGGAAACT
CACGTTGACCAAGTGCGATCCGCACATGAGGCACTCGCTAATGACCCATC
TACTTACGATGACCGACAATTCGGATGCCGAAAAGGCAGTTGCCAGCGAA
GATCCACGTACTCAGTGCGATAATCTCACTCAGATTCTGGTCAGTCGTCT
TAACTCAATAAGTTCCTCCATAGCCAGTCTGAATGAGATGGGAGTGGTCA
ACGGAAATGGAGTAGGAGCAGCAGCGGTAACAGGAGCAGCAGCGGTAACA
GGAGCAGCAGCGGTAACAGGAGCAGCAGCGGTAACAGGAGCAGCAGCAAG
CCACAGTTATGATGCCACACAGTCCAGCATCGGATTGAGAAAACAGTCCT
TGCCCAACTACCTGGATGCAACAAAGATGTTGCCCGAGTCTCGACATGAT
ATAGTGATGAGTGCCATTTACTCCTTCACCGGCGTTCAAGGGAAGTATTT
GAAGAAGGATGTGGTAACGGGCCGTTTCAAGCTGGATCAGCAGAACATCA
AGTTCCTGACCACCGGCCAAGCGGGCATGTTGCTGCGGCTCTCCGAACTT
GGCTACTACCACGATCGAGTGGTCAAGTTTTCGGATGTATCGACCGGTTT
CAATGCCATTGGCAGCATGGGCCAGGCCCTGATTTCCAAACTCAAGGAGG
AGCTGGCGAATTTTCACGGGCAAGTGGCAATGCTTCACGATGAAATGCAG
CGTTTTCGGCAGGCCTCGGTGAATGGAATTGCAAACAAGGGGAAAAAGGA
TAGTGGGCCCGATGCTGGCGATGAAATGACGCTATTCAAGCTGCTCGCCT
GGTATATAAAGCCACTGCACCGGATGCAGTGGTTAACCAAGATTGCCGAC
GCCTGCCAGGTAAAGAAGGGCGGTGATTTGGCATCGACCGTTTATGATTT
CCTTGACAACGGTAACGATATGGTCAATAAATTGGTGGAGGATCTCCTAA
CTGCCATTTGTGGCCCACTGGTGCGCATGATCTCCAAATGGATTCTGGAG
GGCGGCATTAGCGATATGCATAGAGAGTTCTTTGTGAAGTCCATTAAAGA
TGTGGGCGTTGATCGGCTATGGCACGATAAATTCCGCCTACGATTGCCAA
TGCTGCCCAAGTTTGTGCCCATGGATATGGCCAATAAGATACTCATGACG
 
GGCAAATCCATTAATTTCTAAGAGAAATCTGCGAGGAGCAGGGTATGAT
GAAGGAGCGCGACGAACTAATGAAGGTCATGGAATCTAGTGCCTCTCAAA
TCTTTTCGTACACACCGGACACCAGTTGGCATGCGGCCGTGGAAACGTGC
TACCAGCAGACCTCCAAACATGTCCTCGACATTATGGTGGGCCCACACAA
GCTGCTGGATCATTTGCACGGAATGCGGCGCTACTTGCTGTTGGGCCAGG
GCGATTTTATTAGCATTCTGATTGAAAACATGAAGAACGAACTGGAGCGA
CCGGGCCTTGATATATATGCTAACGATCTCACCTCCATGTTGGATTCCGC
TCTGCGCTGTACGAATGCCCAGTACGATGATCCTGATATTCTAAACCATC
TCGATGTGATTGTTCAACGACCGTTCAACGGTGATATTGGCTGGAACATC
ATCTCGCTGCAGTACATTGTCCACGGACCACTGGCCGCCATGCTGGAGTC
GACCATGCCAACGTACAAGGTGCTCTTCAAGCCACTCTGGCGCATGAAGC
ACATGGAGTTTGTGCTCTCGATGAAGATCTGGAAGGAGCAGATGGGCAAC
GCAAAGGCCCTTCGTACAATGAAGTCCGAAATCGGCAAGGCGTCACACCG
CCTCAACCTTTTCACTTCCGAGATCATGCACTTTATCCACCAAATGCAGT
ACTATGTGCTATTTGAGGTCATCGAGTGCAACTGGGTGGAGCTACAGAAG
AAGATGCAGAAGGCTACTACGTTGGACGAAATCCTGGAAGCTCACGAGAA
GTTTCTGCAAACGATTTTGGTGGGCTGTTTTGTCAGCAACAAAGCGAGTG
TGGAGCATTCGCTGGAGGTGGTGTACGAGAACATTATCGAATTGGAGAAG
TGGCAGTCGAGCTTTTACAAGGACTGCTTTAAGGAGCTAAATGCCCGCAA
GGAACTGTCCAAAATTGTGGAGAAATCGGAAAAGAAGGGTGTCTACGGAC
TGACCAACAAGATGATCCTGCAGCGCGACCAGGAGGCGAAGATATTTGCC
GAAAAGATGGACATCGCCTGCCGCGGCTTAGAAGTCATAGCAACCGATTA
CGAAAAGGCTGTCAGCACTTTCCTAATGTCTCTCAACTCTAGCGACGATC
CGAATTTGCAGCTCTTTGGCACTCGGCTGGACTTCAACGAGTACTACAAG
AAGAGGGACACCAATTTGAGCAAACCCCTGACCTTCGAGCACATGCGCAT
GAGCAATGTGTTCGCCGTGAACAGTCGCTTCGTGATATGTACGCCGTCCA
CTCAGGAATAGCGACCAATGTCCATGCAATCGGTTTATCCCAGTGTCCAT
ACATCATACCAAATCCCAAATCCCATACAGCATCAGCACTCCATTCAGTT
CAATTGCTGCTAAATATTTGAGATATCTCGATATCATTGGAGCCAATCCA
ACCAAACAAACTAATCCAATTATTAACTAAGCCTTCGAATCGAAAACAAC
CTCTATACATATATATCTCAAGCTTTGCCGTCAATCGCCTGGCTGCAAGC
CATCAACTTAAGATATCTCCAATACAAAATTATTGAGTAGTTGTAACGAA
AGTATTAAGCGACAATTTGTTTGTCGAAAAACGCAACGTTCTATTTTGTT
TGCGAATCCCATAATTTTTTTACATCGAAGCTTAGTTGAAATAGATTTT
CGTAAGTGCATTTGCCAATTGCCATGTTGTAATTAAAGAGAATAAGAGAA
TGTTACGTACTTTAAAAGAATGTTTTAAAAAGTTAATGTTTTGAACAGT
TTTAAACCGTAATGCGAG
 
MSQDRIAGIDVATNSTDISNIINEMIICIKGKQMPEVHEKAMDHLSKMIA
ANSRVIRDSNMLTERECVQKIMKLLSARNKKEEGKTVSDHFNELYRKLTL
TKCDPHMRHSLMTHLLTMTDNSDAEKAVASEDPRTQCDNLTQILVSRLNS
ISSSIASLNEMGVVNGNGVGAAAVTGAAAVTGAAAVTGAAAVTGAAASHS
YDATQSSIGLRKQSLPNYLDATKMLPESRHDIVMSAIYSFTGVQGKYLKK
DVVTGRFKLDQQNIKFLTTGQAGMLLRLSELGYYHDRVVKFSDVSTGFNA
IGSMGQALISKLKEELANFHGQVAMLHDEMQRFRQASVNGIANKGKKDSG
PDAGDEMTLFKLLAWYIKPLHRMQWLTKIADACQVKKGGDLASTVYDFLD
NGNDMVNKLVEDLLTAICGPLVRMISKWILEGGISDMHREFFVKSIKDVG
VDRLWHDKFRLRLPMLPKFVPMDMANKILMTGKSINFLREICEEQGMMKE
 
RDELMKVMESSASQIFSYTPDTSWHAAVETCYQQTSKHVLDIMVGPHKLL
DHLHGMRRYLLLGQGDFISILIENMKNELERPGLDIYANDLTSMLDSALR
CTNAQYDDPDILNHLDVIVQRPFNGDIGWNIISLQYIVHGPLAAMLESTM
PTYKVLFKPLWRMKHMEFVLSMKIWKEQMGNAKALRTMKSEIGKASHRLN
LFTSEIMHFIHQMQYYVLFEVIECNWVELQKKMQKATTLDEILEAHEKFL
QTILVGCFVSNKASVEHSLEVVYENIIELEKWQSSFYKDCFKELNARKEL
SKIVEKSEKKGVYGLTNKMILQRDQEAKIFAEKMDIACRGLEVIATDYEK
AVSTFLMSLNSSDDPNLQLFGTRLDFNEYYKKRDTNLSKPLTFEHMRMSN
VFAVNSRFVICTPSTQE
**Human homologue of Complete Genome candidate** AAC39727 - spindle pole body protein spc98 homolog GCP3 
1 caggaagggc gcgggccgcg gtccctgcgc gtgcggcggc agtggcggct ctgcccggac
61 caccgtgcac ggctccgggc gaggatggcg accccggacc agaagtcgcc gaacgttctg
121 ctgcagaacc tgtgctgcag gatcctgggc aggagcgaag ctgatgtagc ccagcagttc
181 cagtatgctg tgcgggtgat tggcagcaac ttcgccccaa ctgttgaaag agatgaattt
241 ttagtagctg aaaaaatcaa gaaagagctt attcgacaac gaagagaagc agatgctgca
301 ttattttcag aactccacag aaaacttcat tcacagggag ttttgaaaaa taaatggtca
361 atactctacc tcttgctgag cctcagtgag gacccacgca ggcagccaag caaggtttct
421 agctatgcta cgttatttgc tcaggcctta ccaagagatg cccactcaac cccttactac
481 tatgccaggc ctcagaccct tcccctgagc taccaagatc ggagtgccca gtcagcccag
541 agctccggca gcgtgggcag cagtggcatc agcagcattg gcctgtgtgc cctcagtggc
601 cccgcgcctg cgccacaatc tctcctccca ggacagtcta atcaagctcc aggagtagga
661 gattgccttc gacagcagtt ggggtcacga ctcgcatgga ctttaactgc aaatcagcct
721 tcttcacaag ccactacctc aaaaggtgtc cccagtgctg tgtctcgcaa catgacaagg
781 tccaggagag aaggggatac gggtggtact atggaaatta cagaagcagc tctggtaagg
841 gacattttgt acgtctttca gggcatagat ggcaaaaaca tcaaaatgaa caacactgaa
901 aattgttaca aagtagaagg aaaggcaaat ctaagtaggt ctttgagaga cacagcagtc
961 aggctttctg agttgggatg gttgcataat aaaatcagaa gatacacgga ccagaggagc
1021 ctggaccgct cattcggact cgtcgggcag agcttttgtg ctgccttgca ccaggaactc
1081 agagaatact atcgattgct ctctgtttta cattctcagc tacaactaga ggatgaccag
1141 ggtgtgaatt tgggacttga gagtagttta acacttcggc gcctcctggt ttggacctat
1201 gatcccaaaa tacgactgaa gaccttgcgg gccctagtgg accactgcca aggaaggaaa
1261 ggaggtgagc tggcctcagc tgtccacgcc tacacaaaaa caggagaccc gtacatgcgg
1321 tctctggtgc agcacatcct cagcctcgtg tctcatcctg ttttgagctt cctgtaccgc
1381 tggatatatg atggggagct tgaggacact taccacgaat tttttgtagc atcagatcca
1441 acagttaaaa cagatcgact gtggcacgac aagtatactt tgaggaaatc gatgattcct
1501 tcgtttatga cgatggatca gtctaggaag gtccttttga taggaaaatc aataaatttc
1561 ttgcaccaag tttgtcatga tcagactccc actacaaaga tgatagctgt gaccaagtct
1621 gcagagtcac cccaggacgc tgcagaccta ttcacagact tggaaaatgc atttcagggg
1681 aagattgatg ctgcttattt tgagaccagc aaatacctgt tggatgttct caataaaaag
1741 tacagcttgc tggaccacat gcaggcaatg aggcggtacc tgcttcttgg tcaaggagac
1801 tttataaggc acttaatgga cttgctaaaa ccagaacttg tccgtccagc tacgactttg
1861 tatcagcata acttgactgg aattctagaa accgctgtca gagccaccaa cgcacagttt
1921 gacagtcctg agatcctgcg aaggctggac gtgcggctgc tggaggtctc tccaggtgac
 
1981 actggatggg atgtcttcag cctcgattat catgttgacg gaccaattgc aactgtgttt
2041 actcgagaat gtatgagcca ctacctaaga gtatttaact tcctctggag ggcgaagcgg
2101 atggaataca tcctcactga catacggaag ggacacatgt gcaatgcaaa gctcctgaga
2161 aacatgccag agttctccgg ggtgctgcac cagtgtcaca ttttggcctc tgagatggtc
2221 catttcattc atcagatgca gtattacatc acatttgagg tgcttgaatg ttcttgggat
2281 gagctttgga acaaagtcca gcaggcccag gatttggatc acatcattgc tgcacacgag
2341 gtgttcttag acaccatcat ctcccgctgc ctgctggaca gtgactccag ggcactttta
2401 aatcaactta gagctgtgtt tgatcaaatt attgaacttc agaatgctca agatgcaata
2461 tacagagctg ctctggaaga attgcagaga cgattacagt ttgaagagaa aaagaaacag
2521 cgtgaaattg agggccagtg gggagtgacg gcagcagagg aagaggagga aaataagagg
2581 attggagaat ttaaagaatc tataccaaaa atgtgctcac agttgcgaat attgacccat
2641 ttctaccagg gtatcgtgca gcagtttttg gtgttactga cgaccagctc tgacgagagt
2701 cttcggtttc ttagcttcag gctggacttc aacgagcatt acaaagccag ggagcccagg
2761 ctccgtgtgt ctctgggtac cagggggcgg cgcagctccc acacgtgaag ctcgcggtcc
2821 tcccagggag ctgcgggtga tgttcgttgc actgctagac acgaaattcc cattgacgtc
2881 ctgcaggaac tgcatgctgc aggtgtcctg cccttccgcc cacgagtgcg ccatgtttca
2941 gcggagcggc gtgtgggaga agccacgtcg tgtttcacat gtcggagtcg aatgcatttg
3001 taaatcccta agtcaagtag gctggctgca ctgttcacat ttgtctctaa aagtcttcat
3061 cgctaaaaga taccataatt tgctgaggct tcttaagctt tctatgttat aatttatatt
3121 tgtcacttta aaaaatccat ttcttttaga aaaaattagg gtgataggat attcattagt
3181 taagatggta acgtcattgc tattttttta acatcctctt tagaggtaat tttgttaac
3241 ataaccaaaa attaaattga aacaaaatgt cccaactaag aaaatatata gagcatttta
3301 ttttttttta gtgttgtaaa atattaacct ctgtgagatc ctttgtatct taatgcatta
3361 cctttacaca tatttattct tattttctct cctttcagag tttacatttt tatatttaat
3421 ttactatttc agatttttaa aatagtatag aaaaaagtag gagtgataga gaacaaaaat
3481 actcttatac agtgcaaccc aaataccgcg aatgcatcag ctaaagcagc gtgtaaatag
3541 gagtgatgag aaagttaatg gagtatttta ttttcaaagt tcctgataag cattggaaag
3601 aaatcgacat ggataatgaa gatttccttt ttccttgcct attttttcat tgtaaatatt
3661 tatatactac tgaccaagat gttggggtgg gggggattgt tttttgtaaa aatgtcatta
3721 tcaggtcaca taaatctgcc tttatgttgc ataagtgaaa atttagaaaa ttaaaagcaa
3781 ttatctttca aaaaa
 
1 matpdqkspn vllqnlccri lgrseadvaq qfqyavrvig snfaptverd eflvaekikk
61 elirqrread aalfselhrk lhsqgvlknk wsilylllsl sedprrqpsk vssyatlfaq
121 alprdahstp yyyarpqtlp lsyqdrsaqs aqssgsvgss gissiglcal sgpapapqsl
181 lpgqsnqapg vgdclrqqlg srlawtltan qpssqattsk gvpsavsrnm trsrregdtg
241 gtmeiteaal vrdilyvfqg idgknikmnn tencykvegk anlsrslrdt avrlselgwl
301 hnkirrytdq rsldrsfglv gqsfcaalhq elreyyrlls vlhsqlqled dqgvnlgles
361 sltlrrllvw tydpkirlkt laalvdhcqg rkggelasav haytktgdpy mrslvqhils
421 lvshpvlsfl yrwiydgele dtyheffvas dptvktdrlw hdkytlrksm ipsfmtmdqs
481 rkvlligksi nflhqvchdq tpttkmiavt ksaespqdaa dlftdlenaf qgkidaayfe
541 tskylldvln kkyslldhmq amrrylllgq gdfirhlmdl lkpelvrpat tlyqhnltgi
601 letavratna qfdspeilrr ldvrllevsp gdtgwdvfsl dyhvdgpiat vftrecmshy
661 lrvfnflwra krmeyiltdi rkghmcnakl lrnmpefsgv lhqchilase mvhfihqmqy
721 yitfevlecs wdelwnkvqq aqdldhiiaa hevfldtiis rclldsdsra llnqlravfd
781 qiielqnaqd aiyraaleel qrrlqfeekk kqreiegqwg vtaaeeeeen krigefkesi
841 pkmcsqlril thfyqgivqq flvllttssd eslrflsfrl dfnehykare prlrvslgtr
901 grrssht
**Putative function** Component of the centrosome

### Example 18 (Category 3)

**Line ID** - 237
**Phenotype** - Lethal phase larval stage 3 (few pupae). High mitotic index, colchicine-type overcondensation of chromosomes, polyploid cells, 'mininuclei' formation
**Annotated *Drosophila* genome genomic segment containing P element insertion site (and map position) -** AE0086 (10C4-5)
**P element insertion site** - 182,487
**Annotated *Drosophila* genome Complete Genome candidate** 2 candidates: CG1558 - novel protein 
ATGGAGCCAGCCGAAAGTCCAGAAAAATTAATGAAATTCGTACGCCGCAG
TGACGTACTGGAATACGTGGGCAACACGAGTGCCGTCGATCTATCGAGCG
GTGATCTCTCCGACATCGATCTCAAGGACGTGCCGGCCCAACTGGAGGCC
ACTTTGAAACCGCGTCGCTATGAAGCAAGCACTTTGTTTAACATTGACCT
GGACGATATCTGGGATCCTAGCTGTCAGGAGGACGAGGTGCAGCAGTACA
AGGAGCGCGCCCAGAAGGAGCAGCAAAAGTTCTTCGACTTTGTAATGCAT
GCGGCACTGGACACGGACAATCGCAAGGTTAGCTTCAAGCCAAACAAGGA
GCAGCAGCGTTACCTAGATCAGGGACCCAATTTGCAAAACTTCGTGCGAA
GCTCGTTGGCTTTCACAAACGCGGCCATCCGATTTCAGGCGGAGCACGAG
GACATGATGGAGCTGCAGTGCAATATGGACGATCACTACCTATTCATGCG
GAACACCATGATCAACAACGCTATACACCAGAATATGGCCAACCAACGGT
GACCCTAAGCTATGCATAAATATACATATGTGAATTGTAGATATTGATAA
ATTAAATTAAGACTCAGAGATTGTAAGACGGTTTGCTTTTGGCTTATACA
GTATAATTCGCTTAGCTGCCTCGAGTACTTTGCACAATGCCTCGATGCAG
GTAACTTAAAAATGCAGCTAACTTAATTTTTTTTTTCTATTTCTATTT
TCTATTCACAC
 
MEPAESPEKLMKFVRRSDVLEYVGNTSAVDLSSGDLSDIDLKDVPAQLEA
TLKPRRYEASTLFNIDLDDIWDPSCQEDEVQQYKERAQKEQQKFFDFVMH
AALDTDNRKVSFKPNKEQQRYLDQGPNLQNFVRSSLAFTNAAIRFQAEHE
DMMELQCNMDDHYLFMRNTMINNAIHQNMANQR
 CG11697 - novel protein 
ATGATTTATGCGATCGTGATACACATACTGTCCCTTCTGGTGGGCTGTTT
CTATCCAGCATTCGCGTCCTACAAGATCCTGAAAAGTCAGAATTGTAGCG
TCAATGATCTTCGCGGATGGTTAATCTACTGGATTGCCTATGGAGTTTAT
GTGGCCTTTGATTATTTCACAGCGGGTCTGCTGGCATTTATTCCATTGCT
AAGTGAGTTCAAGGTGCTTCTCCTGTTCTGGATGTTGCCCTCTGTGGGCG
GCGGCAGTGAGGTGATCTACGAGGAGTTCCTGCGATCCTTTAGCTGTAAC
GAATCCTTCGACCAGGTCCTGGGACGTATCACCTTGGAATGGGGCGAATT
GGTGTGGCAACAAGTTTGCTCCGTTCTTAGCCATTTGATGGTTTTGGCAG
 
ATCGCTATCTCCTGCCCAGCGGTCATCGTCCTGCCCTCCAAATAACGCCC
AGCATCGAGGATCTGGTCAACGATGCCATAGCCAAAAGGCAGTTGGAAGA
GAAGCGGAAACAGATGGGTAACTTATCTGATACCATCAACGAGGTTTTGG
GAGAAAATATCGATTTAAATATGGATCTGCTGCACGGATCCGAATCTGAT
TTATTGGTTATTAAGGAGCCTATTTCCAAGCCCAAGGAGAGACCAATACC
GCCGCCGAAGCCAATGCGTCAGCCATCATCAAGCAACCAGCAAGAAATGA
ATCTTTCGTCGCAGTTTATGTGA
 
MIYAIVIHILSLLVGCFYPAFASYKILKSQNCSVNDLRGWLIYWIAYGVY
VAFDYFTAGLLAFIPLLSEFKVLLLFWMLPSVGGGSEVIYEEFLRSFSCN
ESFDQVLGRITLEWGELVWQQVCSVLSHLMVLADRYLLPSGHRPALQITP
SIEDLVNDAIAKRQLEEKRKQMGNLSDTINEVLGENIDLNMDLLHGSESD
LLVIKEPISKPKERPIPPPKPMRQPSSSNQQEMNLSSQFM
**Human homologue of Complete Genome candidate** (CG1558) - none (CG11697) - BAB 14444 unamed protein - similar to a hypothetical protein in the region deleted in human familial adenomatous polyposis 1 
1 aacgccgggc agggcggcgg gcgcgctcag tctggcggcg gctgccgtga gctgactgac
61 gttccgggaa cgccgcagca gcccgcgccg cccgcagcct agccgagccg cgccgcccgg
121 gcctcgcccg cccgcctgcc cgccatggtg tcatggatca tctccaggct ggtggtgctt
181 atatttggca ccctttaccc tgcgtattat tcctacaagg ctgtgaaatc aaaggacatt
241 aaggaatatg tcaaatggat gatgtactgg attatatttg cacttttcac cacagcagag
301 acattcacag acatcttcct ttgttggttt ccattctatt atgaactaaa aatagcattt
361 gtagcctggc tgctgtctcc ctacacaaaa ggctccagcc tcctgtacag gaagtttgta
421 catcccacac tatcttcaaa agaaaaggaa atcgatgatt gtctggtcca agcaaaagac
481 cgaagttacg atgcccttgt gcacttcggg aagcggggct tgaacgtggc cgccacagcg
541 gctgtgatgg ctgcttccaa gggacagggt gccttatcgg agagactgcg gagcttcagc
601 atgcaggacc tcaccaccat caggggagac ggcgcccctg ctccctcggg ccccccacca
661 ccggggtctg ggcgggccag cggcaaacac ggccagccta agatgtccag gagtgcttct
721 gagagcgcta gcagctcagg caccgcctag aatccttcga tctcgcttca ggaagaaaag
781 tacctcatcc tcggccaccg aaaccacgtg agtgagatga gccaacagca ccggatccac
841 agaatgtttc ttctctgcct taaagagcta ttcactaata acatagaaat ccgcaagctg
901 ggtgtgcttt gagtgtgcag cctcacaaac atggcctttt ctctctcccc ttccactttt
961 aaggatttat tttttcccc cttttcttta tttgctggg gagaggctaa agggaaaggt
1021 agtaggggcg ggggtggtga cctttaagtc ttctgaggtt ggtaattttc cacaattgga
1081 ttgtcattat agacagcagt gtgtttttta gaaagataag agaatcaccc ctatgctgct
1141 gagatgtaca tttgtaattt atctgttgca tacttagttt ttagtcctgt aaatgcaaac
1201 acagcatttt ttacaacttt ctttgttctt ggtacttata ctttgaacta tgatgtacat
1261 atttatggct tttggctttt aatataatgg acttgcaagg gctgccagag gttctgatat
1321 gtaagaaaac tgcaaaaaca aatatagaca aatattttga ttctagagaa cgtctcagat
1381 gtgcttataa agcttccaaa tacaactcca gtaagacatc cctttccctg caggagtgtg
1441 gtctatattc tttagatagt tgtttagtca aaagaccaga caagttacaa actaagagaa
1501 acaatatttc acaacacagt aaagtgtgat gagaggtcag gggaacatcc cagtaaaaga
1561 gaagagtcac aggaagctca tctcctccct ggattctgga ttaggagctt ctgaatcttt
 
1621 tccagggata ggcaggtagc tcactcttgg tgcaatttct tgaggatggg aacatgtaga
1681 gctgctggaa ggagtaattc tgtgcttgac aaaggacgat ttctccttta tcgtgaccag
1741 tgctgccgat ttcctgacag aggagcttac actctgagca ccttgtttta gcgaactcta
1801 gcaaaacttg tttagcttag caaaaacaaa cacacaaaaa actgagaact ctgctgtttc
1861 agatatgcca taacatacat ctgaaacaca tgtgtaacaa tcaaaatggt gggctctaga
1921 atggttttgg agctcgagat cttcatgggt tagacttgct ggtcagaccc aggagcacct
1981 gtggctcaca ccttctgttc ccctcctggc ctgtgcagaa tgtaaacagc agactcatac
2041 tcaatgggca ctacaggcct tatcagacgt tttatacaag cctggattgc ttagtagggg
2101 aataaggcat tctctgaggg ggctttccac ttagattgag aattttattt gaaaagaatc
2161 tggtttaaat ggcattgtgg tccgaggtag ctgctctccc cactgagagc tgagccgaaa
2221 tataagaata atatatttgt gcttcgagtt ggtgtttctt tcagtgtaat gcatgcagtg
2281 gtcacaaccc agttactcat aatatttgga ttgtatttgt tcgtagatat gcccagaaga
2341 ctagagaatt agtgttatat accatataga acttactgtc agtcaactat aaacaggccc
2401 aattaaaaac tgttccatta ctacgcaaac acatattaga ggcctttgct gatgacacat
2461 tagctggatc ttagccaccc cagaaagggt ttgatttgaa gctgattgtt gccagatatg
2521 catattggaa tcccatctac ccatagttcc tctgaaggtg attttgtaat ttgcaaaagg
2581 gtataggaaa atatacctaa aagcgaattt gtggctgaga ggataaacag aagctgtttg
2641 ctcatgttct gtgccccaca cccaccaata cctaaatctg ttaaggaaga cagaaaatgt
2701 tttctttgtg ctcattgagt agttccagac agaagaagaa tatactcttt aaaatgtatt
2761 tacctgttag ttggaagtac ccagaattat cagaaacgaa tgcaaaaaaa aaaaaaaaaa
2821 aaaaaagctt acacagcttc ttagcaattt tttttttttt tgccgaaaca ataaattgcc
2881 tttagcagca gtttaaaatc ctatcgtgaa caacctatat tttcgccatt ttacaatgga
2941 gagttgtgac aagtacaggt tatcaagttt gcacttaact atgccaaaaa aagtttgaag
3001 cgctctattc tcagacatgc tgtattatta cttctcattc aagattgaaa aatataaagg
3061 tatccaaact ctgtcttaat gtaaatgtaa ctatttttcc ttcaagtgtt gactagggag
3121 tcggtttctc tcttaaagac actcactgta caactgaaag cagctgtcat atttctggca
3181 aaatgtgttt acgtatctga caagttgtac atttgtgtat gaactgacat aaaatgtgaa
3241 agcctgtaag tgtacatgta gtggtgtggt gttctgtcta gaggatacaa ctgaatgttt
3301 ttaatttgct gacttacaga cacaggctgt ttacaaaatg ctagctggaa agtctgtaat
3361 gttcatgtca taacttttag ttaattgcca ttgagcacct gttctgagga ggtgagatgt
3421 ggacttgtgc ttataaactg gagagtttag tcataatccc tcctggcttt gtgtgaatag
3481 cttgctcact ttgctggcct ttgaaatgtg ttctccgtga taagctatcc atgtgtttgt
3541 gataagagtg cttgtcaacc atgaccatct ttgagccttc ctagtcctcc acctggcaca
3601 gtatttgaaa tggcaaagga tgtgcttcat cctctaacaa acagtgtaca ctcccagagc
3661 tgatattctg gattgtgact gtgcacattt cctctagttc atgtctgtag tccctataga
3721 atgatctgta ataaaatagt atactggact gtgcatcaaa gggatgtaaa attacagtat
3781 tccaaaggtt gaagttctgc tgttttgtta taatgcctga tacacatctt gaataaagtc
3841 ttaacatttt tctttt

1 miyaivihil sllvgcfypa fasykilksq ncsvndlrgw liywiaygvy vafdyftagl
61 lafipllsef kvlllfwmlp svgggseviy eeflrsfscn esfdqvlgri tlewgelvwq
121 qvcsvlshlm vladryllps ghrpalqitp siedlvndai akrqleekrk qmgnlsdtin
181 evlgenidln mdllhgsesd llvikepisk pkerpipppk pmrqpsssnq qemnlssqfm
241
**Putative function** (CG1558) - unknown (CG11697) - may be deleted in human cancers, possibly a receptor.

### Example 19. Corkscrew / Shp2 (Category 3)

Corkscrew (CG3954) as a candidate gene is detected in a screen of a P-element insertion library covering the X chromosome of *Drosophila melanogaster* (Peter et al. 2001) as mutant phenotype in fly line 171, as described above.

Mitotic defects are observed in brain squashes: low mitotic index, few cells in mitosis and metaphases with separated chromosomes, and is placed in Category 3 as described above.

Rescue and sequencing of genomic DNA flanking the P-element insertion site indicates that the P-element is inserted into the 5' region of two genes: CG3954 corkscrew and CG16903 cyclin/non-specific RNA polymersae II transcription factor.
**Line ID** - 171
**Phenotype** - Lethal phase larval stage 1-2. Low mitotic index, few cells in mitosis, metaphase with separated chromosomes
**Annotated *Drosophila* genome genomic segment containing P element insertion site (and map position)** - AE003423 (2D1-2)
**P element insertion site** - 42,253
**Annotated *Drosophila* genome Complete Genome candidate** 2 candidates: CG3954 - corkscrew. Protein tyrosine phosphatase required for cell signaling in eye development (2 splice variants) and CG16903 - cyclin/non-specific RNA polymersae II transcription factor
   CG3954 - corkscrew. Protein tyrosine phosphatase required for cell signaling in eye splice variant 1 
ATGCTGTTCAACAAATGTCTGGAAAAGTTGTCCAGCTCGCTGGGCAATGT
GGTCAATCACAAGCTGCAAGAGAAACAAGTCTACAACAACAACAATATCA
ACAATAACAATAACAATACGCTAAACAACAACAATGCCTACAACAATCAG
CGAAACTTTGAGTACGAAAGAGCCATACAGGCGCACTACGGAAGCAAGGG
AAGACGCTCGGAGGAGCGCGAAAGGAGCGGCAAGTTCAAGGCCAGCAAGG
GTCGGAAAGCAAAGGTCACCCCACCAACGGAGACACCCGAGGCCCAGGAG
CCGGCCTGCAAGAACTGTATGACCCACGACGAGCTGGCCCAGATCATAAA
GGGCGTGGCCAAGGGCGCTGACGCGCAACGTAATCGAGACAACCGACTGC
AGCGCAGACGTCGTCCTCTCTCCGCCCAACCCTCCGCCGCTGCCTCCGCC
 
TCCACATCGACGGAATCTCTGCACCGTCTTACACCCAGCCCGCAGGCTTC
CTACCCGGCCACGCCCACCTCCTGGACAGCCACACCGCCCCAGTTCCCAG
CCGCCTTCGGCGGCGCCAGCTGCTCCAACAGCACACTGTCCCTCTTGGCC
ACCATGCGCGTCCAGCTCCATGGTTACACATGGTTTCATGGCAATCTTTC
CGGAAAGGAAGCGGAAAAATTGATCCTGGAGCGGGGCAAGAATGGTTCGT
TTCTCGTCCGTGAATCTCAGAGCAAGCCTGGCGACTTCGTCCTTTCCGTG
CGCACGGACGACAAAGTAACGCATGTCATGATTCGATGGCAGGACAAGAA
GTACGACGTCGGCGGCGGGGAATCCTTTGGCACCTTGTCGGAACTGATCG
ATCACTACAAGCGTAATCCCATGGTGGAGACGTGCGGAACCGTGGTGCAT
CTGCGACAGCCATTCAACGCCACACGAATCACGGCGGCCGGCATCAATGC
CCGGGTGGAACAGCTGGTCAAGGGAGGTTTCTGGGAGGAATTCGAATCGC
TGCAACAGGACAGTCGGGACACATTCTCGCGCAACGAGGGCTACAAACAG
GAGAACCGCCTCAAGAATCGCTACCGCAACATATTGCCATACGACCACAC
GCGCGTCAAGCTGCTGGACGTGGAGCATAGCGTGGCCGGAGCCGAGTACA
TCAATGCCAACTACATACGGCTGCCCACCGACGGCGACCTGTACAACATG
AGCAGCTCGTCGGAGAGCCTGAACAGCTCGGTGCCCTCGTGCCCCGCCTG
CACGGCTGCCCAGACACAGCGGAACTGCTCCAACTGCCAGCTGCAAAACA
AGACGTGCGTGCAGTGCGCCGTGAAGAGCGCCATTCTGCCGTATAGCAAC
TGTGCCACCTGCAGCCGCAAGTCAGACTCCCTGAGCAAGCACAAGCGGAG
CGAATCCTCGGCCTCTTCATCGCCCTCCTCCGGCTCTGGGTCCGGACCAG
GATCGTCGGGCACCAGCGGAGTGAGCAGCGTCAATGGACCCGGCACACCC
ACCAATCTCACGAGCGGCACAGCCGGATGTCTGGTCGGCCTGCTGAAGAG
ACACTCGAACGACTCGTCCGGAGCTGTTTCTATATCGATGGCCGAACGGG
AACGCGAGAGGGAGCGCGAGATGTTTAAGACCTACATCGCCACCCAGGGC
TGTCTGCTCACCCAGCAAGTGAACACGGTGACGGACTTCTGGAACATGGT
CTGGCAGGAGAACACGCGGGTGATCGTCATGACCACCAAGGAGTACGAGC
GCGGCAAAGAAAAGTGCGCCCGCTACTGGCCGGACGAGGGTAGATCGGAG
CAGTTCGGCCACGCGCGGATACAGTGCGTCTCGGAGAACTCGACCAGTGA
CTATACGCTGCGCGAGTTCCTCGTCTCGTGGCGGGATCAGCCGGCGCGCC
GGATCTTTCACTACCATTTCCAGGTGTGGCCGGATCACGGAGTGCCCGCC
GATCCGGGCTGTGTGCTCAACTTCCTGCAAGATGTCAACACGCGTCAGAG
TCACCTGGCTCAAGCGGGCGAGAAGCCGGGTCCGATCTGCGTGCACTGCT
CTGCGGGCATCGGTCGCACTGGCACCTTTATTGTGATCGATATGATTCTC
GATCAGATTGTGCGCAATGGATTGGATACTGAAATCGACATCCAGCGCAC
CATTCAGATGGTCCGATCGCAGCGTTCCGGTCTTGTGCAAACCGAGGCGC
AATACAAGTTCGTCTACTATGCGGTGCAGCACTATATACAGACCCTGATC
GCCCGGAAACGAGCTGAGGAGCAGAGCCTGCAGGTTGGCCGCGAGTACAC
CAATATAAAGTACACGGGCGAAATTGGAAACGATTCACAAAGATCTCCAT
TACCACCAGCAATTTCTAGCATAAGTTTAGTTCCGAGTAAGACGCCACTG
ACGCCGACATCGGCGGATTTGGGCACTGGGATGGGCCTAAGCATGGGCGT
GGGCATGGGCGTCGGCAACAAGCACGCATCGAAGCAGCAGCCGCCGTTGC
CGGTGGTCAACTGCAACAATAATAACAACGGCATTGGCAATAGCGGCTGC
AGCAACGGCGGCGGGAGCAGCACCACCAGCAGCAGCAACGGCAGCAGCAA
CGGTAACATCAACGCCCTACTGGGCGGCATCGGCTTGGGGCTGGGCGGCA
ATATGCGCAAGTCGAACTTTTACAGCGACTCGCTGAAGCAGCAACAGCAG
CGCGAGGAGCAGGCTCCGGCGGGAGCAGGTAAGATGCAGCAGCCGGCGCC
GCCGCTGCGACCGCGTCCTGGAATACTCAAGTTGCTCACCAGTCCCGTCA
TCTTTCAGCAAAATTCAAAAACATTCCCAAAGACATGA
 
MLFNKCLEKLSSSLGNVVNHKLQEKQVYNNNNINNNNNNTLNNNNAYNNQ
RNFEYERAIQAHYGSKGRRSEERERSGKFKASKGRKAKVTPPTETPEAQE
PACKNCMTHDELAQIIKGVAKGADAQRNRDNRLQRRRRPLSAQPSAAASA
STSTESLHRLTPSPQASYPATPTSWTATPPQFPAAFGGASCSNSTLSLLA
TMRVQLHGYTWFHGNLSGKEAEKLILERGKNGSFLVRESQSKPGDFVLSV
RTDDKVTHVMIRWQDKKYDVGGGESFGTLSELIDHYKRNPMVETCGTVVH
LRQPFNATRITAAGINARVEQLVKGGFWEEFESLQQDSRDTFSRNEGYKQ
 
ENRLKNRYRNILPYDHTRVKLLDVEHSVAGAEYINANYIRLPTDGDLYNM
SSSSESLNSSVPSCPACTAAQTQRNCSNCQLQNKTCVQCAVKSAILPYSN
CATCSRKSDSLSKHKRSESSASSSPSSGSGSGPGSSGTSGVSSVNGPGTP
TNLTSGTAGCLVGLLKRHSNDSSGAVSISMAEREREREREMFKTYIATQG
CLLTQQVNTVTDFWNMVWQENTRVIVMTTKEYERGKEKCARYWPDEGRSE
QFGHARIQCVSENSTSDYTLREFLVSWRDQPARRIFHYHFQVWPDHGVPA
DPGCVLNFLQDVNTRQSHLAQAGEKPGPICVHCSAGIGRTGTFIVIDMIL
DQIVRNGLDTEIDIQRTIQMVRSQRSGLVQTEAQYKFVYYAVQHYIQTLI
ARKRAEEQSLQVGREYTNIKYTGEIGNDSQRSPLPPAISSISLVPSKTPL
TPTSADLGTGMGLSMGVGMGVGNKHASKQQPPLPVVNCNNNNNGIGNSGC
SNGGGSSTTSSSNGSSNGNINALLGGIGLGLGGNMRKSNFYSDSLKQQQQ
REEQAPAGAGKMQQPAPPLRPRPGILKLLTSPVIFQQNSKTFPKT
 CG3954 - corkscrew. Protein tyrosine phosphatase required for cell signaling in eye splice variant 2 
AGTAAAAAAATAGTTTTTTTTTGTATCCAACCAACCAACTGTAAAAATA
AGTTTAAACAAAGCATCTACTCATAAGTTTCATTTTTTCCGTTAAGTGT
CAACATTATTTATTTTTAAGTGTGCATTCAATAAGAAAATGTCATCGCG
AAGATGGTTCCACCCAACGATATCTGGCATCGAAGCTGAGAAACTGCTGC
AGGAGCAGGGATTCGACGGCTCCTTCCTCGCCCGCCTCTCCTCCTCGAAT
CCGGGCGCCTTCACGCTCTCCGTGCGCCGCGGCAACGAGGTGACCCACAT
CAAAATCCAAAACAATGGCGACTTCTTTGATCTCTACGGTGGTGAAAAGT
TCGCCACACTGCCGGAACTGGTACAATACTACATGGAGAATGGCGAGCTA
AAGGAGAAGAACGGCCAGGCCATCGAACTCAAGCAGCCGCTGATCTGCGC
CGAGCCCACCACGGAAAGATGGTTTCATGGCAATCTTTCCGGAAAGGAAG
CGGAAAAATTGATCCTGGAGCGGGGCAAGAATGGTTCGTTTCTCGTCCGT
GAATCTCAGAGCAAGCCTGGCGACTTCGTCCTTTCCGTGCGCACGGACGA
CAAAGTAACGCATGTCATGATTCGATGGCAGGACAAGAAGTACGACGTCG
GCGGCGGGGAATCCTTTGGCACCTTGTCGGAACTGATCGATCACTACAAG
CGTAATCCCATGGTGGAGACGTGCGGAACCGTGGTGCATCTGCGACAGCC
ATTCAACGCCACACGAATCACGGCGGCCGGCATCAATGCCCGGGTGGAAC
AGCTGGTCAAGGGAGGTTTCTGGGAGGAATTCGAATCGCTGCAACAGGAC
AGTCGGGACACATTCTCGCGCAACGAGGGCTACAAACAGGAGAACCGCCT
CAAGAATCGCTACCGCAACATATTGCCATACGACCACACGCGCGTCAAGC
TGCTGGACGTGGAGCATAGCGTGGCCGGAGCCGAGTACATCAATGCCAAC
TACATACGGCTGCCCACCGACGGCGACCTGTACAACATGAGCAGCTCGTC
GGAGAGCCTGAACAGCTCGGTGCCCTCGTGCCCCGCCTGCACGGCTGCCC
AGACACAGCGGAACTGCTCCAACTGCCAGCTGCAAAACAAGACGTGCGTG
CAGTGCGCCGTGAAGAGCGCCATTCTGCCGTATAGCAACTGTGCCACCTG
CAGCCGCAAGTCAGACTCCCTGAGCAAGCACAAGCGGAGCGAATCCTCGG
CCTCTTCATCGCCCTCCTCCGGCTCTGGGTCCGGACCAGGATCGTCGGGC
ACCAGCGGAGTGAGCAGCGTCAATGGACCCGGCACACCCACCAATCTCAC
GAGCGGCACAGCCGGATGTCTGGTCGGCCTGCTGAAGAGACACTCGAACG
ACTCGTCCGGAGCTGTTTCTATATCGATGGCCGAACGGAACGCGAGAGG
GAGCGCGAGATGTTTAAGACCTACATCGCCACCCAGGGCTGTCTGCTCAC
CCAGCAAGTGAACACGGTGACGGACTTCTGGAACATGGTCTGGCAGGAGA
ACACGCGGGTGATCGTCATGACCACCAAGGAGTACGAGCGCGGCAAAGAA
AAGTGCGCCCGCTACTGGCCGGACGAGGGTAGATCGGAGCAGTTCGGCCA
CGCGCGGATACAGTGCGTCTCGGAGAACTCGACCAGTGACTATACGCTGC
GCGAGTTCCTCGTCTCGTGGCGGGATCAGCCGGCGCGCCGGATCTTTCAC
TACCATTTCCAGGTGTGGCCGGATCACGGAGTGCCCGCCGATCCGGGCTG
TGTGCTCAACTTCCTGCAAGATGTCAACACGCGTCAGAGTCACCTGGCTC
AAGCGGGCGAGAAGCCGGGTCCGATCTGCGTGCACTGCTCTGCGGGCATC
 
GGTCGCACTGGCACCTTTATTGTGATCGATATGATTCTCGATCAGATTGT
GCGCAATGGATTGGATACTGAAATCGACATCCAGCGCACCATTCAGATGG
TCCGATCGCAGCGTTCCGGTCTTGTGCAAACCGAGGCGCAATACAAGTTC
GTCTACTATGCGGTGCAGCACTATATACAGACCCTGATCGCCCGGAAACG
AGCTGAGGAGCAGAGCCTGCAGGTTGGCCGCGAGTACACCAATATAAAGT
ACACGGGCGAAATTGGAAACGATTCACAAAGATCTCCATTACCACCAGCA
ATTTCTAGCATAAGTTTAGTTCCGAGTAAGACGCCACTGACGCCGACATC
GGCGGATTTGGGCACTGGGATGGGCCTAAGCATGGGCGTGGGCATGGGCG
TCGGCAACAAGCACGCATCGAAGCAGCAGCCGCCGTTGCCGGTGGTCAAC
TGCAACAATAATAACAACGGCATTGGCAATAGCGGCTGCAGCAACGGCGG
CGGGAGCAGCACCACCAGCAGCAGCAACGGCAGCAGCAACGGTAACATCA
ACGCCCTACTGGGCGGCATCGGCTTGGGGCTGGGCGGCAATATGCGCAAG
TCGAACTTTTACAGCGACTCGCTGAAGCAGCAACAGCAGCGCGAGGAGCA
GGCTCCGGCGGGAGCAGGTAAGATGCAGCAGCCGGCGCCGCCGCTGCGAC
CGCGTCCTGGAATACTCAAGTTGCTCACCAGTCCCGTCATCTTTCAGCAA
AATTCAAAAACATTCCCAAAGACATGA
 
MSSRRWFHPTISGIEAEKLLQEQGFDGSFLARLSSSNPGAFTLSVRRGNE
VTHIKIQNNGDFFDLYGGEKFATLPELVQYYMENGELKEKNGQAIELKQP
LICAEPTTERWFHGNLSGKEAEKLILERGKNGSFLVRESQSKPGDFVLSV
RTDDKVTHVMIRWQDKKYDVGGGESFGTLSELIDHYKRNPMVETCGTVVH
LRQPFNATRITAAGINARVEQLVKGGFWEEFESLQQDSRDTFSRNEGYKQ
ENRLKNRYRNILPYDHTRVKLLDVEHSVAGAEYINANYIRLPTDGDLYNM
SSSSESLNSSVPSCPACTAAQTQRNCSNCQLQNKTCVQCAVKSAILPYSN
CATCSRKSDSLSKHKRSESSASSSPSSGSGSGPGSSGTSGVSSVNGPGTP
TNLTSGTAGCLVGLLKRHSNDSSGAVSISMAEREREREREMFKTYIATQG
CLLTQQVNTVTDFWNMVWQENTRVIVMTTKEYERGKEKCARYWPDEGRSE
QFGHARIQCVSENSTSDYTLREFLVSWRDQPARRIFHYHFQVWPDHGVPA
DPGCVLNFLQDVNTRQSHLAQAGEKPGPICVHCSAGIGRTGTFIVIDMIL
DQIVRNGLDTEIDIQRTIQMVRSQRSGLVQTEAQYKFVYYAVQHYIQTLI
ARKRAEEQSLQVGREYTNIKYTGEIGNDSQRSPLPPAISSISLVPSKTPL
TPTSADLGTGMGLSMGVGMGVGNKHASKQQPPLPVVNCNNNNNGIGNSGC
SNGGGSSTTSSSNGSSNGNINALLGGIGLGLGGNMRKSNFYSDSLKQQQQ
REEQAPAGAGKMQQPAPPLRPRPGILKLLTSPVIFQQNSKTFPKT
 CG 16903 - cyclin/non-specific RNA polymersae II transcription factor 
ATTTAGTATAAAAGCACGCCTGTTATCGGCTAAATTTACAAAAAAAAAGG
GAAAATTAAAAAATTAAAACACTTAAATAAACGCTTTCCTGGGTTAACCG
CGCACGAATGGCCACCCGTGGGGCCGGCTCGACTGTGGTCCACACGACGG
TGACAGCGCTGACGGTGGAGACGATCACCAATGTCCTGACCACGGTGACT
TCGTTCCATTCGAACAGCGTCAACATTTCGAACAACAACAGCAGCAGTGG
AGCGGCCCCGGGGGCGGATGCAGCTGGCGGCGATGCAGGGGGCGTGGCAG
CGGCTCAGGCGGACGCCAACAAGCCTATCTATCCTCGGCTCTTTAACCGC
ATCGTGCTGACGCTGGAGAACAGCCTCATTCCGGAGGGCAAAATCGATGT
GACGCCATCCAGCCAGGATGGACTGGACCATGAGACGGAGAAGGACCTGC
GCATACTGGGCTGCGAGCTTATTCAGACAGCCGGAATTTTGCTGCGCTTG
CCGCAGGTTGCCATGGCCACCGGCCAGGTGCTGTTCCAGCGCTTCTTCTA
CTCGAAGAGCTTTGTGCGGCACAACATGGAGACTGTGGCCATGAGCTGCG
TGTGCCTGGCGTCCAAGATCGAGGAGGCGCCGCGCCGCATTAGAGACGTG
ATCAATGTGTTCCATCACATCAAGCAAGTGCGGGCCCAAAAGGAAATCTC
GCCCATGGTGCTAGATCCTTACTACACGAACCTCAAGATGCAGGTGATCA
AGGCCGAGCGGCGCGTCCTCAAGGAACTGGGCTTCTGTGTACACGTGAAG
CATCCGCACAAGCTGATCGTGATGTATCTGCAGGTGCTTCAGTACGAGAA
GCACGAGAAGCTGATGCAGCTCTCCTGGAACTTCATGAATGACTCGCTGA
GGACGGACGTTTTTATGCGCTACACACCAGAGGCGATTGCATGCGCCTGC
 
ATCTACCTGAGTGCCCGCAAGCTCAACATACCTCTGCCCAACAGCCCGCC
GTGGTTCGGCATTTTTCGGGTGCCCATGGCGGACATTACGGATATCTGCT
ACCGTGTGATGGAGCTGTACATGCGTTCCAAGCCGGTGGTGGAGAAACTG
GAGGCGGCCGTGGACGAGCTGAAAAAGCGGTACATTGATGCGCGCAACAA
AACGAAGGAGGCAAACACACCGCCGGCTGTAATCACCGTGGATCGGAACA
ATGGCTCGCACAATGCGTGGGGTGGCTTCATCCAGCGTGCTATCCCACTG
CCCTTGCCATCGGAAAAGTCGCCGCAAAAGGATTCGAGGTCACGCTCGCG
ATCCAGGACGCGCACCCATTCGCGGACACCTCGCTCCCGATCACCCAGGT
CCAGGTCGCCTAGTCGCGAGCGCACTAAGAAGACCCACCGCAGTCGATCC
TCCCGCTCGCGCTCCCGTTCGCCGCCGAAGCATAAGAAAAAGTCACGTCA
CTACTCGAGGTCGCCCACGCGCTCCAATTCGCCGCACAGCAAGCACAGGA
AGTCGAAATCCTCGCGAGAACGCTCTGAATACTACTCCAAGAAAGATCGG
TCTGGAAACCCAGGCAGTAGCAATAATCTAGGTGATGGCGACAAGTATCG
CAACTCCGTCTCCAATTCCGGCAAGCACAGTCGGTACTCCTCCTCCTCGT
CGCGTCGGAACAGCGGTGGTGGTGGAGACGGAAGAAGCGGAGGAGGAGGT
GGTGGCGGCGGTGGAGGCAACGGGAACCACGGCAGCCGAGGGGGGCACAA
GCATCGGGATGGCGATCGCTCCAGGGATCGCAAGCGCTAGTGATTGATAG
ACAAGCGAGACAAACACTCCCTTATATTTAATTGCTCTTTATTTTACAAA
TTTACAGATTATTTCTACCGATTTAGTAATGCTAATGTGTATTGAAAAAA
CGAACGCGGGTAAACAATAAATGTAACTCTTCAATC
 
MATRGAGSTVVHTTVTALTVETITNVLTTVTSFHSNSVNISNNNSSSGAA
PGADAAGGDAGGVAAAQADANKPIYPRLFNRIVLTLENSLIPEGKIDVTP
SSQDGLDHETEKDLRILGCELIQTAGILLRLPQVAMATGQVLFQRFFYSK
SFVRHNMETVAMSCVCLASKIEEAPRRIRDVINVFHHIKQVRAQKEISPM
VLDPYYTNLKMQVIKAERRVLKELGFCVHVKHPHKLIVMYLQVLQYEKHE
KLMQLSWNFMNDSLRTDVFMRYTPEAIACACIYLSARKLNIPLPNSPPWF
GIFRVPMADITDICYRVMELYMRSKPVVEKLEAAVDELKKRYIDARNKTK
EANTPPAVITVDRNNGSHNAWGGFIQRAIPLPLPSEKSPQKDSRSRSRSR
TRTHSRTPRSRSPRSRSPSRERTKKTHRSRSSRSRSRSPPKHKKKSRHYS
RSPTRSNSPHSKHRKSKSSRERSEYYSKKDRSGNPGSSNNLGDGDKYRNS
VSNSGKHSRYSSSSSRRNSGGGDGRSGGGGGGGGGNGNHGSRGGHKHR
DGDRSRDRKR
**Human homologue of Complete Genome candidate**
   CG3954 homologue is Homo sapiens protein tyrosine phosphatase, non-receptor type 11 (PTPN11), also known as Shp2. Shp2 has 2 alternative transcripts having accession numbers NM_002834 and NM_080601.
   NM 002834 Homo sapiens protein tyrosine phosphatase, non-receptor type 11 (PTPN 11), transcript variant 1, mRNA also known as Shp2. 
1 cggccgcggt ttccaggagg aagcaaggat gctttggaca ctgtgcgtgg cgcctccgcg
61 gagcccccgc gctgccattc ccggccgtcg ctcggtcctc cgctgacggg aagcaggaag
121 tggcggcggg cgtcgcgagc ggtgacatca cgggggcgac ggcggcgaag ggcgggggcg
181 gaggaggagc gagccgggcc ggggggcagc tgcacagtct ccgggatccc caggcctgga
241 ggggggtctg tgcgcggccg gctggctctg ccccgcgtcc ggtcccgagc gggcctccct
301 cgggccagcc cgatgtgacc gagcccagcg gagcctgagc aaggagcggg tccgtcgcgg
361 agccggaggg cgggaggaac atgacatcgc ggagatggtt tcacccaaat atcactggtg
421 tggaggcaga aaacctactg ttgacaagag gagttgatgg cagttttttg gcaaggccta
481 gtaaaagtaa ccctggagac ttcacacttt ccgttagaag aaatggagct gtcacccaca
541 tcaagattca gaacactggt gattactatg acctgtatgg aggggagaaa tttgccactt
 
601 tggctgagtt ggtccagtat tacatggaac atcacgggca attaaaagag aagaatggag
661 atgtcattga gcttaaatat cctctgaact gtgcagatcc tacctctgaa aggtggtttc
721 atggacatct ctctgggaaa gaagcagaga aattattaac tgaaaaagga aaacatggta
781 gttttcttgt acgagagagc cagagccacc ctggagattt tgttctttct gtgcgcactg
841 gtgatgacaa aggggagagc aatgacggca agtctaaagt gacccatgtt atgattcgct
901 gtcaggaact gaaatacgac gttggtggag gagaacggtt tgattctttg acagatcttg
961 tggaacatta taagaagaat cctatggtgg aaacattggg tacagtacta caactcaagc
1021 agccccttaa cacgactcgt ataaatgctg ctgaaataga aagcagagtt cgagaactaa
1081 gcaaattagc tgagaccaca gataaagtca aacaaggctt ttgggaagaa tttgagacac
1141 tacaacaaca ggagtgcaaa cttctctaca gccgaaaaga gggtcaaagg caagaaaaca
1201 aaaacaaaaa tagatataaa aacatcctgc cctttgatca taccagggtt gtcctacacg
1261 atggtgatcc caatgagcct gtttcagatt acatcaatgc aaatatcatc atgcctgaat
1321 ttgaaaccaa gtgcaacaat tcaaagccca aaaagagtta cattgccaca caaggctgcc
1381 tgcaaaacac ggtgaatgac ttttggcgga tggtgttcca agaaaactcc cgagtgattg
1441 tcatgacaac gaaagaagtg gagagaggaa agagtaaatg tgtcaaatac tggcctgatg
1501 agtatgctct aaaagaatat ggcgtcatgc gtgttaggaa cgtcaaagaa agcgccgctc
1561 atgactatac gctaagagaa cttaaacttt caaaggttgg acaagggaat acggagagaa
1621 cggtctggca ataccacttt cggacctggc cggaccacgg cgtgcccagc gaccctgggg
1681 gcgtgctgga cttcctggag gaggtgcacc ataagcagga gagcatcatg gatgcagggc
1741 cggtcgtggt gcactgcagt gctggaattg gccggacagg gacgttcatt gtgattgata
1801 ttcttattga catcatcaga gagaaaggtg ttgactgcga tattgacgtt cccaaaacca
1861 tccagatggt gcggtctcag aggtcaggga tggtccagac agaagcacag taccgattta
1921 tctatatggc ggtccagcat tatattgaaa cactacagcg caggattgaa gaagagcaga
1981 aaagaaagag gaaagggcac gaatatacaa atattaagta ttctctagcg gaccagacga
2041 gtggagatca gagccctctc ccgccttgta ctccaacgcc accctgtgca gaaatgagag
2101 aagacagtgc tagagtctat gaaaacgtgg gcctgatgca acagcagaaa agtttcagat
2161 gagaaaacct gccaaaactt cagcacagaa atagatgtgg actttcaccc tctccctaaa
2221 aagatcaaga acagacgcaa gaaagtttat gtgaagacag aatttggatt tggaaggctt
2281 gcaatgtggt tgactacctt ttgataagca aaatttgaaa ccatttaaag accactgtat
2341 tttaactcaa caatacctgc ttcccaatta ctcatttcct cagataagaa gaaatcatct
2401 ctacaatgta gacaacatta tattttatag aatttgtttg aaattgagga agcagttaaa
2461 ttgtgcgctg tattttgcag attatgggga ttcaaattct agtaataggc tttttttattt
2521 ttatttttat acccttaacc agtttaattt tttttttcct cattgttggg gatgatgaga
2581 agaaatgatt tgggaaaatt aagtaacaac gacctagaaa agtgagaaca atctcattta
2641 ccatcatgta tccagtagtg gataattcat tttgatggct tctattttg gccaaatgag
2701 aattaagcca gtgcctgaga ctgtcagaag ttgacctttg cactggcatt aaagagtcat
2761 agaaaaaa
 
MTSRRWFHPNITGVEAENLLLTRGVDGSFLARPSKSNPGDFTLS
VRRNGAVTHIKIQNTGDYYDLYGGEKFATLAELVQYYMEHHGQLKEKNGDVIELKYPL
NCADPTSERWFHGHLSGKEAEKLLTEKGKHGSFLVRESQSHPGDFVLSVRTGDDKGES
NDGKSKVTHVMIRCQELKYDVGGGERFDSLTDLVEHYKKNPMVETLGTVLQLKQPLNT
TRINAAEIESRVRELSKLAETTDKVKQGFWEEFETLQQQECKLLYSRKEGQRQENKNK
NRYKNILPFDHTRVVLHDGDPNEPVSDYINANIIMPEFETKCNNSKPKKSYIATQGCL
QNTVNDFWRMVFQENSRVIVMTTKEVERGKSKCVKYWPDEYALKEYGVMRVRNVKESA
AHDYTLRELKLSKVGQGNTERTVWQYHFRTWPDHGVPSDPGGVLDFLEEVHHKQESIM
DAGPVVVHCSAGIGRTGTFIVIDILIDIIREKGVDCDIDVPKTIQMVRSQRSGMVQTE
AQYRFIYMAVQHYIETLQRRIEEEQKRKRKGHEYTNIKYSLADQTSGDQSPLPPCTPT
PPCAEMREDSARVYENVGLMQQQKSFR
 NM 080601 Homo sapiens protein tyrosine phosphatase, non-receptor type 11(PTPN11), transcript variant 2, mRNA (version 1) 
1 gcggaggagg agcgagccgg gccggggggc agctgcacag tctccgggat ccccaggcct
61 ggaggggggt ctgtgcgcgg ccggctggct ctgccccgcg tccggtcccg agcgggcctc
121 cctcgggcca gcccgatgtg accgagccca gcggagcctg agcaaggagc gggtccgtcg
181 cggagccgga gggcgggagg aacatgacat cgcggagatg gtttcaccca aatatcactg
241 gtgtggaggc agaaaaccta ctgttgacaa gaggagttga tggcagtttt ttggcaaggc
301 ctagtaaaag taaccctgga gacttcacac tttccgttag aagaaatgga gctgtcaccc
361 acatcaagat tcagaacact ggtgattact atgacctgta tggaggggag aaatttgcca
421 ctttggctga gttggtccag tattacatgg aacatcacgg gcaattaaaa gagaagaatg
481 gagatgtcat tgagcttaaa tatcctctga actgtgcaga tcctacctct gaaaggtggt
541 ttcatggaca tctctctggg aaagaagcag agaaattatt aactgaaaaa ggaaaacatg
601 gtagttttct tgtacgagag agccagagcc accctggaga ttttgttctt tctgtgcgca
 
661 ctggtgatga caaaggggag agcaatgacg gcaagtctaa agtgacccat gttatgattc
721 gctgtcagga actgaaatac gacgttggtg gaggagaacg gtttgattct ttgacagatc
781 ttgtggaaca ttataagaag aatcctatgg tggaaacatt gggtacagta ctacaactca
841 agcagcccct taacacgact cgtataaatg ctgctgaaat agaaagcaga gttcgagaac
901 taagcaaatt agctgagacc acagataaag tcaaacaagg cttttgggaa gaatttgaga
961 cactacaaca acaggagtgc aaacttctct acagccgaaa agagggtcaa aggcaagaaa
1021 acaaaaacaa aaatagatat aaaaacatcc tgccctttga tcataccagg gttgtcctac
1081 acgatggtga tcccaatgag cctgtttcag attacatcaa tgcaaatatc atcatgcctg
1141 aatttgaaac caagtgcaac aattcaaagc ccaaaaagag ttacattgcc acacaaggct
1201 gcctgcaaaa cacggtgaat gacttttggc ggatggtgtt ccaagaaaac tcccgagtga
1261 ttgtcatgac aacgaaagaa gtggagagag gaaagagtaa atgtgtcaaa tactggcctg
1321 atgagtatgc tctaaaagaa tatggcgtca tgcgtgttag gaacgtcaaa gaaagcgccg
1381 ctcatgacta tacgctaaga gaacttaaac tttcaaaggt tggacaaggg aatacggaga
1441 gaacggtctg gcaataccac tttcggacct ggccggacca cggcgtgccc agcgaccctg
1501 ggggcgtgct ggacttcctg gaggaggtgc accataagca ggagagcatc atggatgcag
1561 ggccggtcgt ggtgcactgc aggtgacagc tcctgctgcc cctctaggcc acagcctgtc
1621 cctgtctcct agcgcccagg gcttgctttt acctacccac tcctagctct ttaactgtag
1681 gaagaattta atatctgttt gaggcataga gcaactgcat tgagggacat tttgatccca
1741 aggcatattt ctcctagacc ctacagcact gccattggcc atggccatgg caacatgctc
1801 agttaaaaca gcaaagacta agtcagcatt atctctgagt ccaccagaag ttgtgcatta
1861 aacaacttca tcctggaaaa aaaaaaaaaa aa
 
1 mtsrrwfhpn itgveaenll ltrgvdgsfl arpsksnpgd ftlsvrrnga vthikiqntg
61 dyydlyggek fatlaelvqy ymehhgqlke kngdvielky plncadptse rwfhghlsgk
121 eaeklltekg khgsflvres qshpgdfvls vrtgddkges ndgkskvthv mircqelkyd
181 vgggerfdsl tdlvehykkn pmvetlgtvl qlkqplnttr inaaeiesrv relsklaett
241 dkvkqgfwee fetlqqqeck llysrkegqr qenknknryk nilpfdhtrv vlhdgdpnep
301 vsdyinanii mpefetkcnn skpkksyiat qgclqntvnd fwrmvfqens rvivmttkev
361 ergkskcvky wpdeyalkey gvmrvrnvke saahdytlre lklskvgqgn tertvwqyhf
421 rtwpdhgvps dpggvldfle evhhkqesim dagpvvvhcr
 NM 080601 Homo sapiens protein tyrosine phosphatase, non-receptor type 11(PTPN11), transcript variant 2, mRNA (version 2) 
1 cggccgcggt ttccaggagg aagcaaggat gctttggaca ctgtgcgtgg cgcctccgcg
61 gagcccccgc gctgccattc ccggccgtcg ctcggtcctc cgctgacggg aagcaggaag
121 tggcggcggg cgtcgcgagc ggtgacatca cgggggcgac ggcggcgaag ggcgggggcg
181 gaggaggagc gagccgggcc ggggggcagc tgcacagtct ccgggatccc caggcctgga
241 ggggggtctg tgcgcggccg gctggctctg ccccgcgtcc ggtcccgagc gggcctccct
301 cgggccagcc cgatgtgacc gagcccagcg gagcctgagc aaggagcggg tccgtcgcgg
361 agccggaggg cgggaggaac atgacatcgc ggagatggtt tcacccaaat atcactggtg
421 tggaggcaga aaacctactg ttgacaagag gagttgatgg cagttttttg gcaaggccta
481 gtaaaagtaa ccctggagac ttcacacttt ccgttagaag aaatggagct gtcacccaca
541 tcaagattca gaacactggt gattactatg acctgtatgg aggggagaaa tttgccactt
601 tggctgagtt ggtccagtat tacatggaac atcacgggca attaaaagag aagaatggag
661 atgtcattga gcttaaatat cctctgaact gtgcagatcc tacctctgaa aggtggtttc
721 atggacatct ctctgggaaa gaagcagaga aattattaac tgaaaaagga aaacatggta
781 gttttcttgt acgagagagc cagagccacc ctggagattt tgttctttct gtgcgcactg
841 gtgatgacaa aggggagagc aatgacggca agtctaaagt gacccatgtt atgattcgct
901 gtcaggaact gaaatacgac gttggtggag gagaacggtt tgattctttg acagatcttg
961 tggaacatta taagaagaat cctatggtgg aaacattggg tacagtacta caactcaagc
1021 agccccttaa cacgactcgt ataaatgctg ctgaaataga aagcagagtt cgagaactaa
1081 gcaaattagc tgagaccaca gataaagtca aacaaggctt ttgggaagaa tttgagacac
 
1141 tacaacaaca ggagtgcaaa cttctctaca gccgaaaaga gggtcaaagg caagaaaaca
1201 aaaacaaaaa tagatataaa aacatcctgc cctttgatca taccagggtt gtcctacacg
1261 atggtgatcc caatgagcct gtttcagatt acatcaatgc aaatatcatc atgcctgaat
1321 ttgaaaccaa gtgcaacaat tcaaagccca aaaagagtta cattgccaca caaggctgcc
1381 tgcaaaacac ggtgaatgac ttttggcgga tggtgttcca agaaaactcc cgagtgattg
1441 tcatgacaac gaaagaagtg gagagaggaa agagtaaatg tgtcaaatac tggcctgatg
1501 agtatgctct aaaagaatat ggcgtcatgc gtgttaggaa cgtcaaagaa agcgccgctc
1561 atgactatac gctaagagaa cttaaacttt caaaggttgg acaagggaat acggagagaa
1621 cggtctggca ataccacttt cggacctggc cggaccacgg cgtgcccagc gaccctgggg
1681 gcgtgctgga cttcctggag gaggtgcacc ataagcagga gagcatcatg gatgcagggc
1741 cggtcgtggt gcactgcagg tgacagctcc tgctgcccct ctaggccaca gcctgtccct
1801 gtctcctagc gcccagggct tgcttttacc tacccactcc tagctcttta actgtaggaa
1861 gaatttaata tctgtttgag gcatagagca actgcattga gggacatttt gatcccaagg
1921 catatttctc ctagacccta cagcactgcc attggccatg gccatggcaa catgctcagt
1981 taaaacagca aagactaagt cagcattatc tctgagtcca ccagaagttg tgcattaaac
2041 aacttcatcc tggaaaaaaa aaaaaaaaa
 
MTSRRWFHPNITGVEAENLLLTRGVDGSFLARPSKSNPGDFTLS
VRRNGAVTHIKIQNTGDYYDLYGGEKFATLAELVQYYMEHHGQLKEKNGDVIELKYPL
NCADPTSERWFHGHLSGKEAEKLLTEKGKHGSFLVRESQSHPGDFVLSVRTGDDKGES
NDGKSKVTHVMIRCQELKYDVGGGERFDSLTDLVEHYKKNPMVETLGTVLQLKQPLNT
TRINAAEIESRVRELSKLAETTDKVKQGFWEEFETLQQQECKLLYSRKEGQRQENKNK
NRYKNILPFDHTRVVLHDGDPNEPVSDYINANIIMPEFETKCNNSKPKKSYIATQGCL
QNTVNDFWRMVFQENSRVIVMTTKEVERGKSKCVKYWPDEYALKEYGVMRVRNVKESA
AHDYTLRELKLSKVGQGNTERTVWQYHFRTWPDHGVPSDPGGVLDFLEEVHHKQESIM
DAGPVVVHCR
**Putative function** (CG3954) - protein tyrosine phosphatase (CG16903) - cyclin, potentially involved in differentiation and neural plasticity

### Example 19B. Validation of GENE Function by RNA interference (RNAi) Knockdown in Drosophila Cultured Cells

To confirm the mitotic role of the target protein, knockdown of Corkscrew (CG3954) expression is performed in cultured *Drosophila* Dmel-2 cells using a double stranded RNA (dsRNA) from within the Corkscrew (CG3954) CDS corresponding to the following CDS sequence: 
GCCGAGTACATCAATGCCAACTACATACGGCTGCCCACCGACGGCGACCTGTA
CAACATGAGCAGCTCGTCGGAGAGCCTGAACAGCTCGGTGCCCTCGTGCCCCGCCTGC
ACGGCTGCCCAGACACAGCGGAACTGCTCCAACTGCCAGCTGCAAAACAAGACGTGC
GTGCAGTGCGCCGTGAAGAGCGCCATTCTGCCGTATAGCAACTGTGCCACCTGCAGCC
GCAAGTCAGACTCCCTGAGCAAGCACAAGCGGAGCGAATCCTCGGCCTCTTCATCGCC
CTCCTCCGGCTCTGGGTCCGGACCAGGATCGTCGGGCACCAGCGGAGTGAGCAGCGT
CAATGGACCCGGCACACCCACCAATCTCACGAGCGGCACAGCCGGATGTCTGGTCGG
CCTGCTGAAGAGACACTCGAACGACTCGTCCGGAGCTGTTTCTATATCGATGGCCGAA
CGGGAACGCGAGAGGGAGCGCGAGATGTTTAAGACCTACATCGCCACCCA
 dsRNA is prepared by annealing complimentary RNAs made by *in vitro* transcription from a PCR fragment created with the following PCR primers:
TAATACGACTCACTATAGGGAGAGCCGAGTACATCAATGCCAACTACAT
TAATACGACTCAGTATAGGGAGATGGGTGGCGATGTAGGTCTTAAACAT

Cells are transfected with double stranded RNA in the presence of 'Transfast' transfection reagent. A control transfection of a non-endogenous RNA corresponding to RFP (red fluorescent protein) is carried out in parallel.

### Analysis of Corkscrew CG3954 Knockdown by RNAi in D-Mel2 cells by Cellomics Mitotic Index Assay

For the transfection, 1 µg dsRNA is added to a well of a 96-well Packard viewplate and 35 µl of logarithmically growing DMel-2 cells diluted to 2.3x10⁵ cells/ml in fresh Drosophila-SFM/glutamine/Pen-Strep are added. Cells are incubated with the dsRNA (60nM) in a humid chamber at 28°C for 1 hr before addition of 100 µl Drosophila-SFM/glutamine/Pen-Strep. Cells are incubated at 28°C for 72 hours before analysis. For the assay, cells were fixed and stained using the Cellomics Mitotic Index HitKit following manufacturers instructions. The mitotic index of cells in each well was determined using the ArrayScan HCS System, running the Application protocol Mike_250502_Polgen_MitoticIndex_10x_p2.0 with the 10x objective and the DualBGlp filter set. This automated screening system detects the levels of a specific antigen (phosphorylated histone H3) which is only detectable during mitosis while the chromosomes are condensed.

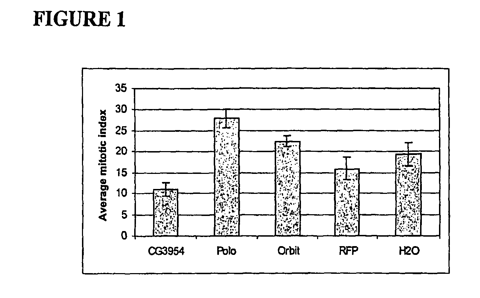

Results for Corkscrew (CG3954) are shown in Figure 1. A reproducible and significant reduction in mitotic index is observed in this assay indicating a reduction in the number of cells able to exit S-phase and enter mitosis after RNAi

### Analysis of Corkscrew CG3954 Knockdown by RNAi in D-Mel2 cells by Microscopy

For transfection 9 µl of Transfast reagent (Promega) is added to 3µg gene specific dsRNA in 500µl Drosophila Schneiders medium (no additives) and incubated at room temperature for 15 min. For control wells an equivalent amount of RFP dsRNA is used. This mix is added to a well of a 6-well tissue culture plate containing a glass coverslip and 500µl of a Dmel-2 cells at 1x10⁶ cells/ml in shneiders medium. After a 1 hour incubation at 28°C, 2mls Schneiders medium + 10% FCS and pen/strep solution is added and cells are incubated at 28°C for 48 hours. Cells on the coverslip are fixed in formaldehyde and stained with antibodies which detect α-tubulin and γ-tubulin (centrosomes), and are co-stained with DAPI to detect DNA.

An increase in the number of cells with chromosomal defects (see Table 1 below) was observed upon RNAi . The phenotypes seen were aneuploidy (65% of mitoses compared to 30% in control cells), misaligned chromosomes (80% compared to 40% in control cells), and polyploidy, however no spindle defects were observed.

**Table 1 shows mitotic defects observed by microscopy after RNAi knockdown of Corkscrew (CG3954) in Dmel2 Drosophila cultured cells.**

| dsRNA | Number cells with chromosomal defects | Number of cells with normal mitosis | % of chromosomal defects (no defects/total cells in mitosis) |
|---|---|---|---|
| No RNA | 135 | 314 | 39.47 |
| RFP | 137 | 309 | 40.29 |
| CG1725 | 186 | 87 | 68.13 |

### Example 19C. Shp2 is a Human Homologue of Drosophila Corkscrew CG3954

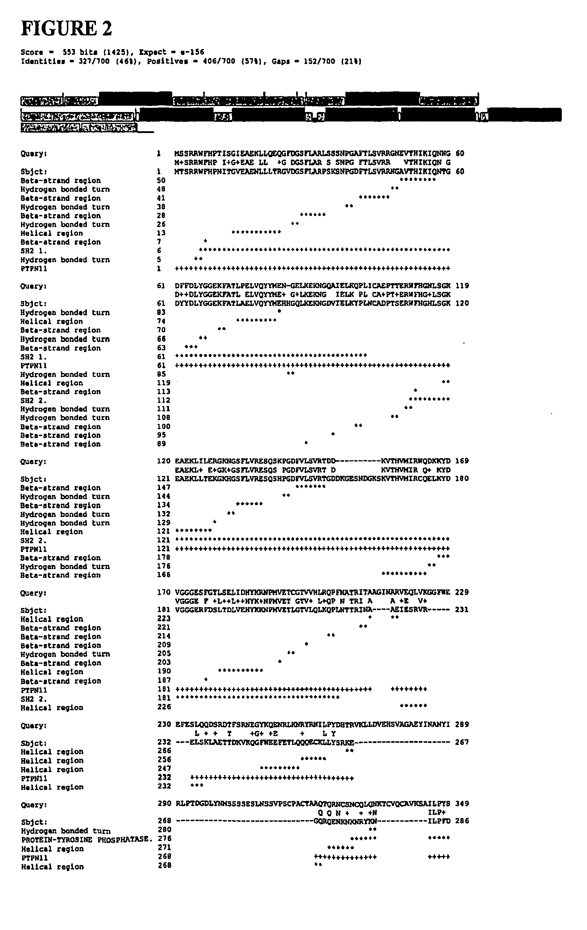

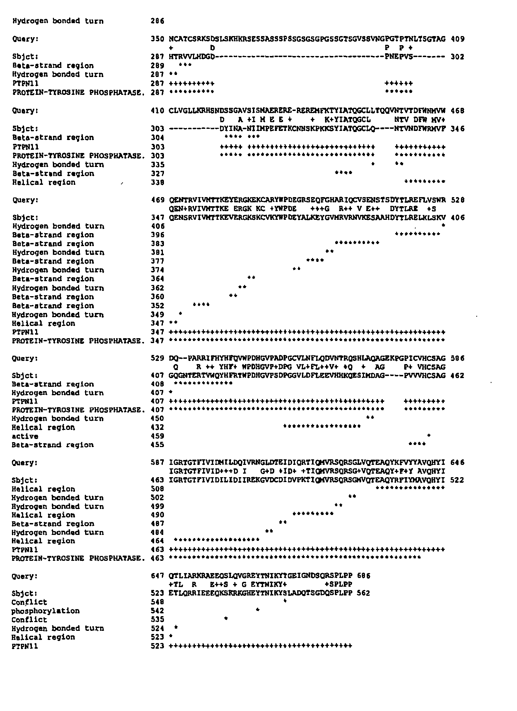

BLASTP with *Drosophila* Corkscrew CG3954 reveals 46% (327/700) sequence identity with the human Shp2 gene (genbank accession D13540), indicating that they are homologues. The BLASTP results are shown in Figure 2.

The sequence of the human Shp2 gene mRNA (2 splice variants is shown in Example 19 above).

### Example 19D. Validation of the Mitotic Role of the Human Homologue by siRNA Knockdown of Shp2 Expression in Human Cultured Cells

### Generation of Shp2 siRNA Knouckdowns

Knockdown of human Shp2 gene expression is achieved by siRNA (short interfering RNA, Elbashir et al, Nature 2001 May 24;411(6836):494-8). We used synthetic double stranded RNAs corresponding to two different regions of the Shp2 mRNA. siRNAs are obtained from Dharmacon (our supplier). The siRNA sequences are:

| | | | |
|---|---|---|---|
| COD1650 | shp2-1 siRNA | AACGUCAAAGAAAGCGC CGCU | Corresponds to nucleotides 1539 - 1559 in human Shp2 splice variants 1 and 2 see example 19 above) |
| COD1651 | shp2-2 siRNA | AAUUGGCCGGACAGGGA CGUU | Corresponds to nucleotides 1766 - 1786 in human Shp2 splice variants 1 and 2 see example 19 above) |

### Analysis of siRNA Hu Shp2 Knockdowns in U2OS Cells by Flow Cytometry Analysis

Cells are seeded in 6-well tissue culture dishes at 1x10⁵ cells/well in 2 ml Dulbecco's Modified Eagle's Medium (DMEM) (Sigma) + 10% Foetal Bovine Serum (FBS) (Perbio), and incubated overnight (37°C/ 5% CO₂).

For each well, 12 µl of 20 µM siRNA duplex (Dharmacon, Inc) (in RNAse-free H₂O) is mixed with 200 µl of Optimem (Invitrogen). In a separate tube 8 µl of oligofectamine reagent (Invitrogen) was mixed with 52 µl of Optimem, and incubated at room temperature for 7-10 mins. The oligofectamine/ Optimem mix is then added dropwise to the siRNA/ Optimem mix, and this is then mixed gently, before being incubated for 15-20 mins at room temperature. During this incubation the cells are washed once with DMEM (with no FBS or antibiotics added). 600 µl of DMEM (no FBS or antibiotics) is then added to each well.

Following the 15-20 min incubation, 128 µl of Optimem is added to the siRNA/oligofectamine/ optimem mix, and this was added to the cells (in 600 µl DMEM). The transfection mix is added at the edge of each well to assist dilution before contact is made with the cells. Cells are then incubated with the transfection mix for 4 h (37°C / 5%CO₂). Subsequently 1 ml DMEM + 20% FBS is added to each well. Cells are then incubated at 37°C / 5% CO₂ for 72 h. Cells are harvested by trypsinisation, washed in PBS, fixed in ice-cold 70% EtOH and stained with propidium iodide before Facs analysis.

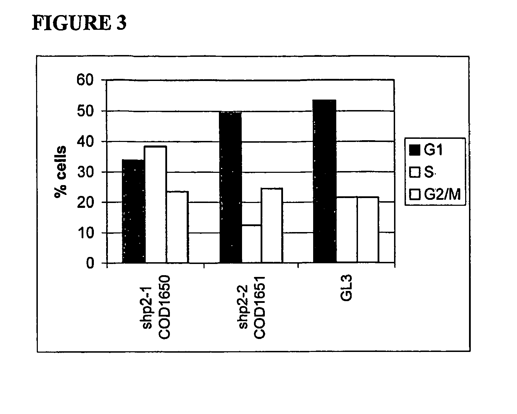

siRNA Hu Shp2 knockdowns are conducted in U2OS. As shown in Figure 3 major changes in the distribution of cells between cell cycle compartments (G1, S, G2 /M) are seen with Shp2 siRNA COD1650 which is directed to both alternative transcripts of Shp2. An accumulation of cells in the S2 compartment cell cycle, is observed with a concomitant reduction in the G1 compartment population. This indicates that a proportion of cells may unable complete S-phase and enter mitosis.

Subsequent microscopic analysis is performed in order to look at phenotypes resulting from the Shp2 siRNA induced defect and check for the presence of large multinucleate cells which may, due to their size and ploidy, be excluded from the FACS analysis.

### Analysis of Hu Shp2 siRNA Knockdowns in U2OS Cells by Microscopy

The transfection method for samples for microscopy is identical to that for Facs except that cells are plated in wells containing a sterile glass coverslip. Cells are incubated with siRNA for 48 hours before formaldehyde fixation and co-staining with Dapi to reveal DNA (blue) and antibodies to reveal microtubules (red) and centrosomes (green). Antibodies used are: rat anti-alpha tubulin (YL12) (supplier Serotec) with secondary antibody goat anti-rat IgG-TRITC (supplier Jackson Immunoresearch) and mouse anti-gamma-tubulin (GTU88) with secondary antibody Alexagreen488-goat anti-mouseIgG (supplier Sigma).

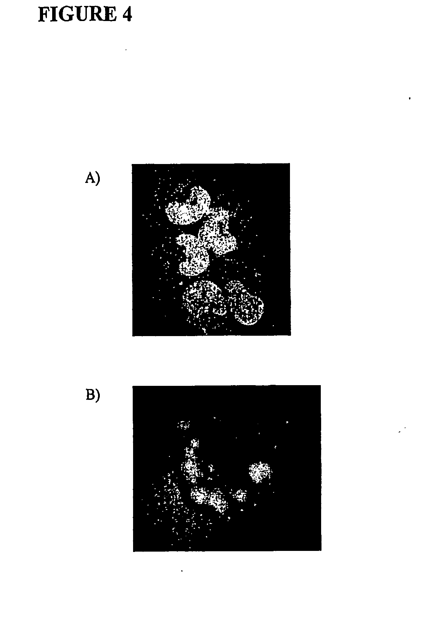

Phenotype analysis by microscopy is conducted on U2OS cells. Results from duplicate experiments in U2OS cells are shown in Figures 4, and Table 2 below. After siRNA no mitotic defects were seen, only a small increase in binucleate and apoptotic cells. These results are consistent with the Facs analysis, and in conjunction with the results of Corkscrew siRNA in Dmel-2 cells, they confirm that Shp2 is involved in cell cycle progression, in particular, in completing S-phase. Accordingly, modulators of Shp2 activity (as identified by the assays described above) may be used to treat any proliferative disease.

**Table 2:**

| Description of significant cell division defects after Shp2 siRNA in U2OS cells. | |
|---|---|
| Gene/siRNA | Shp2/COD1650 |
| Cell Type | U2OS |
| Polyploidy | Normal |
| Mitotic Defects | Normal |
| Main knockout phenotype | No mitotic phenotype observed |
| Additional observations | Increased number of binuclear cells (0.6/ field compared to 0.2/field in untreated) Increase in apoptotic cells |

### Example 19E. Expression of Recombinant Hu Shp2 Protein in Insect Cells

A cDNA encoding the Human Shp2 coding region derived by RT-PCR is inserted into the baculovirus expression vector pFastbacHTc (Life Technologies). A baculovirus stock is generated and western blot of subsequent infections of Sf9 insect cells demonstrates expression of N-terminal 6-His tagged proteins of approximately 68 kD. The recombinant protein is purified by Ni-NTA resin affinity chromatography.

Similarly 6-His tagged Dlg proteins are expressed in bacteria by inserting cDNAs into bacterial expression plamids pDest17 or pET series. Protein expression in cultures of host E.coli cells transformed with recombinant plasmid is induced by addition of inducer chemical IPTG. The recombinant protein is purified by Ni-NTA resin affinity chromatography

### Example 19F. Assay for Modulators of Shp2 Activity

Shp2 is a non-transmembrane-type protein tyrosine phosphatase that participates in the signal transduction pathways of a variety of growth factors and cytokines. Shp2 binds directly to the PDGF receptor, EGF receptor, and c-KIT in response to stimulation of cells with the corresponding receptor ligand and undergoes tyrosine phosphorylation. Shp2 is implicated in PDGF-induced RAS activation and EGF stimulation of the RAS-MAP kinase cascade that leads to DNA synthesis. Corkscrew (the putative *Drosophila* homolog of Shp2) is thought to be required for Ras1 activation or to function in conjunction with Ras1 during signaling by the Sevenless receptor tyrosine kinase. In addition Shp2 is implicated in insulin dependent signaling. Shp2 does not interact directly with the insulin receptor,but it binds through its SH2 domains to tyrosine-phosphorylated docking proteins such as IRS 1, IRS2, and GAB 1 in response to insulin. Overall Shp2 appears to play a role in growth factor-induced cell proliferation, through activation of the RAS-MAP kinase cascade. In addition to its role in receptor tyrosine kinase-mediated MAP kinase activation, Shp2 may play an important role, partly through its interaction with the membrane glycoprotein SHPS-1, in the activation of MAP kinase in response to the engagement of integrins by the extracellular matrix.

phosphotyrosyl proteins or peptides derived from SHPS-1, IRS1 or PDGF. An assay for modulators of Shp2 activity would consist of detection of dephosphorylation of ligand proteins, or phosphotyrosyl peptides derived from ligand proteins, described above e.g. phosphotyrosyl proteins or peptides derived from SHPS-1, IRS1 or PDGF (Takada et al 1998). Dephosphorylation of the substrate would be detected by quantifying the released inorganic phosphate, or by detecting loss of phosphate using an anti-phosphotyrosine antibody.

### Example 20 (Category 3)

**Line ID** - 500
**Phenotype** - Viable, High mitotic index, colchicines-type overcondensed chromosomes, a few polyploid cells
**Annotated *Drosophila* genome genomic segment containing P element insertion site (and map position)** - AE003422 (2C)
**P element insertion site** - 247,403
**Annotated *Drosophila* genome Complete Genome candidate** CG4399 - EAST 
ATGTCTAGCCGGAAGGTGCCAGGAGGCTCTGGAGGAGCTGACGAATCCAC
AGCAGCAGCTGCCCCCCTGGATGATAATGCCAATGCCAGTGTGGAGATTC
CAGACAGCAGCGAGGAGCCAGCAATGGGCGTCGGCGAAGAGATGTCTATC
ATAAGCAAAACACGCACCTCAACTTTGTCAGTGGAGCCCGCTAAGGAGCC
AACAGTAACAGCAGAGCTGGAAGGCGAAAAAGAGCTGGAATCGAATCCAG
TCTCCAAAACTCCTAGGTCCACGCCTACGCCAACCCTTACGCCAGCCGTC
ACGCCTACCGCCAGTGATGGAGTGGCGGCCAAGAGCGTGAGGGTTACCCG
GCACTCGTCGCCACTGCTTCTGATCATCTCGCCCACGACAAGTAGACGTG
AGGTCGGCGACGGAGAGCTAGACACCGAGGAACCAACGGGATCGGGTGGC
CAAAGAAAGAGCTCCGTGGAGCGATCTTTGGCGCCCGTTATACGCGGACG
AAAGTCCATCAAGGATCTGAAAGAAGCCAAAGAAGTCAAGTCCGAGGAGC
CGCCTGCCGCAGCATCAGAGTCACGAGCTGCAAGTGGAGTGACGCCTGGC
CAGGTCAAGGAACAGCATGTCGCGGATGGCAACGAAATGGAATCCTTGCC
AATCACAGACAAGAAAGACCACAAAGACACAAAAGACAAGGGAGATGAGC
GGGAAACCGATCAGGAGGAAGAGAAGGAAAAATCAGCTGATACAGAAATA
ATTGCAGATACAGAAAAAACTTCGGAGAAACAAAAGTATACAGAGAAGGA
CAAAGCTGCCGATAAAGATGGAGGAAAAGAAAAAGATATTGATGCAAATA
AGGATATAGATAAGGAGAAGGAAAAGGTCAAGGAAGTACTTCCGCCAGTG
GTGCCTATAGCACCAGTGACACCCACTTGTAACCGTGTCACACGTAAATC
ACATGCCCAGGAGCAGGCGATTAACACGCGGGTCACTCGCAATCGTCGCC
AGTCCTCTACAGTTGGAGCCAACTCCACCGCGTCTTTGGTAGCTGCATCC
TCCTCAGTAACAGAGCAACCCCCTCCATCTCGCGGTCGACGGAAGAAGCC
AGTGGTGGTGGCTCCTCCCTTGGAGCCTGCGGTAAAACGGAAGCGATCGC
AAGATGTTGAAGCCGACTCAGACGCCAACAACAGCACGAAATACAGCAAG
GTGGAAGTGGTAAAGTCTGAGGAAGCTGAGGCACCAGAGGAGGACTCCAG
TGCCGTGCCCATTAAGCAGGAATCTGTTGATGGCAACGAGGTCAGTTCTA
TTTCTCCAACAGTCACGCCCACACCCACACCTGCGCCAACACCAGCTCCA
GTCCCGGGCAGTCGACGGGGTCGTGGGCGCCCGCAGAACAGGAACTCCTC
TTCGCCTGCAACCACAACGCGGGCAACGCGGCTAAGCAAGGCGGGATCAC
CGGTTATCCTGACGCCAGTAGCCCAGGAACCGGCGCCACCGAAACGGCGG
CGAGTCGGCTCCAGCACACGGAAGACTGTCTCGGCCAGCTCGCTGGCACC
CAGCTCGCAGGGCGGCGCCGGGGATGAGGACTCCAAGGACAGTATGGCCT
CGTCCATGGACGACCTGCTGATGGCCGCAGCAGATATCAAGCAGGAGAAG
CTGACGCCCGATTTCGACGATAGTTTGATGCCAGAAGGCCTGCCCTCTAC
TTCTGGTGCGTCGAGTGCCAATGGTCATTCCTGCACCGAACCGCTTACTG
TGGACACGGAAATTAATGTTAAGCCCGCTGATTCCAAAGTAAAACCAAAG
 
GAGTCACCGGTGGTAGCAGTCGAGGAATCTCCATCACAATCCGAAACGCA
ATCTGCAAAGGTGTCAGCGCATGCGGGAAGGCTCCATCTCTTAGTCCAG
ATATGATAAGTGAAGGCGTGAGCGCGGTCAGTGTTCGAAAGTTTTATAAG
AAGCCTGAGTTCCTGGAAAACAATCTGGGCATTGAAAAGGATCCGGAGCT
AGGTGAAATCGTTCAGACGGTTAGTAACAATGACACGGAAACAGATGTGG
AGATGGCTGTTGATGGCGAGGTGAATCAACCGTCAACTCCCAAGTCGCAG
GATAAAAAGAAAGAGGAGCAGGAAAAGAATCAGAAATCAGGGCTAAAGGC
AGCAAAGAAGGCTCCTGCTAAGTTAGAACCTAAAGCTGAAGACATTTCTG
AAATTCTTACTGACGTTCCTGTTGATATTTCGACTGAGGCAGTAGAAATT
ATAGAAGAAGCAGAGGAAGACACTTGTTCAAATAGCTCAATCAAACCAGG
TGAGCTCCGACTGGACGAGAGCAACGATGAACCTGAACTGCTTCTTGAAG
ACGCCCTCATAGTCAATGGTGATGAGAATGAGACACCAGATCAACCGGAG
GAAAAGGAGGACCAGGTGGAGTTCTTCCATACAGGAGAATACGACGACTT
TGAGCACGAGATTATGGTGGAGCTGGCGAAGGAGGGGGTGCTAGATGCCA
GCGGCAATGCATTAAGTCAGCAAAAGGTAGAACTTGAGCATCCCGAGGAT
GTAACTCTACACGAATCAAAAAATGACATAGAAGCCGAAGAATCGGTTGA
ACGTAAGCCTCTTAAGGACCCGTCGGTTGCGGACGAAATGGAGGACATGA
ATGAGGAATCCTATATTGACATTAAGGACCAGACAAATCAACTGTTAGTT
GAACACTTGGCAGAAGAGGCCATGGAAGCGGACTGCGGTCCCGAGGATAA
CAAGGAGAACTTGTCCACGTCTGCTTCGAGCACCGCTGCCGATGGTCTAG
ATATTCAGTTGGCCATCAAGGAGGATGACGACGAGGAGAAACCGCTTGCA
GTTATCGCTGACGAACAGAAGCCTGGGCTGCTGTTGACCAATGACATGAA
AGTGGATGAGAAACCAAATGGCAAGCAGGAATCGGTCTGTGATGAGCACG
TTCAGCTGGTGCCAAACCTTCGTCAAGAACAGGAAATTCACTTACAAAAT
CTGGGCCTACTCACGCACCAGGCCGCTGAACATAGGCGCAAGTGTCTGCT
TGAGGCACAGGCCCGCCAGGCGCAAATGCAGCTCCAGCAACATCACCACC
ATCAGCACAAGCGACAAGGAGCGCGCGGAGGAGGCAGTGCCACTCATGTG
GAATCCAGCGGTACTTTGAAGACAGTCATCAAGCTGAACAGGAGCAGCAA
CGGAGGAGTAAGCGGTAGTGGCGGCCTGCCTACTGGTACAGTTATCCATG
GAGGCTGTGGCTCCTCTTCAGCTTCTTCCACGTCCTCCTCCTCGGTGGGC
AGTGCCACACGTAAGTCAAGCGGGACCTTGGGCTCAGGAGCGGGAGCAGG
AGCTGGCGTTCGCCGGCAGTCGCTTAAGATGACATTCCAGAAGGGTCGGG
CTCGTGGTCACGGTGCTGCGGATCGATCCGCCGATCAGTATGGCGCCCAC
GCCGAGGACTCCTACTACACCATTCAAAACGAGAACGAAGGTGCGAAAAA
GTTTGTTGTAACTACTGGTAATACCGGCCGCAAGACTAATAACCGTTTCA
GCTCAACTAACAACTACCACTCGACGGTAGCCTTGCACGGTAGCAACTCT
GCGCTCCAGTACTATTCGTCCCACTCGGAAAGTCAGGGACAGACGGACCA
CGGCTTCTATCAGATGGTCAAAAAGGACGAAAAGGAGAAGATCCTCATTC
CGGAAAAGGCCTCCTCGTTTAAGTTTCACCCAGGGAGACTGTGCGAAGAC
CAGTGCTACTACTGTAGCGGAAAGTTTGGCCTCTATGACACCCCCTGCCA
TGTTGGACAAATAAAGTCCGTGGAGCGCCAGCAGAAGATCCTAGCCAACG
AGGAGAAGCTCACCGTGGATAACTGCTTGTGCGACGCATGTTTTCGACAC
GTGGACCGCCGGGCAAATGTGCCATCCTATAAGAAGCGTCTTTCCGCTTC
AGGTCACTTGGAGATGGGGTCTGCAGCGGGATCTGCACTAGAGAAACAGT
TTGCTGGCGACAGCGGCGTCATTACGGAATCGGGTGGCGAAGCTGGTTCT
ACGGCAGCTGTGGCCGTGCAGCAACGATCTTGTGGCGTGAAGGACTGCGT
CGAAGCGGCACGACACTCGCTGCGGCGCAAGTGCATACGCAAGAGAGTAA
AGAAGTATCAGCTCAGCCTGGAGATTCCCGCAGGCTCGTCGAACGTGGGG
 
CTGTGTGAGGCACATTACAATACGGTCATCCAATTTTCCGGCTGCGTTCT
TTGCAAGCGTAGATTAGGCAAGAACCATATGTACAACATAACCACGCAGG
ACACAATTCGACTGGAAAAGGCGCTGTCCGAGATGGGCATCCCAGTTCAG
CTTGGCATGGGCACTGCAGTCTGCAAGCTGTGTCGCTATTTTGCCAACCT
TTTGATAAAGCCACCGGATAGCACCAAGGCACAAAAGGCGGAATTCGTGA
AGAACTACAGAAAGAGGCTCCTCAAGGTGCATAATCTGCAGGATGGCAGT
CATGAGCTGTCCGAAGCGGATGAAGAGGCACCTAATGCAACGGAGAC
AGAAAGGCCAACCTCAGACGGACACGAAGATCCCGAGATGCCCATGGTAG
CGGACTATGATGGACCTACCGACTCCAATTCCAGTAGTTCTTCGACTGCA
GCCCTGGACACCAGCAAACAAATGTCCAAGCTTCAGGCCATCCTGCAGCA
AAATGTGGGAGCGGATGCGGCAGGAGCTGCGGAACAGGAACTGTTGCAG
CAAGTCCCGGAGGAAGCGGATCTGGGGCAGATATCTCTAACGTATTGCGA
GGGAATCCGAACATTTCCATGCGCGAACTTTTCCACGGCGAGGAAGAGCT
GGGTGTGCAGTTCAAGGTGCCGTTCGGATGCAGCAGCAGCCAGCGTACTC
CGGAGGGCTGGACACGAGTGCAGACTTTCCTACAATACGATGAGCCGACG
CGCCGCCTCTGGGAGGAGTTGCAAAAGCCGTACGGAAATCAGAGCTCATT
TCTGCGCCACTTGATACTATTAGAGAAGTACTACCGAAACGGAGATCTCG
TCCTAGCACCGCATGCTTCCTCCAATGCCACGGTTTACACAGAGACTGTT
CGTCAGCGGCTGAATTCGTTTGATCACGGTCACTGCGGTGGATTGAACAT
CGCAGGCAGCCCTTCTTCTTCGGGTTCCGGCAAGCGCAGTGGAGTTCCTC
AACCTACGGGTGCCAGTGTGCTGGCCACCGCCCTCACAACACCCTTGACA
AGCCATTCATCCTCCTCTGCATCCATTTCCTCCGAACAGCATTCGTCGGT
TGATCCTGTCATTCCGCTGGTAGACCTCAATGATGACGATGAAGGCGAAG
ATGGGGCAGGAGGAGCGGGCGAAAGGGAGTCGACAAATAGGCAGCAGGAC
GTAATCTTGGAATGCCTTAGAACTGCCTCTGTGGACAAGCTGACTAAGCA
GCTCAGCTCGAATGCGGTGACGATTATCGCCCGGCCCAAAGACAAATCGC
AGCTCTCCTGCAACAGCGGATCCTCCACGTCCATTTCCAGCTCCTCGTCC
GCTATTTCCTCGCCGGAGGAAGTGGCCGTCACTAAGGTTACAGCAGTCGC
ACCAGTCCAGTCCAAGGATGCACCGCCACTGGCGCCAGCAAGTAGCGGTG
TTAGCAACAGTCGTAGTATCCTTAAAACCAACCTCTTGGGCATGAACAAG
GCCGTGGAACTCGTGCCCTTAACGACTGCCCCCCACGCTTACAAGCCAAC
TGGATGCCATAAGCCTGAGAAACAGCAAAAGATTCTTGACGTGGCCAATA
AGCAGCCCGGTAGCCAGGGGGAACCGGTACCATCAAGCGCCTTGCTTGGC
CTGCAGTCAAAGCTAAAGCCTCCAACGCATCAGCAGCAGGTCAGCGGATC
AGGAGCGGGAACTAGTGGTTCTCAGAAGCCATCTAATGTGGCGCAATTGC
TTAGCTCTCCACCGGAGCTAATCAGCTTGCATCGACGGCAGACCAGCGGA
GCAGCAGCGGGGTCCAGCAGCTTCCTTCAGGGCAAGAGGCTTCAACTTCC
ACGATCTGGAGCAGGGCCTTCAGGAGCGGGAACGGAACAGGCGCTGGAG
CAGCAGGAAGCCGCAGTGCGGGTGGACCACCACCGCCCAATGTGGTCATA
CTGCCGGACGCCTTAACCCCCCAGGAGCGACACGAGAGCAAGAGCTGGAA
GCCAACGCTGATACCGCTGGAGGATCAGCACAAGGTGCCGAACAAATCAC
ATGCTCTTTATCAGACCGCCGACGGTCGAAGGTTGCCCGCCCTGGTGCAA
GTGCAGTCTGGTGGCAAGCCATACCTCATCTCTATCTTCGACTATAACCG
CATGTGCATCTTGCGAAGGGAAAAGCTGATGCGGGACCAGTTGCTCAAGA
GTAACGCCAAGCCAAAGCCGCAGAACCAGCAACAGCAGCAGGGCCAAACG
CACCAGCAGCAGCAGAATTCCGCCGCATCGGCGGCTGCCTTCTCCAACAT
GGTGAAGTTGGCCCAGCAACACACGGCGCGACAGCAGCTTCAGCAGCTGC
AACAGAAGCCACAACAGCAGCAACAATTGCCCACTTTGCAGCCAGGTGGG
 
GTGCGACTTGCCCGGCTGCCGCAAAAACTACTGATGCCACCACTGACTAA
TCCGCAGATTGGCAGTCAAGCACCCAACTTACAGCCGTTGCTGTCTAGTA
CGCTGGATAACAGCAACAACTGCTGGCTGTGGAAAAACTTTCCTGATCCC
AATCAGTATCTGCTAAATGGAAACGGAGGGGGTGCCGGGAGCTCCTCCAG
CAAGTTGCCACATCTCACGGCCAAACCAGCCACGGCAACTAGTAGCTCCG
GAGCGGCCAACAAATCAGCAGGAAGCCTATTTACCCTCAAGCAGCAGCAG
CACCAGCAGAAACTCATCGACAACGCTATCATGTCAAAGATACCCAAAAG
TCTGACAGTAATACCGCAGCAGATGGGTGGTAATACCGGTGGCGATATGG
GGGGCAGCAGCTCCTCCGGCAAGGACTGATGACGGCGAAGGAGGGCGCCA
TGGCCATTAGCCGTAGCGCCGGAGGTAACCCGGCGAAGTAGTAGGATCAA
CAAGCAGGCGACGTGCAGCTTAAGCGGCGATCTTCAGAACAAGAGGTGAC
CAGCGGCGGCTCCATGGATATCACAAACTCCACAATTCCATGGCTGCAGT
AGAATAAGTGATACACT
 
MSSRKVPGGSGGADESTAAAAPLDDNANASVEIPDSSEEPAMGVGEEMSI
ISKTRTSTLSVEPAKEPTVTAELEGEKELESNPVSKTPRSTPTPTLTPAV
TPTASDGVAAKSVRVTRHSSPLLLIISPTTSRREVGDGELDTEEPTGSGG
QRKSSVERSLAPVIRGRKSIKDLKEAKEVKSEEPPAAASESRAASGVTPG
QVKEQHVADGNEMESLPITDKKDHKDTKDKGDERETDQEEEKEKSADTEI
IADTEKTSEKQKYTEKDKAADKDGGKEKDIDANKDIDKEKEKVKEVLPPV
VPIAPVTPTCNRVTRKSHAQEQAINTRVTRNRRQSSTVGANSTASLVAAS
SSVTEQPPPSRGRRKKPVVVAPPLEPAVKRKRSQDVEADSDANNSTKYSK
VEVVKSEEAEAPEEDSSAVPIKQESVDGNEVSSISPTVTPTPTPAPTPAP
VPGSRRGRGRPQNRNSSSPATTTRATRLSKAGSPVILTPVAQEPAPPKRR
RVGSSTRKTVSASSLAPSSQGGAGDEDSKDSMASSMDDLLMAAADIKQEK
LTPDFDDSLMPEGLPSTSGASSANGHSCTEPLTVDTEINVKPADSKVKPK
ESPVVAVEESPSQSETQSAKVSAHAGKAPSLSPDMISEGVSAVSVRKFYK
KPEFLENNLGIEKDPELGEIVQTVSNNDTETDVEMAVDGEVNQPSTPKSQ
DKKKEEQEKNQKSGLKAAKKAPAKLEPKAEDISEILTDVPVDISTEAVEI
IEEAEEDTCSNSSIKPGELRLDESNDEPELLLEDALIVNGDENETPDQPE
EKEDQVEFFHTGEYDDFEHEIMVELAKEGVLDASGNALSQQKVELEHPED
VTLHESKNDIEAEESVERKPLKDPSVADEMEDMNEESYIDIKDQTNQLLV
EHLAEEAMEADCGPEDNKENLSTSASSTAADGLDIQLAIKEDDDEEKPLA
VIADEQKPGLLLTNDMKVDEKPNGKQESVCDEHVQLVPNLRQEQEIHLQN
LGLLTHQAAEHRRKCLLEAQARQAQMLQQHHHHQHKRQGARGGGSATHV
ESSGTLKTVIKLNRSSNGGVSGSGGLPTGTVIHGGCGSSSASSTSSSSVG
SATRKSSGTLGSGAGAGAGVRRQSLKMTFQKGRARGHGAADRSADQYGAH
AEDSYYTIQNENEGAKKFVVTTGNTGRKTNNRFSSTNNYHSTVALHGSNS
ALQYYSSHSESQGQTDHGFYQMVKKDEKEKILIPEKASSFKFHPGRLCED
QCYYCSGKFGLYDTPCHVGQIKSVERQQKILANEEKLTVDNCLCDACFRH
VDRRANVPSYKKRLSASGHLEMGSAAGSALEKQFAGDSGVITESGGEAGS
TAAVAVQQRSCGVKDCVEAARHSLRRKCIRKRVKKYQLSLEIPAGSSNVG
LCEAHYNTVIQFSGCVLCKRRLGKNHMYNITTQDTIRLEKALSEMGIPVQ
LGMGTAVCKLCRYFANLLIKPPDSTKAQKAEFVKNYRKRLLKVHNLQDGS
HELSEADEEEAPNATETERPTSDGHEDPEMPMVADYDGPTDSNSSSSSTA
ALDTSKQMSKLQAILQQNVGADAAGAAGTGTVAASPGGSGSGADISNVLR
GNPNISMRELFHGEEELGVQFKVPFGCSSSQRTPEGWTRVQTFLQYDEPT
 
RRLWEELQKPYGNQSSFLRHLILLEKYYRNGDLVLAPHASSNATVYTETV
RQRLNSFDHGHCGGLNIAGSPSSSGSGKRSGVPQPTGASVLATALTTPLT
SHSSSSASISSEQHSSVDPVIPLVDLNDDDEGEDGAGGAGERESTNRQQD
VILECLRTASVDKLTKQLSSNAVTIIARPKDKSQLSCNSGSSTSISSSSS
AISSPEEVAVTKVTAVAPVQSKDAPPLAPASSGVSNSRSILKTNLLGMNK
AVELVPLTTAPHAYKPTGCHKPEKQQKILDVANKQPGSQGEPVPSSALLG
LQSKLKPPTHQQQVSGSGAGTSGSQKPSNVAQLLSSPPELISLHRRQTSG
AAAGSSSFLQGKRLQLPRSGAGPSGAGTGTGAGAAGSRSAGGPPPPNVVI
LPDALTPQERHESKSWKPTLIPLEDQHKVPNKSHALYQTADGRRLPALVQ
VQSGGKPYLISIFDYNRMCILRREKLMRDQLLKSNAKPKPQNQQQQQGQT
HQQQQNSAASAAAFSNMVKLAQQHTARQQLQQLQQKPQQQQQLPTLQPGG
VRLARLPQKLLMPPLTNPQIGSQAPNLQPLLSSTLDNSNNCWLWKNFPDP
NQYLLNGNGGGAGSSSSKLPHLTAKPATATSSSGAANKSAGSLFTLKQQQ
HQQKLIDNAIMSKIPKSLTVIPQQMGGNTGGDMGGSSSSGKD
**Human homologue of Complete Genome candidate** AAF13722 - neurofilament protein 
1 atgatgagct tcggcggcgc ggacgcgctg ctgggcgccc cgttcgcgcc gctgcatggc
61 ggcggcagcc tccactacgc gctagcccga aagggtggcg caggcgggac gcgctccgcc
121 gctggctcct ccagcggctt ccactcgtgg acacggacgt ccgtgagctc cgtgtccgcc
181 tcgcccagcc gcttccgtgg cgcaggcgcc gcctcaagca ccgactcgct ggacacgctg
241 agcaacgggc cggagggctg catggtggcg gtggccacct cacgcagtga gaaggagcag
301 ctgcaggcgc tgaacgaccg cttcgccggg tacatcgaca aggtgcggca gctggaggcg
361 cacaaccgca gcctggaggg cgaggctgcg gcgctgcggc agcagcaggc gggccgctcc
421 gctatgggcg agctgtacga gcgcgaggtc cgcgagatgc gcggcgcggt gctgcgcctg
481 ggcgcggcgc gcggtcagct acgcctggag caggagcacc tgctcgagga catcgcgcac
541 gtgcgccagc gcctagacga cgaggcccgg cagcgagagg aggccgaggc ggcggcccgc
601 gcgctggcgc gcttcgcgca ggaggccgag gcggcgcgcg tggacctgca gaagaaggcg
661 caggcgctgc aggaggagtg cggctacctg cggcgccacc accaggaaga ggtgggcgag
721 ctgctcggcc agatccaggg ctccggcgcc gcgcaggcgc agatgcaggc cgagacgcgc
781 gacgccctga agtgcgacgt gacgtcggcg ctgcgcgaga ttcgcgcgca gcttgaaggc
841 cacgcggtgc agagcacgct gcagtccgag gagtggttcc gagtgaggct ggaccgactg
901 tcggaggcag ccaaggtgaa cacagacgct atgcgctcag cgcaggagga gataactgag
961 taccggcgtc agctgcaggc caggaccaca gagctggagg cactgaaaag caccaaggac
1021 tcactggaga ggcagcgctc tgagctggag gaccgtcatc aggccgacat tgcctcctac
1081 caggaagcca ttcagcagct ggacgctgag ctgaggaaca ccaagtggga gatggccgcc
1141 cagctgcgag aataccagga cctgctcaat gtcaagatgg ctctggatat agagatagcc
1201 gcttacagaa aactcctgga aggtgaagag tgtcggattg gctttggccc aattcctttc
1261 tcgcttccag aaggactccc caaaattccc tctgtgtcca ctcacataaa ggtgaaaagc
1321 gaagagaaga tcaaagtggt ggagaagtct gagaaagaaa ctgtgattgt ggaggaacag
1381 acagaggaga cccaagtgac tgaagaagtg actgaagaag aggagaaaga ggccaaagag
1441 gaggagggca aggaggaaga agggggtgaa gaagaggagg cagaaggggg agaagaagaa
1501 acaaagtctc ccccagcaga agaggctgca tccccagaga aggaagccaa gtcaccagta
1561 aaggaagagg caaagtcacc ggctgaggcc aagtccccag agaaggagga agcaaaatcc
1621 ccagccgaag tcaagtcccc tgagaaggcc aagtctccag caaaggaaga ggcaaagtca
1681 ccgcctgagg ccaagtcccc agagaaggag gaagcaaaat ctccagctga ggtcaagtcc
 
1741 cccgagaagg ccaagtcccc agcaaaggaa gaggcaaagt caccggctga ggccaagtct
1801 ccagagaagg ccaagtcccc agtgaaggaa gaagcaaagt caccggctga ggccaagtcc
1861 ccagtgaagg aagaagcaaa atctccagct gaggtcaagt ccccggaaaa ggccaagtct
1921 ccaacgaagg aggaagcaaa gtcccctgag aaggccaagt cccctgagaa ggccaagtcc
1981 ccagagaagg aagaggccaa gtcccctgag aaggccaagt ccccagtgaa ggcagaagca
2041 aagtcccctg agaaggccaa gtccccagtg aaggcagaag caaagtcccc tgagaaggcc
2101 aagtccccag tgaaggaaga agcaaagtcc cctgagaagg ccaagtcccc agtgaaggaa
2161 gaagcaaagt cccctgagaa ggccaagtcc ccagtgaagg aagaagcaaa gacccccgag
2221 aaggccaagt ccccagtgaa ggaagaagcc aagtccccag agaaggccaa gtccccagag
2281 aaggccaaga ctcttgatgt gaagtctcca gaagccaaga ctccagcgaa ggaggaagca
2341 aggtcccctg cagacaaatt ccctgaaaag gccaaaagcc ctgtcaagga ggaggtcaag
2401 tccccagaga aggcgaaatc tcccctgaag gaggatgcca aggcccctga gaaggagatc
2461 ccaaaaaagg aagaggtgaa gtccccagtg aaggaggagg agaagcccca ggaggtgaaa
2521 gtcaaagagc ccccaaagaa ggcagaggaa gagaaagccc ctgccacacc aaaaacagag
2581 gagaagaagg acagcaagaa agaggaggca cccaagaagg aggctccaaa gcccaaggtg
2641 gaggagaaga aggaacctgc tgtcgaaaag cccaaagaat ccaaagttga agccaagaag
2701 gaagaggctg aagataagaa aaaagtcccc accccagaga aggaggctcc tgccaaggtg
2761 gaggtgaagg aagacgctaa acccaaagaa aagacagagg tggccaagaa ggaaccagat
2821 gatgccaagg ccaaggaacc cagcaaacca gcagagaaga aggaggcagc accggagaaa
2881 aaagacacca aggaggagaa ggccaagaag cctgaggaga aacccaagac agaggccaaa
2941 gccaaggaag atgacaagac cctctcaaaa gagcctagca agcctaaggc agaaaaggct
3001 gaaaaatcct ccagcacaga ccaaaaagac agcaagcctc cagagaaggc cacagaagac
3061 aaggccgcca aggggaagta aggcagggag aaaggaacat ccggaacagc caaagaaact
3121 cagaagagtc ccggagctca aggatcagag taacacaatt ttcacttttt ctgtctttat
3181 gtaagaagaa actgcttaga tgacggggcc tccttcttca aacaggaatt tctgttagca
3241 atatgttagc aagagagggc actcccaggc ccctgccccc atgccctccc caggcgatgg
3301 acaattatga tagcttatgt agctgaatgt gatacatgcc gaatgccaca cgtaaacact
3361 tgactataaa aactgccccc ctcctttcca aataagtgca tttattgcct ctatgtgcaa
3421 ctgacagatg accgcaataa tgaatgagca gttagaaata cattatgctt gagatgtctt
3481 aacctattcc caaatgcctt ctgttttcca aaggagtggt caagcccttg cccagagctc
3541 tctattctgg aagagcggtc caggtggggc cgggcactgg ccactgaatt atgccagggc
3601 gcactttcca ctggagttca ctttcaattg cttctgtgca ataaaaccaa gtgcttataa
3661 aatgaaaaaa aaaaaaaaaa tgctgttatt ctctttccct gggaaggctg ggggcagggc
3721 aggggaggtc tggatgtgac accccagact gcatgggact gagcaagcat cagt
 
1 mmsfggadal lgapfaplhg ggslhyalar kggaggtrsa agsssgfhsw trtsvssvsa
61 spsrfrgaga asstdsldtl sngpegcmva vatsrsekeq lqalndrfag yidkvrqlea
121 hnrslegeaa alrqqqagrs amgelyerev remrgavlrl gaargqlrle qehllediah
181 vrqrlddear qreeaeaaar alarfaqeae aarvdlqkka qalqeecgyl rrhhqeevge
241 llgqiqgsga aqaqmqaetr dalkcdvtsa lreiraqleg havqstlqse ewfrvrldrl
301 seaakvntda mrsaqeeite yrrqlqartt elealkstkd slerqrsele drhqadiasy
361 qeaiqqldae lrntkwemaa qlreyqdlln vkmaldieia ayrkllegee crigfgpipf
421 slpeglpkip svsthikvks eekikvveks eketviveeq teetqvteev teeeekeake
481 eegkeeegge eeeaeggeee tksppaeeaa spekeakspv keeakspaea kspekeeaks
541 paevkspeka kspakeeaks ppeakspeke eakspaevks pekakspake eakspaeaks
601 pekakspvke eakspaeaks pvkeeakspa evkspekaks ptkeeakspe kakspekaks
661 pekeeakspe kakspvkaea kspekakspv kaeakspeka kspvkeeaks pekakspvke
721 eakspekaks pvkeeaktpe kakspvkeea kspekakspe kaktldvksp eaktpakeea
 
781 rspadkfpek akspvkeevk spekaksplk edakapekei pkkeevkspv keeekpqevk
841 vkeppkkaee ekapatpkte ekkdskkeea pkkeapkpkv eekkepavek pkeskveakk
901 eeaedkkkvp tpekeapakv evkedakpke ktevakkepd dakakepskp aekkeaapek
961 kdtkeekakk peekpkteak akeddktlsk epskpkaeka ekssstdqkd skppekated
1021 kaakgk
**Putative function** unknown

### Example 21 (Category 3)

**Line ID** - 265
**Phenotype** - Lethal phase pharate adult. High mitotic index, rod like overcondensed chromosomes, few anaphases with lagging chromosomes
**Annotated *Drosophila* genome genomic segment containing P element insertion site (and map position)** - AE003509 (17B4-5)
**P element insertion site** - 52,563
Annotated *Drosophila* genome Complete Genome candidate CG6407 - Wnt5 
CAGTTGTTTACAATTTGTCGTTGAGGGTGGATTACTTCGTCGCGAGTTTC
GTTCGTGCATGATGCGGTTGTGGTTGATTGTATACATACATACTATGCAC
AAATCCAGTTCTCATTTTGTTATTTACAAATTCTCAGCGAGCGCATGAA
CTGGCAGCCTATAGCGAGCAGCTAATCACAATATTTACGGCAGATTCGTG
GACTCAAGGAAATTCAGCCAGCAGCCAATCGATTTTCTAGTGTTATCGAA
AAACATTTTTCATTCCTTCATTTCGTTCAACTAACAATACTAGTTACTAC
TAACAATACTCTGTAATAGTAATAGTAAGAGGAACAGGAATAGGAATACA
CATACTCCAAAGCGATAATGAGTTGCTACAGAAAAAGGCACTTTCTATTG
TGGCTCTTGCGTGCTGTGTGTATGTTGCACTTAACCGCGAGAGGGGCATA
TGCCACAGTTGGGTTGCAAGGAGTGCCGACATGGATATATCTCGGCCTCA
AGTCCCCCTTCATCGAGTTTGGCAACCAGGTGGAGCAGCTGGCCAATTCC
AGCATACCACTGAACATGACCAAGGACGAGCAGGCCAATATGCATCAAGA
GGGCCTACGCAAGCTCGGTACGTTCATAAAGCCAGTGGACCTGCGGACT
CGGAGACTGGCTTCGTCAAGGCCGATCTCACCAAGAGACTGGTATTCGAT
AGACCGAACAACATTACATCTCGCCCTATTCACCCGATACAGGAGGAGAT
GGATCAGAAGCAGATAATCCTGCTCGACGAGGATACCGACGAGAATGGCC
TGCCAGCCAGTCTCACCGACGAGGATCGCAAGTTTATAGTGCCGATGGCG
CTCAAGAATATATCGCCCGATCCACGCTGGGCGGCCACTACACCGAGTCC
CTCCGCTTTGCAGCCGAACGCTAAAGCCATCTCGACCATTGTGCCCTCGC
CTCTGGCCCAGGTCGAGGGGGATCCCACGTCCAACATCGATGACCTGAAG
AAGCACATACTCTTCTTGCACAACATGACCAAGACCAATTCGAACTTCGA
GTCGAAATTCGTTAAATTCCCGAGCCTGCAAAAGGACAAGGCCAAGACAT
CGGGAGCTGGCGGTTCGCCGCCCAATCCCAAGCGGCCCCAGCGGCCGATT
CATCAGTATTCCGCGCCCATAGCCCCACCAACACCCAAGGTGCCCGCGCC
AGATGGCGGCGGCGTAGGAGGAGCAGCTTACAATCCCGGAGAGCAGCCAA
TTGGTGGCTACTATCAGAACGAGGAACTAGCGAATAATCAATCCCTTCTT
AAACCAACAGATACCGACTCCCATCCAGCGGCCGGCGGTAGCAGCCATGG
CCAGAAGAATCCCAGCGAGCCCCAGGTGATACTGCTCAACGAGACACTCT
CCACGGAGACCTCAATCGAAGCGGATCGCAGTCCATCGATAAACCAGCCC
AAGGCGGGATCGCCTGCGCGCACAACAAAGCGACCACCTTGCCTGCGCAA
TCCCGAGTCCCCGAAATGCATACGTCAGCGTCGGCGGGAGGAGCAACAGC
GGCAGCGGGAGCGGGACGAGTGGTTCCGCGGTCAGTCGCAGTACATGCAG
CCCCGGTTCGAGCCGATCATACAGACGATTAACAATACGAAGAGATTTGC
CGTATCAATCGAGATTCCAGACTCCTTTAAAGTATCCTCCGAGGGATCGG
ATGGGGAGTTGCTTTCGCGAGTCGAACGCTCGCAGCCCAGCATTAGTAGT
 
AGTAGTAGTAGCAGTAGTAGCAGTAGTAGGAAAATCATGCCAGACTATAT
TAAGGTATCCATGGAGAACAACACATCCGTCACGGATTATTTAAGCACG
ACGTTGTGATGACATCGGCAGATGTCGCCAGCGATAGGGAATTCCTTATC
AAGAACATGGAGGAGCACGGAGGCGCTGGCTCCGCGAACAGTCATCACAA
TGATACGACTCCAACTGCAGACGCATATTCGGAGACAATCGATCTTAATC
CCAATAACTGCTATAGCGCAATAGGTCTAAGCAACAGCCAAAAGAAGCAA
TGTGTTAAGCACACCAGCGTGATGCCGGCCATAAGTCGTGGTGCCCGTGC
CGCCATCCAGGAGTGCCAGTTTCAGTTCAAGAATCGCCGCTGGAACTGCA
GCACAACGAACGACGAGACCGTATTTGGTCCCATGACCAGCCTGGCTGCT
CCCGAAATGGCCTTCATCCACGCCCTGGCCGCGGCCACGGTGACCAGCTT
CATAGCTCGCGCCTGCCGGGATGGCCAACTGGCCTCCTGCAGCTGCTCCC
GCGGCAGTCGACCCAAACAGCTCCACGACGACTGGAAGTGGGGCGGCTGT
GGCGACAACCTGGAGTTCGCCTACAAGTTCGCCACGGACTTCATCGATTC
GCGGGAGAAGGAAACCAATCGCGAGACGCGTGGCGTTAAGAGAAAACGCG
AGGAGATCAACAAGAATCGCATGCATTCCGATGACACGAATGCTTTTAAC
ATAGGTATTAAACGTAACAAAAACGTAGATGCTAAAAACGATACAAGTTT
GGTAGTGAGAAACGTTAGGAAAAGCACTGAGGCTGAAAACAGTCACATAC
TCAATGAGAACTTTGATCAGCACCTATTGGAACTAGAGCAGCGCATTACG
AAGGAGATACTTACATCCAAGATAGACGAGGAGGAGATGATTAAGCTGCA
GGAGAAGATCAAACAGGAGATTGTCAACACCAAGTTCTTCAAGGGTGAGC
AGCAGCCGCGCAAGAAGAAGCGAAAAACCAGAGAGCCGCCGCCGATGCG
CCCGCCTATCCGAGGAACGGCATCAAGGAGAGCTACAAGGATGGCGGCAT
ATTGCCGCGCAGCACGGCCACTGTCAAGGCCAGGAGCCTGATGAACTTGC
ACAACAACGAGGCCGGACGTCGGGCGGTGATCAAGAAGGCCAGGATAACG
TGCAAGTGCCACGGCGTGTCCGGCTCCTGCAGCCTGATCACCTGCTGGCA
GCAATTGTCCTCCATCCGGGAGATTGGCGACTATCTGCGCGAGAAGTACG
AGGGCGCCACCAAGGTGAAGATCAACAAGCGTGGCCGCCTCCAGATCAAG
GACTTGCAATTCAAGGTGCCGACCGCTCACGATCTTATTTACCTAGACGA
AAGTCCCGACTGGTGCCGCAATAGCTATGCGCTGCATTGGCCGGGAACGC
ACGGACGTGTGTGCCACAAAAACTCGTCGGGATTGGAGAGCTGTGCCATC
CTCTGCTGCGGCCGGGGCTATAATACGAAGAACATTATAGTTAACGAACG
CTGCAATTGCAAATTTCACTGGTGTTGCCAGGTTAAATGTGAAGTTTGTA
CGAAGGTACTCGAGGAGCACACATGTAAATAGAGCGTTGATTGAATTCGA
ATGTCTTAATGTTTGTGACTAAGCCATGAAGGAAATAATCGTATTTAAAC
AGTCCTCTCCATTTTAATTGCCATTACCATACACCATCATATTGCTTCTT
CTTAAAATGCT
 
MSCYRKRHFLLWLLRAVCMLHLTARGAYATVGLQGVPTWIYLGLKSPFIE
FGNQVEQLANSSIPLNMTKDEQANMHQEGLRKLGTFIKPVDLRDSETGFV
KADLTKRLVFDRPNNITSRPIHPIQEEMDQKQIILLDEDTDENGLPASLT
DEDRKFIVPMALKNISPDPRWAATTPSPSALQPNAKAISTIVPSPLAQVE
GDPTSNIDDLKKHILFLHNMTKTNSNFESKFVKFPSLQKDKAKTSGAGGS
PPNPKRPQRPIHQYSAPIAPPTPKVPAPDGGGVGGAAYNPGEQPIGGYYQ
NEELANNQSLLKPTDTDSHPAAGGSSHGQKNPSEPQVILLNETLSTETSI
EADRSPSINQPKAGSPARTTKRPPCLRNPESPKCIRQRRREEQQRQRERD
EWFRGQSQYMQPRFEPIIQTINNTKRFAVSIEIPDSFKVSSEGSDGELLS
RVERSQPSISSSSSSSSSSRKIMPDYIKVSMENNTSVTDYFKHDVVMTS
ADVASDREFLIKNMEEHGGAGSANSHHNDTTPTADAYSETIDLNPNNCYS
 
AIGLSNSQKKQCVKHTSVMPAISRGARAAIQECQFQFKNRRWNCSTTNDE
TVFGPMTSLAAPEMAFIHALAAATVTSFIARACRDGQLASCSCSRGSRPK
QLHDDWKWGGCGDNLEFAYKFATDFIDSREKETNRETRGVKRKREEINKN
RMHSDDTNAFNIGIKRNKNVDAKNDTSLVVRNVRKSTEAENSHILNENFD
QHLLELEQRITKEILTSKIDEEEMIKLQEKIKQEIVNTKFFKGEQQPRKK
KRKNQRAAADAPAYPRNGIKESYKDGGILPRSTATVKARSLMNLHNNEAG
RRAVIKKARITCKCHGVSGSCSLITCWQQLSSIREIGDYLREKYEGATKV
KINKRGRLQIKDLQFKVPTAHDLIYLDESPDWCRNSYALHWPGTHGRVCH
KNSSGLESCAILCCGRGYNTKNIIVNERCNCKFHWCCQVKCEVCTKVLEE
HTCK
**Human homologue of Complete Genome candidate** AAA16842 - hWNT5A 
1 attaattctg gctccacttg ttgctcggcc caggttgggg agaggacgga gggtggccgc
61 agcgggttcc tgagtgaatt acccaggagg gactgagcac agcaccaact agagaggggt
121 cagggggtgc gggactcgag cgagcaggaa ggaggcagcg cctggcacca gggctttgac
181 tcaacagaat tgagacacgt ttgtaatcgc tggcgtgccc cgcgcacagg atcccagcga
241 aaatcagatt tcctggtgag gttgcgtggg tggattaatt tggaaaaaga aactgcctat
301 atcttgccat caaaaaactc acggaggaga agcgcagtca atcaacagta aacttaagag
361 acccccgatg ctcccctggt ttaacttgta tgcttgaaaa ttatctgaga gggaataaac
421 atcttttcct tcttccctct ccagaagtcc attggaatat taagcccagg agttgctttg
481 gggatggctg gaagtgcaat gtcttccaag ttcttcctag tggctttggc catatttttc
541 tccttcgccc aggttgtaat tgaagccaat tcttggtggt cgctaggtat gaataaccct
601 gttcagatgt cagaagtata tattatagga gcacagcctc tctgcagcca actggcagga
661 ctttctcaag gacagaagaa actgtgccac ttgtatcagg accacatgca gtacatcgga
721 gaaggcgcga agacaggcat caaagaatgc cagtatcaat tccgacatcg acggtggaac
781 tgcagcactg tggataacac ctctgttttt ggcagggtga tgcagatagg cagccgcgag
841 acggccttca catacgccgt gagcgcagca ggggtggtga acgccatgag ccgggcgtgc
901 cgcgagggcg agctgtccac ctgcggctgc agccgcgccg cgcgccccaa ggacctgccg
961 cgggactggc tctggggcgg ctgcggcgac aacatcgact atggctaccg ctttgccaag
1021 gagttcgtgg acgcccgcga gcgggagcgc atccacgcca agggctccta cgagagtgct
1081 cgcatcctca tgaacctgca caacaacgag gccggccgca ggacggtgta caacctggct
1141 gatgtggcct gcaagtgcca tggggtgtcc ggctcatgta gcctgaagac atgctggctg
1201 cagctggcag acttccgcaa ggtgggtgat gccctgaagg agaagtacga cagcgcggcg
1261 gccatgcggc tcaacagccg gggcaagttg gtacaggtca acagccgctt caactcgccc
1321 accacacaag acctggtcta catcgacccc agccctgact actgcgtgcg caatgagagc
1381 accggctcgc tgggcacgca gggccgcctg tgcaacaaga cgtcggaggg catggatggc
1441 tgcgagctca tgtgctgcgg ccgtgggtac gaccagttca agaccgtgca gacggagcgc
1501 tgccactgca agttccactg gtgctgctac gtcaagtgca agaagtgcac ggagatcgtg
1561 gaccagtttg tgtgcaagta gtgggtgcca cccagcactc agccccgctc ccaggacccg
1621 cttatttata gaaagtacag tgattctggt ttttggtttt tagaaatatt ttttattttt
1681 ccccaagaat tgcaaccgga accatttttt ttcctgttac catctaagaa ctctgtggtt
1741 tattattaat attataatta ttatttggca ataatggggg tgggaaccac gaaaaatatt
1801 tattttgtgg atctttgaaa aggtaataca agacttcttt tggatagtat agaatgaagg
1861 gggaaataac acatacccta acttagctgt gtgggacatg gtacacatcc agaaggtaaa
1921 gaaatacatt ttcttttctt caaatatgcc atcatatggg atgggtaggt tccagttgaa
 
1981 agagggtggt agaaatctat tcacaattca gcttctatga ccaaaatgag ttgtaaattc
2041 tctggtgcaa gataaaaggt cttgggaaaa caaaacaaaa caaaacaaac ctcccttccc
2101 cagcagggct gctagcttgc tttctgcatt ttcaaaatga taatttacaa tggaaggaca
2161 agaatgtcat attctcaagg aaaaaaggta tatcacatgt ctcattctcc tcaaatattc
2221 catttgcaga cagaccgtca tattctaata gctcatgaaa tttgggcagc agggaggaaa
2281 gtccccagaa attaaaaaat ttaaaactct tatgtcaaga tgttgatttg aagctgttat
2341 aagaattggg attccagatt tgtaaaaaga cccccaatga ttctggacac tagatttttt
2401 gtttggggag gttggcttga acataaatga aatatcctgt attttcttag ggatacttgg
2461 ttagtaaatt ataatagtag aaataataca tgaatcccat tcacaggttt ctcagcccaa
2521 gcaacaaggt aattgcgtgc cattcagcac tgcaccagag cagacaacct atttgaggaa
2581 aaacagtgaa atccaccttc ctcttcacac tgagccctct ctgattcctc cgtgttgtga
2641 tgtgatgctg gccacgtttc caaacggcag ctccactggg tcccctttgg ttgtaggaca
2701 ggaaatgaaa cattaggagc tctgcttgga aaacagttca ctacttaggg atttttgttt
2761 cctaaaactt ttattttgag gagcagtagt tttctatgtt ttaatgacag aacttggcta
2821 atggaattca cagaggtgtt gcagcgtatc actgttatga tcctgtgttt agattatcca
2881 ctcatgcttc tcctattgta ctgcaggtgt accttaaaac tgttcccagt gtacttgaac
2941 agttgcattt ataagggggg aaatgtggtt taatggtgcc tgatatctca aagtcttttg
3001 tacataacat atatatatat atacatatat ataaatataa atataaatat atctcattgc
3061 agccagtgat ttagatttac agcttactct ggggttatct ctctgtctag agcattgttg
3121 tccttcactg cagtccagtt gggattattc caaaagtttt ttgagtcttg agcttgggct
3181 gtggccccgc tgtgatcata ccctgagcac gacgaagcaa cctcgtttct gaggaagaag
3241 cttgagttct gactcactga aatgcgtgtt gggttgaaga tatctttttt tcttttctgc
3301 ctcacccctt tgtctccaac ctccatttct gttcactttg tggagagggc attacttgtt
3361 cgttatagac atggacgtta agagatattc aaaactcaga agcatcagca atgtttctct
3421 tttcttagtt cattctgcag aatggaaacc catgcctatt agaaatgaca gtacttatta
3481 attgagtccc taaggaatat tcagcccact acatagatag cttttttttt tttttttttt
3541 ttttaataag gacacctctt tccaaacagg ccatcaaata tgttcttatc tcagacttac
3601 gttgttttaa aagtttggaa agatacacat cttttcatac ccccccttag gaggttgggc
3661 tttcatatca cctcagccaa ctgtggctct taatttattg cataatgata tccacatcag
3721 ccaactgtgg ctctttaatt tattgcataa tgatattcac atcccctcag ttgcagtgaa
3781 ttgtgagcaa aagatcttga aagcaaaaag cactaattag tttaaaatgt cacttttttg
3841 gtttttatta tacaaaaacc atgaagtact tttttatttt gctaaatcag attgttcctt
3901 tttagtgact catgtttatg aagagagttg agtttaacaa tcctagcttt taaaagaaac
3961 tatttaatgt aaaatattct acatgtcatt cagatattat gtatatcttc tagcctttat
4021 tctgtacttt taatgtacat atttctgtct tgcgtgattt gtatatttca ctggtttaaa
4081 aaacaaacat cgaaaggctt attccaaatg gaag
 
1 magsamsskf flvalaiffs faqvvieans wwslgmnnpv qmsevyiiga qplcsqlagl
61 sqgqkklchl yqdhmqyige gaktgikecq yqfrhrrwnc stvdntsvfg rvmqigsret
121 aftyavsaag vvnamsracr egelstcgcs raarpkdlpr dwlwggcgdn idygyrfake
181 fvdarereri hakgsyesar ilmnlhnnea grrtvynlad vackchgvsg scslktcwlq
241 ladfrkvgda lkekydsaaa mrlnsrgklv qvnsrfnspt tqdlvyidps pdycvrnest
301 gslgtqgrlc nktsegmdgc elmccgrgyd qfktvqterc hckfhwccyv kckkcteivd
361 qfvck
**Putative function** Wnt oncogene

### Example 22 (Category 3)

**Line ID** - 392
**Phenotype** - Lethal phase larval stage 3-pharate adult, small brain and optic lobes, high mitotic index, rod-like overcondensed chromosomes, fewer ana- and telophases, overcondensed chromosomes in ana- and telophase
**Annotated *Drosophila* genome genomic segment containing P element insertion site (and map position)** - AE003495 (12D)
**P element insertion site** - 35,688
**Annotated *Drosophila* genome Complete Genome candidate** CG12482 - novel protein 
ATGGGTTGCACCTGCTGTGACAATAAACCCAAGCCGGAGACCATTGAGAT
ATATTCGGTGAAAATCCGTGAGAATGGTACATACAAGTTGATCAAGATGC
AATTGGCGGATATTTGGAGTCACGGATGGGAGCTGCGTATCAATAACTTT
GCCGACAAGGAAAAGGTGCCGCACAACGAGAAGGATATTCGCAATCAGGT
GTCGGTGGCGCGCAAAGCCAAACAGAGTCTGTGGAACAATAATAAGCATT
TTGTGTACTGGTGCCGCTACGGAAGTCGTCAGCAGGATCTGCGAAAGCGA
CAGGTAACGACGAGTGCCAATCACGTGCTGCTGCACCTGATCAATTGA
 
MGCTCCDNKPKPETIEIYSVKIRENGTYKLIKMQLADIWSHGWELRINNF
ADKEKVPHNEKDIRNQVSVARKAKQSLWNNNKHFVYWCRYGSRQQDLRKR
QVTTSANHVLLHLIN
**Human homologue of Complete Genome candidate** none
**Putative function** unknown

### Example 23 (Category 3)

**Line ID** - 37
**Phenotype** - Lethal phase larval stage 3. Small brain, few cells in mitosis, badly defined chromosomes form a broad bend, weak chromosome condensation, abnormal anaphases with broken chromosomes
**Annotated *Drosophila* genome genomic segment containing P element insertion site (and map position) -** AE003418 (1C1-2)
**P element insertion site** - 105,970
**Annotated *Drosophila* genome Complete Genome candidate** CG16983 - skpA, SCF ubiquitin ligase subunit (3 splice variants) 
CCATTTGAAAGTATCGGTGTAATTTGTTTTCAGAGAAATTAATTTCCGTT
TACTGTGCAATTCGGTGTGAAAGTGTTCAGATTTATCAATGCGTATTCTG
CTTTCGACTTCGCCACCAATCTGTGCTGCAAGTTACCATTACCAGGTCCA
CCTGGTTCCCGCCAGTTTTCTTTCATTGTGGCTAGTTGTTGTTCGTGCCT
TCGATAAAGACGTTTAGAGGTGTTTTTAGAGTTTCGCCATCTGGTCACTA
TAGCCGTTTCGTTTTTTACATGCCCAGCATCAAGTTGCAATCTTCGGATG
AGGAGATCTTTGACACGGATATCCAGATCGCCAAGTGCTCCGGCACTATC
AAGACCATGCTGGAGGACTGCGGCATGGAGGACGATGAGAATGCCATTGT
GCCGTTGCCCAATGTGAATTCGACGATTCTTCGCAAGGTGCTTACCTGGG
CTCACTACCACAAGGACGACCCCCAGCCAACGGAGGATGATGAGAGCAAG
GAGAAGCGCACAGACGACATTATCTCATGGGATGCAGATTTCCTAAAAGT
CGACCAGGGCACACTGTTTGAGCTGATATTGGCAGCGAACTATCTGGACA
TTAAGGGCCTTCTGGAGCTCACCTGCAAGACTGTTGCAAACATGATTAAG
GGAAAGACTCCCGAGGAAATACGCAAGACCTTCAACATTAAGAAGGACTT
TTCGCCCGCCGAGGAGGAGCAGGTGCGCAAGGAGAACGAGTGGTGCGAGG
AGAAGTAAAGCGCGGCATTTCGCGGACCAACATTAAGTTGAAACAGCTA
GGGGATTCGGGAACGAATTGGATTTGCAGCATTGCAACTTTACTTAGTTG
CTACTTTCATTTACATTTTTTTTATTTTAACCCCAGCAGAGACTCGAT
TTAAATTGTGTATAAATGATCTGTTGCTGATTTGATTCGCGGGGTTCATT
TTTTGTCGTAAATATATCTCATATACATACATATGCGAGATTGTAACACT
CTCTTTAACCTATTGGAGTAACACTTGATTTCACTTTAATAAATATAACT
ACCCAACAC
 
MPSIKLQSSDEEIFDTDIQIAKCSGTIKTMLEDCGMEDDENAIVPLPNVN
STILRKVLTWAHYHKDDPQPTEDDESKEKRTDDIISWDADFLKVDQGTLF
ELILAANYLDIKGLLELTCKTVANMIKGKTPEEIRKTFNIKKDFSPAEEE
QVRKENEWCEEK
 
TTTCGCCATCTGGTCACTATAGCCGTTTCGTTTTTACGTGAGTATTGTG
AATTTGGTGTGTTGATTTATATCTCAGTTGGAGCCTGCGTGGAAATAGTG
TCAGTACGTTTAAAGGCATCATCGTAAGGAAAGCCCAAAATGCCCAGCAT
CAAGTTGCAATCTTCGGATGAGGAGATCTTTGACACGGATATCCAGATCG
 
CCAAGTGCTCCGGCACTATCAAGACCATGCTGGAGGACTGCGGCATGGAG
GACGATGAGAATGCCATTGTGCCGTTGCCCAATGTGAATTCGACGATTCT
TCGCAAGGTGCTTACCTGGGCTCACTACCACAAGGACGACCCCCAGCCAA
CGGAGGATGATGAGAGCAAGGAGAAGCGCACAGACGACATTATCTCATGG
GATGCAGATTTCCTAAAAGTCGACCAGGGCACACTGTTTGAGCTGATATT
GGCAGCGAACTATCTGGACATTAAGGGCCTTCTGGAGCTCACCTGCAAGA
CTGTTGCAAACATGATTAAGGGAAAGACTCCCGAGGAAATACGCAAGACC
TTCAACATTAAGAAGGACTTTTCGCCCGCCGAGGAGGAGCAGGTGCGCAA
GGAGAACGAGTGGTGCGAGGAGAAGTAAAGCGCGGCATTTCGCGGACCA
ACATTAAGTTGAAACAGCTAGGGGATTCGGGAACGAATTGGATTTGCAGC
ATTGCAACTTTACTTAGTTGCTACTTTCATTTACATTTTTTTTATTTTT
AACCCCAGCAGAGACTCGATTTAAATTGTGTATAAATGATCTGTTGCTGA
TTTGATTCGCGGGGTTCATTTTTTGTCGTAAATATATCTCATATACATAC
ATATGCGAGATTGTAACACTCTCTTTAACCTATTGGAGTAACACTTGATT
TCACTTTAATAAATATAACTACCCAACAC
 
MPSIKLQSSDEEIFDTDIQIAKCSGTIKTMLEDCGMEDDENAIVPLPNVN
STILRKVLTWAHYHKDDPQPTEDDESKEKRTDDIISWDADFLKVDQGTLF
ELILAANYLDIKGLLELTCKTVANMIKGKTPEEIRKTFNIKKDFSPAEEE
QVRKENEWCEEK
 
AAACATCGAAAGTGCACAATCGTTTGTTATCTTTGTACGAAAACAACGGT
GATTTCCACACAGGCATAACCTGCAAGAGAAAGCCCAAAATGCCCAGCAT
CAAGTTGCAATCTTCGGATGAGGAGATCTTTGACACGGATATCCAGATCG
CCAAGTGCTCCGGCACTATCAAGACCATGCTGGAGGACTGCGGCATGGAG
GACGATGAGAATGCCATTGTGCCGTTGCCCAATGTGAATTCGACGATTCT
TCGCAAGGTGCTTACCTGGGCTCACTACCACAAGGACGACCCCCAGCCAA
CGGAGGATGATGAGAGCAAGGAGAAGCGCACAGACGACATTATCTCATGG
GATGCAGATTTCCTAAAAGTCGACCAGGGCACACTGTTTGAGCTGATATT
GGCAGCGAACTATCTGGACATTAAGGGCCTTCTGGAGCTCACCTGCAAGA
CTGTTGCAAACATGATTAAGGGAAAGACTCCCGAGGAAATACGCAAGACC
TTCAACATTAAGAAGGACTTTTCGCCCGCCGAGGAGGAGCAGGTGCGCAA
GGAGAACGAGTGGTGCGAGGAGAAGTAAAGCGCGGCATTTCGCGGGACCA
ACATTAAGTTGAAACAGCTAGGGGATTCGGGAACGAATTGGATTTGCAGC
ATTGCAACTTTACTTAGTTGCTACTTTCATTTACATTTTTTTTTATTTTT
AACCCCAGCAGAGACTCGATTTAAATTGTGTATAAATGATCTGTTGCTGA
TTTGATTCGCGGGGTTCATTTTTTGTCGTAAATATATCTCATATACATAC
ATATGCGAGATTGTAACACTCTCTTTAACCTATTGGAGTAACACTTGATT
TCACTTTAATAAATATAACTACCCAACAC
 
MPSIKLQSSDEEIFDTDIQIAKCSGTIKTMLEDCGMEDDENAIVPLPNVN
STILRKVLTWAHYHKDDPQPTEDDESKEKRTDDIISWDADFLKVDQGTLF
ELILAANYLDIKGLLELTCKTVANMIKGKTPEEIRKTFNIKKDFSPAEEE
QVRKENEWCEEK
**Human homologue of Complete Genome candidate** XP_054159 - hypothetical protein 
1 gcctcccagc tctcgtcagc ctcctgctgg ccatctcctt aacaccaaac actatgcctt
61 caattcagtt gcagagtttt gatggagaga tatttgcagt tgatgtggaa attgccaaac
121 aatctgtgac tatcaagacc acgttggaag atttgggaat ggatgatgaa ggagatgacc
181 cagttcctct accaaatgtg aatgcagcag tattaaaaaa ggtcattcag tggtgcaccc
241 accacaagga tgaccctcct ccccctgaag atgatgagaa caaagaaaag caaacagacg
301 atatccctgt ttgggaccaa gaattcctga aagttgctca aggaacactt tttgaactca
361 ttcgggctgc aaactactta gacatcaaag gtttgcttga tgttacatgc aagactgttg
421 ccaatatgat caaggggaaa actcctgagg agattcgcaa gacattcaat atcaaaaatg
481 actttactga agaggaggaa gcccaggtac gcaaagagaa ccagtggtgt gaagagaagt
541 gaaatgttgt gcctgacact gtaacactgt aaggat

1 
61 
121 
**Putative function** Cell cycle protein, ubiquitin ligase

### Example 24 (Category 3)

**Line ID** - 186
**Phenotype** - Lethal phase larval stage 3. Small brain, high mitotic index, rod-like overcondensed chromosomes, fewer ana- and telophases.
**Annotated *Drosophila* genome genomic segment containing P element insertion site (and map position) -** AE003494 (12C6-7)
**P element insertion site** - 123,540
**Annotated *Drosophila* genome Complete Genome candidate** CG18319 - bendless ubiquitin conjugating enzyme 
TTAGTCACAGCAACGCACACACACACTACCAAACGGCTACATTTTTTTC
GAGTGTGTTCGACATTCATAATTTTGTGGTGGAGCTGCCTGCAAAATCG
AATTTATCAGTTTGCCAACGAAGTTATCGGCCATAACTGCAAATAAAGT
TCAGCAATAACTTGGCGCTGTTACGATCTCAACGAGAAGGTCCAGACTCA
ACCCGCGTTTCCAGTTCACCGCGTAAAAGGAACCAGCTAAACGATGTCCA
GCCTGCCACGTCGCATCATCAAGGAGACTCAACGTTTGATGCAGGAGCCA
GTGCCTGGGATCAATGCCATTCCCGATGAGAACAATGCCCGTTACTTCCA
TGTGATCGTGACCGGACCGAACGATTCGCCCTTCGAGGGCGGCGTGTTCA
AGCTGGAGCTGTTCCTACCGGAGGACTATCCAATGTCAGCGCCCAAAGTG
CGCTTCATCACGAAGATCTACCATCCGAACATCGATCGTTTGGGCCGCAT
TTGCCTCGACGTGCTGAAGGACAAGTGGAGTCCAGCCCTGCAGATCCGGA
CCATATTGCTATCCATTCAGGCACTGCTCAGTGCACCCAATCCCGACGAT
CCGCTGGCCAACGATGTGGCTGAGTTGTGGAAGGTCAACGAGGCGGAGGC
CATTCGCAATGCCCGCGAGTGGACCCAGAAATATGCCGTCGAAGACTGAA
CGCCCGAGGTCAGGAGGAAAGTCAGAAAGCGGATCCGTCAGTTGTATCGG
CGTTTTTCCAGAAAGTGGGTGCGTGACATGAACGGGCGGGTGGGTAAATT
GAATACTTTAAAAGCAACCAGAAAAACCTAAAACATACGAAAGAAAACAT
AAAATAAGAAAAAAGTAAGGAAGCAAACATAAAAAAAAACGATTTAAGAA
CACATTTTTTTTCGAACCTTCTGGGGCGGATATACATATAAAATATTA
ATATATATATTTTTTCAACCAATCGATCGGGGCGATCGGCGAAATGGAG
GAGAGATAGCGAAAGCATTCTTTATGTAAGACGTATACATGTATCCGAAA
CAAACTAAAAACGAAAAAAAAAAAAAAAAAAAAAAAACAGTAATTGGTTTT
AGTCGTTTCTATTGATTTGTTCGAGGGTTCTGGTGTCTATATACATATAG
CCGTATATAATTCTATGTGTAACTGAAATAACCAACCCATAACCATTAAC
ACATGTAGCATCAGATATGATAAATCAATTGGAAAGGCAAACAAGAAGGG
ATTTTGATTTCCTTTAACTCGTCATTTGAAAACTCGGCTTAAATGTCAAT
TCAAAATAGAGAATTTTGATTGTATCATTTTCAGTGTTTCAGAAAATTTA
AGATGTGATCGTCCAACTTGTAGACTTTACTTTTCTTAACTAAGAGTTCA
CCATTTCGATTGATACTTGAGCTTTGCCTGGGTTGTGTCAGAGTCCCTTT
GATAAACGATAAATAGTTTTACTCGAAAACAATTTTTTTAACCAAACA
ATGAAGCCTTTAAGCTATTAGTAATTTTGAAAAAAAAAAAAATAAAAAA
TATATATATAAAAAATATACAAAAATATGATACATGATCAAAATACAATG
AATGCATACACTATATATTTATACAAAAAAATACAAAAAGAAAAACAAA
AGTAGTGGCTTGATTGCGTGAAAATTTCAAGTGCAGTTCTCAACAAAAAT
TGTGTACAGTAATTAAATGTTTGTCACCGAAATCACTAAAGGATAATCCA
 
AAAAACAATAGCAACCGAAAAGCAACCATAAATCAAAGAGTAAGCGAAAA
TAAAAATTCAGTTTTCTTTAATTTTAATTAATTTTTTTCTAAGAAAAATA
AATAAAAACGAAAAATTCAAAT
 
MSSLPRRIIKETQRLMQEPVPGINAIPDENNARYFHVIVTGPNDSPFEGG
VFKLELFLPEDYPMSAPKVRFITKIYHPNIDRLGRICLDVLKDKWSPALQ
IRTILLSIQALLSAPNPDDPLANDVAELWKVNEAEAIRNAREWTQKYAVE
D
**Human homologue of Complete Genome candidate** BAA11675 - ubiquitin-conjugating enzyme E2 UbcH-ben 
1 actcgtgcgt gaggcgagag gagccggaga cgagaccaga ggccgaactc gggttctgac
61 aagatggccg ggctgccccg caggatcatc aaggaaaccc agcgtttgct ggcagaacca
121 gttcctggca tcaaagccga accagatgag agcaacgccc gttattttca tgtggtcatt
181 gctggccctc aggattcccc ctttgaggga gggactttta aacttgaact attccttcca
241 gaagaatacc caatggcagc ccctaaagta cgtttcatga ccaaaattta tcatcctaat
301 gtagacaagt tgggaagaat atgtttagat attttgaaag ataagtggtc cccagcactg
361 cagatccgca cagttctgct atcgatccag gccttgttaa gtgctcccaa tccagatgat
421 ccattagcaa atgatgtagc ggagcagtgg aagaccaacg aagcccaagc catagaaaca
481 gctagagcat ggactaggct atatgccatg aataatattt aaattgatac gatcatcaag
541 tgtgcatcac ttctcctgtt ctgccaagac ttcctcctct ttgtttgcat ttaatggaca
601 cagtcttaga aacattacag aataaaaaag cccagacatc ttcagtcctt tggtgattaa
661 atgcacatta gcaaatctat gtcttgtcct gattcactgt cataaagcat gagcagaggc
721 tagaagtatc atctggattg ttgtgaaacg tttaaaagca gtggcccctc cctgctttta
781 ttcatttccc ccatcctggt ttaagtataa agcactgtga atgaaggtag ttgtcaggtt
841 agctgcaggg gtgtgggtgt ttttatttta ttttatttta ttttattttt gaggggggag
901 gtagtttaat tttatgggct cctttccccc ttttttggtg atctaattgc attggttaaa
961 agcagctaac caggtcttta gaatatgctc tagccaagtc taactttatt tagacgctgt
1021 agatggacaa gcttgattgt tggaaccaaa atgggaacat taaacaaaca tcacagccct
1081 cactaataac attgctgtca agtgtagatt ccccccttca aaaaaagctt gtgaccattt
1141 tgtatggctt gtctggaaac ttctgtaaat cttatgtttt agtaaaatat tttttgttat
1201 tct
 
1 maglprriik etqrllaepv pgikaepdes naryfhvvia gpqdspfegg tfklelflpe
61 eypmaapkvr fmtkiyhpnv dklgricldi lkdkwspalq irtvllsiqa llsapnpddp
121 landvaeqwk tneaqaieta rawtrlyamn ni
**Putative function** Ubiquitin conjugating enzyme

### Example 25 (Category 3)

**Line ID** - 301
**Phenotype** - semilethal male and female, Low mitotic index, badly defined chromosomes, weak/uneven staining, fewer ana- and telophases
**Annotated *Drosophila* genome genomic segment containing P element insertion site (and map position) -** AE003422 (2B7-10)
**P element insertion site -** 96,307
**Annotated *Drosophila* genome Complete Genome candidate** CG14813 - deltaCOP, component of cotamer involved in retrograde (golgi to ER) transport 
TCGCAGAACCGAACACGTCAGCTACGGGGATTGATTGTTAAACAACGTTT
CTATCGCCCCGCAAATCCGATCCGTAGCAGCAGTCCATCCTGCGCCGTCC
GCATCCGATCCGCGAAGTATTTCCAGGGCAAAAACGTCAAACGCAGCAG
CAAAATGGTATTAATTGCTGCGGCTGTCTGCACGAAGAATGGCAAAGTGA
TTCTGTCACGTCAGTTCGTCGAGATGACGAAGGCACGCATCGAGGGACTG
CTGGCTGCCTTTCCCAAGCTGATGACTGCTGGCAAGCAGCACACTTACGT
GGAGACGGACTCCGTGCGCTACGTCTACCAGCCGATGGAGAAACTATATA
TGCTGCTCATCACCACTAAGGCCAGCAACATTCTGGAGGATCTGGAGACC
CTGCGCCTCTTCTCGAAAGTGATTCCCGAGTACAGCCACTCGCTCGACGA
GAAGGAGATTGTGGAGAATGCCTTCAATCTGATCTTCGCATTTGACGAGA
TCGTGGCACTCGGCTACAGGGAGAGCGTCAACTTGGCCCAGATCAAGACC
TTCGTGGAGATGGACTCACATGAGGAGAAGGTCTACCAGGCAGTGCGTCA
GACGCAGGAGCGTGATGCGCGCCAGAAGATGCGCGAGAAGGCCAAGGAAC
TGCAGCGGCAGCGCATGGAGGCCAGCAAACGGGGTGGTCCCTCCCTGGGT
GGCATTGGCAGCCGCAGCGGCGGCTTTAGCGCCGACGGAATTGGCAGTAG
CGGCGTGAGCAGCAGTTCCGGTGCCTCCAGCGCCAACACCGGCATCACCT
CCATCGATGTGGACACCAAATCCAAGGCGGCTGCCAGTAAACCAGCTTCC
CGCAATGCCCTCAAGCTAGGTGGCAAGTCCAAGGACGTCGATAGTTTCGT
GGATCAGCTGAAGAACGAGGGCGAGAAGATTGCCAATCTGGCACCGGCGG
CGCCCGCTGGAGGTTCCAGTGCTGCAGCTAGCGCCAGTGCAGCGGCCAAG
GCAGCTATCGCGTCGGACATTCACAAAGAGAGCGTACATCTGAAGATTGA
GGACAAGCTAGTAGTGCGTCTGGGACGCGATGGTGGCGTGCAGCAGTTCG
AGAACTCGGGCCTCCTGACGTTGCGCATTACGGACGAGGCCTACGGACGC
ATTTTGCTGAAGCTGTCTCCCAACCACACACAGGGCCTGCAGTTGCAGAC
CCACCCCAACGTGGACAAGGAGCTGTTCAAGTCGCGCACTACCATCGGAC
TAAAGAACTTGGGCAAGCCGTTTCCCCTTAACACCGATGTGGGTGTGCTC
AAGTGGCGCTTCGTCTCGCAGGACGAGTCGGCAGTCCCGCTGACCATTAA
CTGCTGGCCATCGGATAATGGAGAGGGTGGATGCGATGTTAACATTGAGT
ATGAACTGGAGGCGCAGCAGCTAGAGCTGCAGGACGTGGCCATTGTCATT
CCCTTGCCAATGAATGTGCAGCCTTCGGTGGCGGAGTACGACGGCACCTA
CAACTACGATTCACGCAAGCATGTGCTCCAGTGGCACATTCCAATAATCG
ATGCCGCCAACAAGTCCGGTTCTATGGAGTTCAGCTGCAGTGCCTCCATT
CCCGGTGACTTCTTCCCCTTGCAGGTGTCCTTCGTCTCGAAAACGCCGTA
TGCGGGCGTCGTGGCCCAGGATGTGGTGCAGGTGGACAGCGAGGCGGCGG
 
TCAAGTATTCAAGCGAGTCCATTCTGTTCGTGGAAAAGTACGAGATCGTG
TAGGCCGCGCCGCTGGCCACGCCCACCTAAGTAGTACATAAATATACATA
ATTTCCCGGGGTCATCCGATGCGATGCAATTAATTCAACTGCTGCAGCAT
GTTGAGAATTATTTTTCCATGTGCGAACTTTACATATTTATGGCGCAGAC
AGCTTCTCAGAGCGAGTAATTGATTCC
 
MVLIAAAVCTKNGKVILSRQFVEMTKARIEGLLAAFPKLMTAGKQHTYVE
TDSVRYVYQPMEKLYMLLITTKASNILEDLETLRLFSKVIPEYSHSLDEK
EIVENAFNLIFAFDEIVALGYRESVNLAQIKTFVEMDSHEEKVYQAVRQT
QERDARQKMREKAKELQRQRMEASKRGGPSLGGIGSRSGGFSADGIGSSG
VSSSSGASSANTGITSIDVDTKSKAAASKPASRNALKLGGKSKDVDSFVD
QLKNEGEKIANLAPAAPAGGSSAAASASAAAKAAIASDIHKESVHLKIED
KLVVRLGRDGGVQQFENSGLLTLRITDEAYGRILLKLSPNHTQGLQLQTH
PNVDKELFKSRTTIGLKNLGKPFPLNTDVGVLKWRFVSQDESAVPLTINC
WPSDNGEGGCDVNIEYELEAQQLELQDVAIVIPLPMNVQPSVAEYDGTYN
YDSRKHVLQWHIPIIDAANKSGSMEFSCSASIPGDFFPLQVSFVSKTPYA
GVVAQDVVQVDSEAAVKYSSESILFVEKYEIV
**Human homologue of Complete Genome candidate** CAA57071 - archain, possible role in vesicle structure or trafficking 
1 cgggcggttc ctgtcaaggg ggcagcaggt ccagagctgc tggtgctccc gttccccaga
61 ccctacccct atccccagtg gagccggagt gcggcgcgcc ccaccaccgc cctcaccatg
121 gtgctgttgg cagcagcggt ctgcacaaaa gcaggaaagg ctattgtttc tcgacagttt
181 gtggaaatga cccgaactcg gattgagggc ttattagcag cttttccaaa gctcatgaac
241 actggaaaac aacatacgtt tgttgaaaca gagagtgtaa gatatgtcta ccagcctatg
301 gagaaactgt atatggtact gatcactacc aaaaacagca acattttaga agatttggag
361 accctaaggc tcttctcaag agtgatccct gaatattgcc gagccttaga agagaatgaa
421 atatctgagc actgttttga tttgattttt gcttttgatg aaattgtcgc actgggatac
481 cgggagaatg ttaacttggc acagatcaga accttcacag aaatggattc tcatgaggag
541 aaggtgttca gagccgtcag agagactcaa gaacgtgaag ctaaggctga gatgcgtcgt
601 aaagcaaagg aattacaaca ggcccgaaga gatgcagaga gacagggcaa aaaagcacca
661 ggatttggcg gatttggcag ctctgcagta tctggaggca gcacagctgc catgatcaca
721 gagaccatca ttgaaactga taaaccaaaa gtggcacctg caccagccag gccttcaggc
781 cccagcaagg ctttaaaact tggagccaaa ggaaaggaag tagataactt tgtggacaaa
841 ttaaaatctg aaggtgaaac catcatgtcc tctagtatgg gcaagcgtac ttctgaagca
901 accaaaatgc atgctccacc cattaatatg gaaagtgtac atatgaagat tgaagaaaag
961 ataacattaa cctgtggacg agacggagga ttacagaata tggagttgca tggcatgatc
1021 atgcttagga tctcagatga caagtatggc cgaattcgtc ttcatgtgga aaatgaagat
1081 aagaaagggg tgcagctaca gacccatcca aatgtggata aaaaactttt cactgcagag
1141 tctctaattg gcctgaagaa tccagagaag tcatttccag tcaacagtga cgtaggggtg
1201 ctaaagtgga gactacaaac cacagaggaa tcttttattc cactgacaat taattgctgg
1261 ccctcggaga gtggaaatgg ctgtgatgtc aacatagaat atgagctaca agaagataat
1321 ttagaactga atgatgtggt tatcaccatc ccactcccgt ctggtgtcgg cgcgcctgtt
1381 atcggtgaga tcgatgggga gtatcgacat gacagtcgac gaaataccct ggagtggtgc
1441 ctgcctgtga ttgatgccaa aaataagagt ggcagcctgg agtttagcat tgctgggcag
 
1501 cccaatgact tcttccctgt tcaagtttcc tttgtctcca agaaaaatta ctgtaacata
1561 caggttacca aagtgaccca ggtagatgga aacagccccg tcaggttttc cacagagacc
1621 actttcctag tggataagta tgaaatcctg taataccaag aagagggagc tgaaaaggaa
1681 aattttcaga ttaataaaga agacgccaat gatggctgaa gagtttttcc cagatttaca
1741 agccactgga gacccctttt ttctgataca atgcacgatt ctctgcgcgc aaggaccctc
1801 gactcacccc catgtttcag tgtcacagag acattctttg ataaggaaat ggcacaaaca
1861 taaagggaaa ggctgctaat tttctttggc agattgtatt ggccagcagg aaagcaagct
1921 ctccagagaa tgcccccagt taaatacctc ctctaccttt acctaagttg ctcctttatt
1981 tttattttat aataataa
 
1 mvllaaavct kagkaivsrq fvemtrtrie gllaafpklm ntgkqhtfve tesvryvyqp
61 meklymvlit tknsniledl etlrlfsrvi peycraleen eisehcfdli fafdeivalg
121 yrenvnlaqi rtftemdshe ekvfravret qereakaemr rkakelqqar rdaerqgkka
181 pgfggfgssa vsggstaami tetiietdkp kvapaparps gpskalklga kgkevdnfvd
241 klksegetim sssmgkrtse atkmhappin mesvhmkiee kitltcgrdg glqnmelhgm
301 imlrisddky grirlhvene dkkgvqlqth pnvdkklfta esliglknpe ksfpvnsdvg
361 vlkwrlqtte esfipltinc wpsesgngcd vnieyelqed nlelndvvit iplpsgvgap
421 vigeidgeyr hdsrrntlew clpvidaknk sgslefsiag qpndffpvqv sfvskknycn
481 iqvtkvtqvd gnspvrfste ttflvdkyei l
**Putative function** Role in vesicle trafficking

### Example 26 (Category 3)

**Line ID** - 148
**Phenotype** - Lethal phase pupal to pharate adult. Lagging chromosomes and bridges in ana- and telophase
**Annotated *Drosophila* genome genomic segment containing P element insertion site (and map position) -** AE003438 (6B-C)
**P element insertion site** - 116,914
**Annotated *Drosophila* genome Complete Genome candidate** CG8655 - cdc7 kinase 
ATGCGTTATGACGCCTCCGCCGCTTTCGTGATGCCCTTCATGGCACATGA
CCGATTCCAGGACTTTACACGCGCATGGATGTGCCCGAGATCCGGCAGT
ATATGCGCAATCTCCTGGTGGCACTGCGTCATGTCCACAAGTTCGATGTC
ATCCATCGCGACGTGAAGCCGAGCAACTTTCTCTACAATCGACGTCGGCG
AGAGTTTCTCCTCGTCGATTTCGGTCTGGCCCAGCATGTGAATCCTCCGG
CTGCGCGATCTTCCGGAAGTGCCGCCGCCATCGCCGCAGCCAACAACAAA
AACAACAACAATAATAACAATAATAATAGCAAACGGCCACGAGAGCGCGA
ATCAAAGGGGGATGTGCAGCAAATTGCGCTGGATGCTGGTTTGGGTGGAG
CAGTGAAGCGTATGCGTTTGCACGAGGAGTCCAACAAGATGCCCCTGAAA
CCGGTCAACGATATTGCGCCAAGCGATGCGCCGGAGCAGTCAGTAGATGG
GTCCAATCACGTCCAGCCACAGCTAGTGCAGCAAGAGCAGCAACAACTGC
AGCCGCAACAGCAGCAGCAACAACAGCAGCAGCAACAACAGTCGCAACAG
CAGCAGCAGCCGCAGCAGCAGTCGCAACAGCAGCACCCACAACGACAGCC
ACAACTGGCGCAGATGGATCAAACAGCATCGACGCCATCTGGCAGCAAGT
ACAATACGAATCGAAATGTCTCGGCAGCAGCGGCTAATAATGCCAAGTGC
GTTTGCTTTGCAAATCCCTCAGTTTGCCTCAACTGTCTGATGAAGAAGGA
GGTGCACGCCTCCAGGGCAGGAACACCTGGCTATCGGCCGCCCGAGGTTC
TGCTCAAGTACCCAGATCAGACCACTGCCGTGGACGTTTGGGCGGCGGGT
GTGATATTCCTTTCGATCATGTCAACGGTGTATCCGTTTTTCAAAGCGCC
CAACGATTTTATCGCGCTGGCCGAGATTGTAACAATATTTGGAGATCAGG
CGATACGGAAGACGGCCTTGGCTCTCGACCGTATGATCACCCTGAGCCAG
AGGTCCAGGCCACTGAATCTGCGAAAGTTGTGCCTGCGCTTTCGCTATCG
TTCCGTTTTTAGTGATGCCAAGCTCCTCAAGAGCTACGAATCTGTGGACG
GAAGCTGCGAAGTGTGCCGGAATTGTGATCAATACTTCTTCAACTGCCTA
TGCGAGGATAGCGATTACTTGACAGAGCCACTGGACGCATACGAATGTTT
TCCACCCAGCGCCTATGACCTACTGGATCGCCTGCTCGAGATTAATCCCC
ATAAACGAATTACCGCCGAAGAGGCACTAAAGCATCCATTCTTTACGGCC
GCCGAGGAGGCCGAGCAGACGGAGCAGGATCAGTTGGCCAATGGAACGCC
GCGCAAGATGCGTCGACAAAGATATCAAAGTCACAGAACGGTGGCCGCCT
CACAGGAGCAGGTCAAGCAGCAGGTTGCCCTTGATCTGCAGCAAGCGGCC
ATTAACAAGCTGTGA
 
MRYDASAAFVMPFMAHDRFQDFYTRMDVPEIRQYMRNLLVALRHVHKFDV
IHRDVKPSNFLYNRRRREFLLVDFGLAQHVNPPAARSSGSAAAIAAANNK
NNNNNNNNNSKRPRERESKGDVQQIALDAGLGGAVKRMRLHEESNKMPLK
 
PVNDIAPSDAPEQSVDGSNHVQPQLVQQEQQQLQPQQQQQQQQQQQSQQ
QQQPQQQSQQQHPQRQPQLAQMDQTASTPSGSKYNTNRNVSAAAANNAKC
VCFANPSVCLNCLMKKEVHASRAGTPGYRPPEVLLKYPDQTTAVDVWAAG
VIFLSIMSTVYPFFKAPNDFIALAEIVTIFGDQAIRKTALALDRMITLSQ
RSRPLNLRKLCLRFRYRSVFSDAKLLKSYESVDGSCEVCRNCDQYFFNCL
CEDSDYLTEPLDAYECFPPSAYDLLDRLLEINPHKRITAEEALKHPFFTA
AEEAEQTEQDQLANGTPRKMRRQRYQSHRTVAASQEQVKQQVALDLQQAA
INKL
**Human homologue of Complete Genome candidate** AAB97512 - HsCdc7 
1 atggaggcgt ctttggggat tcagatggat gagccaatgg ctttttctcc ccagcgtgac
61 cggtttcagg ctgaaggctc tttaaaaaaa aacgagcaga attttaaact tgcaggtgtt
121 aaaaaagata ttgagaagct ttatgaagct gtaccacagc ttagtaatgt gtttaagatt
181 gaggacaaaa ttggagaagg cactttcagc tctgtttatt tggccacagc acagttacaa
241 gtaggacctg aagagaaaat tgctgtaaaa cacttgattc caacaagtca tcctataaga
301 attgcagctg aacttcagtg cctaacagtg gctggggggc aagataatgt catgggagtt
361 aaatactgct ttaggaagaa tgatcatgta gttattgcta tgccatatct ggagcatgag
421 tcgtttttgg acattctgaa ttctctttcc tttcaagaag tacgggaata tatgcttaat
481 ctgttcaaag ctttgaaacg cattcatcag tttggtattg ttcaccgtga tgttaagccc
541 agcaattttt tatataatag gcgcctgaaa aagtatgcct tggtagactt tggtttggcc
601 caaggaaccc atgatacgaa aatagagctt cttaaatttg tccagtctga agctcagcag
661 gaaaggtgtt cacaaaacaa atcccacata atcacaggaa acaagattcc actgagtggc
721 ccagtaccta aggagctgga tcagcagtcc accacaaaag cttctgttaa aagaccctac
781 acaaatgcac aaattcagat taaacaagga aaagacggaa aggagggatc tgtaggcctt
841 tctgtccagc gctctgtttt tggagaaaga aatttcaata tacacagctc catttcacat
901 gagagccctg cagtgaaact catgaagcag tcaaagactg tggatgtact gtctagaaag
961 ttagcaacaa aaaagaaggc tatttctacg aaagttatga atagtgctgt gatgaggaaa
1021 actgccagtt cttgcccagc tagcctgacc tgtgactgct atgcaacaga taaagtttgt
1081 agtatttgcc tttcaaggcg tcagcaggtt gcccctaggg caggtacacc aggattcaga
1141 gcaccagagg tcttgacaaa gtgccccaat caaactacag caattgacat gtggtctgca
1201 ggtgtcatat ttctttcttt gcttagtgga cgatatccat tttataaagc aagtgatgat
1261 ttaactgctt tggcccaaat tatgacaatt aggggatcca gagaaactat ccaagctgct
1321 aaaacttttg ggaaatcaat attatgtagc aaagaagttc cagcacaaga cttgagaaaa
1381 ctctgtgaga gactcagggg tatggattct agcactccca agttaacaag tgatatacag
1441 gggcatgctt ctcatcaacc agctatttca gagaagactg accataaagc ttcttgcctc
1501 gttcaaacac ctccaggaca atactcaggg aattcattta aaaaggggga tagtaatagc
1561 tgtgagcatt gttttgatga gtataatacc aatttagaag gctggaatga ggtacctgat
1621 gaagcttatg acctgcttga taaacttcta gatctaaatc cagcttcaag aataacagca
1681 gaagaagctt tgttgcatcc attttttaaa gatatgagct tgtga
 
1 
61 
121 
181 
 
241 
301 
361 
421 
481 
541 
**Putative function** Protein kinase which regulates the G1/S phase transition and/or DNA replication in mammalian cells.

### Example 27 (Category 3)

**Line ID** - 335
**Phenotype** - Lethal phase, pupal. Uneven chromosome condensation, lagging chromosomes in anaphase
**Annotated *Drosophila* genome genomic segment containing P element insertion site (and map position) -** AE003424 (3B1-2)
**P element insertion site** - 286,560
**Annotated *Drosophila* genome Complete Genome candidate** CG2621- shaggy, protein serine/threonine kinase 
ATGTTTACCTTCTACACCAATATAAATAATACACTGATCAACAACAACAA
TAATAATAATAATACTAGTAACAGTAATAATAATAATAACAACGTTATAA
GCCAGCCGATTAAAATACCGCTAACCGAGCGCTTCTCATCGCAAACATCG
ACGGGCTCGGCGGATAGCGGTGTAATTGTTTCCAGTGCATCGCAGCAGCA
ACTGCAGTTGCCACCACCACGCAGTAGCAGTGGATCGCTGAGTCTGCCAC
AAGCGCCACCTGGCGGCAAGTGGCGGCAGAAGCAGCAGCGCCAACAGTTG
CTGCTCAGCCAGGACAGCGGCATCGAAAATGGTGTCACCACTCGTCCATC
GAAAGCCAAGGACAACCAGGGTGCGGAAAAGCCAGTCACAATGCCACAA
GCTCGAAGGAGAGCGGCGCGCAGTCGAACAGCAGCAGCGAGAGCCTGGGC
AGCAATTGCTCCGAGGCCCAGGAGCAGCAGAGAGTAAGAGCCTCCTCCGC
TCTGGAGCTCAGCAGCGTGGACACTCCCGTGATCGTCGGCGGTGTGGTCA
GTGGAGGCAACAGCATCTTGCGCAGCCGCATTAAGTACAAGAGTACGAAC
AGCACCGGAACCCAGGGATTCGATGTGGAGGATCGCATCGATGAGGTGGA
TATCTGTGATGATGATGATGTCGACTGCGATGATCGCGGATCGGAGATCG
AGGAGGAGGAGGAGGACCAAACCGAACAAGAGGAGGAGGTCGATGAGGTG
GATGCCAAGCCGAAGAACCGACTTTTGCCACCGGATCAGGCGGAACTCAC
AGTGGCGGCGGCCATGGCACGTCGACGCGATGCCAAGAGCCTGGCCACCG
ACGGTCACATATATTTCCCACTGCTCAAGATCAGCGAGGATCCGCACATT
GATTCGAAGCTGATCAATCGCAAGGATGGCCTCCAGGACACCATGTATTA
TTTGGACGAATTCGGCAGTCCAAAGTTGCGAGAGAAGTTCGCCCGCAAGC
AGAAGCAGCTGCTCGCCAAGCAGCAGAAGCAGTTGATGAAACGTGAAAGG
AGGAGCGAGGAGCAGCGCAAGAAGCGAAACACCACCGTGGCATCCAACTT
GGCGGCCAGCGGAGCGGTGGTGGACGACACCAAAGATGATTACAAACAAC
AACCACACTGTGATACTAGCTCTAGGAGCAAAAATAACTCGGTACCCAAT
CCACCCAGCAGCCATCTCCATCAGAACCACAATCATCTCGTTGTGGATGT
GCAAGAGGATGTGGATGATGTGAATGTGGTTGCCACCAGCGACGTGGACA
GTGGTGTCGTCAAGATGCGCCGCCATAGCCACGATAACCACTACGACCGA
ATTCCCCGGAGCAATGCTGCCACCATTACCACCCGCCCTCAAATCGACCA
ACAGTCGTCGCACCACCAGAACACCGAGGATGTGGAGCAAGGAGCTGAGC
CCCAAATCGATGGCGAAGCGGATCTGGATGCGGATGCGGATGCGGACAGC
GATGGGAGTGGCGAGAACGTTAAGACTGCCAAATTGGCCAGAACACAGTC
CTGCAAAAACCAAACAGGTCGCGATGGTTCTAAAATCACAACAGTTGTTG
CAACACCCGGCCAAGGCACCGATCGCGTACAAGAGGTCTCCTATACAGAC
ACAAAGGTCATCGGCAATGGCAGCTTCGGCGTCGTGTTCCAGGCAAAGCT
CTGCGATACCGGCGAACTGGTGGCAATCAAAAAAGTTTTACAAGACAGAC
 
GATTTAAGAATCGCGAATTGCAAATAATGCGCAAATTGGAGCATTGTAAT
ATTGTGAAGCTTTTGTACTTTTTCTATTCGAGTGGTGAAAAGCGTGATGA
AGTATTTTTGAATTTAGTCCTCGAATATATACCAGAAACCGTATACAAAG
TGGCTCGCCAATATGCCAAAACCAAGCAAACGATACCAATCAACTTTATT
CGGCTCTACATGTATCAACTGTTCAGAAGTTTGGCCTACATCCACTCGCT
GGGCATTTGCCATCGTGATATCAAGCCGCAGAATCTTCTGCTCGATCCGG
AGACGGCTGTGCTGAAGCTCTGTGACTTTGGCAGCGCCAAACAGCTGCTG
CACGGCGAGCCGAATGTATCGTATATCTGCTCCCGGTATTACCGCGCCCC
CGAGCTCATCTTTGGCGCCATCAATTATACAACAAAGATCGATGTCTGGA
GTGCCGGTTGCGTTTTGGCCGAACTGCTGCTGGGCCAGCCCATCTTCCCT
GGCGATTCCGGTGTGGATCAGCTCGTCGAGGTCATCAAGGTCCTGGGCAC
ACCGACAAGAGAACAGATACGCGAAATGAATCCAAACTACACGGAATTCA
AGTTCCCTCAGATTAAGAGTCATCCATGGCAGAAAGTTTTCCGTATACGC
ACTCCTACAGAAGCTATCAACTTGGTGTCCCTGCTGCTCGAGTATACGCC
CAGTGCCAGGATCACACCGCTCAAGGCCTGCGCACATCCGTTCTTCGATG
AGCTACGCATGGAGGGTAATCACACCTTGCCCAACGGTCGCGATATGCCG
CCGCTGTTCAACTTCACAGAGCATGAGCTCTCAATACAGCCCAGCCTAGT
GCCGCAGTTGTTGCCCAAGCATCTGCAGAACGCATCCGGACCTGGCGGCA
ATCGACCCTCGGCCGGCGGAGCAGCCTCCATTGCGGCCAGCGGCTCCACC
AGCGTCTCGTCAACGGGCAGTGGTGCCTCGGTGGAAGGATCCGCCCAGCC
ACAGTCGCAGGGTACAGCAGCAGCTGCGGGATCCGGATCGGGCGGAGCAA
CAGCAGGAACCGGCGGAGCGAGTGCCGGTGGACCCGGATCTGGTAACAAC
AGTAGCAGCGGCGGAGCATCGGGAGCGCCGTCCGCTGTGGCTGCCGGAGG
AGCCAATGCCGCCGTCGCTGGCGGTGCTGGTGGTGGTGGCGGAGCCGGTG
CGGCGACCGCAGCTGCAACAGCAACTGGCGCTATAGGCGCGACTAATGCC
GGCGGCGCCAATGTAACAGATTCATAGGGGAAATAGTAACATACATACAC
ACACTAAATATATATCCAAGCATATATATATAGTAATCATTATATATAAC
ACCTACACCCACAACAACAACAACAGCAATTATATATAATAACCATAAAC
AAGAATGGAGAAAGCCAATCCAGCAATCACAGCAAACTATATACACAACA
ACAACAATTAAATTAATTAATGCAATTGATGAAAGAACAGCAGCAGCAGC
AGCAGCAGCAGCAGCAGCAGCATCAACCGCAATTTCAAAAGAACTCTAGA
AACAGCAAAGGCATAAAATATAACAAAAGAAATATTTTACTTAGGTAAAA
CATTAAATTTATTTTAAATCTAAAATAAACTAATAAGCATTAAATAATAC
ATGATAATGGTAAATAAACACACAATAATTATAATAGTAGAGCGAGCGCT
GATCGATTGTCATTTTATTGCTGCCGC
 
MFTFYTNINNTLINNNNNNNNTSNSNNNNNNVISQPIKIPLTERFSSQTS
TGSADSGVIVSSASQQQLQLPPPRSSSGSLSLPQAPPGGKWRQKQQRQQL
LLSQDSGIENGVTTRPSKAKDNQGAGKASHNATSSKESGAQSNSSSESLG
SNCSEAQEQQRVRASSALELSSVDTPVIVGGVVSGGNSILRSRIKYKSTN
STGTQGFDVEDRIDEVDICDDDDVDCDDRGSEIEEEEEDQTEQEEEVDEV
DAKPKNRLLPPDQAELTVAAAMARRRDAKSLATDGHIYFPLLKISEDPHI
DSKLINRKDGLQDTMYYLDEFGSPKLREKFARKQKQLLAKQQKQLMKRER
RSEEQRKKRNTTVASNLAASGAVVDDTKDDYKQQPHCDTSSRSKNNSVPN
PPSSHLHQNHNHLVVDVQEDVDDVNVVATSDVDSGVVKMRRHSHDNHYDR
IPRSNAATITTRPQIDQQSSHHQNTEDVEQGAEPQIDGEADLDADADADS
DGSGENVKTAKLARTQSCKNQTGRDGSKITTVVATPGQGTDRVQEVSYTD
TKVIGNGSFGVVFQAKLCDTGELVAIKKVLQDRRFKNRELQIMRKLEHCN
 
IVKLLYFFYSSGEKRDEVFLNLVLEYIPETVYKVARQYAKTKQTIPINFI
RLYMYQLFRSLAYIHSLGICHRDIKPQNLLLDPETAVLKLCDFGSAKQLL
HGEPNVSYICSRYYRAPELIFGAINYTTKIDVWSAGCVLAELLLGQPIFP
GDSGVDQLVEVIKVLGTPTREQIREMNPNYTEFKFPQIKSHPWQKVFRIR
TPTEAINLVSLLLEYTPSARITPLKACAHPFFDELRMEGNHTLPNGRDMP
PLFNFTEHELSIQPSLVPQLLPKHLQNASGPGGNRPSAGGAASIAASGST
SVSSTGSGASVEGSAQPQSQGTAAAAGSGSGGATAGTGGASAGGPGSGNN
SSSGGASGAPSAVAAGGANAAVAGGAGGGGGAGAATAAATATGAIGATNA
GGANVTDS
**Human homologue of Complete Genome candidate** NP_002084 - glycogen synthase kinase 3 beta 
1 ggagaaggaa ggaaaaggtg attcgcgaag agagtgatca tgtcagggcg gcccagaacc
61 acctcctttg cggagagctg caagccggtg cagcagcctt cagcttttgg cagcatgaaa
121 gttagcagag acaaggacgg cagcaaggtg acaacagtgg tggcaactcc tgggcagggt
181 ccagacaggc cacaagaagt cagctataca gacactaaag tgattggaaa tggatcattt
241 ggtgtggtat atcaagccaa actttgtgat tcaggagaac tggtcgccat caagaaagta
301 ttgcaggaca agagatttaa gaatcgagag ctccagatca tgagaaagct agatcactgt
361 aacatagtcc gattgcgtta tttcttctac tccagtggtg agaagaaaga tgaggtctat
421 cttaatctgg tgctggacta tgttccggaa acagtataca gagttgccag acactatagt
481 cgagccaaac agacgctccc tgtgatttat gtcaagttgt atatgtatca gctgttccga
541 agtttagcct atatccattc ctttggaatc tgccatcggg atattaaacc gcagaacctc
601 ttgttggatc ctgatactgc tgtattaaaa ctctgtgact ttggaagtgc aaagcagctg
661 gtccgaggag aacccaatgt ttcgtatatc tgttctcggt actatagggc accagagttg
721 atctttggag ccactgatta tacctctagt atagatgtat ggtctgctgg ctgtgtgttg
781 gctgagctgt tactaggaca accaatattt ccaggggata gtggtgtgga tcagttggta
841 gaaataatca aggtcctggg aactccaaca agggagcaaa tcagagaaat gaacccaaac
901 tacacagaat ttaaattccc tcaaattaag gcacatcctt ggactaaggt cttccgaccc
961 cgaactccac cggaggcaat tgcactgtgt agccgtctgc tggagtatac accaactgcc
1021 cgactaacac cactggaagc ttgtgcacat tcattttttg atgaattacg ggacccaaat
1081 gtcaaacatc caaatgggcg agacacacct gcactcttca acttcaccac tcaagaactg
1141 tcaagtaatc cacctctggc taccatcctt attcctcctc atgctcggat tcaagcagct
1201 gcttcaaccc ccacaaatgc cacagcagcg tcagatgcta atactggaga ccgtggacag
1261 accaataatg ctgcttctgc atcagcttcc aactccacct gaacagtccc gacgagccag
1321 ctgcacagga aaaaccacca gttacttgag tgtcactcag caacactggt cacgtttgga
1381 aagaatatt
 
1 msgrprttsf aesckpvqqp safgsmkvsr dkdgskvttv vatpgqgpdr pqevsytdtk
61 vigngsfgvv yqaklcdsge lvaikkvlqd krfknrelqi mrkldhcniv rlryffyssg
121 ekkdevylnl vldyvpetvy rvarhysrak qtlpviyvkl ymyqlfrsla yihsfgichr
181 dikpqnllld pdtavlklcd fgsakqlvrg epnvsyicsr yyrapelifg atdytssidv
241 wsagcvlael llgqpifpgd sgvdqlveii kvlgtptreq iremnpnyte fkfpqikahp
301 wtkvfrprtp peaialcsrl leytptarlt pleacahsff delrdpnvkh pngrdtpalf
361 nfttqelssn pplatilipp hariqaaast ptnataasda ntgdrgqtnn aasasasnst
421
**Putative function** Serine/threonine kinase involved in winglwess signaling pathway

### Example 28 (Category 3)

Dlg1 (CG1725) as a candidate gene is detected in a screen of a P-element insertion library covering the X chromosome of *Drosophila melanogaster* (Peter et al. 2001) as mutant phenotype in fly line 342 , as described above.

Mitotic defects are observed in brain squashes: high mitotic index, overcondensed chromosomes, lagging chromosomes and a high proportion of anaphases and telophases compared to normal brains.

Rescue and sequencing of genomic DNA flanking the P-element insertion site indicates that the P-element is inserted into the 5' region of gene Dlg1 (CG1725).
**Line ID** - 342
**Phenotype** - Lethal phase pupal. Higher mitotic index, colchicine-like overcondensed chromosomes, many ana- and telophases, lagging chromosomes
**Annotated *Drosophila* genome genomic segment containing P element insertion site (and map position) -** AE003486 (10B8-10)
**P element insertion site** - 1128 and 3755
**Annotated *Drosophila* genome Complete Genome candidate** CG1725 - dlg, membrane-associated guanylate kinase homologs, role in cell junctions and proliferation (version 1) 
CACAAACAACACGCTCGTGCGTGCGATTTAAATATATAGATGTTTCAAAA
GTCAACCTCTCTGTTCGCAATTGTGTGCATTTTCGTTTGTCTAGTGCAAA
AAGTTGGATAATCACAGGCGGCAAATAAAATAGTAACGAATCGAGTTCAA
GAAGAAGAAGAGAAGAGGAAGCAGAGGCAGCAGCGCCGGCATTTGTC
CGTGTGTTGTTGTTGTTGTTTGTGCGCGGCTGTAACTTTAACCCTCGAAC
GCCATAAGATTAAAAAACCAAGTATAACAATAAGTTATAAAATCAATTAA
ACAAAAGCCGCTGCGATATGACAACGAGGAAAAAGAAGCGCGACGGCGGC
GGCAGCGGCGGCGGATTCATCAAGAAAGTTTCGTCACTCTTCAATCTGGA
TTCGGTGAATGGCGATGATAGCTGGTTATACGAGGACATTCAGCTGGAGC
GCGGCAACTCCGGATTGGGCTTTTCCATTGCCGGCGGTACGGATAATCCG
CACATCGGCACCGACACCTCCATCTACATCACCAAGCTCATTTCCGGTGG
AGCAGCTGCCGCCGATGGACGTCTGAGCATCAACGATATCATCGTATCGG
TGAACGATGTGTCCGTGGTGGATGTGCCACATGCCTCCGCCGTGGATGCC
 
CTCAAGAAGGCGGGCAATGTTGTTAAGCTGCATGTGAAGCGAAAACGTGG
AACGGCCACCACCCCGGCAGCGGATCGGCGGCAGGAGATGCTCGGGATA
GTGCGGCCAGCGGACCGAAGGTCATCGAAATCGATCTGGTCAAGGGCGGC
AAGGGACTGGGCTTCTCAATTGCCGGCGGCATTGGCAACCAGCACATCCC
CGGCGACAATGGCATCTATGTGACCAAGTTGATGGACGGCGGAGCAGCGC
AGGTGGACGGACGTCTCTCCATCGGAGATAAGCTGATTGCAGTGCGCACC
AACGGAGCGAGAAGAACCTGGAGAACGTAACGCACGAACTGGCGGTGGC
CACGTTGAAATCGATCACCGACAAGGTGACGCTGATCATTGGAAAGACAC
AGCATCTGACCACCAGTGCGTCCGGCGGCGGAGGAGGAGGCCTTTCATCC
GGACAACAATTGTCGCAGTCCCAATCGCAGTTGGCCACCAGCCAGAGCCA
AAGTCAGGTGCATCAGCAGCAGCATGCGACGCCGATGGTCAATTCGCAGT
CGACAGGTGCGCTAAATAGTATGGACAGACGGTTGTCGATTCACCATCA
ATACCACAAGCAGCCGCAGCAGTAGCAGCAGCAGCAAATGCATCTGCATC
TGCATCAGTCATTGCAAGCAACAACACAATCAGCAACACCACAGTCACCA
CAGTCACGGCCACGGCCACAGCCAGCAACAGTAGCAGCAAGTTGCCGCCG
TCGCTTGGCGCTAACAGCAGCATTAGCATTAGCAATAGCAATAGCAATAG
CAACAGCAATAATATCAACAACATTAATAGCATCAACAACAACAACAGTA
GCAGCAGCAGCACGACGGCAACTGTTGCAGCAGCAACACCAACAGCAGCA
TCAGCAGCAGCAGCAGCAGCATCATCTCCACCCGCCAACTCCTTCTATAA
CAATGCTTCCATGCCCGCCCTGCCTGTCGAATCCAATCAAACAAACAACC
GATCCCAATCACCCCAGCCGCGCCAGCCCGGGTCGCGATACGCCTCTACA
AATGTCCTAGCCGCCGTTCCACCAGGAACTCCACGCGCTGTCAGCACCGA
GGATATAACCAGAGAACCGCGCACCATCACCATCCAGAAGGACCGCAGG
GCCTGGGCTTCAATATCGTTGGCGGCGAGGATGGCCAGGGTATCTATGTG
TCCTTCATCCTGGCCGGCGGCCCAGCGGATCTCGGGTCGGAGTTGAAGCG
TGGCGACCAGCTGCTCAGCGTGAACAATGTCAATCTCACGCACGCCACCC
ACGAAGAGGCAGCCCAGGCGCTCAAGACTTCTGGCGGTGTGGTGACCCTG
TTGGCGCAGTACCGCCCAGAGGAGTACAATCGCTTCGAGGCACGCATTCA
AGAGTTGAAACAACAGGCTGCCCTCGGTGCCGGCGGATCGGGAACGCTGC
TGCGCACCACGCAAAAGCGATCGCTGTATGTGCGCGCCCTGTTTGACTAC
GATCCGAATCGGGATGATGGATTGCCCTCGCGAGGATTGCCCTTTAAGCA
CGGCGATATCCTGCACGTGACCAATGCCTCCGACGATGAATGGTGGCAGG
CACGACGAGTTCTCGGCGACAACGAGGACGAGCAAATCGGTATTGTACCA
TCGAAAAGGCGTTGGGAGCGCAAAATGCGAGCTAGGGACCGCAGCGTTAA
GTTCCAGGGACATGCGGCAGCTAATAATAATCTGGATAAGCAATCGACAT
TGGATCGAAAGAAAAAGAATTTCACATTCTCGCGCAAATTTCCGTTTATG
AAGAGTCGCGATGAGAAGAATGAAGATGGCAGCGACCAAGAGCCAATGG
AGTTGTGAGCAGCACCAGCGAGATTGACATCAATAATGTCAACAACAACC
AGTCAAATGAACCGCAACCTTCCGAGGAGAACGTGTTGTCCTACGAGGCC
GTACAGCGTTTGTCCATCAACTACACGCGCCCGGTGATTATTCTGGGACC
CCTGAAGGATCGCATCAACGATGACCTTATATCAGAGTATCCCGACAAGT
TCGGCTCTTGTGTGCCACACACCACCCGACCCAAGCGAGAGTACGAGGTG
GATGGTAGGGACTACCACTTTGTATCCTCTCGCGAGCAAATGGAACGGGA
TATTCAGAATCATCTGTTCATCGAGGCGGGACAGTATAACGACAATCTGT
ACGGCACATCGGTGGCCAGCGTGCGCGAAGTGGCCGAGAAGGGTAAACAC
TGCATCCTGGACGTGTCCGGGAACGCCATCAAGCGACTCCAAGTTGCCCA
GCTGTATCCCGTCGCCGTGTTCATCAAGCCCAAGTCGGTGGATTCAGTGA
TGGAAATGAATCGTCGCATGACGGAGGAGCAGGCCAAGAAGACTTACGAG
 
CGGGCGATTAAAATGGAGCAAGAATTCGGCGAATACTTTACGGGCGTTGT
CCAAGGCGATACCATCGAGGAGATTTACAGCAAAGTGAAATCGATGATTT
GGTCCCAGTCGGGACCAACCATTTGGGTACCTTCCAAGGAATCTCTATGA
CCAACAGCCACCACAACTTGGACACTGCCGCCTCGAGTTCGATGTCGACC
AGTCTCGAGAACAACAATAGGAGCAACAGCAGCAGCAACAAATCAGCAGC
CGCAGCAGAAGACGCCGCACTGATGATGCATCACAGTAACAACAGATACT
AATACAACTACAACAACAACAAGAACAACAACAACAACAGCAACCACAGC
AGCAGCCACAGCGACAACAACAAAAACAACAACACTGACAACGACAGGAA
ACGG
 
MTTRKKKRDGGGSGGGFIKKVSSLFNLDSVNGDDSWLYEDIQLERGNSGLGFSIA
GGTDNPHIGTDTSIYITKLISGGAAAADGRLSINDIIVSVNDVSVVDVPHASAVDAL
KKAGNVVKLHVKRKRGTATTPAAGSAAGDARDSAASGPKVIEIDLVKGGKGLGF
SIAGGIGNQHIPGDNGIYVTKLTDGGRAQVDGRLSIGDKLIAVRTNGSEKNLENVT
HELAVATLKSITDKVTLIIGKTQHLTTSASGGGGGGLSSGQQLSQSQSQLATSQSQ
SQVHQQQHATPMVNSQSTGALNSMGQTVVDSPSIPQAAAAVAAAANASASASVI
ASNNTISNTTVTTVTATATASNDSSKLPPSLGANSSISISNSNSNSNSNNINNINSINN
NNSSSSSTTATVAAATPTAASAAAAAASSPPANSFYNNASMPALPVESNQTNNRS
QSPQPRQPGSRYASTNVLAAVPPGTPRAVSTEDITREPRTITIQKGPQGLGFNIVGG
EDGQGIYVSFILAGGPADLGSELKRGDQLLSVNNVNLTHATHEEAAQALKTSGGV
VTLLAQYRPEEYNRFEARIQELKQQAALGAGGSGTLLRTTQKRSLYVRALFDYDP
NRDDGLPSRGLPFKHGDILHVTNASDDEWWQARRVLGDNEDEQIGIVPSKRRWE
RKMRARDRSVKFQGHAAANNNLDKQSTLDRKKKNFTFSRKFPFMKSRDEKNED
GSDQEPNGVVSSTSEIDINNVNNNQSNEPQPSEENVLSYEAVQRLSINYTRPVIILG
PLKDRINDDLISEYPDKFGSCVPHTTRPKREYEVDGRDYHFVSSREQMERDIQNHL
FIEAGQYNDNLYGTSVASVREVAEKGKHCILDVSGNAIKRLQVAQLYPVAVFIKP
KSVDSVMEMNRRMTEEQAKKTYERAIKMEQEFGEYFTGVVQGDTIEEIYSKVKS
MIWSQSGPTIWVPSKESL
 CG1725 - dlg, membrane-associated guanylate kinase homologs, role in cell junctions and proliferation, genbank accession number M73529 (version 2) 
1 cccccccccc cccagttggg tgtgttgttt tcgtcgcgtt cggttgctcg ctttattttt
61 ttgtttgttt attttgtttt gtgcaatgga aatgtgaaca caaatgtttc aaaagtcaac
121 ctctctgttc gcaattgtgt gcattttcgt ttgtctagtg caaaaagttg gataacacag
181 gcggcaaata aaatagtaac gaatcgagtt caagaagaag aagaagagaa gaggaagcag
241 aggcagcagc gccggcattt gtccgtgtgt tgttgttgtt gtttgtgcgc ggctgtaact
301 ttaaccctcg aacgccataa gattaaaaaa ccaactataa caataagtta taaaatcaat
361 taaacaaaag ccgctgcgat atgacaacga ggaaaaagaa gcgcgacggc ggcggcagcg
421 gcggcggatt catcaagaaa gtttcgtcac tcttcaatct ggattcggtg aatggcgatg
481 atagctggtt atacgaggac attcagctgg agcgcggcaa ctccggattg ggcttttcca
541 ttgccggcgg tacggataat ccgcacatcg gcaccgacac ctccatctac atcaccaagc
601 tcatttccgg tggagcagct gccgccgatg gacgtctgag catcaacgat atcatcgtat
661 cggtgaacga tgtgtccgtg gtggatgtgc cacatgcctc cgccgtggat gccctcaaga
721 aggcgggcaa tgttgttaag ctgcatgtga agcgaaaacg tggaacggcc accaccccgg
781 cagcgggatc ggcggcagga gatgctcggg atagtgcggc cagcggaccg aaggtcatcg
841 aaatcgatct ggtcaagggc ggcaagggac tgggcttctc aattgccggc ggcattggca
901 accagcacat ccccggcgac aatggcatct atgtgaccaa gttgacggac ggcggacgag
961 cgcaggtgga cggacgtctc tccatcggag ataagctgat tgcagtgcgc accaacggga
1021 gcgagaagaa cctggagaac gtaacgcacg aactggcggt ggccacgttg aaatcgatca
1081 ccgacaaggt gacgctgatc attggaaaga cacagcatct gaccaccagt gcgtccggcg
1141 gcggaggagg aggcctttca tccggacaac aattgtcgca gtcccaatcg cagttggcca
1201 ccagccagag ccaaagtcag gtgcatcagc agcagcatgc gacgccgatg gtcaattcgc
1261 agtcgacagg tgcgctaaat agtatgggac agacggttgt cgattcacca tcaataccac
1321 aagcagccgc agcagtagca gcagcagcaa atgcatctgc atctgcatca gtcattgcaa
1381 gcaacaacac aatcagcaac accacagtca ccacagtcac ggccacggcc acagccagca
1441 acgatagcag caagttgccg ccgtcgcttg gcgctaacag cagcattagc attagcaata
 
1501 gcaatagcaa tagcaacagc aataatatca acaacattaa tagcatcaac aacaacaaca
1561 gtagcagcag cagcacgacg gcaactgttg cagcagcaac accaacagca gcatcagcag
1621 cagcagcagc agcatcatct ccacccgcca actccttcta taacaatgct tccatgcccg
1681 ccctgcctgt cgaatccaat caaacaaaca accgatccca atcaccccag ccgcgccagc
1741 ccgggtcgcg atacgcctct acaaatgtcc tagccgccgt tccaccagga actccacgcg
1801 ctgtcagcac cgaggatata accagagaac cacgcaccat caccatccag aagggaccgc
1861 agggcctggg cttcaatatc gttggcggcg aggatggcca gggtatctat gtgtccttca
1921 tcctggccgg cggcccagcg gatctcgggt cggagttgaa gcgtggcgac cagctgctca
1981 gcgtgaacaa tgtcaatctc acgcacgcca cccacgaaga ggcagcccag gcgctcaaga
2041 cttctggcgg tgtggtgacc ctgttggcgc agtaccgccc agaggagtac aatcgcttcg
2101 aggcacgcat tcaagagttg aaacaacagg ctgccctcgg tgccggcgga tcgggaacgc
2161 tgctgcgcac cacgcaaaag cgatcgctgt atgtgcgcgc cctgtttgac tacgatccga
2221 atcgggatga tggattgccc tcgcgaggat tgccctttaa gcacggcgat atcctgcacg
2281 tgaccaatgc ctccgacgat gaatggtggc aggcacgacg agttctcggc gacaacgagg
2341 acgagcaaat cggtattgta ccatcgaaaa ggcgttggga gcgcaaaatg cgagctaggg
2401 accgcagcgt taagttccag ggacatgcgg cagctaataa taatctggat aagcaatcga
2461 cattggatcg aaagaaaaag aatttcacat tctcgcgcaa atttccgttt atgaagagtc
2521 gcgatgagaa gaatgaagat ggcagcgacc aagagcccaa tggagttgtg agcagcacca
2581 gcgagattga catcaataat gtcaacaaca accagtcaaa tgaaccgcaa ccttccgagg
2641 agaacgtgtt gtcctacgag gccgtacagc gtttgtccat caactacacg cgcccggtga
2701 ttattctggg acccctgaag gatcgcatca acgatgacct tatatcagag tatcccgaca
2761 agttcggctc ctgtgtgcca cacaccaccc gacccaagcg agagtacgag gtggatggta
2821 gggactacca ctttgtatcc tctcgcgagc aaatggaacg ggatattcag aatcatctgt
2881 tcatcgaggc gggacagtat aacgacaatc tgtacggcac atcggtggcc agcgtgcgcg
2941 aagtggccga gaagggtaaa cactgcatcc tggacgtgtc cgggaacgcc atcaagcgac
3001 tccaagttgc ccagctgtat cccgtcgccg tgttcatcaa gcccaagtcg gtggattcag
3061 tgatggaaat gaatcgtcgc atgacggagg agcaggccaa gaagacttac gagcgggcga
3121 ttaaaatgga gcaagaattc ggcgaatact ttacgggcgt tgtccagggc gataccatcg
3181 aggagatcta cagcaaagtg aaatcgatga tttggtccca gtcgggacca accatttggg
3241 taccttccaa ggaatctcta tga
 
MTTRKKKRDGGGSGGGFIKKVSSLFNLDSVNGDDSWLYEDIQLE
RGNSGLGFSIAGGTDNPHIGTDTSIYITKLISGGAAAADGRLSINDIIVSVNDVSVVD
VPHASAVDALKKAGNVVKLHVKRKRGTATTPAAGSAAGDARDSAASGPKVIEIDLVKG
GKGLGFSIAGGIGNQHIPGDNGIYVTKLTDGGRAQVDGRLSIGDKLIAVRTNGSEKNL
ENVTHELAVATLKSITDKVTLIIGKTQHLTTSASGGGGGGLSSGQQLSQSQSQLATSQ
SQSQVHQQQHATPMVNSQSTGALNSMGQTVVDSPSIPQAAAAVAAAANASASASVIAS
NNTISNTTVTTVTATATASNDSSKLPPSLGANSSISISNSNSNSNSNNINNINSINNN
NSSSSSTTATVAAATPTAASAAAAAASSPPANSFYNNASMPALPVESNQTNNRSQSPQ
PRQPGSRYASTNVLAAVPPGTPRAVSTEDITREPRTITIQKGPQGLGFNIVGGEDGQG
IYVSFILAGGPADLGSELKRGDQLLSVNNVNLTHATHEEAAQALKTSGGVVTLLAQYR
PEEYNRFEARIQELKQQAALGAGGSGTLLRTTQKRSLYVRALFDYDPNRDDGLPSRGL
PFKHGDILHVTNASDDEWWQARRVLGDNEDEQIGIVPSKRRWERKMRARDRSVKFQGH
AAANNNLDKQSTLDRKKKNFTFSRKFPFMKSRDEKNEDGSDQEPNGVVSSTSEIDINN
VNNNQSNEPQPSEENVLSYEAVQRLSINYTRPVIILGPLKDRINDDLISEYPDKFGSC
VPHTTRPKREYEVDGRDYHFVSSREQMERDIQNHLFIEAGQYNDNLYGTSVASVREVA
EKGKHCILDVSGNAIKRLQVAQLYPVAVFIKPKSVDSVMEMNRRMTEEQAKKTYERAI
KMEQEFGEYFTGVVQGDTIEEIYSKVKSMIWSQSGPTIWVPSKESL
**Human homologue of Complete Genome candidate** XP_012060 - discs, large (Drosophila) homolog 2, channel-associated protein of synapses-110' (version 1) 
1 gggaattctg gcctgggatt cagtattgct ggggggacag ataatcccca cattggagat
61 gaccctggca tatttattac gaagattata ccaggaggtg ctgcagcaga ggatggcaga
121 ctcagggtca atgattgtat cttgcgggtg aatgaggttg atgtgtcaga ggtttcccac
181 agtaaagcgg tggaagccct gaaggaagca gggtctatcg ttcggctgta tgtgcgtaga
241 agacgaccta ttttggagac cgttgtggaa atcaaactgt tcaaaggccc taaaggttta
301 ggcttcagta ttgcaggagg tgtggggaac caacacattc ctggagacaa cagcatttat
361 gtaactaaaa ttatagatgg aggagctgca caaaaagatg gaaggttgca agtaggagat
421 agactactaa tggtaaacaa ctacagttta gaagaagtaa cacacgaaga ggcagtagca
481 atattaaaga acacatcaga ggtagtttat ttaaaagttg gcaaacccac taccatttat
541 atgactgatc cttatggtcc acctgatatt actcactctt attctccacc aatggaaaac
601 catctactct ctggcaacaa tggcacttta gaatataaaa cctccctgcc acccatctct
 
661 ccaggaaggt actcaccaat tccaaagcac atgcttgttg acgacgacta caccaggcct
721 ccggaacctg tttacagcac tgtgaacaaa ctatgtgata agcctgcttc tcccaggcac
781 tattcccctg ttgagtgtga caaaagcttc ctcctctcag ctccctattc ccactaccac
841 ctaggcctgc tacctgactc tgagatgacc agtcattccc aacatagcac cgcaactcgt
901 cagccttcaa tgactctcca acgggccgtc tccctggaag gagagcctcg caaggtagtc
961 ctgcacaaag gctccactgg cctgggcttc aacattgtcg gtggggaaga tggagaaggt
1021 atttttgtgt ccttcattct ggctggtgga ccagcagacc taagtgggga gctccagaga
1081 ggagaccaga tcctatcggt gaatggcatt gacctccgtg gtgcatccca cgagcaggca
1141 gctgctgcac taaagggggc tggacagaca gtgacgatta tagcacaata tcaacctgaa
1201 gattacgctc gatttgaggc caaaatccat gacctacgag agcagatgat gaaccacagc
1261 atgagctccg ggtccggatc cctgcgaacc aatcagaaac gctccctcta cgtcagagcc
1321 atgttcgact acgacaagag caaggacagt gggctgccaa gtcaaggact tagttttaaa
1381 tatggagata ttctccacgt tatcaatgcc tctgatgatg agtggtggca agccaggaga
1441 gtcatgctgg agggagacag tgaggagatg ggggtcatcc ccagcaaaag gagggtggaa
1501 agaaaggaac gtgcccgatt gaagacagtg aagtttaatg ccaaacctgg agtgattgat
1561 tcgaaagggt cattcaatga caagcgtaaa aagagcttca tcttttcacg aaaattccca
1621 ttctacaaga acaaggagca gagtgagcag gaaaccagtg atcctgaacg tggacaagaa
1681 gacctcattc tttcctatga gcctgttaca aggcaggaaa taaactacac ccggccggtg
1741 attatcctgg ggcccatgaa ggatcggatc aatgacgact tgatatctga attccctgat
1801 aaatttggct cctgtgtgcc tcatactacg aggccaaagc gagactacga ggtggatggc
1861 agagactatc actttgtcat ttccagagaa caaatggaga aagatatcca agagcacaag
1921 tttatagaag ccggccagta caatgacaat ttatatggaa ccagtgtgca gtctgtgaga
1981 tttgtagcag aaagaggcaa acactgtata cttgatgtat caggaaatgc tatcaagcgg
2041 ttacaagttg cccagctcta tcccattgcc atcttcataa aacccaggtc tctggaacct
2101 cttatggaga tgaataagcg tctaacagag gaacaagcca agaaaaccta tgatcgagca
2161 attaagctag aacaagaatt tggagaatat tttacagcta ttgtccaagg agatacttta
2221 gaagatatat ataaccaatg caagcttgtt attgaagagc aatctgggcc tttcatctgg
2281 attccctcaa aggaaaagtt ataaattagc tactgcgcct ctgacaacga cagaagagca
2341 tttagaagaa caaaatatat ataacatact acttggaggc ttttatgttt ttgttgcatt
2401 tatgtttttg cagtcaatgt gaattcttac gaatgtacaa cacaaactgt atgaagccat
2461 gaaggaaaca gaggggccaa agggtg
 
1 mvnnysleev theeavailk ntsevvylkv gkpttiymtd pygppdiths ysppmenhll
61 sgnngtleyk tslppispgr yspipkhmlv dddytrppep vystvnklcd kpasprhysp
121 vecdksflls apyshyhlgl lpdsemtshs qhstatrqps mtlqravsle geprkvvlhk
181 gstglgfniv ggedgegifv sfilaggpad lsgelqrgdq ilsvngidlr gasheqaaaa
241 lkgagqtvti iaqyqpedya rfeakihdlr eqmmnhsmss gsgslrtnqk rslyvramfd
301 ydkskdsglp sqglsfkygd ilhvinasdd ewwqarrvml egdseemgvi pskrrverke
361 rarlktvkfn akpgvidskg sfndkrkksf ifsrkfpfyk nkeqseqets dpergqedli
421 lsyepvtrqe inytrpviil gpmkdrindd lisefpdkfg scvphttrpk rdyevdgrdy
481 hfvisreqme kdiqehkfie agqyndnlyg tsvqsvrfva ergkhcildv sgnaikrlqv
541 aqlypiaifi kprsleplme mnkrlteeqa kktydraikl eqefgeyfta ivqgdtledi
601 ynqcklviee qsgpfiwips kekl
 DLG2: discs, large homolog 2, chapsyn-110 channel-associated protein of synapses-110' genbank accession number U32376 (version 2) 
1 aaaagcaact gaggtcttaa ctttcagacg ctgaattctc atctaattga aattactggg
61 cataatgcta tatatagcca atgaagagat tttgagctct cactcagtgc cttcaagaca
121 tgtcgttttg tagtcagaga aaacagagat caatgcattt tcaaactgac agagggaacg
181 gatgctcttt agtagcacat gcccaggatc gtgtgtgtgg ggcttgcgct gtgctgagaa
241 gctgaatacc ggtccatatg ctccttattt actgcaatgt tctttgcatg ttactgtgca
301 ctccggacta acgtgaagaa gtatcgatat caagatgagg acgctccaca tgatcattcc
361 ttacctcgac taacccacga agtaagaggc ccagaactcg tgcatgtatc agaaaagaac
421 ctctctcaaa tagaaaatgt ccatggatat gtcctgcagt ctcatatttc tcctctgaag
481 gccagtcctg ctcctataat tgtcaacaca gatactttgg acacaattcc ttatgtcaat
541 gggacagaaa ttgaatatga atttgaagaa attacactgg agagggggaa ttctggcctg
601 ggattcagta ttgctggggg gacagataat ccccacattg gagatgaccc tggcatattt
661 attacgaaga ttataccagg aggtgctgca gcagaggatg gcagactcag ggtcaatgat
721 tgtatcttgc gggtgaatga ggttgatgtg tcagaggttt cccacagtaa agcggtggaa
781 gccctgaagg aagcagggtc tatcgctcgg ctgtatgtgc gtagaagacg acctattttg
841 gagaccgttg tggaaatcaa actgttcaaa ggccctaaag gtttaggctt cagtattgca
901 ggaggtgtgg ggaaccaaca cattcctgga gacaacagca tttatgtaac taaaattata
961 gatggaggag ctgcacaaaa agatggaagg ttgcaagtag gagatagact actaatggta
1021 aacaactaca gtttagaaga agtaacacac gaagaggcag tagcaatatt aaagaacaca
1081 tcagaggtag tttatttaaa agttggcaac cccactacca tttatatgac tgatccttat
1141 ggtccacctg atattactca ctcttattct ccaccaatgg aaaaccatct actctctggc
1201 aacaatggca ctttagaata taaaacctcc ctgccaccca tctctccagg gaggtactca
1261 ccaattccaa agcacatgct tgttgacgac gactacacca ggcctccgga acctgtttac
1321 agcactgtga acaaactatg tgataagcct gcttctccca ggcactattc ccctgttgag
1381 tgtgacaaaa gcttcctcct ctcagctccc tattcccact accacctagg cctgctacct
1441 gactctgaga tgaccagtca ttcccaacat agcaccgcaa ctcgtcagcc ttcaatgact
1501 ctccaacggg ccgtctccct ggaaggagag cctcgcaagg tagtcctgca caaaggctcc
1561 actggcctgg gcttcaacat tgtcggtggg gaagatggag aaggtatttt tgtgtccttc
1621 attctggctg gtggaccagc agacctaagt ggggagctcc agagaggaga ccagatccta
1681 tcggtgaatg gcattgacct ccgtggtgca tcccacgagc aggcagctgc tgcactaaag
1741 ggggctggac agacagtgac gattatagca caatatcaac ctgaagatta cgctcgattt
1801 gaggccaaaa tccatgacct acgagagcag atgatgaacc acagcatgag ctccgggtcc
1861 ggatccctgc gaaccaatca gaaacgctcc ctctacgtca gagccatgtt cgactacgac
1921 aagagcaagg acagtgggct gccaagtcaa ggacttagtt ttaaatatgg agatattctc
1981 cacgttatca atgcctctga tgatgagtgg tggcaagcca ggagagtcat gctggaggga
2041 gacagtgagg agatgggggt catccccagc aaaaggaggg tggaaagaaa ggaacgtgcc
2101 cgattgaaga cagtgaagtt taatgccaaa cctggagtga ttgattcgaa agggtcattc
2161 aatgacaagc gtaaaaagag cttcatcttt tcacgaaaat tcccattcta caagaacaag
2221 gagcagagtg agcaggaaac cagtgatcct gaacgtggac aagaagacct cattctttcc
2281 tatgagcctg ttacaaggca ggaaataaac tacacccggc cggtgattat cctggggccc
2341 atgaaggatc ggatcaatga cgacttgata tctgaattcc ctgataaatt tggctcctgt
2401 gtgcctcata ctacgaggcc aaagcgagac tacgaggtgg atggcagaga ctatcacttt
2461 gtcatttcca gagaacaaat ggagaaagat atccaagagc acaagtttat agaagccggc
2521 cagtacaatg acaatttata tggaaccagt gtgcagtctg tgagatttgt agcagaaaga
2581 ggcaaacact gtatacttga tgtatcagga aatgctatca agcggttaca agttgcccag
2641 ctctatccca ttgccatctt cataaaaccc aggtctctgg aatctcttat ggagatgaat
2701 aagcgtctaa cagaggaaca agccaagaaa acctatgatc gagcaattaa gctagaacaa
2761 gaatttggag aatattttac agctattgtc caaggagata ctttagaaga tatatataac
2821 caatgcaagc ttgttattga agagcaatct gggcctttca tctggattcc ctcaaaggaa
2881 aagttataaa ttagctactg cgcctctgac aacgacagaa gagcatttag aagaacaaaa
2941 tatatataac atactacttg gaggctttta tgtttttgtt gcatttatgt ttttgcagtc
3001 aatgtgaatt cttacgaatg tacaacacaa actgtatgaa gccatgaagg aaacagaggg
3061 gccaaagggt g
 
FFACYCALRTNVKKYRYQDEDAPHDHSLPRLTHEVRGPELVHV
EKNLSQIENVHGYVLQSHISPLKASPAPIIVNTDTLDTIPYVNGTEIEYEFEEITLE
GNSGLGFSIAGGTDNPHIGDDPGIFITKIIPGGAAAEDGRLRVNDCILRVNEVDVSE
SHSKAVEALKEAGSIARLYVRRRRPILETVVEIKLFKGPKGLGFSIAGGVGNQHIPG
NSIYVTKIIDGGAAQKDGRLQVGDRLLMVNNYSLEEVTHEEAVAILKNTSEVVYLKV
NPTTIYMTDPYGPPDITHSYSPPMENHLLSGNNGTLEYKTSLPPISPGRYSPIPKHM
VDDDYTRPPEPVYSTVNKLCDKPASPRHYSPVECDKSFLLSAPYSHYHLGLLPDSEM
SHSQHSTATRQPSMTLQRAVSLEGEPRKVVLHKGSTGLGFNIVGGEDGEGIFVSFIL
GGPADLSGELQRGDQILSVNGIDLRGASHEQAAAALKGAGQTVTIIAQYQPEDYARF
AKIHDLREQMMNHSMSSGSGSLRTNQKRSLYVRAMFDYDKSKDSGLPSQGLSFKYGD
LHVINASDDEWWQARRVMLEGDSEEMGVIPSKRRVERKERARLKTVKFNAKPGVIDS
GSFNDKRKKSFIFSRKFPFYKNKEQSEQETSDPERGQEDLILSYEPVTRQEINYTRP
IILGPMKDRINDDLISEFPDKFGSCVPHTTRPKRDYEVDGRDYHFVISREQMEKDIQ
HKFIEAGQYNDNLYGTSVQSVRFVAERGKHCILDVSGNAIKRLQVAQLYPIAIFIKP
SLESLMEMNKRLTEEQAKKTYDRAIKLEQEFGEYFTAIVQGDTLEDIYNQCKLVIEE
SGPFIWIPSKEKL
 DLG1: discs, large (Drosophila) homolog 1, genbank accession number U13896 
1 gttggaaacg gcactgctga gtgaggttga ggggtgtctc ggtatgtgcg ccttggatct
61 ggtgtaggcg aggtcacgcc tctcttcaga cagcccgagc cttcccggcc tggcgcgttt
121 agttcggaac tgcgggacgc cggtgggcta gggcaaggtg tgtgccctct tcctgattct
181 ggagaaaaat gccggtccgg aagcaagata cccagagagc attgcacctt ttggaggaat
241 atcgttcaaa actaagccaa actgaagaca gacagctcag aagttccata gaacgggtta
301 ttaacatatt tcagagcaac ctctttcagg ctttaataga tattcaagaa ttttatgaag
361 tgaccttact ggataatcca aaatgtatag atcgttcaaa gccgtctgaa ccaattcaac
421 ctgtgaatac ttgggagatt tccagccttc caagctctac tgtgacttca gagacactgc
481 caagcagcct tagccctagt gtagagaaat acaggtatca ggatgaagat acacctcctc
541 aagagcatat ttccccacaa atcacaaatg aagtgatagg tccagaattg gttcatgtct
601 cagagaagaa cttatcagag attgagaatg tccatggatt tgtttctcat tctcatattt
661 caccaataaa gccaacagaa gctgttcttc cctctcctcc cactgtccct gtgatccctg
721 tcctgccagt ccctgctgag aatactgtca tcctacccac cataccacag gcaaatcctc
781 ccccagtact ggtcaacaca gatagcttgg aaacaccaac ttacgttaat ggcacagatg
841 cagattatga atatgaagaa atcacacttg aaaggggaaa ttcagggctt ggtttcagca
901 ttgcaggagg tacggacaac ccacacattg gagatgactc aagtattttc attaccaaaa
961 ttatcacagg gggagcagcc gcccaagatg gaagattgcg ggtcaatgac tgtatattac
1021 aagtaaatga agtagatgtt cgtgatgtaa cacatagcaa agcagttgaa gcgttgaaag
1081 aagcagggtc tattgtacgc ttgtatgtaa aaagaaggaa accagtgtca gaaaaaataa
1141 tggaaataaa gctcattaaa ggtcctaaag gtcttgggtt tagcattgct ggaggtgttg
1201 gaaatcagca tattcctggg gataatagca tctatgtaac caaaataatt gaaggaggtg
1261 cagcacataa ggatggcaaa cttcagattg gagataaact tttagcagtg aataacgtat
1321 gtttagaaga agttactcat gaagaagcag taactgcctt aaagaacaca tctgattttg
1381 tttatttgaa agtggcaaaa cccacaagta tgtatatgaa tgatggctat gcaccacctg
1441 atatcaccaa ctcttcttct cagcctgttg ataaccatgt tagcccatct tccttcttgg
1501 gccagacacc agcatctcca gccagatact ccccagtttc taaagcagta cttggagatg
1561 atgaaattac aagggaacct agaaaagttg ttcttcatcg tggctcaacg ggccttggtt
1621 tcaacattgt aggaggagaa gatggagaag gaatatttat ttcctttatc ttagccggag
1681 gacctgctga tctaagtgga gagctcagaa aaggagatcg tattatatcg gtaaacagtg
1741 ttgacctcag agctgctagt catgagcagg cagcagctgc attgaaaaat gctggccagg
1801 ctgtcacaat tgttgcacaa tatcgacctg aagaatacag tcgttttgaa gctaaaatac
1861 atgatttacg ggagcagatg atgaatagta gtattagttc agggtcaggt tctcttcgaa
1921 ctagccagaa gcgatccctc tatgtcagag ccctttttga ttatgacaag actaaagaca
1981 gtgggcttcc cagtcaggga ctgaacttca aatttggaga tatcctccat gttattaatg
2041 cttctgatga tgaatggtgg caagccaggc aggttacacc agatggtgag agcgatgagg
2101 tcggagtgat tcccagtaaa cgcagagttg agaagaaaga acgagcccga ttaaaaacag
2161 tgaaattcaa ttctaaaacg agagataaag ggcagtcatt caatgacaag cgtaaaaaga
2221 acctcttttc ccgaaaattc cccttctaca agaacaagga ccagagtgag caggaaacaa
2281 gtgatgctga ccagcatgta acttctaatg ccagcgatag tgaaagtagt taccgtggtc
2341 aagaagaata cgtcttatct tatgaaccag tgaatcaaca agaagttaat tatactcgac
2401 cagtgatcat attgggacct atgaaagaca ggataaatga tgacttgatc tcagaatttc
2461 ctgacaaatt tggatcctgt gttcctcata caactagacc aaaacgagat tatgaggtag
2521 atggaagaga ttatcatttt gtgacttcaa gagagcagat ggaaaaagat atccaggaac
2581 ataaattcat tgaagctggc cagtataaca atcatctata tggaacaagt gttcagtctg
2641 tacgagaagt agcaggaaag ggcaaacact gtatccttga tgtgtctgga aatgccataa
2701 agagattaca gattgcacag ctttacccta tctccatttt tattaaaccc aaatccatgg
2761 aaaatatcat ggaaatgaat aagcgtctaa cagaagaaca agccagaaaa acatttgaga
2821 gagccatgaa actggaacag gagtttactg aacatttcac agctattgta cagggggata
2881 cgctggaaga catttacaac caagtgaaac agatcataga agaacaatct ggttcttaca
2941 tctgggttcc ggcaaaagaa aagctatgaa aactcatgtt tctctgtttc tcttttccac
3001 aattccattt tctttggcat ctctttgccc tttcctctgg aaaaaa
 
MPVRKQDTQRALHLLEEYRSKLSQTEDRQLRSSIERVINIFQSN
LFQALIDIQEFYEVTLLDNPKCIDRSKPSEPIQPVNTWEISSLPSSTVTSETLPSSLS
PSVEKYRYQDEDTPPQEHISPQITNEVIGPELVHVSEKNLSEIENVHGFVSHSHISPI
KPTEAVLPSPPTVPVIPVLPVPAENTVILPTIPQANPPPVLVNTDSLETPTYVNGTDA
DYEYEEITLERGNSGLGFSIAGGTDNPHIGDDSSIFITKIITGGAAAQDGRLRVNDCI
LQVNEVDVRDVTHSKAVEALKEAGSIVRLYVKRRKPVSEKIMEIKLIKGPKGLGFSIA
GGVGNQHIPGDNSIYVTKIIEGGAAHKDGKLQIGDKLLAVNNVCLEEVTHEEAVTALK
NTSDFVYLKVAKPTSMYMNDGYAPPDITNSSSQPVDNHVSPSSFLGQTPASPARYSPV
SKAVLGDDEITREPRKVVLHRGSTGLGFNIVGGEDGEGIFISFILAGGPADLSGELRK
GDRIISVNSVDLRAASHEQAAAALKNAGQAVTIVAQYRPEEYSRFEAKIHDLREQMMN
SSISSGSGSLRTSQKRSLYVRALFDYDKTKDSGLPSQGLNFKFGDILHVINASDDEWW
QARQVTPDGESDEVGVIPSKRRVEKKERARLKTVKFNSKTRDKGQSFNDKRKKNLFSR
KFPFYKNKDQSEQETSDADQHVTSNASDSESSYRGQEEYVLSYEPVNQQEVNYTRPVI
ILGPMKDRINDDLISEFPDKFGSCVPHTTRPKRDYEVDGRDYHFVTSREQMEKDIQEH
KFIEAGQYNNHLYGTSVQSVREVAGKGKHCILDVSGNAIKRLQIAQLYPISIFIKPKS
MENIMEMNKRLTEEQARKTFERAMKLEQEFTEHFTAIVQGDTLEDIYNQVKQIIEEQS
GSYIWVPAKEKL
**Putative function** Component of cell junctions, possible role in proliferation

### Example 28B. Validation of GENE Function by RNA interference (RNAi) Knockdown in Drosophila Cultured Cells

To confirm the mitotic role of the target protein, knockdown of GENE expression is performed in cultured *Drosophila* Dmel-2 cells using a double stranded RNA (dsRNA) from within the Dlg1 (CG1725) gene corresponding to the following sequence: 
GGAGGCCTTTCATCCGGACAACAATTGTCGCAGTCCCAATCGCAGTTGGCCACCAGCCAGAGCCAA
AGTCAGGTGCATCAGCAGCAGCATGCGACGCCGATGGTCAATTCGCAGTCGACAGGTGCGCTAAAT
AGTATGGGACAGACGGTTGTCGATTCACCATCAATACCACAAGCAGCCGCAGCAGTAGCAGCAGCA
GCAAATGCATCTGCATCTGCATCAGTCATTGCAAGCAACAACACAATCAGCAACACCACAGTCACC
ACAGTCACGGCCACGGCCACAGCCAGCAACAGTAGCAGCAAGTTGCCGCCGTCGCTTGGCGCTAAC
AGCAGCATTAGCATTAGCAATAGCAATAGCAATAGCAACAGCAATAATATCAACAACATTAATAGC
ATCAACAACAACAACAGTAGCAGCAGCAGCACGACGGCAACTGTTGCAGCAGCAACACCAACAGCA
GCATCAGCAGCAGCAGCAGCAGCATCATCTCCACCCGCCAACTCCTTCTATAA
 dsRNA is prepared by annealing complimentary RNAs made by *in vitro* transcription from a PCR fragment created with the following PCR primers:
TAATACGACTCACTATAGGGAGAGGAGGCCTTTCATCCGGACAACAAT
TAATACGACTCACTATAGGGAGATTATAGAAGGAGTTGGCGGGTGGAG

Cells are transfected with double stranded RNA in the presence of 'Transfast' transfection reagent. A control transfection of a non-endogenous RNA corresponding to RFP (red fluorescent protein) is carried out in parallel.

### Analysis of Dlg1 Knockdown by RNAi in D-Mel2 cells by Cellomics Mitotic Index Assay

For the transfection, 1 µg dsRNA is added to a well of a 96-well Packard viewplate and 35 µl of logarithmically growing DMel-2 cells diluted to 2.3x10⁵ cells/ml in fresh Drosophila-SFM/glutamine/Pen-Strep are added. Cells are incubated with the dsRNA (60nM) in a humid chamber at 28°C for 1 hr before addition of 100 µl Drosophila-SFM/glutamine/Pen-Strep. Cells are incubated at 28°C for 72 hours before analysis. For the assay, cells were fixed and stained using the Cellomics Mitotic Index HitKit following manufacturers instructions. The mitotic index of cells in each well was determined using the ArrayScan HCS System, running the Application protocol Mike_250502_Polgen_MitoticIndex_10x_p2.0 with the 10x objective and the DualBGlp filter set. This automated screening system detects the levels of a specific antigen (phosphorylated histone H3) which is only detectable during mitosis while the chromosomes are condensed.

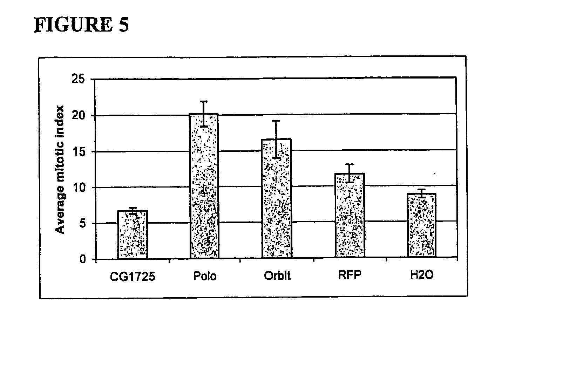

Results for Dlgl (CG1725) are shown in Figure 5. A reproducible and significant reduction in mitotic index is observed in this assay indicating a reduction in the number of cells entering mitosis after RNAi

### Analysis of Dlg1 Knockdown by RNAi in D-Mel2 cells by Microscopy

For transfection 9 µl of Transfast reagent (Promega) is added to 3µg gene specific dsRNA in 500µl Drosophila Schneiders medium (no additives) and incubated at room temperature for 15 min. For control wells an equivalent amount of RFP dsRNA is used. This mix is added to a well of a 6-well tissue culture plate containing a glass coverslip and 500µl of a Dmel-2 cells at 1x10⁶ cells/ml in shneiders medium. After a 1 hour incubation at 28°C, 2mls Schneiders medium + 10% FCS and pen/strep solution is added and cells are incubated at 28°C for 48 hours. Cells on the coverslip are fixed in formaldehyde and stained with antibodies which detect α-tubulin and γ-tubulin (centrosomes), and are co-stained with DAPI to detect DNA.

Although no pronounced increase in the frequency of chromosomal defects (see Table 3 below) was observed upon RNAi, there was a small increase (30% compared to 10% in control cells) of spindle defects, of which the majority (>90%) had multiple centrosomes (more than 2).

**Table 3**

| Mitotic defects observed in Dmel-2 cells after siRNA with Dlg1 (CG1725) | | | |
|---|---|---|---|
| dsRNA | Number cells with chromosomal defects | Number of cells with normal mitisis | % of chromosomal defects (no defects/total cells in mitosis) |
| No RNA | 135 | 314 | 39.47 |
| RFP | 137 | 309 | 40.29 |
| CG1725 | 152 | 169 | 47.35 |

### Example 28B. Human Dlg1 and Dlg2 are Human Homologues of Drosophila Dlg1

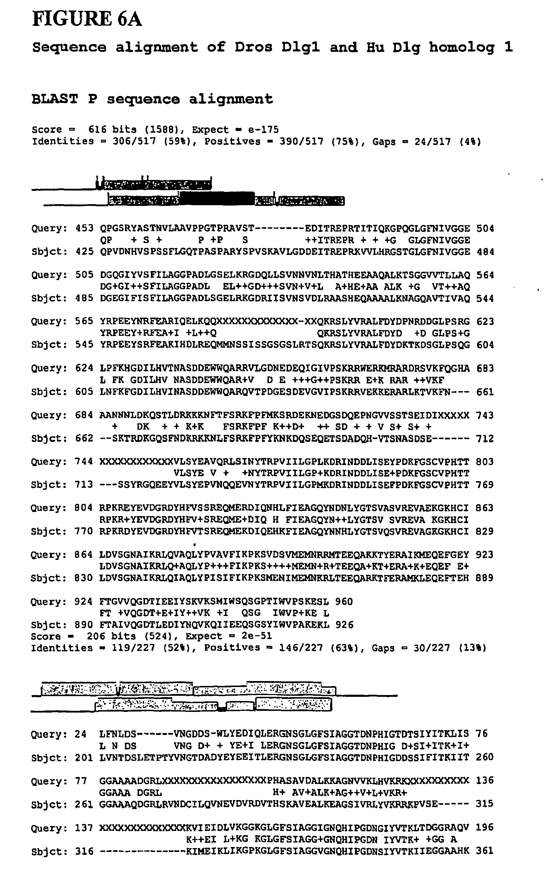

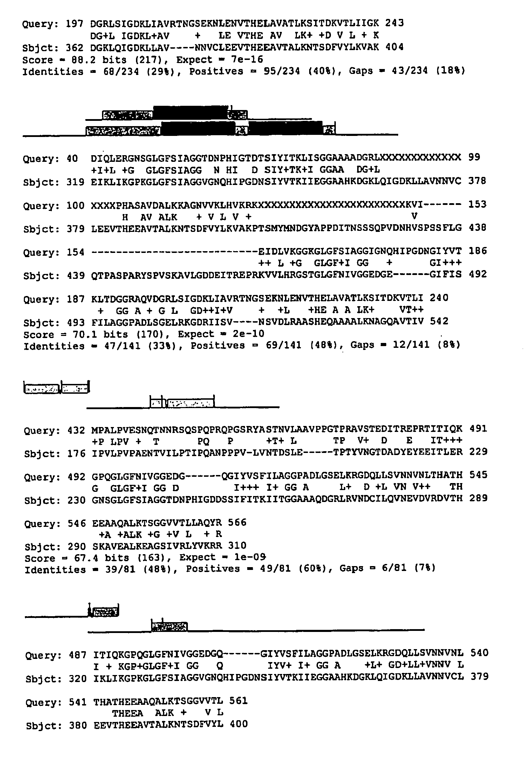

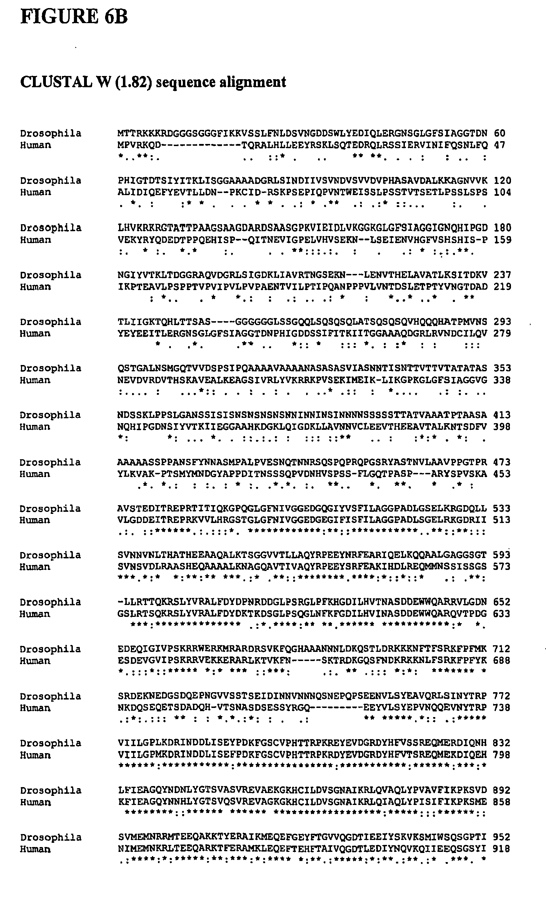

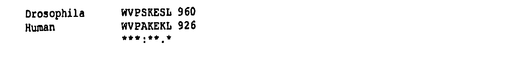

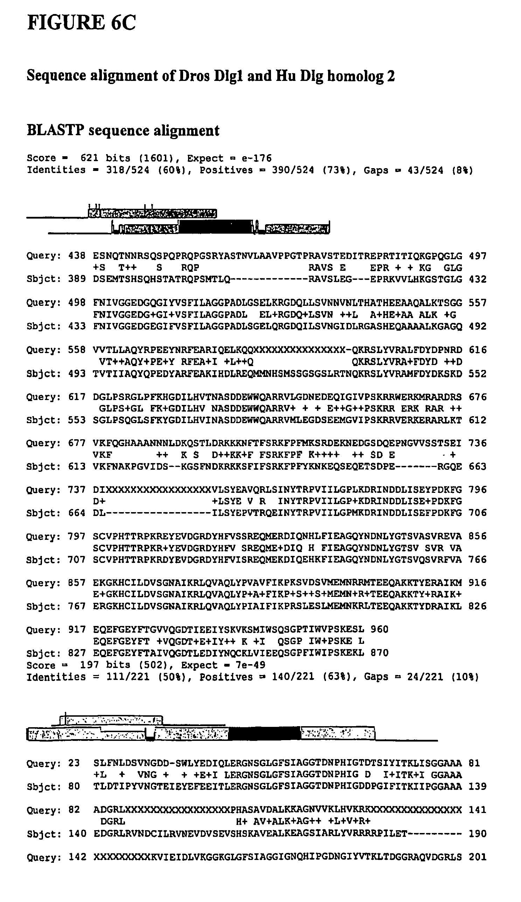

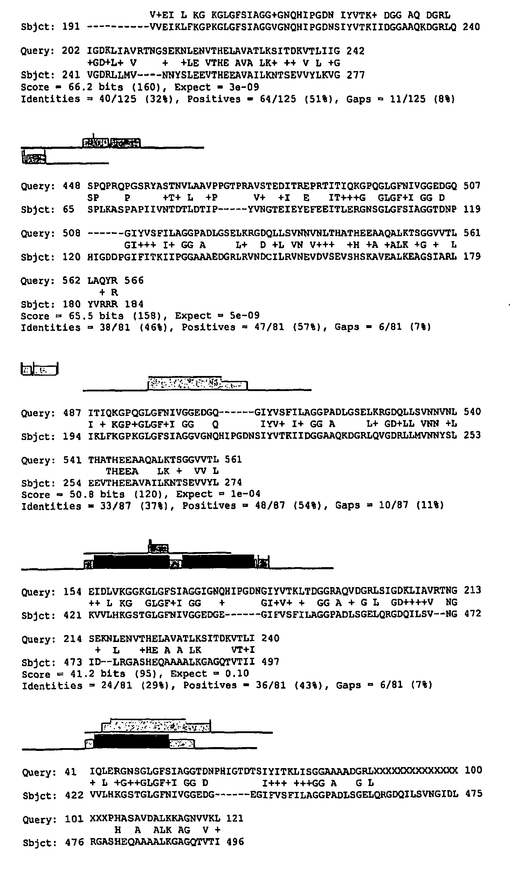

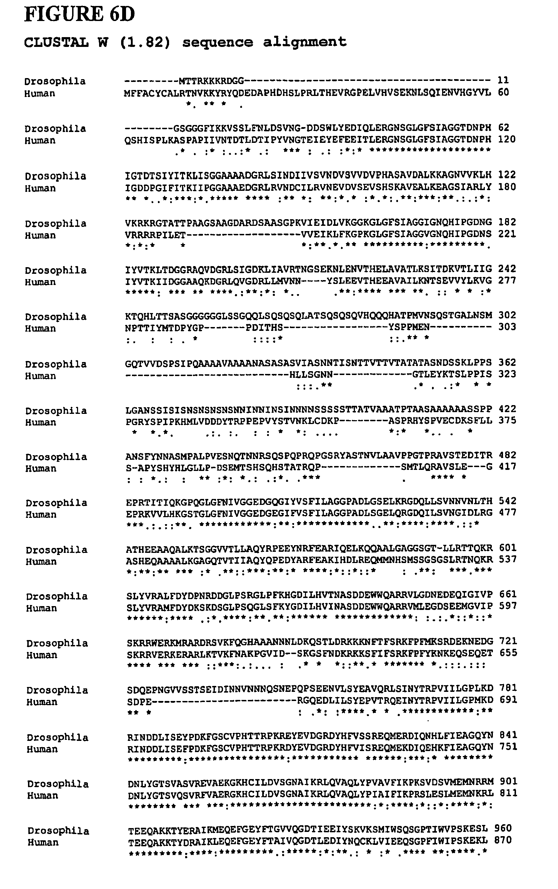

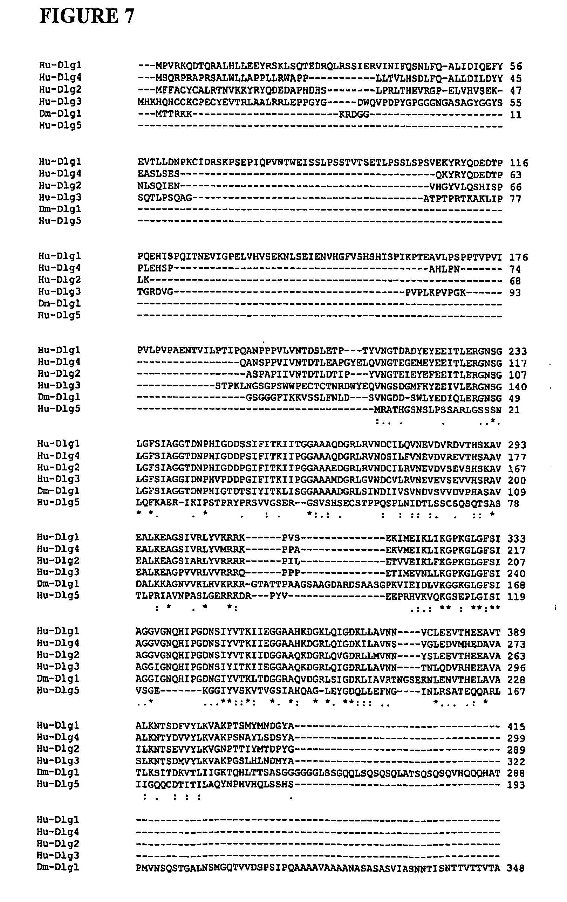

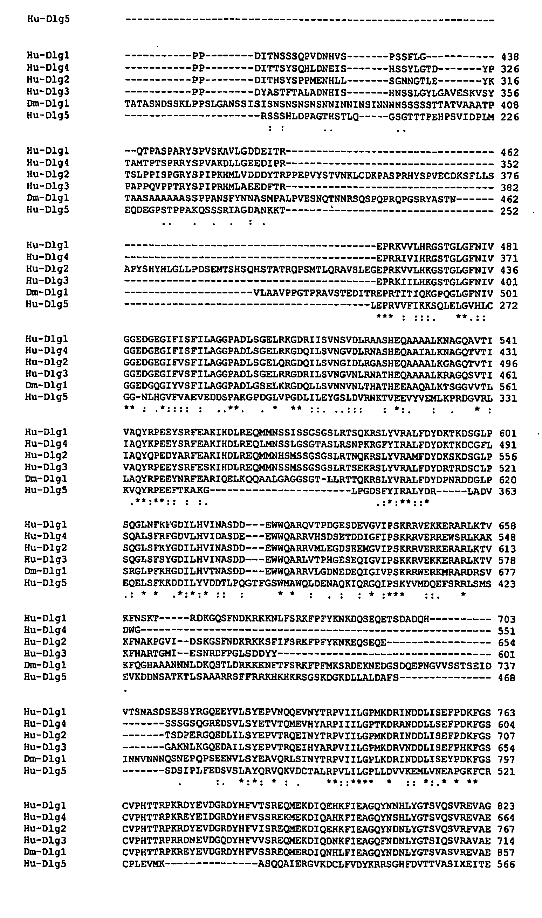

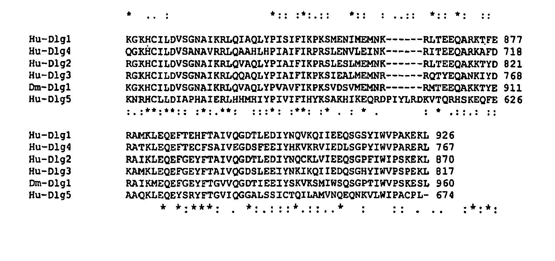

BLASTP with *Drosophila* Dlg1 reveals 59% (306/517) sequence identity with regions of the human discs, large (Drosophila) homolog 1 (GENBANK ACCESSION U13896), and 60% (318/524) sequence identity with regions of human discs, large (Drosophila) homolog 2 (GENBANK ACCESSION U32376) that human Dlg1 and Dlg2 are is a homologues of *Drosophila* Dlg1. The BLASTP results are shown in Figure 6. Figure7 shows a Clustal W alignment of Drosophila Dlg1 and the five human Dlg homologues that are currently detailed in the NCBI database. Considering the homology between the human Dlg proteins, it is probable that some or all of them are functionally similar to Drosophila Dlg1.

The nucleotide sequence of the human Dlg1 and human Dlg2 genes and their deduced amino acid sequences are shown in example 28 above.

### Example 28C. Validation of the Mitotic Role of the Human Homologue by siRNA Knockdown of GENE Expression in Human Cultured Cells

### Generation of siRNA human Dlg1 and Dlg2 Knockdowns

Knockdown of human Dlg1 and Dlg2 gene expression is achieved by siRNA (short interfering RNA, Elbashir et al, Nature 2001 May 24;411(6836):494-8). We used synthetic double stranded RNAs corresponding to two different regions of each of the human Dlg1 and Dlg2 mRNAs. Synthetic siRNAs are obtained from Dharmacon Inc (our supplier). The siRNA sequences are:

| | | | |
|---|---|---|---|
| COD1652 | dlg2-1 | AACAUUGUCGGUGGGAA GAU | Corresponds to nucleotides 1576 - 1596 in human Dlg-2 (see example 28 above) |
| COD1653 | dlg2-2 | AAAACCCAGGUCUCUGGA ACC | Corresponds to nucleotides 2664 - 2684 in human Dlg-2 (see example 28 above) |
| COD1654 | dlg1-1 | AAAGGGGAAAUUCAGGGC [?] UUG [?] | Corresponds to nucleotides 871 - 891 in human Dlg-1 (see example 28 above) |
| COD1655 | dlg1-2 | AAGUAGCAGGAAAGGGCA AAC | Corresponds to nucleotides 2647-2667 in human Dlg-1 (see example 28 above) |

### Analysis of siRNA Hu Dlg1 and Dlg2 Knockdowns in U2OS Cells by Flow Cytometry Analysis

Cells are seeded in 6-well tissue culture dishes at 1x10⁵ cells/well in 2 ml Dulbecco's Modified Eagle's Medium (DMEM) (Sigma) + 10% Foetal Bovine Serum (FBS) (Perbio), and incubated overnight (37°C/ 5% CO₂).

For each well, 12 µl of 20 µM siRNA duplex (Dharmacon, Inc) (in RNAse-free H₂O) is mixed with 200 µl of Optimem (Invitrogen). In a separate tube 8 µl of oligofectamine reagent (Invitrogen) was mixed with 52 µl of Optimem, and incubated at room temperature for 7-10 mins. The oligofectamine/ Optimem mix is then added dropwise to the siRNA/ Optimem mix, and this is then mixed gently, before being incubated for 15-20 mins at room temperature. During this incubation the cells are washed once with DMEM (with no FBS or antibiotics added). 600 µl of DMEM (no FBS or antibiotics) is then added to each well.

Following the 15-20 min incubation, 128 µl of Optimem is added to the siRNA/oligofectamine/ optimem mix, and this was added to the cells (in 600 µl DMEM). The transfection mix is added at the edge of each well to assist dilution before contact is made with the cells. Cells are then incubated with the transfection mix for 4 h (37°C / 5%CO₂). Subsequently 1 ml DMEM + 20% FBS is added to each well. Cells are then incubated at 37°C / 5% CO₂ for 72 h. Cells are harvested by trypsinisation, washed in PBS, fixed in ice-cold 70% EtOH and stained with propidium iodide before Facs analysis.

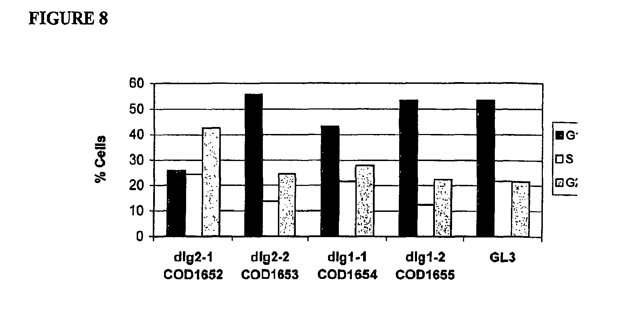

siRNA Hu Dlg1 and Dlg2 knockdowns are conducted in U2OS. As shown in Figure 8 major changes in the distribution of cells between cell cycle compartments (G1, S, G2 /M) are seen with Dlgl siRNA COD1564 and DIg2 siRNA COD1562. In both cases an accumulation of cells with a 2N DNA content, indicated as the G2/M compartment of the cell cycle, is observed with a concomitant reduction in the 1N DNA content G1 compartment population. This indicates that a proportion of cells may unable to exit mitosis and renter G1 and so may be unable to complete cytokinesis, or have entered the next cycle as polyploid cells.

Subsequent microscopic analysis is performed in order to phenotype the Hu Dlg1 and Dlg2 siRNA induced defect and check for the presence of large multinucleate cells which may, due to their size and ploidy, be excluded from the FACS analysis.

### Analysis of Hu Dlg1 and Dlg2 siRNA Knockdowns in U2OS Cells by Microscopy

The transfection method for samples for microscopy is identical to that for Facs except that cells are plated in wells containinmg a sterile glass coverslip. Cells are incubated with siRNA for 48 hours before formaldehyde fixation and co-staining with Dapi to reveal DNA (blue) and antibodies to reveal microtubules (red) and centrosomes (green). Antibodies used are: rat anti-alpha tubulin (YL12) (supplier Serotec) with secondary antibody goat anti-rat IgG-TRITC (supplier Jackson Immunoresearch) and mouse anti-gamma-tubulin (GTU88) with secondary antibody Alexagreen488-goat anti-mouseIgG (supplier Sigma).

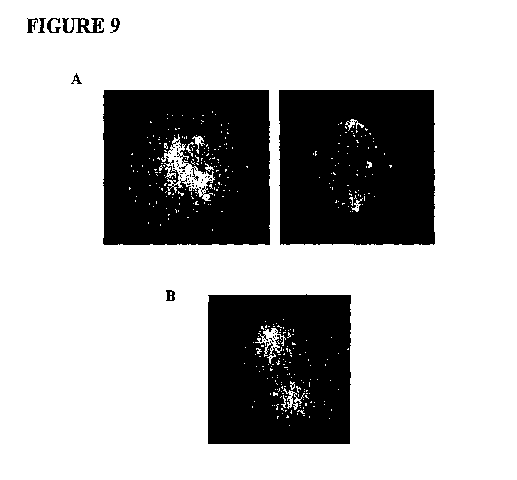

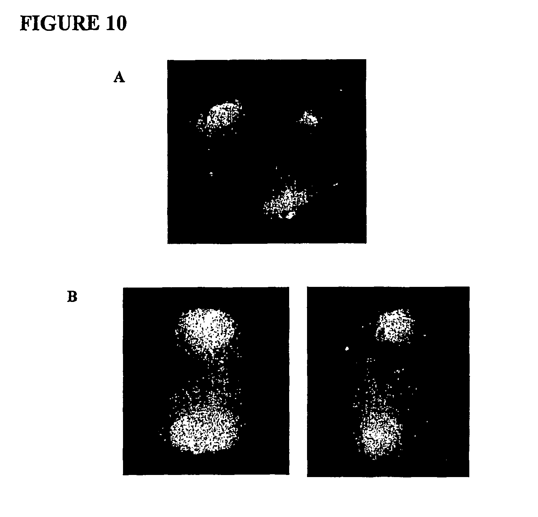

Phenotype analysis by microscopy is conducted on U2OS cells. Results from duplicate experiments in U2OS cells are shown in Figures 9 and 10, and Table 4 below. Generally after siRNA more of the cells in mitosis seem to be in the early stages, prometaphase rather than the later stages (metaphase, anaphase telophase) a high frequency of cells have multiple centrosomes as is also observed in RNAi with Dmel-2 cell siRNA (see above). In addition transfected cells appear to be unable to successfully carry out cytokinesis which may account for the increase in polyploid cells.

**Table 4:**

| Brief description of significant cell division defects after Dlg1 and 2siRNA in U2OS cells. | | |
|---|---|---|
| Gene/siRNA | Dlg1/COD1564 | Dlg2/COD1562 |
| Cell Type | U2OS | U2OS |
| Polyploidy | Increased (4.8/field compared to 1.6/field in nuntreated) | Increased (4.8/field compared to 1.6/field in nuntreated) |
| Mitotic Defects | Increased (23% compared to 13% in untreated) | Increased (36% compared to 13% in untreated) |
| Main knockout phenotype | Increased number of multi -centrosomal cells (7.3% compared to 2.6% in untreated) Cytokinesis defects (10% compared to 0% in - untreated) Large increase in apoptotic cells | Increased number of multi -centrosomal cells (6.6% compared to 2.6%) in untreated) Cytokinesis defects (23% compared to 0% in untreated) Large increase in apoptotic cells |
| Additional observations | Increase in ratio of prophase to prometaphase (61% compared to 43% in untreated cells) Decrease in ratio of metaphase (5% compared to 22% in untreated cells) | Increase in ratio of prophase to prometaphase (72% compared to 43% in untreated cells) Decrease in ratio of metaphase (6% compared to 22% in untreated cells) Decrease in ratio of anaphase and telophase (19% compared to 27% in untreated cells) |

The above results confirm that Dlg1 and Dlg2 are involved in cell cycle progression, in particular, in achieving successful cell separation during cytokinesis. The mutiplication of centrosomes in many cells after Dlg 1 or 2 RNAi may reflect failure to undergo cytokinesis so that cells prematurely enter the next cycle, or may indicate that the centrosome duplication cycle is overriding normal cell cycle checkpoints. Accordingly, modulators of Dlg1 and Dlg2 activity (as identified by the assays described above) may be used to treat any proliferative disease.

### Example 28D. Expression of Recombinant Hu Dlg Protein in Insect Cells

A cDNA encoding the Human Dlg1 or Dlg2 coding region derived by RT-PCR is inserted into the baculovirus expression vector pFastbacHTc (Life Technologies). A baculovirus stock is generated and western blot of subsequent infections of Sf9 insect cells demonstrates expression of N-terminal 6-His tagged proteins of approximately 100 kD (Dlg1) and 97kD (Dlg2). The recombinant protein is purified by Ni-NTA resin affinity chromatography.

Similarly 6-His tagged Dlg proteins are expressed in bacteria by inserting cDNAs into bacterial expression plamids pDest17 or pET series. Protein expression in cultures of host E.coli cells transformed with recombinant plasmid is induced by addition of inducer chemical IPTG. The recombinant protein is purified by Ni-NTA resin affinity chromatography

### Example 28E. Assay for Modulators of Dlg Activity

Dlgs are Membrane-associated guanylate kinase (MAGUK) homologues and contain several protein - protein interaction domains including PDZ domains, SH3 domains and a C-terminal guanylate kinase homology region that does not possess guanylate kinase activities but may act as a protein - protein interaction domain. Several proteins are known to bind huDlg1 including the adenomatous polposis coli (APC) tumour suppressor protein, the human papillomavirus E6 transforming protein, transforming adenovirus E4 protein, and the PDZ-binding kinase PBK (Gaudet et al 2000). An assay for modulators of Dlg activity would consist of an ELISA type assay where full length Dlg protein, or individual PDZ domains of Dlg protein expressed in bacteria or insect cells (as described above) are bound to a solid support, and interaction with the PDZ binding proteins described above could be measured by antibody detection of, or radioactive labelling of the PDZ binding proteins.

### Example 29 (Category 3)

**Line ID** - 419
**Phenotype** - Lethal phase, prepupal - pupal. High mitotic index, colchicines-like chromosome condensation, metaphase arrest
**Annotated *Drosophila* genome genomic segment containing P element insertion site (and map position)** - AE003450 (9C)
**P element insertion site** - 292,726
**Annotated *Drosophila* genome Complete Genome candidate** CG12638 - sprint, ras associated protein 
ATGTTTGCCATATCATTGCAGCTGCTCAGCTCGCTGGCCAGCGATTTGGA
CATAATGCTAAACGATCTTCGATCGGCGCCGAGTCATGCTGCAACAGCAA
CAGCAACAGCAACAACAACGGCAACAGTTGCAACTGCAACCGCAACAACA
ACGGCCAACCGGCAGCAGCAACATCATAATCACCATAATCAGCAGCAAAT
GCAATCAAGGCAATTGCATGCACATCATTGGCAGAGCATTAACAACAATA
AGAATAACAACATTAGTAACAAAAACAACAACAACAACAACAATAATAAC
AATAACATTAATAACAATAATAATAATAATAATCATTCGGCACACCCACC
TTGCCTGATCGATATTAAGCTGAAGTCAAGCCGATCGGCAGCAACAAAAA
TAACCCATACAACAACCGCCAATCAGCTGCAGCAACAACAACGCCGCCGT
GTGGCACCCAAGCCACTGCCACGCCCACCGCGACGTACCCGCCCAACGGG
ACAAAAGGAGGTGGGGCCGTCTGAAGAGGATGGGGACACGGATGCCAGTG
ACCTGGCCAATATGACATCACCGCTGAGCGCCAGTGCAGCGGCCACTCGA
ATCAACGGCCTCTCGCCGGAAGTGAAGAAAGTCCAGCGGTTGCCACTGTG
GAATGCGCGAAACGGAAACGGAAGTACCACCACCCACTGTCACCCAACCG
GCGTCTCTGTGCAACGCCGTCTGCCCATCCAAAGTCATCAGCAGCGAATT
CTAAACCAACGATTTCATCACCAGCGAATGCATCATGGGTAA
 
MFAISLQLLSSLASDLDIMLNDLRSAPSHAATATATATTTATVATATATATT
TANRQQHHNHHNQQMQSRQLHAHHWQSI NNKNNNISNKNNNNNNNNN
NNINNNNNNNNHSAHPPCLIDIKLKSSRSAATKITHTTTANQLQQQQRRR
VAPKPLPRPPRRTRPTGQKEVGPSEEDGDTDASDLANMTSPLSASAAATR
INGLSPEVKKVQRLPLWNARNGNGSTTTHC HPTGVSVQRRLPIQSHQQRI
LNQRFHHQRM HHG
**Human homologue of Complete Genome candidate** B38637 - Ras inhibitor (clone JC265) - human (fragment) 1 ggccggcagc ggctgagcga catgagcatt tctacttcct cctccgactc gctggagttc 
61 gaccggagca tgcctctgtt tggctacgag gcggacacca acagcagcct ggaggactac
121 gagggggaaa gtgaccaaga gaccatggcg ccccccatca agtccaaaaa gaaaaggagc
181 agctccttcg tgctgcccaa gctcgtcaag tcccagctgc agaaggtgag cggggtgttc
241 agctccttca tgaccccgga gaagcggatg gtccgcagga tcgccgagct ttcccgggac
301 aaatgcacct acttcgggtg cttagtgcag gactacgtga gcttcctgca ggagaacaag
361 gagtgccacg tgtccagcac cgacatgctg cagaccatcc ggcagttcat gacccaggtc
421 aagaactatt tgtctcagag ctcggagctg gaccccccca tcgagtcgct gatccctgaa
481 gaccaaatag atgtggtgct ggaaaaagcc atgcacaagt gcatcttgaa gcccctcaag
541 gggcacgtgg aggccatgct gaaggacttt cacatggccg atggctcatg gaagcaactc
601 aaggagaacc tgcagcttgt gcggcagagg aatccgcagg agctgggggt cttcgccccg
661 acccctgatt ttgtggatgt ggagaaaatc aaagtcaagt tcatgaccat gcagaagatg
721 tattcgccgg aaaagaaggt catgctgctg ctgcgggtct gcaagctcat ttacacggtc
781 atggagaaca actcagggag gatgtatggc gctgatgact tcttgccagt cctgacctat
841 gtcatagccc agtgtgacat gcttgaattg gacactgaaa tcgagtacat gatggagctc
901 ctagacccat cgctgttaca tggagaagga ggctattact tgacaagcgc atatggagca
961 ctttctctga taaagaattt ccaagaagaa caagcagcgc gactgctcag ctcagaaacc
1021 agagacaccc tgaggcagtg gcacaaacgg agaaccacca accggaccat cccctctgtg
1081 gacgacttcc agaattacct ccgagttgca tttcaggagg tcaacagtgg ttgcacagga
1141 aagaccctcc ttgtgagacc ttacatcacc actgaggatg tgtgtcagat ctgcgctgag
1201 aagttcaagg tgggggaccc tgaggagtac agcctctttc tcttcgttga cgagacatgg
1261 cagcagctgg cagaggacac ttaccctcaa aaaatcaagg cggagctgca cagccgacca
1321 cagccccaca tcttccactt tgtctacaaa cgcatcaaga acgatcctta tggcatcatt
1381 ttccagaacg gggaagaaga cctcaccacc tcctagaaga caggcgggac ttcccagtgg
1441 tgcatccaaa ggggagctgg aagccttgcc ttcccgcttc tacatgcttg agcttgaaaa
1501 gcagtcacct cctcggggac ccctcagtgt agtgactaag ccatccacag gccaactcgg
1561 ccaagggcaa ctttagccac gcaaggtagc tgaggtttgt gaaacagtag gattctcttt
1621 tggcaatgga gaattgcatc tgatggttca agtgtcctga gattgtttgc tacctacccc
1681 cagtcaggtt ctaggttggc ttacaggtat gtatatgtgc agaagaaaca cttaagatac
1741 aagttctttt gaattcaaca gcagatgctt gcgatgcagt gcgtcaggtg attctcactc
1801 ctgtggatgg cttcatccct g
 
1 grqrlsdmsi stsssdslef drsmplfgye adtnssledy egesdqetma ppikskkkrs
61 ssfvlpklvk sqlqkvsgvf ssfmtpekrm vrriaelsrd kctyfgclvq dyvsflqenk
121 echvsstdml qtirqfmtqv knylsqssel dppieslipe dqidvvleka mhkcilkplk
181 ghveamlkdf hmadgswkql kenlqlvrqr npqelgvfap tpdfvdveki kvkfmtmqkm
241 yspekkvmll lrvckliytv mennsgrmyg addflpvlty viaqcdmlel dteieymmel
301 ldpsllhgeg gyyltsayga lsliknfqee qaarllsset rdtlrqwhkr rttnrtipsv
361 ddfqnylrva fqevnsgctg ktllvrpyit tedvcqicae kfkvgdpeey slflfvdetw
421 qqlaedtypq kikaelhsrp qphifhfvyk rikndpygii fqngeedltt s
**Putative function** Ras associated effector protein

### REFERENCES

Altschul, S.F. and Lipman, D. J. (1990) Protein database searches for multiple alignments. Proc. Natl. Acad. Sci. USA 87: 5509-5513
Burge, C. and Karlin, S. (1997) Prediction of complete gene structures in human genomic DNA. J. Mol. Biol. 268, 78-94.
Deak, P., Omar, M.M., Saunders, R.D.C., Pal, M., Komonyi, O., Szidonya, J., Maroy, P., Zhang, Y., Ashbumer, M., Benos, P., Savakis, C., Siden-Kiamos, I., Louis, C., Bolshakov, V.N., Kafatos, F.C., Madueno, E., Modolell, J., Glover, D.M. (1997) Correlating physical and cytogenetic maps in chromosomal region 86E-87F of Drosophila melanogaster. Genetics 147:1697-1722.
Gaudet S, Branton D and Lue RA (2000) Characterisation of PDZ-binding kinase, a mitotic kinase PNAS 97, 5167-5172
Jowett, T. (1986) Preparation of nucleic acids. In "Drosophila: A Practical Approach." Ed Roberts,D.B. IRL Press Oxford.
Lefevre, G. (1976) A photographic representation and interpretation of the polytene chromosomes of Drosophila melanogaster salivary glands. In: The Genetics and Biology of Drosophila, Eds Ashburner,M. and Novitski,E. Academic Press.
Pirrotta, V. (1986) Cloning Drosophila genes. In: . In "Drosophila: A Practical Approach." Ed Roberts,D.B. IRL Press Oxford.
Saunders, R.D.C., Glover, D.M., Ashbumer, M., Siden-Kiamos, I., Louis, C., Monastirioti, M., Savakis, C., Kafatos, F.C.(1989) PCR amplification of DNA microdissected from a single polytene chromosome band: a comparison with conventional microcloning. Nucleic Acids Res. 17:9027-9037
Takada T, Matozaki T, Takeda H, Fukunaga K, Noguchi T, Fujioka Y, Okazaki I, Tsuda M, Yamao T, Ochi F, Kasuga M. (1998) Roles of the complex formation of SHPS-1 with SHP-2 in insulin-stimulated mitogen-activated protein kinase activation. J Biol Chem 1998 Apr 10;273(15):9234-42
Torok, T., Tick, G., Alvarado, M., Kiss, I. (1993) P-lacW insertional mutagenesis on the second chromosome of Drosophila melanogaster: isolation of lethals with different overgrowth phenotypes. Genetics 135(1):71-80
Each of the applications and patents mentioned in this document, and each document cited or referenced in each of the above applications and patents, including during the prosecution of each of the applications and patents ("application cited documents") and any manufacturer's instructions or catalogues for any products cited or mentioned in each of the applications and patents and in any of the application cited documents, are hereby incorporated herein by reference. Furthermore, all documents cited in this text, and all documents cited or referenced in documents cited in this text, and any manufacturer's instructions or catalogues for any products cited or mentioned in this text, are hereby incorporated herein by reference.
Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the claims.

### Annex to the application documents - subsequently filed sequences listing

SEQUENCE LISTING

<110> Cyclacel Limited
      Deak, Peter
      Frenz, Lisa
      Glover, David
      Midgley, Carol

<120> Cell Cycle Progression Proteins

<130> P012431WO CYK

<140> PCT/GB02/04780
<141> 2002-10-23

<150> GB 0126506.5
<151> 2001-11-05

<150> GB 0128384.5
<151> 2001-11-27

<150> GB 0203185.4
<151> 2002-02-11

<160> 306

<170> PatentIn version 3.1

<210> 1
<211> 16
<212> PRT
<213> Artificial sequence

<220>
<223> Penetratin

<400> 1

Arg Gln Ile Lys Ile Trp Phe Gln Asn Arg Arg Met Lys Trp Lys Lys
1               5                   10                  15


<210> 2
<211> 23
<212> DNA
<213> Artificial sequence

<220>
<223> T7 polymerase binding site

<400> 2
taatacgact cactataggg aca                                              23


<210> 3
<211> 48
<212> DNA
<213> Artificial sequence

<220>
<223> Primer

## Claims

1. Use of a polynucleotide as set out in Table 5, or a polypeptide encoded by the polypeptide, in a method of prevention, treatment or diagnosis of a disease in an individual.

2. A use as claimed in Claim 1, in which the polynucleotide comprises a human polypeptide as set out in column 3 of Table 5.

3. A use as claimed in Claim 1 or 2, in which the polynucleotide or polypeptide is used to identify a substance capable of binding to the polypeptide, which method comprises incubating the polypeptide with a candidate substance under suitable conditions and determining whether the substance binds to the polypeptide.

4. A use as claimed in Claim 1, 2 or 3, in which the polynucleotide or polypeptide is used to identify a substance capable of modulating the function of the polypeptide, the method comprising the steps of: incubating the polypeptide with a candidate substance and determining whether activity of the polypeptide is thereby modulated.

5. A use as claimed in any preceding claim, in which the polynucleotide or polypeptide is administered to an individual in need of such treatment.

6. A use as claimed in any preceding claim, in which the substance identified by the method is administered to an individual in need of such treatment.

7. A use as claimed in Claim 1 or 2 in a method of diagnosis, in which the presence or absence of a polynucleotide is detected in a biological sample in a method comprising: (a) bringing the biological sample containing nucleic acid such as DNA or RNA into contact with a probe comprising a fragment of at least 15 nucleotides of the polynucleotide as set out in Table 5 under hybridising conditions; and (b) detecting any duplex formed between the probe and nucleic acid in the sample.

8. A use as claimed in Claim 1 or 2 in a method of diagnosis, in which the presence or absence of a polypeptide is detected in a biological sample in a method comprising: (a) providing an antibody capable of binding to the polypeptide; (b) incubating a biological sample with said antibody under conditions which allow for the formation of an antibody-antigen complex; and (c) determining whether antibody-antigen complex comprising said antibody is formed.

9. A use as claimed in any preceding claim, in which the disease comprises a proliferative disease such as cancer.

10. A method of modulating, preferably down-regulating, the expression of a polynucleotide as set out in Table 5 in a cell, the method comprising introducing a double stranded RNA (dsRNA) corresponding to the polynucleotide, or an antisense RNA corresponding to the polynucleotide, or a fragment thereof, into the cell.

11. A polynucleotide selected from:
(a) polynucleotides comprising any one of the nucleotide sequences set out in Example 19, preferably Shp2 polynucleotide, or the complement thereof;
(b) polynucleotides comprising a nucleotide sequence capable of hybridising to the nucleotide sequences set out in Example 19, preferably Shp2 polynucleotide, or a fragment thereof;
(c) polynucleotides comprising a nucleotide sequence capable of hybridising to the complement of the nucleotide sequences set out in Example 19, preferably Shp2 polynucleotide, or a fragment thereof;
(d) polynucleotides comprising a polynucleotide sequence which is degenerate as a result of the genetic code to the polynucleotides defined in (a), (b) or (c).

12. A polynucleotide selected from:
(a) polynucleotides comprising any one of the nucleotide sequences set out in Example 28, preferably Dlg1 or Dlg2 polynucleotide, or the complement thereof;
(b) polynucleotides comprising a nucleotide sequence capable of hybridising to the nucleotide sequences set out in Example 28, preferably Dlg1 or Dlg2 polynucleotide, or a fragment thereof;
(c) polynucleotides comprising a nucleotide sequence capable of hybridising to the complement of the nucleotide sequences set out in Example 28, preferably Dlg1 or Dlg2 polynucleotide, or a fragment thereof;
(d) polynucleotides comprising a polynucleotide sequence which is degenerate as a result of the genetic code to the polynucleotides defined in (a), (b) or (c).

13. A polynucleotide selected from:
(a) polynucleotides comprising any one of the nucleotide sequences set out in Table 5 or the complement thereof;
(b) polynucleotides comprising a nucleotide sequence capable of hybridising to the nucleotide sequences set out in Table 5, or a fragment thereof;
(c) polynucleotides comprising a nucleotide sequence capable of hybridising to the complement of the nucleotide sequences set out in Table 5, or a fragment thereof;
(d) polynucleotides comprising a polynucleotide sequence which is degenerate as a result of the genetic code to the polynucleotides defined in (a), (b) or (c).

14. A polynucleotide selected from:
(a) polynucleotides comprising any one of the nucleotide sequences set out in Examples 1 to 18, 20 to 27 and 29 or the complement thereof;
(b) polynucleotides comprising a nucleotide sequence capable of hybridising to the nucleotide sequences set out in Examples 1 to 18, 20 to 27 and 29, or a fragment thereof;
(c) polynucleotides comprising a nucleotide sequence capable of hybridising to the complement of the nucleotide sequences set out in Examples 1 to 18, 20 to 27 and 29, or a fragment thereof;
(d) polynucleotides comprising a polynucleotide sequence which is degenerate as a result of the genetic code to the polynucleotides defined in (a), (b) or (c).

15. A polynucleotide selected from:
(a) polynucleotides comprising any one of the nucleotide sequences set out in Examples 1, 2, 2A, 2B and 2C or the complement thereof;
(b) polynucleotides comprising a nucleotide sequence capable of hybridising to the nucleotide sequences set out in Examples 1, 2, 2A, 2B and 2C, or a fragment thereof;
(c) polynucleotides comprising a nucleotide sequence capable of hybridising to the complement of the nucleotide sequences set out in Examples 1, 2, 2A, 2B and 2C, or a fragment thereof;
(d) polynucleotides comprising a polynucleotide sequence which is degenerate as a result of the genetic code to the polynucleotides defined in (a), (b) or (c).

16. A polynucleotide selected from:
(a) polynucleotides comprising any one of the nucleotide sequences set out in Examples 3 to 9 and 9A or the complement thereof;
(b) polynucleotides comprising a nucleotide sequence capable of hybridising to the nucleotide sequences set out in Examples 3 to 9 and 9A, or a fragment thereof;
(c) polynucleotides comprising a nucleotide sequence capable of hybridising to the complement of the nucleotide sequences set out in Examples 3 to 9 and 9A, or a fragment thereof;
(d) polynucleotides comprising a polynucleotide sequence which is degenerate as a result of the genetic code to the polynucleotides defined in (a), (b) or (c).

17. A polynucleotide selected from:
(a) polynucleotides comprising any one of the nucleotide sequences set out in Examples 10 to 29 or the complement thereof;
(b) polynucleotides comprising a nucleotide sequence capable of hybridising to the nucleotide sequences set out in Examples 10 to 29, or a fragment thereof;
(c) polynucleotides comprising a nucleotide sequence capable of hybridising to the complement of the nucleotide sequences set out in Examples 10 to 29, or a fragment thereof;
(d) polynucleotides comprising a polynucleotide sequence which is degenerate as a result of the genetic code to the polynucleotides defined in (a), (b) or (c).

18. A polynucleotide probe which comprises a fragment of at least 15 nucleotides of a polynucleotide according to any of Claims 11 to 16.

19. A polypeptide which comprises any one of the amino acid sequences set out in any of the following:
(a) Example 19, preferably Shp2 polypeptide;
(b) Example 28, preferably Dlg1 or Dlg2 polypeptide;
(c) Table 5;
(d) Examples 1 to 18, 20 to 27 and 29;
(e) Examples 1 to 2, 2A, 2B and 2C;
(f) Examples 3 to 9 and 9A;
(g) Examples 10 to 29;
or a homologue, variant, derivative or fragment thereof.

20. A polynucleotide encoding a polypeptide according to Claim 19.

21. A vector comprising a polynucleotide according to any of Claims 11 to 18 and 20.

22. An expression vector comprising a polynucleotide according to any of Claims 11 to 19 and 20 operably linked to a regulatory sequence capable of directing expression of said polynucleotide in a host cell.

23. An antibody capable of binding a polypeptide according to Claim 19.

24. A method for detecting the presence or absence of a polynucleotide according to any of Claims 11 to 18 and 20 in a biological sample which comprises:
(a) bringing the biological sample containing DNA or RNA into contact with a probe according to Claim 18 under hybridising conditions; and
(b) detecting any duplex formed between the probe and nucleic acid in the sample.

25. A method for detecting a polypeptide according to Claim 19 present in a biological sample which comprises:
(a) providing an antibody according to Claim 23;
(b) incubating a biological sample with said antibody under conditions which allow for the formation of an antibody-antigen complex; and
(c) determining whether antibody-antigen complex comprising said antibody is formed.

26. A polynucleotide according to according to any of Claims 11 to 18 and 20 for use in therapy.

27. A polypeptide according to Claim 19 for use in therapy.

28. An antibody according to Claim 23 for use in therapy.

29. A method of treating a tumour or a patient suffering from a proliferative disease comprising administering to a patient in need of treatment an effective amount of a polynucleotide according to any of Claims 11 to 18 and 20.

30. A method of treating a tumour or a patient suffering from a proliferative disease, comprising administering to a patient in need of treatment an effective amount of a polypeptide according to Claim 17.

31. A method of treating a tumour or a patient suffering from a proliferative disease, comprising administering to a patient in need of treatment an effective amount of an antibody according to Claim 23 to a patient.

32. Use of a polypeptide according to Claim 19 in a method of identifying a substance capable of affecting the function of the corresponding gene.

33. Use of a polypeptide according to Claim 19 in an assay for identifying a substance capable of inhibiting the cell division cycle.

34. Use as claimed in Claim 33, in which the substance is capable of inhibiting mitosis and/or meiosis.

35. A method for identifying a substance capable of binding to a polypeptide according to Claim 19, which method comprises incubating the polypeptide with a candidate substance under suitable conditions and determining whether the substance binds to the polypeptide.

36. A method for identifying a substance capable of modulating the function of a polypeptide according to Claim 19 or a polypeptide encoded by a polynucleotide according to any of Claims 11 to 18 and 20, the method comprising the steps of:
incubating the polypeptide with a candidate substance and determining whether activity of the polypeptide is thereby modulated.

37. A substance identified by a method or assay according to any of Claims 32 to 36.

38. Use of an antibody according to Claim 23 or a substance according to Claim 36 in a method of inhibiting the function of a polypeptide.

39. Use of an antibody according to Claim 23 or a substance according to Claim 37 in a method of regulating a cell division cycle function.

40. A method of identifying a human nucleic acid sequence, by: (a) selecting a *Drosophila* polypeptide identified in any of Examples 11 to 39; (b) identifying a corresponding human polypeptide; (c) identifying a nucleic acid encoding the polypeptide of (b).

41. A method according to Claim 40, in which a human homologue of the *Drosophila* sequence, or a human sequence similar to the *Drosophila* sequence, is identified in step (b).

42. A method according to Claim 40 or 41, in which the human polypeptide has at least one of the biological activities, preferably substantially all the biological activities of the *Drosophila* polypeptide.

43. A human polypeptide identified by a method according to Claim 40, 41 or 42.
